# EUROPEAN PATENT SPECIFICATION

(11) **EP 2 474 530 B1**
(45) Date of publication and mention of the grant of the patent: **03.09.2014**
(21) Application number: 10804369.6
(22) Date of filing: 26.07.2010
(51) Int. Cl.: C07D 209/18, C07D 401/04, C07D 401/06, C07D 401/14, C07D 405/04, C07D 405/06, C07D 405/14, C07D 409/04, C07D 409/06, C07D 409/14, C07D 413/14, C07D 417/06, C07D 417/14, A61K 31/404, A61K 31/422, A61K 31/427, A61K 31/4439, A61P 13/02, A61P 43/00

(54) **INDOLE DERIVATIVE AND PHARMACOLOGICALLY ACCEPTABLE SALT THEREOF**
INDOLDERIVAT UND PHARMAZEUTISCH AKZEPTABLES SALZ DARAUS
DÉRIVÉ D'INDOLE ET SEL PHARMACOLOGIQUEMENT ACCEPTABLE DE CELUI-CI

(30) Priority: 27.07.2009 JP 2009173852; 04.11.2009 JP 2009252846
(43) Date of publication of application: 11.07.2012
(73) Proprietor: Kissei Pharmaceutical Co., Ltd., Matsumoto-shi Nagano 399-8710 (JP)
(72) Inventor: KONDO, Tatsuhiro, Azumino-shi Nagano 399-8304 (JP); KONDO, Atsushi, Azumino-shi Nagano 399-8304 (JP); TATANI, Kazuya, Azumino-shi Nagano 399-8304 (JP); KAWAMURA, Naohiro, Azumino-shi Nagano 399-8304 (JP)
(74) Representative: Boult Wade Tennant
(86) International application number: PCT/JP2010/062532
(87) International publication number: WO 2011/013623

(56) References cited:
- WO-A1-98/51667
- WO-A2-2009/156462
- US-A- 4 510 158
- HALL, A. ET AL.: 'Discovery of a novel indole series of EP1 receptor antagonists by scaffold hopping' BIOORGANIC & MEDICINAL CHEMISTRY LETTERS vol. 18, no. 8, 2008, pages 2684 - 2690, XP022606372

## Description

### Technical Field

The present invention relates to an indole derivative having an EP₁ receptor antagonism, which is useful as a pharmaceutical, or a pharmaceutically acceptable salt thereof, a pharmaceutical composition comprising the same, and a pharmaceutical use thereof.

### Background Art

With an increasingly aging and stressed society, the number of patients with lower urinary tract dysfunction (LUTD) has increased. LUTD is a generic term for urine collection disorder and dysuria, and the symptoms derived from LUTD are lower urinary tract symptoms (LUTS). One of the LUTS is an overactive bladder syndrome (OABs). OABs may be generally called overactive bladder (OAB) in some cases. In any case, it is a disease defined as "a symptom syndrome which essentially has urinary urgency and which is usually accompanied by urinary frequency and nocturia. Urge urinary incontinence is not necessary". The symptoms associated with OABs interfere with general life such as work, daily life, mental activity, and the like, and thus lower the quality of life (QOL). Currently, a first choice drug as an agent for treating OABs is an anticholinergic agent. However, it is necessary for the anticholinergic agent to be used also in due consideration of an anti-muscarinic effect such as thirst and residual urine, and thus, is not always effective for all patients (see, for example, Non-patent literature 1). Under these circumstances, there is a demand for development of a therapeutic agent which has a different mechanism from that of the anticholinergic agent (see, for example, Non-patent literature 1).

Recently, in LUTS, particularly in OABs, the role of urothelium has attracted attention. In LUTS, it has become clear that various chemical mediators are released in the urothelial cells, which cause a micturition reflex through the receptors of bladder sensory nerve terminals. Among them, one of the chemical mediators, prostaglandin E₂ (PGE₂), binds with a prostaglandin E receptor 1 (EP₁ receptor) in the afferent nerves (especially C fibers) in the urothelium to increase the micturition reflex. In addition, PGE₂ binds with the EP₁ receptors present in the bladder smooth muscle to contract the bladder. In fact, it has been reported that the EP₁ receptor antagonists inhibit both of the increase in the micturition reflex and the increase in the afferent nerve activities by PGE₂ (see, for example, Non-patent literature 2 and Non-patent literature 3). From these, it is suggested that PGE₂ is involved in contraction of the bladder smooth muscle and increase in the bladder sensory nerves through the EP₁ receptors. Furthermore, it is reported that EP₁ receptor antagonists do not increase the amount of the residual urine, but increase the bladder capacity (see, for example, Non-patent literature 4).

There exist four subtypes, EP₂, EP₃, and EP₄ as well as EP₁, of the PGE₂ receptor. The EP₁ receptor exists in the lungs as well as the bladder and the urothelium, the skeletal muscle, the renal collecting duct, and the like (see, for example, Non-patent literature 2). Therefore, it is expected that by changing the selectivity of the subtypes of the PGE₂ receptor, the target organs of the drugs, or the target tissues, a therapeutic agent for desired diseases can be developed.

WO-A-2009/156462 describes organic compounds of Formula (I), methods of use, and pharmaceutical compositions thereof.

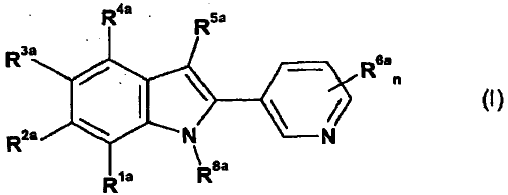

Meanwhile, a compound represented by the general formula (A) is disclosed as a traditional complement pathway inhibitor (see, for example, Patent literature 1). 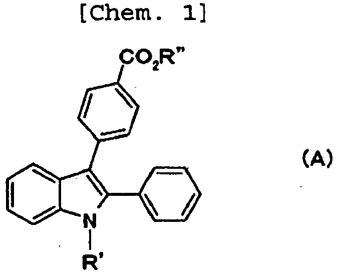 [wherein R' and R" independently represent a hydrogen atom or a lower alkyl group].

However, there is no suggestion or disclosure that these compounds have an EP₁ receptor antagonism.

As an indole derivative having an EP₁ receptor antagonism, a compound represented by the chemical structural formula (B) (sodium 6-(6-chloro-3-isobutylindol-1-yl)pyridine-2-carboxylate) and an analog thereof are disclosed (see, for example, Non-patent literature 5).

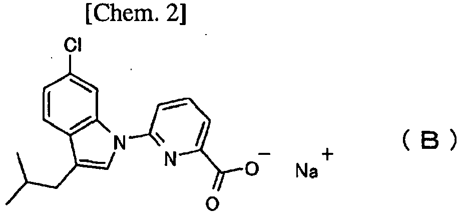

However, these compounds differ from the compounds of the present invention in the chemical structural formula in terms of the position, the type, or the like of a substituent.

### Citation List

### Patent Literature

Patent literature 1: U.S. Patent No. 4510158

### Non-Patent Literature

Non-patent literature 1: Narihito Seki, "Folia Pharmacologia Japonica", 2007, Vol. 129, p. 368-373
Non-patent literature 2: Xiaojun Wang, et al., "Biomedical Research", 2008, Vol. 29, p. 105-111
Non-patent literature 3: Masahito Kawatani, "PAIN RESEARCH", 2004, Vol. 19, p. 185-190
Non-patent literature 4: Masanobu Maegawa, "The Journal of The Japan Neurogenic Bladder Society", 2008, Vol. 19, p. 169
Non-patent literature 5: Adrian Hall, et al., "Bioorganic & Medicinal Chemistry Letters", 2008, p. 2684-2690

### Summary of the Invention

### Objects to Be Solved by the Invention

The present invention is to provide a compound having an EP₁ receptor antagonism or a pharmaceutically acceptable salt thereof, a pharmaceutical composition comprising the same, and a pharmaceutical use thereof.

### Means for Solving the Objects

The present inventors have conducted extensive studies on a compound having an EP₁ receptor antagonism, and as a result, they have found that the compounds (I) of the present invention or a pharmaceutically acceptable salt thereof exhibit a potent EP₁ receptor antagonism, thereby completing the present invention.

That is, the means for solving the above described objects are presented below.
[1] A compound represented by the general formula (I) or a pharmaceutically acceptable salt thereof: 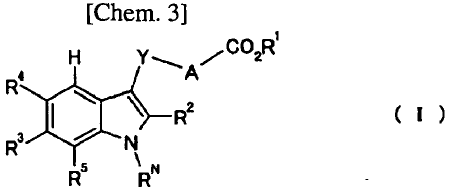 [wherein
   A represents a group selected from the group consisting of the following a) to h): 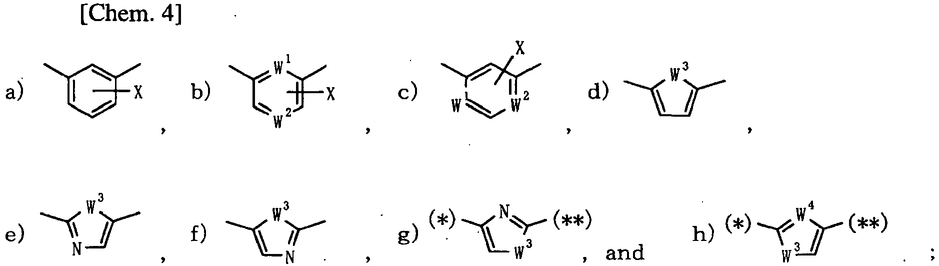
   one of W¹ and W² represents a nitrogen atom and the other represents =CH- or a nitrogen atom;
   W³ represents an oxygen atom or a sulfur atom;
   W⁴ represents =CH- or a nitrogen atom;
   X represents a hydrogen atom or a halogen atom;
   Y represents a C₁₋₆ alkylene group or a halo-C₁₋₆ alkylene group;
   R^{N} represents a hydrogen atom or a C₁₋₆ alkyl group;
   R¹ represents a hydrogen atom, a C₁₋₆ alkyl group or a C₇₋₁₀ aralkyl group;
   R² represents a group selected from the group consisting of the following i) to m):
      i) a branched C₃₋₆ alkyl group,
      j) a C₃₋₆ cycloalkyl group,
      k) a phenyl group, in which the ring is unsubstituted or substituted with 1 to 5 groups independently selected from the group consisting of: a halogen atom, a C₁₋₆ alkyl group, a halo-C₁₋₆ alkyl group, a hydroxy-C₁₋₆ alkyl group, a C₁₋₆ alkoxy group and a cyano group,
      l) a 6-membered aromatic heterocyclic group, in which the ring is unsubstituted or substituted with 1 to 4 groups independently selected from the group consisting of: a halogen atom, a C₁₋₆ alkyl group, a halo-C₁₋₆ alkyl group, a hydroxy-C₁₋₆ alkyl group, a C₁₆ alkoxy group and a cyano group, and
      m) a 5-membered aromatic heterocyclic group, in which the ring is unsubstituted or substituted with 1 to 3 groups independently selected from the group consisting of: a halogen atom, a C₁₋₆ alkyl group, a halo-C₁₋₆ alkyl group, a hydroxy-C₁₋₆ alkyl group, a C₁₆ alkoxy group and a cyano group;
   R³ represents a halogen atom, a C₁₋₆ alkyl group, a halo-C₁₋₆ alkyl group, a hydroxy-C₁₋₆ alkyl group, a C₁₋₆ alkoxy group, a halo-C₁₋₆ alkoxy group, a C₁₋₆ alkylsulfanyl group, a C₁₋₆ alkylsulfinyl group, a C₁₋₆ alkylsulfonyl group, a C₃₋₆cycloalkyl group, a cyano group, an amino group or a nitro group;
   R⁴ represents a hydrogen atom, a halogen atom, a C₁₋₆ alkyl group or a C₁₋₆ alkoxy group; and
   R⁵ represents a hydrogen atom, a halogen atom, a C₁₋₆ alkyl group, or a C₁₋₆ alkoxy group;
   with the proviso that the bonds with (*) represent binding to Y; and the bonds with (**) represent binding to CO₂R¹.].
[2] The compound as set forth in (1) or a pharmaceutically acceptable salt thereof, wherein A is a group selected from the group consisting of the following a) to d): 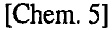 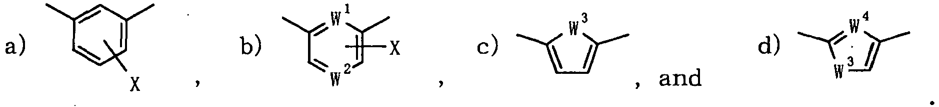
[3] The compound as set forth in (2) or a pharmaceutically acceptable salt thereof, wherein A is a group selected from the group consisting of the following a) to c): 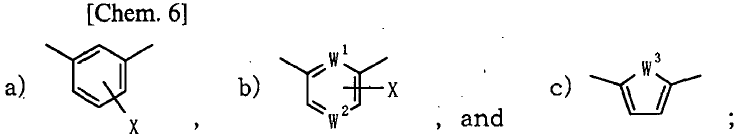
   W¹ is a nitrogen atom;
   W² is =CH-; and
   X is a hydrogen atom.
[4] The compound as set forth in (3) or a pharmaceutically acceptable salt thereof, wherein R² is a group selected from the group consisting of the following a) to c):
   a) a branched C₃₋₆ alkyl group,
   b) a phenyl group, and
   c) a 5-membered aromatic heterocyclic group or a 6-membered aromatic heterocyclic group;
   R³ is a halogen atom, a C₁₋₆ alkyl group, a halo-C₁₋₆ alkyl group, a C₁₋₆ alkoxy group, a halo-C₁₋₆ alkoxy group or a cyano group;
   R⁴ is a hydrogen atom; and
   R⁵ is a hydrogen atom.
[5] The compound as set forth in (4) or a pharmaceutically acceptable salt thereof, wherein R¹ is a hydrogen atom.
[6] The compound as set forth in (5) or a pharmaceutically acceptable salt thereof, wherein Y is a methylene group and R^{N} is a hydrogen atom.
[7] The compound as set forth in (6) or a pharmaceutically acceptable salt thereof, wherein R² is a phenyl group, a 5-membered aromatic heterocyclic group or a 6-membered aromatic heterocycle.
[8] The compound as set forth in (7) or a pharmaceutically acceptable salt thereof, wherein R³ is a halogen atom or a C₁₋₆ alkoxy group.
[9] The compound as set forth in (6) or a pharmaceutically acceptable salt thereof, wherein R² is an isopropyl group, an isobutyl group, a sec-butyl group or a tert-butyl group.
[10] The compound as set forth in (9) or a pharmaceutically acceptable salt thereof, wherein R³ is a halogen atom or a C₁₋₆ alkoxy group.
[11] The compound as set forth in (2) or a pharmaceutically acceptable salt thereof, wherein A is a group represented by the following formula: 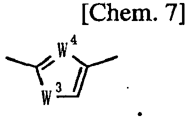
[12] The compound as set forth in (11) or a pharmaceutically acceptable salt thereof, wherein R¹ is a hydrogen atom.
[13] The compound as set forth in (3) or a pharmaceutically acceptable salt thereof, wherein R² is a group selected from the group consisting of the following a) to d): 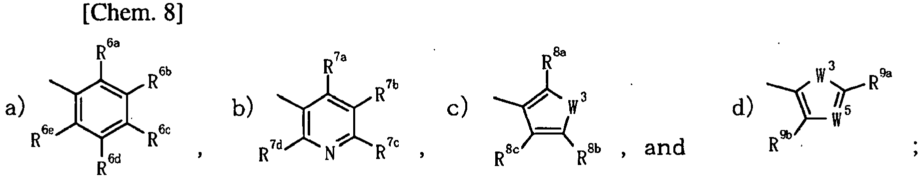
   W⁵ is a nitrogen atom or -CR^{9c}=; and
   R^{6a}, R^{6b}, R^{6c}, R^{6d}, R^{6e}, R^{7a}, R^{7b}, R^{7c}, R^{7d}, R^{8a}, R^{8b}, R^{8c}, R^{9a}, R^{9b} and R^{9c} are each independently a hydrogen atom, a halogen atom, a C₁₋₆ alkyl group, a halo-C₁₋₆ alkyl group, a hydroxy-C₁₋₆ alkyl group, a C₁₋₆ alkoxy group or a cyano group.
[14] The compound as set forth in (13) or a pharmaceutically acceptable salt thereof, wherein R¹ is a hydrogen atom.
[15] A pharmaceutical composition comprising the compound as set forth in any one of (1) to (14) or a pharmaceutically acceptable salt thereof as an active ingredient.
[16] An EP₁ receptor antagonist comprising the compound as set forth in any one of (1) to (14) or a pharmaceutically acceptable salt thereof as an active ingredient.
[17] An agent for treating or preventing lower urinary tract symptoms, comprising the compound as set forth in any one of (1) to (14) or a pharmaceutically acceptable salt thereof as an active ingredient.
[19] A use of the compound as set forth in any one of (1) to (14) or a pharmaceutically acceptable salt thereof for the manufacture of a pharmaceutical composition for preventing or treating lower urinary tract symptoms.

### Effects of the Invention

The compound (1) of the present invention or a pharmaceutically acceptable salt thereof exhibits a potent EP₁ receptor antagonism, for example, in a test for confirmation of an EP₁ receptor antagonism. Therefore, the compound (I) of the present invention or a pharmaceutically acceptable salt thereof is useful as an agent for treating or preventing lower urinary tract symptoms (LUTS), in particular, overactive bladder syndrome (OABs) or the like, based on its EP₁ receptor antagonism.

### Embodiments for Carrying Out the Invention

The terms in the specification are defined.

The "halogen atom" means a fluorine atom, a chlorine atom, a bromine atom, or an iodine atom. It is preferably a fluorine atom or a chlorine atom.

The "C₁₋₆ alkyl group" means an alkyl group having 1 to 6 carbon atoms, which may be branched. Examples thereof include a methyl group, an ethyl group, an n-propyl group, an isopropyl group, an *n*-butyl group, an isobutyl group, a sec-butyl group, a *tert-*butyl group, an *n*-pentyl group, an isopentyl group, a neopentyl group, a *tert*-pentyl group, a 1-methylbutyl group, a 2-methylbutyl group, a 1,2-dimethylpropyl group, an *n*-hexyl group, an isohexyl group and the like. In R³, a methyl group, an ethyl group or a propyl group is preferable, a methyl group or an ethyl group is more preferable, and a methyl group is further preferable. In R^{N}, a methyl group is preferred.

The "branched C₃₋₆ alkyl group" means a branched alkyl group having 3 to 6 carbon atoms. Examples thereof include an isopropyl group, an isobutyl group, a *sec-*butyl group, a *tert*-butyl group, an isopentyl group, a neopentyl group, a *tert*-pentyl group, a 1-methylbutyl group, a 2-methylbutyl group, a 1,2-dimethylpropyl group, a 1-ethylpropyl group, an isohexyl group and the like. It is preferably an isopropyl group, an isobutyl group, a *sec*-butyl group, a *tert*-butyl group or a 1-ethylpropyl group. It is more preferably an isopropyl group, an isobutyl group, a *sec*-butyl group or a *tert*-butyl group. It is further preferably a *tert*-butyl group.

The "C₁₋₆ alkoxy group" means an alkoxy group having 1 to 6 carbon atoms, which may be branched. Examples thereof include a methoxy group, an ethoxy group, a propoxy group, an isopropoxy group, a butoxy group, an isobutoxy group, a sec-butoxy group, a *tert*-butoxy group, a pentyloxy group, a hexyloxy group and the like. In R³, a methoxy group or an ethoxy group is preferable, and a methoxy group is more preferable.

The "halo-C₁₋₆ alkyl group" means a C₁₋₆ alkyl group substituted with the same or different 1 to 5 halogen atoms. Examples thereof include a monofluoromethyl group, a difluoromethyl group, a trifluoromethyl group, a 2-chloroethyl group, a 2-fluoroethyl a group, a 2,2-difluoroethyl group, a 1,1-difluoroethyl group, a 1,2-difluoroethyl group, a 2,2,2-trifluoroethyl group, a 1,1,2,2,2 pentafluoroethyl group, a 2,2,2-trichloroethyl group, a 3-fluoropropyl group, a 2-fluoropropyl group, a 1-fluoropropyl group, a 3,3-difluoropropyl group, a 2,2-difluoropropyl group, a 1,1-difluoropropyl group, a 1-fluorobutyl group, a 1-fluoropentyl group, a 1-fluorohexyl group and the like. It is preferably a monofluoromethyl group, a difluoromethyl group, a trifluoromethyl group, a 2-fluoroethyl group, a 2,2-difluoroethyl group, a 1,1-difluoroethyl group, a 1,2-difluoroethyl group and a 2,2,2-trifluoroethyl group. It is more preferably a monofluoromethyl group, a trifluoromethyl group, a 2-fluoroethyl group, a 1,1-difluoroethyl group, a 1,2-difluoroethyl group or a 2,2,2-trifluoroethyl group. It is further preferably a monofluoromethyl group or a trifluoromethyl group.

The "hydroxy-C₁₋₆ alkyl group" means a C₁₋₆ alkyl group substituted with a hydroxy group. Examples thereof include a hydroxymethyl group, a 1-hydroxyethyl group, a 1-hydroxy-1,1-dimethylmethyl group, a 2-hydroxyethyl group, a 2-hydroxy-2-methylpropyl group, a 3-hydroxypropyl group and the like.

The "C₁₋₆ alkylsulfanyl" means a group represented by (C₁₋₆ alkyl)-S-. Examples thereof include a methylsulfanyl group, an ethylsulfanyl group, a propylsulfanyl group, a butylsulfanyl group, a pentylsulfanyl group, a hexylsulfanyl group and the like.

The "C₁₋₆ alkylsulfinyl group" means a group represented by (C₁₋₆ alkyl)-S(=O)-. Examples thereof include a methylsulfinyl group, an ethylsulfinyl group, a propylsulfinyl group, a butylsulfinyl group, a pentylsulfinyl group, a hexylsulfinyl group, and the like.

The "C₁₋₆ alkylsulfonyl group" means a group represented by (C₁₋₆ alkyl)-SO₂-. Examples thereof include a methanesulfonyl group, an ethanesulfonyl group, a propanesulfonyl group, a butanesulfonyl group, a pentanesulfonyl group, a hexanesulfonyl group and the like.

The "C₃₋₆ cycloalkyl group" means a monocyclic saturated alicyclic hydrocarbon group having 3 to 6 carbon atoms. Examples thereof include a cyclopropyl group, a cyclobutyl group, a cyclopentyl group, a cyclohexyl group and the like. It is preferably a cyclopropyl group or a cyclopentyl group. In R³, it is more preferably a cyclopropyl group.

The "halo-C₁₋₆ alkoxy group" means a C₁₋₆ alkoxy group substituted with the same or different 1 to 5 halogen atoms. Examples thereof include a monofluoromethoxy group, a difluoromethoxy group, a trifluoromethoxy group, a 2-chloroethoxy group, a 2-fluoroethoxy group, a 2,2-difluoroethoxy group, a 1,1-difluoroethoxy group, a 1,2-difluoroethoxy group, a 2,2,2-trifluoroethoxy group, a 1,1,2,2,2 pentafluoroethoxy group, a 2,2,2-trichloroethoxy group, a 3-fluoropropoxy group, a 2-fluoropropoxy group, a 1-fluoropropoxy group, a 3,3-difluoropropoxy group, a 2,2-difluoropropoxy group, a 1,1-difluoropropoxy group, a 4-fluorobutoxy group, a 5-fluoropentyloxy group, a 6-fluorohexyloxy group and the like. It is preferably a monofluoromethoxy group, a difluoromethoxy group, a trifluoromethoxy group, a 2-fluoroethoxy group, a 2,2-difluoroethoxy group, a 1,1-difluoroethoxy group, a 1,2-difluoroethoxy group or a 2,2,2-trifluoroethoxy group. It is more preferably a monofluoromethoxy group, a difluoromethoxy group, a trifluoromethoxy group, a 2-fluoroethoxy group, a 1,1-difluoroethoxy group, a 1,2-difluoroethoxy group or a 2,2,2-trifluoroethoxy group. It is further preferably a difluoromethoxy group or a trifluoromethoxy group.

The "C₇₋₁₀ aralkyl group" means an alkyl group having 1 to 4 carbon atoms, which is substituted with an aryl group. Examples thereof include a benzyl group, a phenethyl group, a 1-phenylethyl group, a 3-phenylpropyl group, a 4-phenylbutyl group and the like.

The "5- or 6-membered aromatic heterocyclic group" means a 5- or 6-membered ring group containing 1 to 4 hetero atoms selected from an oxygen atom, a nitrogen atom, and a sulfur atom in the ring. Examples thereof include a pyridyl group, a pyrimidinyl group, a pyrazinyl group, a pyridazinyl group, a furyl group, a pyrrolyl group, a thienyl group, an imidazolyl group, a pyrazolyl group, a 1,2,4-triazolyl group, an isothiazolyl group, an isoxazolyl group, an oxazolyl group, a thiazolyl group, a 1,3,4-oxadiazolyl group, a 1,2,4-oxadiazolyl group and the like. It is preferably a 5-membered aromatic heterocyclic group, and more preferably a 2-furyl group, a 3-furyl group, a 2-thienyl group or a 3-thienyl group. It is further preferably a 3-furyl group or a 3-thienyl group.

The "5-membered aromatic heterocyclic group" means a 5-membered ring group containing 1 to 4 hetero atoms selected from an oxygen atom, a nitrogen atom, and a sulfur atom in the ring. Examples thereof include a furyl group, pyrrolyl group, a thienyl group, an imidazolyl group, a pyrazolyl group, a 1,2,4-triazolyl group, an isothiazolyl group, an isoxazolyl group, an oxazolyl group, a thiazolyl group, a 1,3,4-oxadiazolyl group, a 1,2,4-oxadiazolyl group and the like, preferably a 2-furyl group, a 3-furyl group, a 2-thienyl group or a 3-thienyl group, and more preferably a 3-furyl group or a 3-thienyl group.

The "6-membered aromatic heterocyclic group" means a 6-membered ring group containing 1 to 4 nitrogen atoms in the ring. Examples thereof include a pyridyl group, a pyrimidinyl group, a pyrazinyl group, a pyridazinyl group and the like. It is preferably a pyridyl group, and more preferably a 3-pyridyl group.

The "C₁₋₆ alkylene group" means a divalent linear or molecular-chained saturated hydrocarbon chain having 1 to 6 carbon atoms. Examples thereof include -CH₂-,-CH₂CH₂-, -CH(CH₃)-, -(CH₂)₃-, -CH(CH₃)CH₂-, -CH₂CH(CH₃)-, -CH(CH₂CH₃)-,-C(CH₃)₂-, -(CH₂)₄-, -CH(CH₃)-(CH₂)₂-, -(CH₂)₂-CH(CH₃)-, -CH(CH₂CH₃)-CH₂-,-C(CH₃)₂CH₂-, -CH₂-C(CH₃)₂-, -CH(CH₃)-CH(CH₃)-, -(CH₂)₅-, -CH(CH₃)-(CH₂)₃-,-C(CH₃)₂CH₂CH₂-, -(CH₂)₆-, -C(CH₃)₂(CH₂)₃- and the like.

The "C₁₋₅ alkylene group" means a divalent linear or molecular-chained saturated hydrocarbon chain having 1 to 5 carbon atoms. Examples thereof include -CH₂-, -(CH₂)₂-, -CH(CH₃)-, -(CH₂)₃-, -CH₂-CH(CH₃)-, -C(CH₃)₂-, -(CH₂)₄-, -(CH₂)₅- and the like.

The "halo-C₁₋₆ alkylene group" means a group in which the hydrogen atom(s) of a C₁₋₆ alkylene group is(are) substituted with 1 to 4 halogen atoms. As the halogen atom for substituting the hydrogen atom, a fluorine atom or a chlorine atom is preferable, and more preferably a fluorine atom. Examples of the halo-C₁₋₆ alkylene group include -CHF-, -CF₂-, -CF₂CH₂-, -CH₂CF₂-, -CF₂CF₂-, -CCl₂-, -CCl₂CH₂-, -CH₂CCl₂-, -CCl₂CCl₂-, and the like.

Hereinafter, the present invention is described in more detail.

In the case that one or more asymmetric carbon atoms exist in the compound (I) of the present invention, the present invention includes each of compounds in which the respective asymmetric carbon atoms are in an R configuration or S configuration, and compounds having any combination of the configurations. Also, the racemic compound, the racemic mixture, the singular enantiomer, and the diastereomer mixture are encompassed within the scope of the present invention. In the case that geometrical isomerism exists in the compound (I) of the present invention, the present invention includes any of the geometrical isomers.

The compound (I) of the present invention can be converted to a pharmaceutically acceptable salt thereof according to a usual method, as necessary. Such a salt may be presented as an acid addition salt or a salt with a base.

Examples of the acid addition salt include acid addition salts with mineral acids such as hydrochloric acid, hydrobromic acid, hydroiodic acid, sulfuric acid, nitric acid, phosphoric acid and the like, and acid addition salts with organic acids such as formic acid, acetic acid, trifluoroacetic acid, methanesulfonic acid, benzenesulfonic acid, *p-*toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid, aspartic acid and the like.

Examples of the salt with a base include salts with inorganic bases, such as a sodium salt, a potassium salt, a calcium salt, a magnesium salt and the like, and salts with organic bases or the like such as piperidine, morpholine, pyrrolidine, arginine, lysine and the like.

In addition, the compound (I) of the present invention or a pharmaceutically acceptable salt thereof also encompasses hydrates, and solvates with pharmaceutically acceptable solvents such as ethanol and the like.

The "EP₁ receptor antagonism" as mentioned in the present invention means an action of inhibiting the binding of a prostaglandin E₂ (PGE₂) to a prostaglandin E receptor 1 (EP₁ receptor).

The EP₁ receptor antagonism reduces the influx amount of calcium into cells and thus decreased or suppressed the intracellular calcium concentration. As the result, the EP₁ receptor antagonism exhibits an action of relaxation of smooth muscles, inhibition of sensory nerve stimulation or the like. Particularly, the EP₁ receptor antagonist acts on the bladder, the urothelium or the like, whereby it is useful as an agent for treating or preventing LUTS, in particular, the symptoms of OABs or the like.

Furthermore, the EP₁ receptor antagonism can be evaluated based on the efficacy of inhibiting the influx amount of calcium into cells by a PGE₂. This efficacy can be evaluated by an in vitro test or an in vivo test in accordance with "Pharmacological Test Examples" described in JP2008-214224A.

Examples of the preferable substituents for the compound (I) of the present invention or a pharmaceutically acceptable salt thereof are as follows.
(I-1) A is preferably a benzene ring, a pyridine ring, or a thiazole ring.
(1-2) Y is preferably a methylene group, -CH(CH₃)- or -C(CH₃)₂-, and more preferably a methylene group.
(I-3) R¹ is preferably a hydrogen atom or a C₁₋₆ alkyl group, and more preferably a hydrogen atom.
(I-4) R² is preferably a *tert*-butyl group, a phenyl group, or a 5-membered aromatic heterocyclic group, or a 6-membered aromatic heterocyclic group, a phenyl group, in which the ring is substituted with 1 to 3 groups independently selected from the group consisting of: a halogen atom, a C₁₋₆ alkyl group, a halo-C₁₋₆ alkyl group, a hydroxy-C₁₋₆ alkyl group, a C₁₋₆ alkoxy group and a cyano group, a 6-membered aromatic heterocyclic group, in which the ring is substituted with 1 to 2 groups independently selected from the group consisting of: a halogen atom, a C₁₋₆ alkyl group, a halo-C₁₋₆ alkyl group, a hydroxy-C₁₋₆ alkyl group, a C₁₋₆ alkoxy group and a cyano group, or a 5-membered aromatic heterocyclic group, in which the ring is substituted with 1 to 2 groups independently selected from the group consisting of: a halogen atom, a C₁₋₆ alkyl group, a halo-C₁₋₆ alkyl group, a hydroxy-C₁₋₆ alkyl group, a C₁₋₆ alkoxy group and a cyano group. It is more preferably a *tert*-butyl group, a phenyl group, a 3-furyl group, a 3-thienyl group or a group selected from the group consisting of the following a) to d): 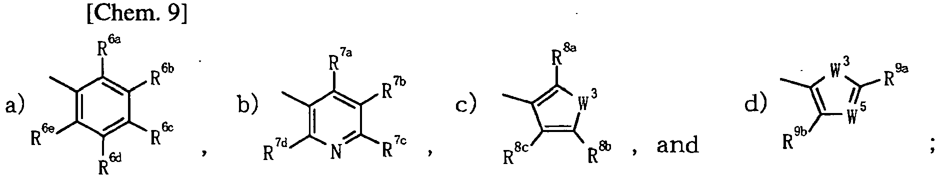 (wherein
   R^{6a}, R^{6b}, R^{6c}, R^{6d} and R^{6e} are each a group selected from the group consisting of the following e) to g):
      e) one group of R^{6a}, R^{6b}, R^{6c}, R^{6d} and R^{6e} is a halogen atom, a C₁₋₆ alkyl group, a halo-C₁₋₆ alkyl group, a hydroxy-C₁₋₆ alkyl group, a C₁₋₆ alkoxy group or a cyano group, and the other four groups are hydrogen atoms,
      f) two groups of R^{6a}, R^{6b}, R^{6c}, R^{6d}, and R^{6e} are each independently a halogen atom, a C₁₋₆ alkyl group, a halo-C₁₋₆ alkyl group, a hydroxy-C₁₋₆ alkyl group, a C₁₋₆ alkoxy group or a cyano group, and the other three groups are hydrogen atoms, and
      g) three groups of R^{6a}, R^{6b}, R^{6c}, R^{6d} and R^{6e} are each independently a halogen atom, a C₁₋₆ alkyl group, a halo-C₁₋₆ alkyl group, a hydroxy-C₁₋₆ alkyl group, a C₁₋₆ alkoxy group or a cyano group, and the other two groups are hydrogen atoms;
   R^{7a}, R^{7b}, R^{7c} and R^{7d} are each a group selected from the group consisting of the following h) and i):
      h) one group of R^{7a}, R^{7b}, R^{7c} and R^{7d} is a halogen atom, a C₁₋₆ alkyl group, a halo-C₁₋₆ alkyl group, a hydroxy-C₁₋₆ alkyl group, a C₁₋₆ alkoxy group or a cyano group, and the other three groups are hydrogen atoms, and
      i) two groups of R^{7a}, R^{7b}, R^{7c} and R^{7d} are each independently a halogen atom, a C₁₆ alkyl group, a halo-C₁₋₆ alkyl group, a hydroxy-C₁₋₆ alkyl group, a C₁₋₆ alkoxy group or a cyano group, and the other two groups are hydrogen atoms;
   R^{8a}, R^{8b} and R^{8c} are each a group selected from the group consisting of following j) and k):
      j) one group of R^{8a}, R^{8b} and R^{8c} is a halogen atom, a C₁₋₆ alkyl group, a halo-C₁₋₆ alkyl group, a hydroxy-C₁₋₆ alkyl group, a C₁₋₆ alkoxy group or a cyano group, and the other two groups are hydrogen atoms, and
      k) two groups of R^{8a}, R^{8b} and R^{8c} are each independently a halogen atom, a C₁₋₆ alkyl group, a halo-C₁₋₆ alkyl group, a hydroxy-C₁₋₆ alkyl group, a C₁₋₆ alkoxy group or a cyano group, and the others are hydrogen atoms;
   for R^{9a} and R^{9b}, when W⁵ is -CR^{9c}=, one group of R^{9a}, R^{9b} and R^{9c} are a halogen atom, a C₁₋₆ alkyl group, a halo-C₁₋₆ alkyl group, a hydroxy-C₁₋₆ alkyl group, a C₁₋₆ alkoxy group or a cyano group, the other two groups are hydrogen atoms, and when w⁵ is a nitrogen atom, one group of R^{9a} and R^{9b} is a halogen atom, a C₁₋₆ alkyl group, a halo-C₁₋₆ alkyl group, a hydroxy-C₁₋₆ alkyl group, a C₁₋₆ alkoxy group or a cyano group, and the other group is a hydrogen atom). It is further preferably a *tert*-butyl group or a phenyl group.
(I-5) R³ is preferably a chlorine atom, a fluorine atom, a methyl group, a trifluoromethyl group, a methoxy group, an ethoxy group or a trifluoromethoxy group, more preferably a chlorine atom, a fluorine atom, a methyl group, a methoxy group, an ethoxy group or a trifluoromethoxy group, further preferably a chlorine atom or a methoxy group, and particularly preferably a methoxy group.
(I-6) R^{N} is preferably a hydrogen atom or a methyl group, and more preferably a hydrogen atom.

A preferable embodiment of the compound (I) of the present invention or a pharmaceutically acceptable salt thereof is a compound formed by combinations of preferable substituents described in (I-1) to (I-6).

### Embodiment 1

A preferable embodiment of the present invention is as follows:
Y is a methylene group;
A is a benzene ring or a pyridine ring;
R¹ is a hydrogen atom;
R² is a phenyl group or a 5-membered aromatic heterocyclic group;
R³ is a methoxy group or an ethoxy group; and
R^{N} is a hydrogen atom.

Examples of the concrete compound included in the present embodiment include the following compounds:
6-((6-methoxy-2-phenyl-1*H*-indol-3-ylmethyl)pyridine-2-carboxylic acid (Example 4-1), 3-(6-methoxy-2-phenyl-1*H*-indol-3-ylmethyl)benzoic acid (Example 4-2), 3-(6-methoxy-2-thiophen-3-yl-1*H*-indol-3-ylmethyl)benzoic acid (Example 4-12), 6-(6-ethoxy-2-phenyl-1*H*-indol-3-ylmethyl)pyridine-2-carboxylic acid (Example 4-34), 6-(6-methoxy-2-thiophen-3-yl-1*H*-indol-3-ylmethyl)pyridine-2-carboxylic acid (Example 11-1), 6-(2-(3-fluorophenyl)-6-methoxy-1*H*-indol-3-ylmethyl]pyridine-2-carboxylic acid (Example 11-5), 6-(2-furan-3-yl-6-methoxy-1*H*-indol-3-ylmethyl)pyridine-2-carboxylic acid (Example 11-10), 6-[2-(4-fluorophenyl)-6-methoxy-1*H*-indol-3-ylmethyl]pyridine-2-carboxylic acid (Example 11-13), 3-(2-furan-3-yl-6-methoxy-1*H*-indol-3-ylmethyl)benzoic acid (Example 11-15), 6-(6-methoxy-2-thiophen-2-yl-1*H*-indol-3-ylmethyl)pyridine-2-carboxylic acid (Example 11-17), 6-[6-methoxy-2-(5-methylthiophen-3-yl)-1*H*-indol-3-ylmethyl]pyridine-2-carboxylic acid (Example 11-20), 6-[2-(3-chlorophenyl)-6-methoxy-1*H*-indol-3-ylmethyl]pyridine-2-carboxylic acid (Example 11-21), 6-(6-methoxy-2-thiazol-5-yl-1*H-*indol-3-ylmethyl)pyridine-2-carboxylic acid (Example 11-24), 6-[6-methoxy-2-(3-methoxyphenyl)-1*H*-indol-3-ylmethyl]pyridine-2-carboxylic acid (Example 11-34), 6-[2-(3,4-difluorophenyl)-6-methoxy-1*H*-indol-3-ylmethyl]pyridine-2-carboxylic acid (Example 16-2), 6-[6-methoxy-2-(3,4,5-trifluorophenyl)-1*H*-indol-3-ylmethyl]pyridine-2-carboxylic acid (Example 16-6), 2-fluoro-3-(6-methoxy-2-phenyl-1*H*-indol-3-ylmethyl)benzoic acid (Example 16-9), 6-(6-methoxy-5-methyl-2-phenyl-1*H*-indol-3-ylmethyl)pyridine-2-carboxylic acid (Example 16-12), 6-(5-chloro-6-methoxy-2-phenyl-1*H*-indol-3-ylmethyl)pyridine-2-carboxylic acid (Example 16-13), 6-(5-fluoro-6-methoxy-2-phenyl-1*H*-indol-3-ylmethyl)pyridine-2-carboxylic acid (Example 16-16), 2-fluoro-5-(6-methoxy-2-phenyl-1*H*-indol-3-ylmethyl)benzoic acid (Example 16-18), 6-(6-methoxy-5-methyl-2-thiophen-3-yl-1*H*-indol-3-ylmethyl)pyridine-2-carboxylic acid (Example 16-24), 6-(5-fluoro-6-methoxy-2-thiophen-3-yl-1*H*-indol-3-ylmethyl)pyridine-2-carboxylic acid (Example 16-28), 6-(5-chloro-6-methoxy-2-thiophen-3-yl-1*H*-indol-3-ylmethyl)pyridine-2-carboxylic acid (Example 16-29), 6-[6-ethoxy-2-(4-fluorophenyl)-1*H*-indol-3-ylmethyl]pyridine-2-carboxylic acid (Example 16-60), 6-(6-ethoxy-2-thiophen-3-yl-1*H-*indol-3-ylmethyl)pyridine-2-carboxylic acid (Example 16-61), 6-(6-ethoxy-5-fluoro-2-thiophen-3-yl-1*H*-indol-3-ylmethyl)pyridine-2-carboxylic acid (Example 16-79), 6-(6-ethoxy-5-methyl-2-phenyl-1*H*-indol-3-ylmethyl)pyridine-2-carboxylic acid (Example 16-84), 6-[2-(4-fluorophenyl)-6-methoxy-5-methyl-1*H*-indol-3-ylmethyl]pyridine-2-carboxylic acid (Example 16-85), and 6-(6-ethoxy-5-methyl-2-thiophen-3-yl-1*H*-indol-3-ylmethyl)pyridine-2-carboxylic acid (Example 16-93).

### Embodiment 2

Another preferable embodiment of the present invention is as follows:
Y is a methylene group;
A is a benzene ring or a pyridine ring;
R¹ is a hydrogen atom;
R² is an unsubstituted phenyl group;
R³ is a methoxy group; and
R^{N} is a hydrogen atom.

Examples of the concrete compound included in the present embodiment include the following compounds:
6-((6-methoxy-2-phenyl-1*H*-indol-3-ylmethyl)pyridine-2-carboxylic acid (Example 4-1), 3-(6-methoxy-2-phenyl-1*H*-indol-3-ylmethyl)benzoic acid (Example 4-2), 2-fluoro-3-(6-methoxy-2-phenyl-1*H*-indol-3-ylmethyl)benzoic acid (Example 16-9), 6-(6-methoxy-5-methyl-2-phenyl-1*H*-indol-3-ylmethyl)pyridine-2-carboxylic acid (Example 16-12), 6-(5-chloro-6-methoxy-2-phenyl-1*H*-indol-3-ylmethyl)pyridine-2-carboxylic acid (Example 16-13), 6-(5-fluoro-6-methoxy-2-phenyl-1*H*-indol-3-ylmethyl)pyridine-2-carboxylic acid (Example 16-16), and 2-fluoro-5-(6-methoxy-2-phenyl-1*H*-indol-3-ylmethyl)benzoic acid (Example 16-18).

### Embodiment 3

Another preferable embodiment of the present invention is as follows:
Y is a methylene group;
A is a benzene ring or a pyridine ring;
R¹ is a hydrogen atom;
R² is a substituted phenyl group;
R³ is a methoxy group; and
R^{N} is a hydrogen atom.

Examples of the concrete compound included in the present embodiment include the following compounds:
6-[2-(3-fluorophenyl)-6-methoxy-1*H*-indol-3-ylmethyl]pyridine-2-carboxylic acid (Example 11-5), 6-[2-(4-fluorophenyl)-6-methoxy-1*H*-indol-3-ylmethyl]pyridine-2-carboxylic acid (Example 11-13), 6-[2-(3-chlorophenyl)-6-methoxy-1*H*-indol-3-ylmethyl]pyridine-2-carboxylic acid (Example 11-21), 6-[6-methoxy-2-(3-methoxyphenyl)-1*H*-indol-3-ylmethyl]pyridine-2-carboxylic acid (Example 11-34), 6-[2-(3,4-difluorophenyl)-6-methoxy-1*H*-indol-3-ylmethyl]pyridine-2-carboxylic acid (Example 16-2), 6-[6-methoxy-2-(3,4,5-trifluorophenyl)-1*H*-indol-3-ylmethyl]pyridine-2-carboxylic acid (Example 16-6), 6-[2-(5-fluoropyridin-3-yl)-6-methoxy-1*H*-indol-3-ylmethyl]pyridine-2-carboxylic acid (Example 16-11), 6-[2-(4-fluorophenyl)-6-methoxy-5-methyl-1*H*-indol-3-ylmethyl]pyridine-2-carboxylic acid (Example 16-85).

### Embodiment 4

Another preferable embodiment of the present invention is as follows:
Y is a methylene group;
A is a benzene ring or a pyridine ring;
R¹ is a hydrogen atom;
R² is a 5-membered aromatic heterocyclic group which may have a substituent;
R³ is a methoxy group; and
R^{N} is a hydrogen atom.

Examples of the concrete compound included in the present embodiment include the following compounds:
3-((6-methoxy-2-thiophen-3-yl-1*H*-indol-3-ylmethyl)benzoic acid (Example 4-12), 6-(6-methoxy-2-thiophen-3-yl-1*H*-indol-3-ylmethyl)pyridine-2-carboxylic acid (Example 11-1), 6-(2-furan-3-yl-6-methoxy-1*H*-indol-3-ylmethyl)pyridine-2-carboxylic acid (Example 11-10), 3-(2-furan-3-yl-6-methoxy-1*H*-indol-3-ylmethyl)benzoic acid (Example 11-15), 6-(6-methoxy-2-thiophen-2-yl-1*H*-indol-3-ylmethyl)pyridine-2-carboxylic acid (Example 11-17), 6-[6-methoxy-2-(5-methylthiophen-3-yl)-1*H*-indol-3-ylmethyl]pyridine-2-carboxylic acid (Example 11-20), 6-(6-methoxy-5-methyl-2-thiophen-3-yl-1*H*-indol-3-ylmethyl)pyridine-2-carboxylic acid (Example 16-24), 6-(5-fluoro-6-methoxy-2-thiophen-3-yl-1*H*-indol-3-ylmethyl)pyridine-2-carboxylic acid (Example 16-28), and 6-(5-chloro-6-methoxy-2-thiophen-3-yl-1*H*-indol-3-ylmethyl)pyridine-2-carboxylic acid (Example 16-29).

### Embodiment 5

Another preferable embodiment of the present invention is as follows:
Y is a methylene group;
A is a benzene ring or a pyridine ring;
R¹ is a hydrogen atom;
R² is a 6-membered aromatic heterocyclic group which may have a substituent;
R³ is a methoxy group; and
R^{N} is a hydrogen atom.

Examples of the concrete compound included in the present embodiment include the following compounds:
6-((6-methoxy-2-pyridin-3-yl-1*H*-indol-3-ylmethyl)pyridine-2-carboxylic acid (Example 11-11), 6-[2-(5-fluoropyridin-3-yl)-6-methoxy-1*H*-indol-3-ylmethyl]pyridine-2-carboxylic acid (Example 16-11), and 6-(6-methoxy-5-methyl-2-pyridin-3-yl-1*H*-indol-3-ylmethyl)pyridine-2-carboxylic acid (Example 16-66).

### Embodiment 6

Another preferable embodiment of the present invention is as follows:
Y is a methylene group;
A is a benzene ring or a pyridine ring;
R¹ is a hydrogen atom;
R² is a phenyl group which may have a substituent or a 5-membered aromatic heterocyclic group which may have a substituent;
R³ is a halogen atom; and
R^{N} is a hydrogen atom.

Examples of the concrete compound included in the present embodiment include the following compounds:
6-((6-chloro-2-phenyl-1*H*-indol- 3-ylmethyl)pyridine-2-carboxylic acid (Example 4-17), 6-(6-chloro-5-methoxy-2-phenyl-1*H*-indol-3-ylmethyl)pyridine-2-carboxylic acid (Example 16-23), 6-(6-chloro-5-fluoro-2-thiophen-3-yl-1*H*-indol-3-ylmethyl)pyridine-2-carboxylic acid (Example 16-52), 6-(6-chloro-5-methyl-2-phenyl-1*H*-indol-3-ylmethyl)pyridine-2-carboxylic acid (Example 16-59), 6-(6-chloro-5-methoxy-2-thiophen-3-yl-1*H*-indol-3-ylmethyl)pyridine-2-carboxylic acid (Example 16-86), 6-(6-chloro-5-methyl-2-thiophen-3-yl-1*H*-indol-3-ylmethyl)pyridine-2-carboxylic acid (Example 16-92), and 6-(6-chloro-2-furan-3-yl-5-methoxy-1*H*-indol-3-ylmethyl)pyridine-2-carboxylic acid (Example 16-105).

### Embodiment 7

Another preferable embodiment of the present invention is as follows:
Y is a methylene group;
A is a benzene ring or a pyridine ring;
R¹ is a hydrogen atom;
R² is an isopropyl group, an isobutyl group, a sec-butyl group or a tert-butyl group;
R³ is a methoxy group or an ethoxy group; and
R^{N} is a hydrogen atom.

Examples of the concrete compound included in the present embodiment include the following compounds:
6-((2-*tert*-butyl-5-chloro-6-methoxy-1*H*-indol-3-ylmethyl)pyridine-2-carboxylic acid (Example 16-89), 6-(2-*tert*-butyl-5-fluoro-6-methoxy-1*H*-indol-3-ylmethyl)pyridine-2-carboxylic acid (Example 16-97), 6-(2-*tert*-butyl-6-methoxy-5-methyl-1*H*-indol-3-ylmethyl)pyridine-2-carboxylic acid (Example 16-98), 6-(2-*sec*-butyl-5-chloro-6-methoxy-1*H*-indol-3-ylmethyl)pyridine-2-carboxylic acid (Example 16-107), 6-(5-chloro-2-isopropyl-6-methoxy-1*H*-indol-3-ylmethyl)pyridine-2-carboxylic acid (Example 16-109), and 6-(5-fluoro-2-isopropyl-6-methoxy-1*H*-indol-3-ylmethyl)pyridine-2-carboxylic acid (Example 18-1).

### Embodiment 8

Another preferable embodiment of the present invention is as follows:
Y is a methylene group;
A is a benzene ring or a pyridine ring;
R¹ is a hydrogen atom;
R² is a *tert*-butyl group, a phenyl group, or a 5-membered aromatic heterocyclic group;
R³ is a chlorine atom, a fluorine atom, a methyl group, a trifluoromethyl group, a methoxy group, an ethoxy group or a trifluoromethoxy group; and
R^{N} is a methyl group.

### Embodiment 9

Another preferable embodiment of the present invention is as follows:
Y is a methylene group;
A is a benzene ring or a pyridine ring;
R¹ is a C₁₋₆ alkyl group;
R² is a *tert*-butyl group, a phenyl group or a 5-membered aromatic heterocyclic group;
R³ is a chlorine atom, a fluorine atom, a methyl group, a trifluoromethyl group, a methoxy group, an ethoxy group or a trifluoromethoxy group; and
R^{N} is a hydrogen atom or a methyl group.

### Embodiment 10

Another preferable embodiment of the present invention is as follows:
Y is a methylene group;
A is a benzene ring or a pyridine ring;
R¹ is a hydrogen atom;
R² is a phenyl group which may have a substituent, a 5-membered aromatic heterocyclic group which may have a substituent or a 6-membered aromatic heterocyclic group which may have a substituent;
R³ is a cyclopropyl group; and
R^{N} is a hydrogen atom.

Examples of the concrete compound included in the present embodiment include the following compounds:
6-((6-cyclopropyl-2-phenyl-1*H*-indol-3-ylmethyl)pyridine-2-carboxylic acid (Example 16-8), 6-[6-cyclopropyl-2-(4-fluorophenyl)-1*H*-indol-3-ylmethyl]pyridine-2-carboxylic acid (Example 16-62), 6-(6-cyclopropyl-2-thiophen-3-yl-1*H*-indol-3-ylmethyl)pyridine-2-carboxylic acid (Example 16-65), 6-(6-cyclopropyl-2-pyridine-3-yl-1*H*-indol-3-ylmethyl)pyridine-2-carboxylic acid (Example 16-67), 6-(6-cyclopropyl-5-fluoro-2-phenyl-1*H*-indol-3-ylmethyl)pyridine-2-carboxylic acid (Example 16-68), 6-[6-cyclopropyl-5-fluoro-2-(4-fluorophenyl)-1*H*-indol-3-ylmethyl]pyridine-2-carboxylic acid (Example 16-74), 6-(6-cyclopropyl-5-fluoro-2-thiophen-3-yl-1*H*-indol-3-ylmethyl)pyridine-2-carboxylic acid (Example 16-75), 6-(6-cyclopropyl-5-fluoro-2-pyridine-3-yl-1*H*-indol-3-ylmethyl)pyridine-2-carboxylic acid (Example 16-76), 6-(6-cyclopropyl-5-methyl-2-phenyl-1*H*-indol-3-ylmethyl)pyridine-2-carboxylic acid (Example 16-100), 6-(6-cyclopropyl-5-methyl-2-pyridine-3-yl-1*H*-indol-3-ylmethyl)pyridine-2-carboxylic acid (Example 16-101), and 6-(6-cyclopropyl-5-methyl-2-thiophen-3-yl-1*H-*indol-3-ylmethyl)pyridine-2-carboxylic acid (Example 16-102).

### Embodiment 11

Another preferable embodiment of the present invention is as follows:
Y is a methylene group;
A is a benzene ring or a pyridine ring;
R¹ is a hydrogen atom;
R² is a phenyl group which may have a substituent, a 5-membered aromatic heterocyclic group which may have a substituent, or a 6-membered aromatic heterocyclic group which may have a substituent;
R³ is a methyl group; and
R^{N} is a hydrogen atom.

Examples of the concrete compound included in the present embodiment include the following compounds:
6-((6-methyl-2-phenyl-1*H*-indol-3-ylmethyl)pyridine-2-carboxylic acid (Example 4-35), 6-(6-methyl-2-thiophen-3-yl-1*H*-indol-3-ylmethyl)pyridine-2-carboxylic acid (Example 16-21), 6-(5,6-dimethyl-2-phenyl-1*H*-indol-3-ylmethyl)pyridine-2-carboxylic acid (Example 16-44), 6-[2-(4-fluorophenyl)-6-methyl-1*H*-indol-3-ylmethyl]pyridine-2-carboxylic acid (Example 16-48), 6-(5,6-dimethyl-2-thiophen-3-yl-1*H*-indol-3-ylmethyl)pyridine-2-carboxylic acid (Example 16-51), 6-(5-fluoro-6-methyl-2-phenyl-1*H-*indol-3-ylmethyl)pyridine-2-carboxylic acid (Example 16-53), 6-[5-fluoro-2-(4-fluorophenyl)-6-methyl-1*H*-indol-3-ylmethyl]pyridine-2-carboxylic acid (Example 16-54), 6-(5-fluoro-6-methyl-2-thiophen-3-yl-1*H*-indol-3-ylmethyl)pyridine-2-carboxylic acid (Example 16-56), 6-(5,6-dimethyl-2-pyridin-3-yl-1*H*-indol-3-ylmethyl)pyridine-2-carboxylic acid (Example 16-58), 6-(5-methoxy-6-methyl-2-phenyl-1*H*-indol-3-ylmethyl)pyridine-2-carboxylic acid (Example 16-78), 6-(5-methoxy-6-methyl-2-thiophen-3-yl-1*H*-indol-3-ylmethyl)pyridine-2-carboxylic acid (Example 16-108).

### Production Process of Compound (I) of the Present Invention

The compound (I) of the present invention or a pharmaceutically acceptable salt thereof can be prepared by a method described in the following Schemes 1 to 2 or 4, or a similar method thereto, or by a method described in other literature, or a similar method thereto.

### [A] Synthesis of Compounds (Ia) to (Id)

The compound (I) of the present invention can be prepared by the method shown in Schemes 1 to 2 or 4 as compounds (Ia) to (Id). Further, when a protective group is needed, combinations of introduction and cleavage can appropriately be carried out according to a usual method. 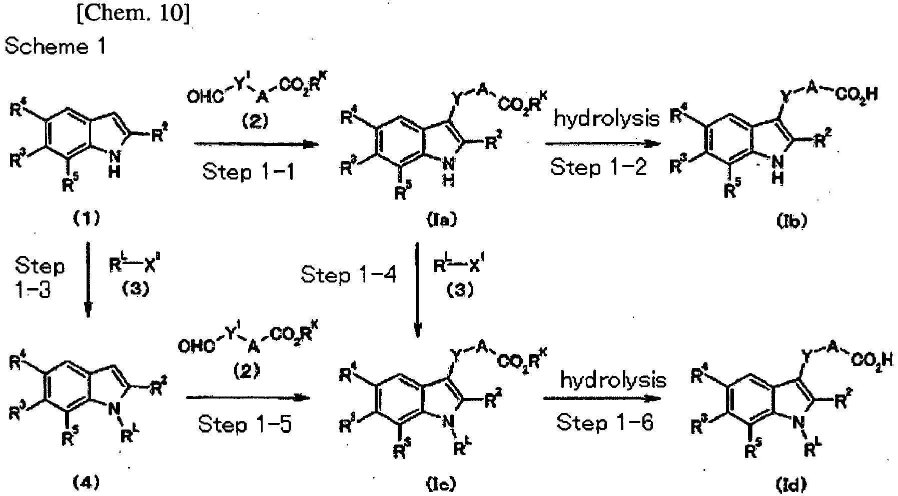 (wherein R², R³, R⁴, R⁵, A, and Y have the same meanings as defined above; R^{K} is a C₁₋₆ alkyl group or a C₇₋₁₀ aralkyl group; X¹ represents a leaving group such as a chlorine atom, a bromine atom, an iodine atom, a *p*-toluenesulfonyloxy group, a methanesulfonyloxy group and the like; Y¹ represents a single bond or a C₁₋₅ alkylene group; and R^{L} represents a C₁₋₆ alkyl group).

### Step 1-1

A compound (Ia) can be obtained by reacting a compound (1) with a compound (2) in a solvent in the presence of a reducing agent and an acid. Examples of the solvent to be used include methylene chloride, tetrahydrofuran and the like. Examples of the reducing agent include triethylsilane and the like, and examples of the acid include trifluoroacetic acid, acetic acid, trimethylsilyl trifluoromethanesulfonate, a borontrifluoride ethyl ether complex and the like. The reaction temperature is usually -70°C to 50°C, and the reaction time varies depending on a starting material to be used, a solvent to be used, or a reaction temperature, but it is usually 30 minutes to 3 days.

Furthermore, the compound (2) used in the present step may be commercially available or can be prepared according to a method described in other literature or a similar method thereto.

### Step 1-2

A compound (Ib) can be obtained by treating the compound (Ia) according to a method such as alkali hydrolysis and the like that are usually used for organic synthesis. Examples of the solvent to be used include methanol, ethanol, acetonitrile, tetrahydrofuran, 1,4-dioxane, water, a mixed solvent thereof and the like. Examples of the base include sodium hydroxide, potassium hydroxide, lithium hydroxide and the like. The reaction temperature usually ranges from room temperature to a solvent reflux temperature, and the reaction time varies depending on a starting material to be used, a solvent to be used, a reaction temperature or the like, but it is usually 30 minutes to 3 days. Further, the present step may be performed according to a method of acid hydrolysis or hydrogenolysis, or it may also be performed according to the method described in "Greene's Protective Groups in Organic Synthesis", edited by Theodra W. Greene & Peter G. M. Wuts, fourth edition, Wiley-Interscience, 2006.

### Step 1-3

A compound (4) can be obtained by reacting the compound (1) with a compound (3) in a solvent in the presence of a base. Examples of the solvent to be used include *N,N*-dimethylformamide, *N,N*-dimethylacetamide, *N,N*-dimethylimidazolidinone, 1-methyl-2-pyrrolidone, tetrahydrofuran, a mixed solvent thereof and the like. Examples of the base include sodium hydride, cesium carbonate, potassium carbonate, potassium *tert-*butoxide, lithium bis(trimethylsilyl)amide and the like. The reaction temperature usually ranges from -20°C to a solvent reflux temperature, and the reaction time varies depending on a starting material to be used, a solvent to be used, a reaction temperature or the like, but it is usually 30 minutes to 3 days. Further, in the present step, an additive such as sodium iodide and the like may be used, if necessary.

Furthermore, the compound (3) used in the present step may be commercially available or can be prepared according to a method described in other literature or a similar method thereto.

### Step 1-4

In the same manner as in Step 1-3, a compound (Ic) can be obtained from the compound (Ia) and the compound (3).

### Step 1-5

In the same manner as in Step 1-1, the compound (Ic) can be obtained from the compound (4) and the compound (2).

### Step 1-6

In the same manner as in Step 1-2, a compound (Id) can be obtained by hydrolyzing the compound (Ic).

Further, the compound (1) can be obtained according to the method shown in the following Scheme 3. 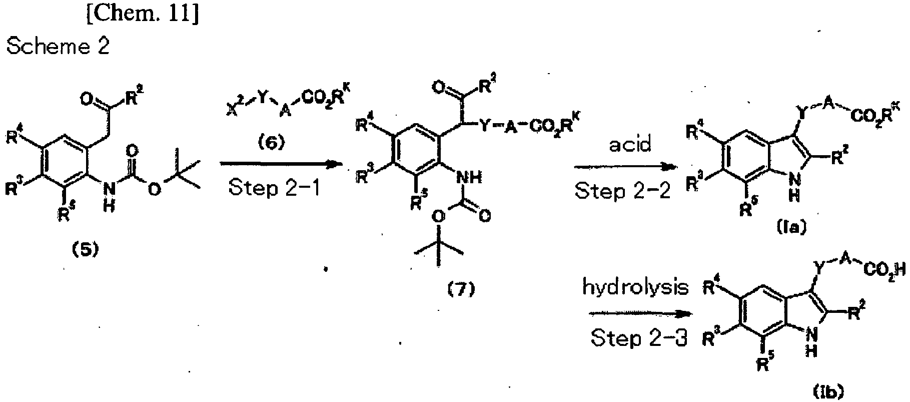 (wherein R², R³, R⁴, R⁵, R^{K}, A and Y have the same meanings as defined above; and X² represents a leaving group such as a chlorine atom, a bromine atom, an iodine atom, a p-toluenesulfonyloxy group, a methanesulfonyloxy group and the like.)

### Step 2-1

A compound (7) can be obtained by reacting a compound (5) with a compound (6) in a solvent in the presence of a base. Examples of the solvent to be used include *N,N-*dimethylformamide, *N,N*-dimethylacetamide, *N,N*-dimethylimidazolidinone, 1-methyl-2-pyrrolidone, tetrahydrofuran and the like, and examples of the base include sodium hydride, cesium carbonate, potassium carbonate, potassium *tert*-butoxide, 1,8-diazabicycloundec[5.4.0]-7-ene (DBU), lithium bis(trimethylsilyl)amide and the like. The reaction temperature usually ranges from -20°C to a solvent reflux temperature, and the reaction time varies depending on a starting material to be used, a solvent to be used, a reaction temperature or the like, but it is usually 30 minutes to 3 days. Further, in the present step, an additive such as sodium iodide and the like may be used, if necessary.

### Step 2-2

The compound (Ia) can be obtained by reacting the compound (7) under acidic conditions in a solvent or without a solvent. Examples of the solvent to be used include methylene chloride, tetrahydrofuran and the like, and examples of the acid include trifluoroacetic acid, acetic acid, hydrogen chloride, sulfuric acid and the like. The reaction temperature usually ranges from 0°C to a solvent reflux temperature, and the reaction time varies depending on a starting material to be used, a solvent to be used, a reaction temperature or the like, but it is usually 30 minutes to 24 hours.

### Step 2-3

In the same manner as in Step 1-2, the compound (Ib) can be obtained by hydrolyzing the compound (Ia).

Further, the compound (5) can be obtained by the Steps 3-6 to 3-8 shown in the following Scheme 3.

### [B] Synthesis of Compound (1) and Compound (5)

The compound (1) used in Scheme 1 and the compound (5) used in Scheme 2 can be obtained by the method shown in Scheme 3. 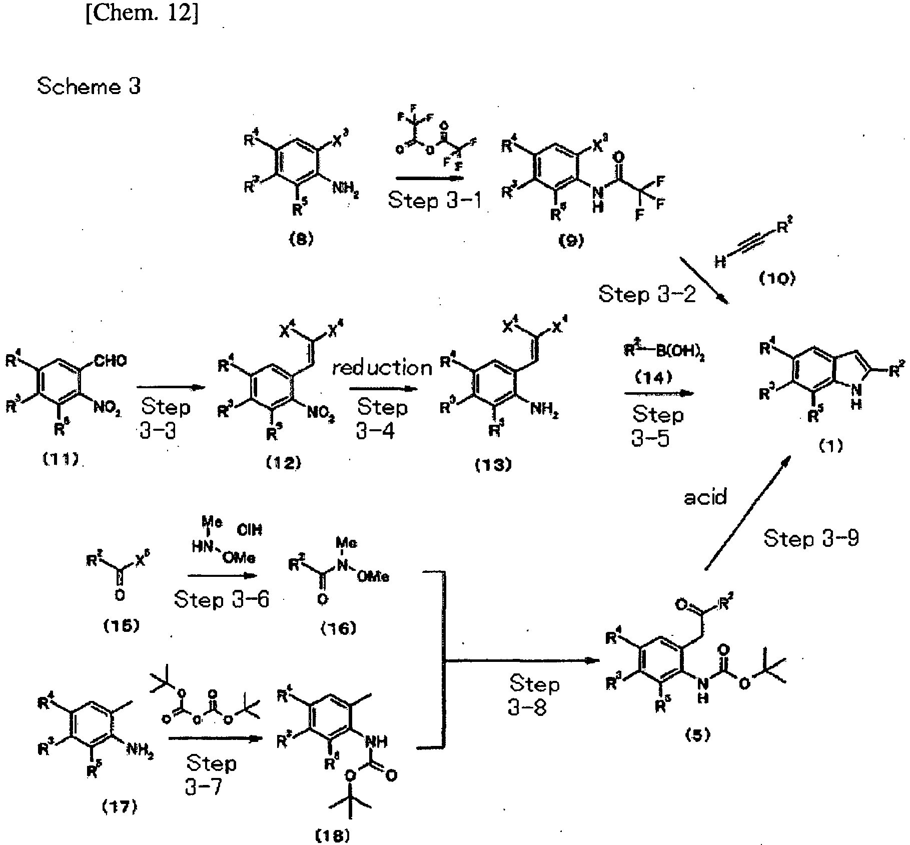 (wherein R², R³, R⁴ and R⁵ have the same meanings as defined above; X³ represents a leaving group such as a chlorine atom, a bromine atom, an iodine atom, a trifluoromethanesulfonyloxy group and the like; and X⁴ and X⁵ represent a halogen atom such as a chlorine atom, a bromine atom and the like.)

### Step 3-1

A compound (9) can be obtained by acylating the compound (8) using trifluoroacetic anhydride. Such an acylation reaction is well-known to a skilled person in the art, and can also be carried out according to the method described in "Greene's Protective Groups in Organic Synthesis", edited by Theodra W., Greene & Peter G. M. Wuts, fourth edition, Wiley-Interscience, 2006.

Furthermore, the compound (8) used in the present step may be commercially available or can be prepared according to a method described in other literature or a similar method thereto.

### Step 3-2

The compound (1) can be obtained by reacting the compound (9) with a compound (10) in a solvent in the presence of a base, a copper catalyst, and a palladium catalyst. Examples of the solvent to be used include tetrahydrofuran, acetonitrile, *N,N-*dimethylformamide and the like. Examples of the base include triethylamine, *N,N-*diisopropylethylamine, potassium carbonate, potassium phosphate and the like. Examples of the palladium catalyst include dichlorobis(triphenylphosphine)palladium (II), tetrakis(triphenylphosphine)palladium (0) and the like, and examples of the copper catalyst include copper (I) iodide and the like. The reaction temperature usually ranges from room temperature to a solvent reflux temperature, and the reaction time varies depending on a starting material to be used, a solvent to be used, a reaction temperature or the like, but it is usually 30 minutes to 3 days. Further, the present step can be carried even in the absence of a palladium catalyst, and in this case, an amino acid derivative such as *N,N-*dimethylglycine, L-proline and the like as an additive may be used.

### Step 3-3

A compound (12) can be obtained by reacting the compound (11) with a halomethane in a solvent in the presence of a base, and triphenylphosphine. Examples of the solvent to be used include methylene chloride, tetrahydrofuran and the like. Examples of the halomethane to be used include carbon tetrabromide, carbon tetrachloride, chloroform and the like. Examples of the base include potassium *tert*-butoxide and the like. The reaction temperature usually ranges from room temperature to a solvent reflux temperature, and the reaction time varies depending on a starting material to be used, a solvent to be used, a reaction temperature or the like, but it is usually 30 minutes to 24 hours. Further, the present step can be carried out in the absence of a base when carbon tetrabromide is used as the halomethane.

Furthermore, the compound (11) used in the present step may be commercially available or can be prepared according to a method described in other literature or a similar method thereto.

### Step 3-4

A compound (13) can be obtained by reducing the compound (12) by a general catalytic reduction method, a reduction method using a metal or a metal salt or the like.
1) The catalytic reduction method can be carried out with the compound (12) using a catalyst in a solvent under a hydrogen atmosphere. Examples of the solvent to be used include methanol, ethanol, ethyl acetate, tetrahydrofuran, acetic acid and the like. Examples of the catalyst include palladium carbon powders, rhodium carbon powders, platinum carbon powders, vanadium-doped platinum carbon powders and the like, and preferably vanadium-doped platinum carbon powders. The reaction temperature usually ranges from room temperature to a solvent reflux temperature, and the reaction time varies depending on a starting material to be used, a solvent to be used, a reaction temperature or the like, but the treatment may be conducted for 1 hour to 3 days.
2) The reduction method using a metal or a metal salt may be carried out with the compound (12) using a reducing agent in a solvent under acidic conditions. Examples of the solvent to be used include methanol, ethanol, tetrahydrofuran, and the like. Examples of the acid include acetic acid, ammonium chloride, hydrochloric acid, sulfuric acid and the like. Examples of the reducing agent include tin (II) chloride dihydrate, iron, zinc and the like. The reaction temperature usually ranges from room temperature to a solvent reflux temperature, and the reaction time varies depending on a starting material to be used, a solvent to be used, a reaction temperature or the like, but the treatment may be conducted for 30 minutes to 3 days.

### Step 3-5

The compound (1) can be obtained by reacting the compound (13) with a compound (14) in a solvent in the presence of a base, and a palladium catalyst. Examples of the solvent to be used include toluene, tetrahydrofuran, 1,4-dioxane, ethanol, water, a mixed solvent thereof and the like. Examples of the base include potassium phosphate, potassium phosphate monohydrate, potassium carbonate, cesium carbonate, cesium fluoride, sodium carbonate and the like. Examples of the palladium catalyst include bis(triphenylphosphine)palladium (II) dichloride, tetrakis (triphenylphosphine)palladium (0), palladium (II) acetate, tris(dibenzylideneacetone)dipalladium (0) and the like. The reaction temperature usually ranges from room temperature to a solvent reflux temperature, and the reaction time varies depending on a starting material to be used, a solvent to be used, a reaction temperature or the like, but it is usually 30 minutes to 3 days. Further, the present step may be carried out by addition of a ligand such as 2-dicyclohexylphosphino-2',6'-dimethoxybiphenyl, bis(diphenylphosphino)ferrocene and the like, as necessary.

### Step 3-6

A compound (16) can be obtained by reacting a compound (15) with *N,O-*dimethylhydroxylamine hydrochloride in a solvent under basic conditions. Examples of the solvent to be used include tetrahydrofuran, methylene chloride and the like, and examples of the base include triethylamine, pyridine, *N,N*-diisopropylethylamine and the like. The reaction temperature usually ranges from -20°C to room temperature, and the reaction time varies depending on a starting material to be used, a solvent to be used, a reaction temperature or the like, but it is usually 30 minutes to 24 hours.

Furthermore, the compound (15) used in the present step may be commercially available or can be prepared according to a method described in other literature or a similar method thereto.

### Step 3-7

A compound (18) can be obtained by *tert*-butoxycarbonylating a compound (17) using di-*tert*-butyl dicarbonate. This reaction is well-known to a skilled person in the art and can also be carried out according to the method described in "Greene's Protective Groups in Organic Synthesis" edited by Theodra W. Greene & Peter G. M. Wuts, fourth edition, Wiley-Interscience, 2006.

Furthermore, the compound (17) used in the present step may be commercially available or can be prepared according to a method described in other literature or a similar method thereto.

### Step 3-8

The compound (5) can be obtained by lithiating the compound (18) in a solvent using alkyllithium and then adding the compound (16) thereto. Examples of the solvent to be used include tetrahydrofuran, 1,4-dioxane, diethyl ether and the like, and examples of the alkyllithium include *n*-butyllithium, *sec*-butyllithium, *tert*-butyllithium and the like, and preferably *sec*-butyllithium. The reaction temperature usually ranges from -78°C to room temperature, and the reaction time varies depending on a starting material to be used, a solvent to be used, a reaction temperature or the like, but it is usually 30 minutes to 6 hours.

### Step 3-9

In the same manner as in Step 2-2, the compound (1) can be obtained from the compound (5).

Further, the compound (Ia) can also be obtained by the method shown in the following Scheme 4. 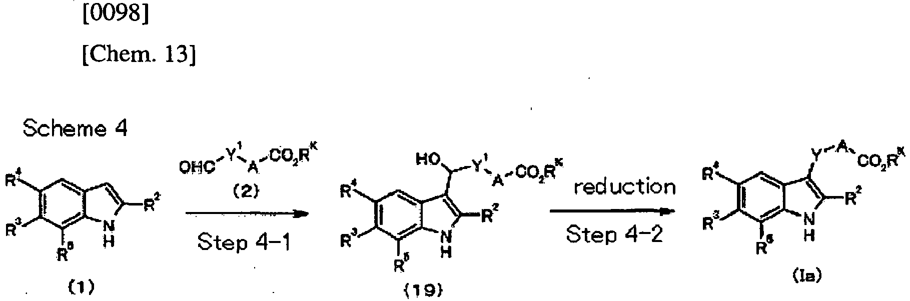 (wherein R², R³, R⁴, R⁵, A, R^{K}, Y, and Y¹ have the same meanings as defined above).

### Step 4-1

A compound (19) can be obtained by reacting the compound (1) with the compound (2) in a solvent in the presence of a base. Examples of the solvent to be used include methylene chloride, tetrahydrofuran, acetonitrile and the like. Examples of the base include 1,8-diazabicycloundec[5.4.0]-7-ene (DBU), sodium hydroxide, sodium methoxide, sodium ethoxide and the like. The reaction temperature is usually -20°C to 50°C, and the reaction time varies depending on a starting material to be used, a solvent to be used, or a reaction temperature, but it is usually 30 minutes to 3 days.

Furthermore, the compound (2) used in the present step may be commercially available or can be prepared according to a method described in other literature or a similar method thereto.

### Step 4-2

The compound (Ia) can be obtained by reacting the compound (19) with a reducing agent in a solvent. Examples of the solvent to be used include acetonitrile, ethyl acetate, tetrahydrofuran and the like. Examples of the reducing agent include sodium iodide/trialkylchlorosilane (for example, trimethylchlorosilane, triethylchlorosilane, t-butyldimethylchlorosilane) and the like. The reaction temperature is -30°C to room temperature, and the reaction time varies depending on a starting material to be used, a solvent to be used, or a reaction temperature, but it is usually 10 minutes to 24 hours.

Furthermore, the compound (Ia) can also be obtained by reacting the compound (19) with a reducing agent in a solvent under acidic conditions. Examples of the solvent to be used include tetrahydrofuran, methylene chloride, a mixed solvent thereof, and the like. Examples of the reducing agent include triethylsilane and the like, and examples of the acid include trifluoroacetic acid, acetic acid, trimethylsilyl trifluoromethanesulfonate, a borontrifluoride ethyl ether complex and the like. The reaction temperature is usually - 70°C to 50°C, and the reaction time varies depending on a starting material to be used, a solvent to be used, or a reaction temperature, but it is usually 30 minutes to 3 days.

These schemes shown above are exemplification of the method for preparing the compound (I) of the present invention or an intermediate for preparation. These are allowed to be modified to such a scheme that can be readily understood by a person skilled in the art.

Also, in the case that there is a need of a protective group according to the kind of the functional group, combinations of introduction and cleavage can be appropriately carried out according to a usual method. The type, introduction, and cleavage of the protective group can be illustrated in reference to the method described in, for example, "Greene's Protective Groups in Organic Synthesis", edited by Theodra W. Greene & Peter G. M. Wuts, fourth edition, Wiley-Interscience, 2006.

The intermediates used for preparation of the compound (I) of the present invention or a pharmaceutically acceptable salt thereof can be isolated/purified, as necessary, by solvent extraction, crystallization, recrystallization, chromatography, preparative high performance liquid chromatography or the like, that is an isolation/purification means well-known to a skilled person in the art.

### Pharmaceutical Composition Comprising Compound (I) of the Present Invention or Pharmaceutically Acceptable Salt thereof

The pharmaceutical composition comprising the compound (I) of the present invention or a pharmaceutically acceptable salt thereof as an active ingredient is used in various dosage forms according to the usages. Examples of the dosage forms include powders, granules, fine granules, dry syrups, tablets, capsules, injections, liquids, ointments, suppositories, plasters, sublinguals and the like, which are administered orally or parenterally.

These pharmaceutical compositions can be prepared by appropriately admixing or diluting/dissolving with pharmaceutical additives such as an excipient, a disintegrant, a binder, a lubricant, a diluent, a buffering agent, a tonicity agent, a preservative, a wetting agent, an emulsifier, a dispersant, a stabilizer, a solubilizing aid and the like by a well-known method according to the dosage forms. In addition, when used in combination with agents other than the EP₁ receptor antagonist, the pharmaceutical compositions can be prepared by formulating the respective active ingredients simultaneously or separately as described above.

### Pharmaceutical Use of Compound (I) of the Present Invention or Pharmaceutically Acceptable Salt thereof

The compound (I) of the present invention or a pharmaceutically acceptable salt thereof exhibits a potent EP₁ receptor antagonism in a test for confirmation of an EP₁ receptor antagonism. Therefore, the compound (I) of the present invention can suppress or decrease the intracellular calcium concentration. Accordingly, a pharmaceutical composition comprising the compound (I) of the present invention or a pharmaceutically acceptable salt thereof as an active ingredient can be used as an agent for treating or preventing diseases or symptoms caused by activation of the EP₁ receptor due to stimulus of a PGE₂.

In addition, examples of the diseases with the activation of the EP₁ receptor due to the PGE₂ stimulus include lower urinary tract symptoms (LUTS), inflammatory diseases, pain diseases, osteoporosis, cancer and the like. The pharmaceutical composition comprising the compound (I) of the present invention or a pharmaceutically acceptable salt thereof as an active ingredient is preferably used as an agent for treating or preventing LUTS, inflammatory diseases, or pain diseases. It is more preferably LUTS.

Examples of the disease that causes the lower urinary tract symptoms include overactive bladder (OAB), benign prostatic hyperplasia (BPH), cystitis such as interstitial cystitis and the like, prostatitis, and the like.

The "lower urinary tract symptoms" means storage symptoms, voiding symptoms, post micturition symptoms or the like. The compound (I) of the present invention or a pharmaceutically acceptable salt thereof is preferably used for treatment or prevention of storage symptoms.

Examples of the "storage symptoms" include urinary urgency, increased daytime frequency, nocturia, urinary incontinence (stress urinary incontinence, urge urinary incontinence, mixed urinary incontinence, enuresis, nocturnal enuresis, continuous urinary incontinence and the like), and bladder sensation (increased bladder sensation, reduced bladder sensation, absent bladder sensation, non-specific bladder sensation and the like). The compound (I) of the present invention or a pharmaceutically acceptable salt thereof is preferably used for treatment or prevention of urinary urgency, increased daytime frequency, nocturia, urge urinary incontinence, mixed urinary incontinence, enuresis, nocturnal enuresis, increased bladder sensation or non-specific bladder sensation. It is more preferably urinary urgency, increased daytime frequency, nocturia, urge urinary incontinence, or increased bladder sensation. Further, the compound (I) of the present invention or a pharmaceutically acceptable salt thereof is particularly preferable to use for the treatment or prevention of OABs.

### Combinations or Mixtures of Compound (I) of the Present Invention or Pharmaceutically Acceptable Salt thereof

The compound (I) of the present invention or a pharmaceutically acceptable salt thereof can be appropriately used in combination with at least one agent other than the EP₁ receptor antagonist.

Examples of the agent that can be used in combination with the compound (I) of the present invention or a pharmaceutically acceptable salt thereof include agents for the treatment of overactive bladder (OAB), benign prostatic hyperplasia (BPH), cystitis such as interstitial cystitis and the like, prostatitis, and the like, which have different action mechanisms from that of the EP₁ receptor antagonist. Examples of the agent include an anticholinergic agent, an α₁ antagonist, a β agonist, a 5α-reductase inhibitor, a PDE inhibitor, an acetylcholine esterase inhibitor, an anti-androgen, a progesterone-based hormone, an LH-RH analog, a neurokinin inhibitor, an antidiuretic, a calcium channel blocker, a direct smooth muscle agonist, a tricyclic antidepressant, a K channel modulator, a sodium channel blocker, an H₁ blocker, a serotonin reuptake inhibitor, a norepinephrine reuptake inhibitor, a dopamine reuptake inhibitor, a GABA agonist, a TRPV1 modulator, an endothelin antagonist, a 5-HT_{1A} antagonist, an α₁ agonist, an opioid agonist, a P₂X antagonist, a COX inhibitor, a σ agonist, a muscarinic agonist and the like. It is preferably an anticholinergic agent, an α₁ antagonist, a β agonist, a 5α-reductase inhibitor, a PDE inhibitor, a progesterone-based hormone, an anti-diuretic, a direct smooth muscle agonist or a tricyclic antidepressant.

Furthermore, concrete examples of the agent that is used in combination are illustrated as below, but the context of the present invention is not limited thereto. Further, examples of the concrete compound include a free form thereof, and other pharmaceutically acceptable salts.

Examples of the "anticholinergic agent" include oxybutynin, propiverine, solifenacin, tolterodine, imidafenacin, temiberin, darifenacin, fesoterodine, trospium, propantheline and the like.

Examples of the "α₁ antagonist" include urapidil, naphthopidil, tamsulosin, silodosin, prazosin, terazosin, alfuzosin, doxazosin, CR-2991, fiduxosin and the like.

Examples of the "β agonist" include YM-178, KUC-7483, KRP-204, SM-350300, TRK-380, amibegron, clenbuterol, SAR-150640, solabegron and the like.

Examples of the "5α-reductase inhibitor" include dutasteride, TF-505, finasteride, izonsteride and the like.

Examples of the "PDE inhibitor" include tadalafil, vardenafil, sildenafil, avanafil, UK-369003, T-0156, AKP-002, etazolate and the like.

Examples of the "acetylcholine esterase inhibitor" include distigmine, donepezil, Z-338, rivastigmine, ganstigmine, BGC-20-1259, galantamine, itopride, NP-61, SPH-1286, tolserine, ZT-1 and the like.

Examples of the "anti-androgen" include gestonorone, oxendolone, bicalutamide, BMS-641988, CB-03-01, CH-4892789, flutamide, MDV-3100, nilutamide, TAK-700, YM-580 and the like.

Examples of the "progesterone-based hormone" include chlormadinone, allylestrenol and the like.

Examples of the "LH-RH analog" include AEZS-108, buserelin, deslorelin, goserelin, histrelin, leuprorelin, lutropin, nafarelin, triptorelin, AEZS-019, cetrorelix, degarelix, elagolix, ganirelix, ozarelix, PTD-634, TAK-385, teverelix, TAK-448, TAK-683 and the like.

Examples of the "neurokinin inhibitor" include KRP-103, aprepitant, AV-608, casopitant, CP-122721, DNK-333, fosaprepitant, LY-686017, netupitant, orvepitant, rolapitant, TA-5538, T-2328, vestipitant, AZD-2624, Z-501, 1144814, MEN-15596, MEN-11420, SAR-102779, SAR-102279, saredutant, SSR-241586 and the like.

Examples of the "anti-diuretic" include desmopressin, VA-106483 and the like.

Examples of the "calcium channel blocker" include amlodipine, cilnidipine, propiverine, temiverine, PD-299685, aranidipine, azelnidipine, barnidipine, benidipine, bevantolol, clevidipine, CYC-381, diltiazem, efonidipine, fasudil, felodipine, gabapentin, gallopamil, isradipine, lacidipine, lercanidipine, lomerizine, manidipine, MEM-1003, nicardipine, nifedipine, nilvadipine, nimodipine, nisoldipine, SB-751689, verapamil, YM-58483, ziconotide and the like.

Examples of the "direct smooth muscle agonist" include flavoxate and the like.

Examples of the "tricyclic antidepressant" include imipramine, clomipramine, amitriptyline and the like.

Examples of the "K channel modulator" include nicorandil, NIP-141, NS-4591, NS-1643, andolrast, diazoxide, ICA-105665, minoxidil, pinacidil, tilisolol, VRX-698 and the like.

Examples of the "sodium channel blocker" include bepridil, dronedarone, propafenone, safinamide, SUN-N8075, SMP-986,1014802,552-02, A-803467, brivaracetam, cibenzoline, eslicarbazepine, F-15845, flecainide, fosphenytoin, lacosamide, lamotrigine, levobupivacaine, M-58373, mexiletine, moracizine, nerispirdine, NW-3509, oxcarbazepine, pilsicainide, pirmenol, propafenone, NW-1029, ropivacaine, vemakalant and the like.

Examples of the "H1 blocker" include acrivastine, alcaftadine, bepotastine, bilastine, cetirizine, desloratadine, ebastine, efletirizine, epinastine, fexofenadine, GSK-835726, levocabastine, levocetirizine, loratadine, mequitazine, mizolastine, NBI-75043, ReN-1869, terfenadine, UCB-35440, vapitazine, YM-344484, diphenhydramine, chlorpheniramine and the like.

Examples of the "serotonin reuptake inhibitor" include UCB-46331, 424887, AD-337, BGC-20-1259, BMS-505130, citalopram, dapoxetine, desvenlafaxine, DOV-102677, DOV-216303, DOV-21947, duloxetine, escitalopram, F-2695, F-98214-TA, fluoxetine, fluvoxamine, IDN-5491, milnacipran, minaprine, NS-2359, NSD-644, paroxetine, PF-184298, SD-726, SEP-225289, SEP-227162, SEP-228425, SEP-228432, sertraline, sibutramine, tesofensine, tramadol, trazodone, UCB-46331, venlafaxine, vilazodone, WAY-426, WF-516 and the like.

Examples of the "norepinephrine reuptake inhibitor" include AD-337, desvenlafaxine, DOV-102677, DOV-216303, DOV-21947, duloxetine, F-2695, F-98214-TA, milnacipran, NS-2359, NSD-644, PF-184298, SD-726, SEP-225289, SEP-227162, SEP-228425, SEP-228432, sibutramine, tesofensine, tramadol, venlafaxine, bupropion, radafaxine, atomoxetine, DDP-225, LY-2216684, neboglamine, NRI-193, reboxetine, tapentadol, WAY-256805, WAY-260022 and the like.

Examples of the "dopamine reuptake inhibitor" include DOV-102677, DOV-216303, DOV-21947, IDN-5491, NS-2359, NSD-644, SEP-225289, SEP-228425, SEP-228432, sibutramine, tesofensine, tramadol, brasofensine, bupropion, NS-27100, radafaxine, safinamide and the like.

Examples of the "GABA agonist" include retigabine, eszopiclone, indiplon, pagoclone, SEP-225441, acamprosate, baclofen, AZD-7325, BL-1020, brotizolam, DP-VPA, progabide, propofol, topiramate, zopiclone, EVT-201, AZD-3043, ganaxolone, NS-11394, arbaclofen, AZD-3355, GS-39783, ADX-71441, ADX-71943 and the like.

Examples of the "TRPV1 modulator" include capsaicin, resiniferatoxin, DE-096, GRC-6211, AMG-8562, JTS-653, SB-705498, A-425619, A-784168, ABT-102, AMG-628, AZD-1386, JNJ-17203212, NGD-8243, PF-3864086, SAR-115740, SB-782443 and the like.

Examples of the "endothelin antagonist" include SB-234551, ACT-064992, ambrisentan, atrasentan, bosentan, clazosentan, darusentan, fandosentan, S-0139, TA-0201, TBC-3711, zibotentan, BMS-509701, PS-433540 and the like.

Examples of the "5-HT_{1A} antagonist" include espindolol, lecozotan, lurasidone, E-2110, REC-0206, SB-649915, WAY-426, WF-516 and the like.

Examples of the "α₁ agonist" include CM-2236, armodafinil, midodrine, modafinil and the like.

Examples of the "opioid agonist" include morphine, TRK-130, DPI-125, DPI-3290, fentanyl, LIF-301, loperamide, loperamide oxide, remifentanil, tapentadol, WY-16225, oxycodone, PTI-202, PTI-721, ADL-5747, ADL-5859, DPI-221, DPI-353, IPP-102199, SN-11, ADL-10-0101, ADL-10-0116, asimadoline, buprenorphine, CR-665, CR-845, eptazocine, nalbuphine, nalfurafine, pentazocine, XEN-0548, W-212393, ZP-120, nalmefene and the like.

Examples of the "P₂X antagonist" include A-740003, AZ-11657312, AZD-9056, GSK-1482160, GSK-31481A and the like.

Examples of the "COX inhibitor" include aceclofenac, ST-679, aspirin, bromfenac, dexketoprofen, flurbiprofen, FYO-750, ibuprofen, ketoprofen, ketorolac, licofelone, lornoxicam, loxoprofen, LT-NS001, diclofenac, mofezolac, nabumetone, naproxen, oxaprozin, piroxicam, pranoprofen, suprofen, tenoxicam, tiaprofenic acid, tolfenamic acid, zaltoprofen, 644784, ABT-963, ajulemic acid, apricoxib, celecoxib, cimicoxib, etoricoxib, iguratimod, lumiracoxib, meloxicam, nimesulide, parecoxib, RO-26-2198, valdecoxib and the like.

Examples of the "σ agonist" include ANAVEX-27-1041, PRS-013, SA-4503, ANAVEX-2-73, siramesine, ANAVEX-7-1037, ANAVEX-1-41 and the like.

Examples of the "muscarinic agonist" include AC-260584, cevimeline, MCD-386, NGX-267, NGX-292, sabcomeline, pilocarpine, bethanechol and the like.

When the compound (I) of the present invention or a pharmaceutically acceptable salt thereof is used in combination with one or more of the above-described agents, the present invention includes at least one administration method selected from the following 1) to 5):
1) simultaneous administration by a combination preparation,
2) simultaneous administration by the same administration pathway as a separate formulation,
3) simultaneous administration by a different administration pathway as a separate formulation,
4) administration at different times by the same administration pathway as a separate formulation, or
5) administration at different times by a different administration pathway as a separate formulation. Further, in the case of administration at different times as a separate formulation as in 4) or 5), the order of administration of the compound (I) of the present invention and the above-described agents is not particularly limited.

Furthermore, the compound (I) of the present invention or a pharmaceutically acceptable salt thereof can be used appropriately in combination of one or more of the above-described agents to attain an advantageous effect that is equal to or more than an additive effect in the prevention or treatment of the above-described diseases. Alternatively, as compared with a case of being used alone, the amount used can be reduced, the side effects of the agent used together can be reduced, or the side effects of the agent used together can be avoided or mitigated.

### Usage/Dose of Compound (I) of the Present Invention

The pharmaceutical of the present invention can be administered systematically or locally, orally or parenterally (nasal, pulmonary, intravenous, rectal, subcutaneous, intramuscular, transdermal and the like).

When the pharmaceutical composition of the present invention is used for practical treatments, the dose of the compound (I) of the present invention or a pharmaceutically acceptable salt thereof that is the active ingredient is appropriately determined by taking the patient's age, gender, weight, medical condition, degree of the treatment and the like into consideration. For example, in case of oral administration, administration can be conducted appropriately at a daily dose in the range from about 0.01 to 1000 mg for an adult (as a body weight of 60 kg), and in case of parenteral administration, administration can be conducted appropriately at a daily dose in the range from about 0.001 to 300 mg for an adult in one portion or in several divided portions. In addition, the dose of the compound (I) of the present invention or a pharmaceutically acceptable salt thereof can be reduced according to the amount of the agent other than an EP₁ receptor antagonist.

Hereinbelow, the present invention is illustrated in detail with reference to Examples, Reference Examples, and Test Examples.

### Examples

In the symbols used in each of Reference Examples, Examples, and Tables, Ref. No. means Reference Example No., Ex. No. means Example No., Strc means a chemical structural formula, Physical data means physical property values, 1H-NMR means a proton nuclear magnetic resonance spectrum, CDCl3 means chloroform-d, and DMSO-d6 means dimethylsulfoxide-d₆. Further, MS means mass spectroscopy, and ESI means measurement by an electrospray ionization method.

### (Reference Example 1-1)

### N-(2-Bromo-5-trifluoromethoxyphenyl)-2,2,2-trifluoroacetamide

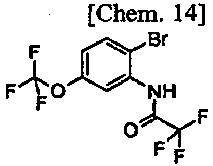

Under ice-cooling, to a solution of 2-bromo-5-trifluoromethoxyaniline (0.98 g) in tetrahydrofuran (10 mL) were added trifluoroacetic anhydride (0.79 mL) and pyridine (0.61 mL). The solution was stirred at room temperature for 16 hours. The reaction mixture was concentrated under reduced pressure. To the residue was added ethyl acetate, and the organic layer was washed with 10% aqueous citric acid solution, a saturated aqueous sodium hydrogen carbonate solution, and saturated brine successively, and then dried over anhydrous sodium sulfate. The solvent was evaporated under reduced pressure and the obtained crude product was purified by silica gel column chromatography (eluting solvent: hexane-ethyl acetate) to obtain the title compound (1.30 g).

1H-NMR (CDCl3) δ ppm: 8.60-8.30 (2H, m), 7.65 (1H, d, J=8.9Hz), 7.10-7.00 (1H, m)

In the same manner as in Reference Example 1-1 using the corresponding aniline derivatives, Reference Examples 1-2 to 1-3 below were obtained. The structural formulae and the physical property values thereof were shown in Table 1.

**[Table 1]**

| Ref. No. | Strc | Physical data |
|---|---|---|
| 1-2 | 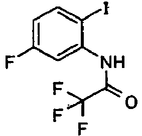 | 1H-NMR (CDCl3) *δ* ppm:8.45-8.25 (1H, br), 8.15-8.10 (1H, m), 7.85-7.75 (1H, m), 6.85-6.75 (1H, m) |
| 1-3 | 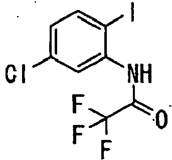 | 1H-NMR (CDCl3) *δ* ppm:8.40-8.15 (2H, m), 7.75 (1H, d, J=8.5Hz), 7.00 (1H, dd, J=8.5, 2.4Hz) |

### (Reference Example 2-1)

### 2-Phenyl-6-trifluoromethoxy-1H-indole

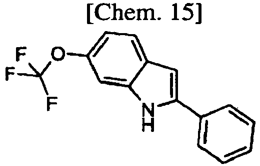

To a solution of *N*-(2-bromo-5-trifluoromethoxyphenyl)-2,2,2-trifluoroacetamide (0.513 g) in acetonitrile (10 mL) were added bis(triphenylphosphine)palladium (II) dichloride (30.7 mg), copper iodide (16.6 mg), and triethylamine (0.51 mL), and the mixture was stirred at 120°C for 10 minutes under microwave irradiation. After cooling to room temperature, to the reaction mixture was added potassium carbonate (0.503 g), and the mixture was stirred at 120°C for 10 minutes under microwave irradiation. The reaction mixture was filtered through Celite (registered trademark) to remove the insoluble materials. To the filtrate were added ethyl acetate and saturated brine. The organic layer was separated, dried over anhydrous sodium sulfate, and concentrated under reduced pressure. The obtained crude product was purified by aminopropylated silica gel column chromatography (eluting solvent: hexane-ethyl acetate) to obtain the title compound (0.233 g).

1H-NMR (CDCl3) δ ppm: 8.50-8.35 (1H, br), 7.70-7.55 (3H, m), 7.50-7.30 (3H, m), 7.05-6.95 (1H, m), 6.85-6.80 (1H, m)

In the same manner as in Reference Example 2-1 using the corresponding trifluoroacetanilide derivatives, Reference Examples 2-2 to 2-3 below were obtained. The structural formulae and the physical property values thereof were shown in Table 2.

**[Table 2]**

| Ref. No. | Strc | Physical data |
|---|---|---|
| 2-2 | 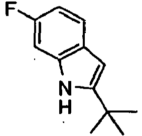 | 1H-NMR (CDCl3) *δ* ppm:8.05-7.80 (1H, br), 7.45-7.35 (1H, m), 7.05-6.95 (1H, m), 6.90-6.75 (1H, m), 6.25-6.20 (1H, m), 1.38 (9H, s) |
| 2-3 | 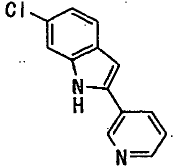 | 1H-NMR (CDCl3) *δ* ppm:9.00-8.90 (1H, m), 8.65-8.40 (2H, m), 8.00-7.85 (1H, m), 7.55 (1H, d, J=8.5Hz), 7.50-7.30 (2H, m), 7.12 (1H, dd, J=8.5, 1.8Hz), 6.90-6.80 (1H, m) |
| | | MS(ESI, m/z) = 229 (M+H)+ |

### (Reference Example 3-1)

### tert-Butyl (5-methoxy-2-methylphenyl)carbamate

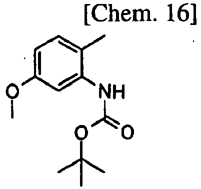

At room temperature, to a solution of 5-methoxy-2-methylaniline (2.74 g) in tetrahydrofuran (80 mL) was added di-*tert*-butyl dicarbonate (4.80 g), and the solution was heated to reflux at 75°C overnight. After being left to be cooled, the solution was concentrated under reduced pressure. The residue was purified by aminopropylated silica gel column chromatography (eluting solvent: hexane-ethyl acetate) to obtain the title compound (4.00 g).

1H-NMR (CDCl3) δ ppm: 7.65-7.45 (1H, m), 7.02 (1H, d, J=8.4Hz), 6.55 (1H, dd, J=8.4, 2.7Hz), 6.27(1H, br s), 3.80 (3H, s), 2.17 (3H, s), 1.53 (9H, s)

In the same manner as in Reference Example 3-1 using the corresponding 2-methylaniline derivatives, Reference Examples 3-2 to 3-6 below were obtained. The structural formulae and the physical property values thereof were shown in Table 3.

**[Table 3]**

| Ref. No. | Strc | Physical data |
|---|---|---|
| 3-2 | 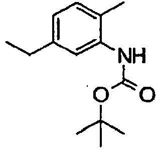 | 1H-NMR (CDCl3) *δ* ppm:7.75-7.65 (1H, m), 7.05 (1H, d, J=7.7Hz), 6.83 (1H, dd, J=7.7, 1.7Hz), 6.23 (1H, br s), 2.61 (2H, q, J=7.6Hz), 2.21 (3H, s), 1.53 (9H, s), 1.22 (3H, t, J=7.6Hz) |
| 3-3 | 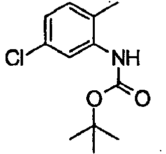 | 1H-NMR (CDCl3) *δ* ppm:8.05-7.85 (1H, m), 7.04 (1H, d, J=8.1Hz), 6.94 (1H, dd, J=8.1, 2.1 Hz), 6.26 (1H, br s), 2.20 (3H, s), 1.53 (9H, s) |
| 3-4 | 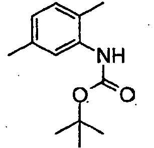 | 1H-NMR (CDCl3) *δ* ppm:7.75-7.60 (1H, m), 7.05-6.95 (1H, m), 6.85-6.75 (1H, m), 6.23 (1H, br s), 2.31 (3H, s), 2.20 (3H, s), 1.52 (9H, s) |
| 3-5 | 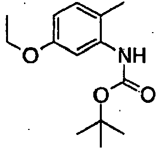 | 1H-NMR (CDCl3) *δ* ppm:7.65-7.45 (1H, m), 7.00 (1H, d, J=8.3Hz), 6.54 (1H, dd, J=8.3, 2.6Hz), 6.25 (1H, br s), 4.02 (2H, q, J=7.0Hz), 2.16 (3H, s), 1.52 (9H, s), 1.38 (3H, t, J=7.0Hz) |
| 3-6 | 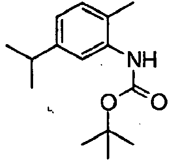 | 1H-NMR (CDCl3) *δ* ppm:7.80-7.60 (1H, m), 7.06 (1H, d, J=7.8Hz), 6.87 (1H, dd, J=7.8, 1.8Hz), 6.23 (1H, s), 2.95-2.80 (1H, m), 2.21 (3H, s), 1.53 (9H, s), 1.24 (6H, d, J=6.9Hz) |

### (Reference Example 4-1)

### tert-Butyl [5-methoxy-2-(2-oxo-2-phenylethyl)phenyl]carbamate

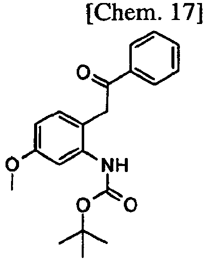

Under an argon gas atmosphere, to a solution of *tert*-butyl (5-methoxy-2-methylphenyl)carbamate (3.87 g) in tetrahydrofuran (70 mL) was added dropwise a *sec-*butyl lithium solution (1.04 mol/L *n*-hexane-cyclohexane solution, 31.36 mL) over 19 minutes while keeping the inner temperature at -50°C or lower. The solution was stirred for 20 minutes while keeping the inner temperature at -48°C. And a solution of *N-*methoxy-*N*-methylbenzamide (2.69 g) in tetrahydrofuran (32 mL) was added thereto over 15 minutes while keeping the inner temperature at -40°C or lower. The solution was stirred for 20 minutes while keeping the inner temperature at -40°C or lower, and further stirred at room temperature for 1 hour and 20 minutes. Under ice-cooling, 2 mol/L hydrochloric acid was added to the reaction mixture, and the mixture was stirred at room temperature for 5 minutes, followed by addition of ethyl acetate. The organic layer was separated, washed with water and saturated brine successively, and dried over anhydrous magnesium sulfate. The solvent was evaporated under reduced pressure. The obtained crude product was purified by silica gel column chromatography (eluting solvent: hexane-ethyl acetate), and then by aminopropylated silica gel column chromatography (eluting solvent: hexane-ethyl acetate) to obtain the title compound (2.49 g).

1H-NMR (CDCl3) δ ppm: 8.10-8.00 (2H, m), 7.90-7.70 (1H, br), 7.65-7.40 (4H, m), 7.09 (1H, d, J=8.5Hz), 6.59 (1H, dd, J=8.5, 2.6Hz), 4.19 (2H, s), 3.79 (3H, s), 1.53 (9H, s)

In the same manner as in Reference Example 4-1 using the corresponding *tert-*butyl (2-methylphenyl)carbamate derivatives, Reference Examples 4-2 to 4-11 below were obtained. The structural formulae and the physical property values thereof were shown in Tables 4 and 5.

**[Table 4]**

| Ref. No. | Strc | Physical data |
|---|---|---|
| 4-2 | 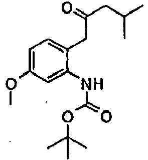 | 1H-NMR (CDCl3) *δ* ppm:7.65-7.50 (2H, m), 7.02 (1H, d, J=8.4Hz), 6.60 (1H, dd, J=8.4, 2.7Hz), 3.80 (3H, s), 3.58 (2H, s), 2.41 (2H, d, J=6.9Hz), 2.25-2.05 (1H, m), 1.52 (9H, s), 0.88 (6H, d, J=6.6Hz) |
| 4-3 | 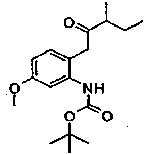 | 1H-NMR (CDCl3) *δ* ppm:7.78 (1H, br s), 7.55-7.40 (1H, m), 7.01 (1H, d, J=8.4Hz), 6.59 (1H, dd, J=8.4, 2.7Hz), 3.80 (3H, s), 3.65 (2H, s), 2.70-2.55 (1H, m), 1.80-1.35 (11H, m), 1.09 (3H, d, J=7.0Hz), 0.82 (3H, t, J=7.5Hz) |
| 4-4 | 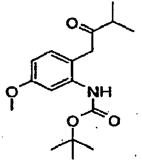 | 1H-NMR (CDCl3) *δ* ppm:7.72(1H, br s), 7.55-7.40 (1H, m), 7.02 (1H, d, J=8.4Hz), 6.59 (1H, dd, J=8.4, 2.7Hz), 3.80 (3H, s), 3.66 (2H, s), 2.85-2.70 (1H, m), 1.52 (9H, s), 1.13 (6H, d, J=7.0Hz) |
| 4-5 | 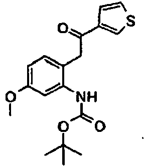 | 1H-NMR (CDCl3) *δ* ppm:8.23 (1H, dd, J=2.9, 1.2Hz), 7.96 (1H, br s), 7.56 (1H, dd, J=5.2, 1.2Hz), 7.55-7.45 (1H, m), 7.35 (1H, dd, J=5.2, 2.9Hz), 7.08 (1H, d, J=8.5Hz), 6.59 (1H, dd, J=8.5, 2.7Hz), 4.08 (2H, s), 3.79 (3H, s), 1.53 (9H, s) |
| 4-6 | 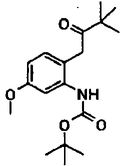 | 1H-NMR (CDCl3) *δ* ppm:8.06 (1H, br s), 7.60-7.30 (1H, m), 6.98 (1H, d, J=8.5Hz), 6.57 (1H, dd, J=8.5, 2.7Hz), 3.80 (3H, s), 3.72 (2H, s), 1.52 (9H, s), 1.22 (9H,s) |

**[Table 5]**

| Ref. No. | Strc | Physical data |
|---|---|---|
| 4-7 | 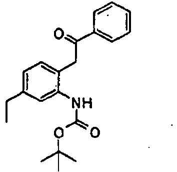 | 1H-NMR (CDCl3) *δ* ppm:8.15-8.00 (2H, m), 7.75-7.45 (5H, m), 7.15-7.05 (1H, m), 6.95-6.85 (1H, m), 4.24 (2H, s), 2.62 (2H, q, J=7.6Hz), 1.52 (9H, s), 1.21 (3H, t, J=7.6Hz) |
| 4-8 | 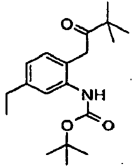 | 1H-NMR (CDCl3) *δ* ppm:8.05-7.45 (2H, m), 7.10-6.75 (2H, m), 3.76 (2H, s), 2.70-2.50 (2H, m), 1.60-1.10 (21 H, m) |
| 4-9 | 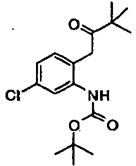 | 1H-NMR (CDCl3) *δ* ppm:8.20-7.80(2H, m), 7.10-6.95 (2H, m), 3.76 (2H, s), 1.52 (9H, s), 1.23 (9H, s) |
| 4-10 | 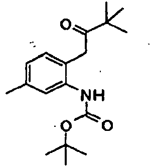 | 1H-NMR (CDCl3) *δ* ppm:7.88 (1H, br s), 7.65-7.50 (1H, m), 7.00-6.95 (1H, m), 6.90-6.80 (1H, m), 3.76 (2H, s), 2.32 (3H, s), 1.52 (9H, s), 1.23 (9H, s) |
| 4-11 | 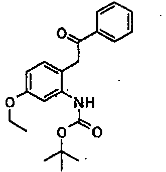 | 1H-NMR (CDCl3) *δ* ppm:8.10-8.00 (2H, m), 7.77 (1H, br s), 7.65-7.40 (4H, m), 7.08 (1H, d, J=8.5Hz), 6.58 (1H, dd, J=8.5, 2.6Hz), 4.18 (2H, s), 4.02 (2H, q, J=7.0Hz), 1.52 (9H, s), 1.37 (3H, t, J=7.0Hz) |

### (Reference Example 5-1)

### 6-Methoxy-2-phenyl-1H-indole

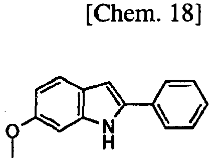

A solution of *tert*-butyl [5-methoxy-2-(2-oxo-2-phenylethyl)phenyl]carbamate (170 mg) in trifluoroacetic acid (0.80 mL)-methylene chloride (2.0 mL) was stirred at room temperature overnight. The solvent was evaporated under reduced pressure. To the residue was added methylene chloride, and to the solution was added a saturated aqueous sodium hydrogen carbonate solution. The organic layer was separated and concentrated under reduced pressure. The obtained crude product was purified by aminopropylated silica gel column chromatography (eluting solvent: hexane-ethyl acetate) to obtain the title compound (80.1 mg).

1H-NMR (CDCl3) δ ppm: 8.30-8.10 (1H, br), 7.65-7.35 (5H, m), 6.91 (1H, d, J=2.0Hz), 6.85-6.70 (2H, m), 3.87 (3H, s)

In the same manner as in Reference Example 5-1 using the corresponding ketones, Reference Examples 5-2 to 5-10 below were obtained. The structural formulae and the physical property values thereof were shown in Tables 6 and 7.

**[Table 6]**

| Ref. No. | Strc | Physical data |
|---|---|---|
| 5-2 | 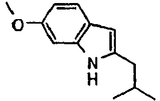 | 1H-NMR (CDCl3) *δ* ppm: 7.80-7.60 (1H, br), 7.39 (1H, d, J=8.6Hz), 6.83 (1H, d, J=2.3Hz), 6.74 (1H, dd, J=8.6, 2.3Hz), 6.15 (1H, s), 3.84 (3H, s), 2.58 (2H, d, J=7.2Hz), 1.95 (1H, m), 0.97 (6H, d, J=6.6Hz) |
| 5-3 | 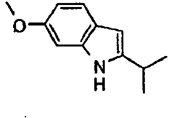 | 1H-NMR (CDCl3) *δ* ppm:7.90-7.65 (1H, br), 7.40 (1H, d, J=8.6Hz), 6.83 (1H, d, J=2.3Hz), 6.74 (1H, dd, J=8.6, 2.3Hz), 6.20-6.10 (1H, m), 3.83 (3H, s), 3.10-2.95 (1H, m), 1.34 (6H, d, J=7.0Hz) |
| 5-4 | 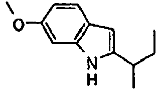 | 1H-NMR (CDCl3) *δ* ppm:7.85-7.65 (1H, br), 7.40 (1H, d, J=8.6Hz), 6.84 (1H, d, J=2.2Hz), 6.74 (1H, dd, J=8.6, 2.2Hz), 6.20-6.10 (1H, m), 3.84 (3H, s), 2.90-2.70 (1H, m), 1.80-1.55 (2H, m), 1.32 (3H, d, J=7.0Hz), 0.91 (3H, t, J=7.4Hz) |
| 5-5 | 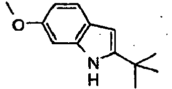 | 1H-NMR (CDCl3) *δ* ppm:8.00-7.65 (1H, br), 7.40 (1H, d, J=8.6Hz), 6.84 (1H, d, J=2.2Hz), 6.74 (1H, dd, J=8.6, 2.2Hz), 6.20-6.10 (1H, m), 3.83 (3H, s), 1.37 (9H, s) |
| 5-6 | 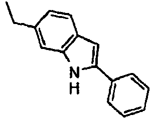 | 1H-NMR (CDCl3) *δ* ppm:8.40-8.10 (1H, br), 7.70-7.40 (5H, m), 7.35-7.20 (2H, m), 7.05-6.95 (1H, m), 6.85-6.75 (1H, m), 2.77 (2H, q, J=7.6Hz), 1.30 (3H, t, J=7.6Hz) |

**[Table 7]**

| Ref. No. | Strc | Physical data |
|---|---|---|
| 5-7 | 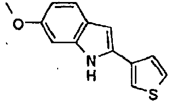 | 1H-NMR (CDCl3) δ ppm:8.20-7.95 (1H, br), 7.47 (1H, d, J=8.6Hz), 7.45-7.30 (3H, m), 6.88 (1H, d, J=2.3Hz), 6.79 (1H, dd, J=8.6, 2.3Hz), 6.65-6.60 (1H, m), 3.86 (3H, s) |
| 5-8 | 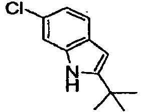 | 1H-NMR (CDCl3) δ ppm:8.10-7.80 (1H, br), 7.45-7.25 (2H, m), 7.03 (1H, dd, J=8.4, 1.8Hz), 6.25-6.20 (1H, m), 1.38 (9H, s) |
| 5-9 | 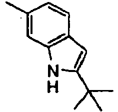 | 1H-NMR (CDCl3) δ ppm:7.81 (1H, br s), 7.41 (1H, d, J=8.0Hz), 7.15-6.85 (2H, m), 6.25-6.15 (1H, m), 2.44 (3H, s), 1.38 (9H, s) |
| 5-10 | 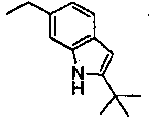 | 1H-NMR (CDCl3) δ ppm:7.43 (1H, d, J=8.0Hz), 7.20-6.85 (2H, m), 6.25-6.15 (1H, m), 2.73 (2H, q, J = 7.6Hz), 1.38 (9H, s), 1.27 (3H, t, J=7.6Hz) |

### (Reference Example 6-1)

### Methyl 6-[2-(2-tert-butoxycarbonylamino-4-methoxyphenyl)-3-oxo-3-phenylpropyl]pyridine-2-carboxylate

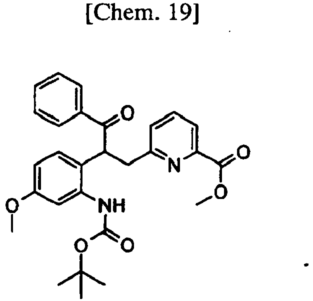

Under ice-cooling, to a solution of *tert*-butyl [5-methoxy-2-(2-oxo-2-phenylethyl)phenyl]carbamate (2.39 g) in *N*,*N*-dimethylformamide (50 mL) were added sodium hydride (content of about 55%, 321 mg) under an argon gas atmosphere. The suspension was stirred at room temperature for 30 minutes under an argon gas atmosphere. At room temperature, a solution of methyl 6-chloromethylpyridine-2-carboxylic acid (1.37 g) in *N*,*N*-dimethylformamide (15 mL) was added thereto over 5 minutes under an argon gas atmosphere, and the mixture was stirred at room temperature for 3 hours under an argon gas atmosphere. To the reaction mixture was added water, followed by addition of ethyl acetate, and the organic layer was separated. The aqueous layer was extracted with ethyl acetate and the organic layer was combined. The combined organic layer was washed with water and saturated brine successively, then dried over anhydrous magnesium sulfate, and concentrated under reduced pressure. The obtained crude product was purified by aminopropylated silica gel column chromatography (eluting solvent: hexane-ethyl acetate) to obtain the title compound (3.27.g).

MS (ESI, m/z): 491(M+H)+

In the same manner as in Reference Example 6-1 using the corresponding ketones, Reference Examples 6-2 to 6-6 below were obtained. The structural formulae and the physical property values thereof were shown in Table 8.

**[Table 8]**

| Ref. No. | Strc | Physical data |
|---|---|---|
| 6-2 | 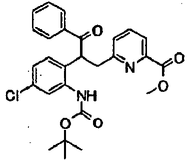 | MS(ESI, m/z) : 495 (M+H)+ |
| 6-3 | 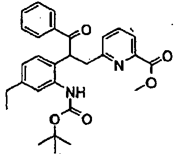 | MS(ESI, m/z) : 489 (M+H)+ |
| | | MS(ESI, m/z) : 487 (M-H)- |
| 6-4 | 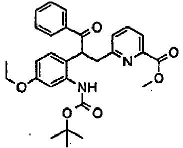 | MS(ESI, m/z) : 505 (M+H)+ |
| 6-5 | 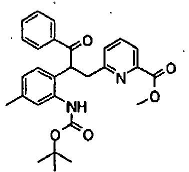 | 1H-NMR (CDCl3) *δ* ppm:8.05-7.15 (10H, m), 7.10-7.00 (1H, m), 6.80-6.75 (1H, m), 5.59 (1H, dd, J=10.0, 5.1Hz), 4.03 (3H, s), 3.72 (1H, dd, J=15.8, 5.1 Hz), 3.58 (1H, dd, J=15.8, 10.1 Hz), 2.19 (3H, s), 1.61 (9H, s) |
| 6-6 | 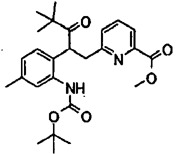 | 1H-NMR (CDCl3) *δ* ppm:8.20-6.75 (7H, m), 5.20-5.10 (1H, m), 4.05 (3H, s), 3.50-3.35 (2H, m), 2.23 (3H, s), 1.54 (9H, s), 1.03 (9H, s) |

### (Reference Example 7)

### 2,2,2-Trifluoro-N-(5-methoxy-2-trimethylsilanylethynylphenyl)acetamide

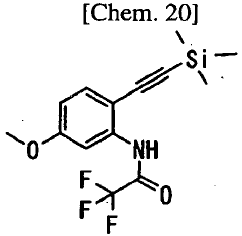

At room temperature, to a solution of 2,2,2-trifluoro-*N*-(2-iodo-5-methoxyphenyl)acetamide (500 mg), ethynyltrimethylsilane (157 mg), and triethylamine (0.403 mL) in acetonitrile (8.0 mL) were added bis(triphenylphosphine)palladium (II) dichloride (25 mg) and copper iodide (14 mg), and the mixture was stirred for 4 hours under an argon gas atmosphere. To the reaction mixture was added saturated brine, followed by extraction with ethyl acetate. The organic layer was dried over anhydrous magnesium sulfate and then concentrated under reduced pressure. The obtained crude product was purified by silica gel column chromatography (eluting solvent: hexane-ethyl acetate) to obtain the title compound (361 mg).

1H-NMR (CDCl3) δ ppm: 9.00-8.80 (1H, br), 8.00 (1H, d, J=2.5Hz), 7.39 (1H, d, J=8.7Hz), 6.70 (1H, dd, J=8.7, 2.5Hz), 3.85 (3H, s), 0.28 (9H, s)

### (Reference Example 8)

### N-(2-ethynyl-5-methoxyphenyl)-2,2,2-trifluoroacetamide

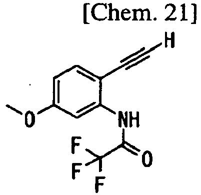

At room temperature, to a solution of 2,2,2-trifluoro-*N*-(5-methoxy-2-trimethylsilanylethynylphenyl)acetamide (360 mg) in tetrahydrofuran (2.0 mL) was added tetrabutylammonium fluoride (1.0 mol/L tetrahydrofuran solution, 1.37 mL), and the mixture was stirred for 30 minutes. The reaction mixture was concentrated under reduced pressure. The obtained crude product was purified by silica gel column chromatography (eluting solvent: hexane-ethyl acetate) to obtain the title compound (210 mg).

1H-NMR (CDCl3) δ ppm: 8.90-8.65 (1H, br), 8.01 (1H, d, J=2.5Hz), 7.43 (1H, d, J=8.7Hz), 6.73 (1H, dd, J=8.7, 2.5Hz), 3.85 (3H, s), 3.53 (1H, s)

### (Reference Example 9-1)

### 6-Methoxy-2-thiazol-4-yl-1H-indole

To a solution of *N*-(2-ethynyl-5-methoxyphenyl)-2,2,2-trifluoroacetamide (209 mg), 4-bromothiazole (0.115 mL) and triethylamine (0.298 mL) in acetonitrile (5.0 mL) were added bis(triphenylphosphine)palladium (II) dichloride (18 mg) and copper iodide (10 mg), and the mixture was stirred at 120°C for 10 minutes under microwave irradiation. After cooling to room temperature, to the mixture was added potassium carbonate (297 mg), and the mixture was stirred at 120°C for 10 minutes under microwave irradiation. To the reaction mixture was added saturated brine, followed by extraction with ethyl acetate. The organic layer was dried over anhydrous magnesium sulfate and then concentrated under reduced pressure. The obtained crude product was purified by silica gel column chromatography (eluting solvent: hexane-ethyl acetate) to obtain the title compound (60.0 mg).

Further, the structural formula and the spectral data of the title compound were shown in Table 9.

In the same manner as in Reference Example 9-1 using the corresponding starting material, Reference Example 9-2 below was obtained. The structural formula and the spectral data thereof were shown in Table 9.

**[Table 9]**

| Ref. No. | Strc | Physical data |
|---|---|---|
| 9-1 | 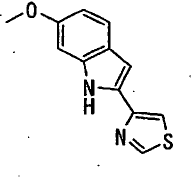 | 1H-NMR (CDCl3) *δ* ppm:9.05-8.85 (1H, br), 8.83 (1H, d, J=2.0Hz), 7.55-7.45 (2H, m), 6.89 (1H, d, J=2.0Hz), 6.85-6.75 (2H, m), 3.87 (3H, s) |
| | | MS(ESI, m/z) = 231 (M+H)+ |
| 9-2 | 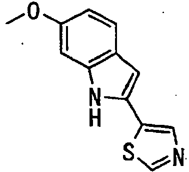 | 1H-NMR (CDCl3) *δ* ppm:8.72 (1H, s), 8.35-8.15 (1H, br), 8.00 (1H, s), 7.48 (1H, d, J=8.7Hz), 6.95-6.85 (1H, m), 6.81 (1H, dd, J=8.7, 2.2Hz), 6.75-6.65 (1H, m), 3.86 (3H, s) |
| | | MS(ESI, m/z) = 231 (M+H)+ |

### (Reference Example 10-1)

### Ethyl 6-[(6-chloro-2-pyridin-3-yl-1H-indol-3-yl) hydroxymethyl]pyridine-2-carboxylate

At room temperature, to a suspension of 6-chloro-2-pyridin-3-yl-1*H*-indole (62.6 mg) and ethyl 6-formylpyridine-2-carboxylate (49.2 mg) in methylene chloride (2.7 mL) was added 1,8-diazabicycloundec[5.4.0]-7-ene (0.004 mL), and the mixture was stirred overnight. The reaction mixture was purified by aminopropylated silica gel column chromatography (eluting solvent: hexane-ethyl acetate-methanol) to obtain the title compound (84.1 mg).

Further, the structural formula and the spectral data of the title compound were shown in Table 10.

In the same manner as in Reference Example 10-1 using the corresponding starting material, Reference Examples 10-2 to 10-3 below were obtained. The structural formulae and the spectral data thereof were shown in Table 10.

**[Table 10]**

| Ref. No. | Strc | Physical Data |
|---|---|---|
| 10-1 | 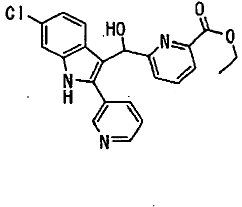 | 1H-NMR (CDCl3) *δ* ppm:9.00-8.90 (1H, m), 8.70-8.60 (1H, m), 8.47 (1H, s), 8.20-8.00 (2H, m), 7.80-7.65 (1H, m), 7.45-7.30 (3H, m), 7.09 (1H, d, J=8.5Hz), 6.93 (1H, dd, J=8.5, 1.8Hz), 6.08 (1H, s), 4.50 (2H, q, J=7.2Hz), 1.47 (3H, t, J=7.2Hz) |
| 10-2 | 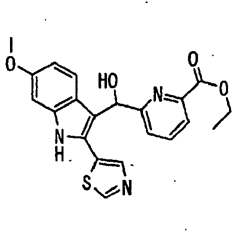 | 1H-NMR (CDCl3) *δ* ppm:8.86 (1H, s), 8.24 (1H, s), 8.15 (1H, s), 8.10-8.00 (1H, m), 7.80-7.65 (1H, m), 7.40-7.30 (1H, m), 7.13 (1H, d, J=8.8Hz), 6.83 (1H, d, J=2.2Hz), 6.64 (1H, dd, J=8.8, 2.2Hz), 6.22 (1H, s), 4.50 (2H, q, J=7.2Hz), 3.80 (3H, s), 1.48 (3H, t, J=7.2Hz) |
| 10-3 | 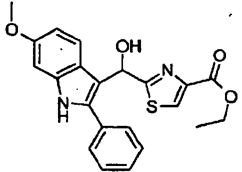 | 1H-NMR (CDCl3) *δ* ppm:8.18 (1H, br s), 8.13 (1H, s), 7.70-7.60 (2H, m), 7.55-7.35 (4H, m), 6.86 (1H, d, J=2.3Hz), 6.72 (1H, dd, J=8.7, 2.3Hz), 6.34 (1H, d, J=2.9Hz), 4.40 (2H, q, J=7.2Hz), 3.83 (3H, s), 3.41 (1H, d, J=2.9Hz), 1.39 (3H, t, J=7.2Hz) |

### (Reference Example 11-1)

### 5-Methylthiophene-3-carboxylic acid methoxymethylamide

At room temperature, to a solution of 5-methylthiophene-3-carboxylic acid (0.500 g) in tetrahydrofuran (10 mL) were added 2-chloro-4, 6-dimethoxy-1, 3,5-triazine (0.738 g) and *N*-methylmorpholine (1.20 mL). The mixture was stirred for 1 hour, and then *N*,*O-*dimethylhydroxylamine hydrochloride (0.376 g) was added thereto. The mixture was stirred for 23 hours. To the reaction mixture were added water and diethyl ether, and the organic layer was separated. The aqueous layer was extracted with diethyl ether, and the organic layer was combined. The combined organic layer was washed with saturated brine, then dried over anhydrous sodium sulfate, and evaporated under reduced pressure. The obtained crude product was purified by aminopropylated silica gel column chromatography (eluting solvent: hexane-ethyl acetate) to obtain the title compound (0.626 g).

Further, the structural formula and the spectral data of the title compound were shown in Table 11.

In the same manner as in Reference Example 11-1 using the corresponding starting material, Reference Example 11-2 below was obtained. The structural formula and the spectral data thereof were shown in Table 11.

**[Table 11]**

| Ref. No. | Strc | Physical data |
|---|---|---|
| 11-1 | 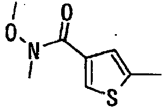 | 1H-NMR (CDCl3) δ ppm:7.81 (1H, d, J=1.4Hz), 7.25-7.15 (1H, m), 3.66 (3H, s), 3.34 (3H, s), 2.48 (3H, d, J=1.1Hz) |
| 11-2 | 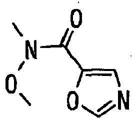 | 1H-NMR (CDCl3) δ ppm:8.22 (1H, d, J=0.8Hz), 7.93 (1H, d, J=0.8Hz), 3.78 (3H, s), 3.40 (3H, s) |

### (Reference Example 12)

### 5-Methylfuran-2-carboxylic acid methoxymethylamide

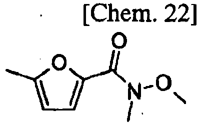

Under ice-cooling, to a suspension of 5-methylfuran-2-carbonyl chloride (0.573 g) and *N*,*O*-dimethylhydroxylamine hydrochloride (0.426 g) in methylene chloride (15 mL) was added pyridine (0.670 mL), and the solution was stirred at room temperature overnight. To the reaction mixture was added water, followed by extraction with ethyl acetate. The organic layer was washed with saturated brine and then dried over anhydrous magnesium sulfate. The solvent was evaporated under reduced pressure to obtain the title compound (0.670 g).

1H-NMR (CDCl3) δ ppm: 7.05 (1H, d, J=3.4Hz), 6.15-6.05 (1H, m), 3.75 (3H, s), 3.34 (3H, s), 2.39 (3H, s)

### (Reference Example 13-1)

### tert-Butyl [5-methoxy-2-(2-oxo-2-thiophen-3-yl-ethyl)phenyl]carbamate

Under an argon gas atmosphere, to a solution of *tert*-butyl (5-methoxy-2-methylphenyl)carbamate (0.596 g) in tetrahydrofuran (6.0 mL) was added dropwise a *sec-*butyllithium solution (1.07 mol/L *n*-hexane-cyclohexane solution, 4.7 mL) at -43°C over 5 minutes. At the same temperature, the mixture was stirred for 30 minutes, and to the mixture was added dropwise a solution of 5-methylthiophene-3-carboxylic acid methoxymethylamide (0.430 g) in tetrahydrofuran (4.0 mL). The mixture was stirred at-45°C for 30 minutes and at room temperature for 1 hour. Under ice-cooling, to the reaction mixture was added 1 mol/L hydrochloric acid, followed by extraction with diethyl ether. The aqueous layer was extracted with diethyl ether, and the organic layer was combined. The combined organic layer was washed with saturated brine, then dried over anhydrous sodium sulfate, and concentrated under reduced pressure. The obtained crude product was purified by aminopropylated silica gel column chromatography (eluting solvent: hexane-ethyl acetate) to obtain the title compound (0.424 g).

Further, the structural formula and the spectral data of the title compound were shown in Table 12.

In the same manner as in Reference Example 13-1 using the corresponding starting materials, Reference Examples 13-2 to 13-23 below were obtained. The structural formulae and the spectral data thereof were shown in Tables 12 to 16.

**[Table 12]**

| Ref. No. | Strc | Physical data |
|---|---|---|
| 13-1 | 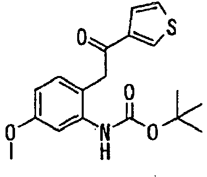 | 1H-NMR (CDCl3) *δ* ppm:8.22 (1H, dd, J=2.7, 1.1Hz), 8.00-7.80 (1H, br), 7.65-7.40 (2H, m), 7.35 (1H, dd, J=5.2, 2.7Hz), 7.08 (1H, d, J=8.5Hz), 6.59 (1H, dd, J=8.5, 2.7Hz), 4.08 (2H, s), 3.79 (3H, s), 1.53 (9H, s) |
| 13-2 | 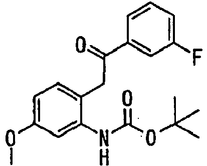 | 1H-NMR (CDCl3) δ ppm:7.95-7.20 (6H, m), 7.08 (1H, d, J=8.5Hz), 6.61 (1H, dd, J=8.5, 2.7Hz), 4.17 (2H, s), 3.79 (3H, s), 1.52 (9H, s) |
| 13-3 | 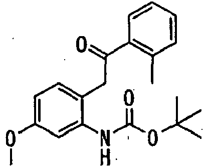 | 1H-NMR (CDCl3) *δ* ppm:7.80-7.70 (1H, m), 7.65-7.20 (5H, m), 6.99 (1H, d, J=8.5Hz), 6.58 (1H, dd, J=8.5, 2.7Hz), 4.13 (2H, s), 3.80 (3H, s), 2.42 (3H, s), 1.52 (9H, s) |
| 13-4 | 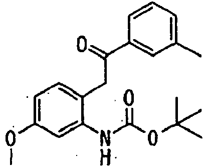 | 1H-NMR (CDCl3) *δ* ppm:7.95-7.70 (3H, m), 7.55-7.30 (3H, m), 7.08 (1H, d, J=8.5Hz), 6.59 (1H, dd, J=8.5, 2.7Hz), 4.18 (2H, s), 3.79 (3H, s), 2.42 (3H, s), 1.53 (9H, s) |
| 13-5 | 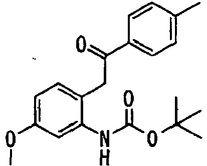 | 1H-NMR (CDCl3) *δ* ppm:8.05-7.80 (3H, m), 7.55-7.40 (1H, m), 7.35-7.20 (2H, m), 7.08 (1H, d, J=8.5Hz), 6.58 (1H, dd, J=8.5, 2.7Hz), 4.16 (2H, s), 3.79 (3H, s), 2.42 (3H, s), 1.53. (9H, s) |

**[Table 13]**

| Ref. No. | Strc | Physical data |
|---|---|---|
| 13-6 | 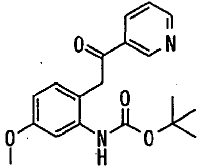 | 1H-NMR (CDCl3) δ ppm:9.35-9.25 (1H, m), 8.85-8.75 (1H, m), 8.35-8.25 (1H, m), 7.60-7.35 (3H, m), 7.10 (1H, d, J=8.5Hz), 6.62 (1H, dd, J=8.5, 2.7Hz), 4.21 (2H, s), 3.79 (3H, s), 1.52 (9H, s) |
| 13-7 | 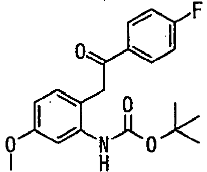 | 1H-NMR (CDCl3) δ ppm:8.15-8.00 (2H, m), 7.80-7.60 (1H, m), 7.55-7.40 (1H, m), 7.20-7.10 (2H, m), 7.07 (1H, d, J=8.5Hz), 6.60 (1H, dd, J=8.5, 2.7Hz), 4.16 (2H, s), 3.79 (3H, s), 1.52 (9H, s) |
| 13-8 | 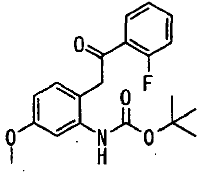 | 1H-NMR (CDCl3) δ ppm:7.90-7.75 (1H, m), 7.65-7.50 (2H, m), 7.43 (1H, br s), 7.30-7.15 (2H, m), 7.09 (1H, d, J=8.5Hz), 6.59 (1H, dd, J=8.5, 2.7Hz), 4.25-4.20 (2H, m), 3.79 (3H, s), 1.52 (9H, s) |
| 13-9 | 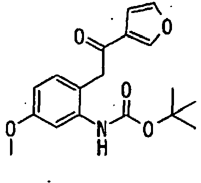 | 1H-NMR(CDCl3) δ ppm:8.20-8.10 (1H, m), 8.10-7.90 (1H, br), 7.55-7.40 (2H, m), 7.05 (1H, d, J=8.5Hz), 6.85-6.75 (1H, m), 6.59 (1H, dd, J=8.5, 2.7Hz), 3.93 (2H, s), 3.79 (3H, s), 1.53 (9H, s) |
| 13-10 | 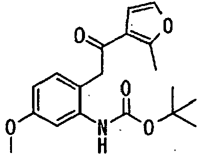 | 1H-NMR (CDCl3) δ ppm:8.05-7.40 (2H, m), 7.28 (1H, d, J=2.1Hz), 7.04 (1H, d, J=8.5Hz), 6.76 (1H, d, J=2.1Hz), 6.59 (1H, dd, J=8.5, 2.7Hz), 3.92 (2H, s), 3.79 (3H, s), 2.59 (3H, s), 1.53 (9H, s) |

**[Table 14]**

| Ref. No. | Strc | Physical data |
|---|---|---|
| 13-11 | 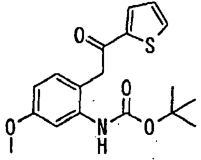 | 1H-NMR (CDCl3) *δ* ppm:8.20-8.00 (1H, br), 7.90 (1H, dd, J=3.9, 1.1Hz), 7.71 (1H, dd, J=5.0, 1.1Hz), 7.55-7.45 (1H, m), 7.18 (1H, dd, J=5.0, 3.9Hz), 7.11 (1H, d, J=8.5Hz), 6.59 (1H, dd, J=8.5, 2.7Hz), 4.09 (2H, s), 3.79 (3H, s), 1.53 (9H, s) |
| 13-12 | 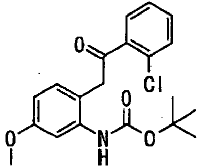 | 1H-NMR (CDCl3) *δ* ppm:7.60-7.20 (6H, m), 6.97 (1H, d, J=8.5Hz), 6.58 (1H, dd, J=8.5, 2.7Hz), 4.18 (2H, s), 3.80 (3H, s), 1.52 (9H, s) |
| 13-13 | 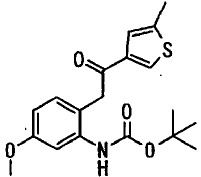 | 1H-NMR (CDCl3) *δ* ppm:8.10-7.90 (2H, m), 7.55-7.45 (1H, m), 7.30-7.20 (1H, m), 7.07 (1H, d, J=8.5Hz), 6.58 (1H, dd, J=8.5, 2.7Hz), 4.02 (2H, s), 3.79 (3H, s), 2.48 (3H, d, J=1.1Hz), 1.53 (9H, s) |
| 13-14 | 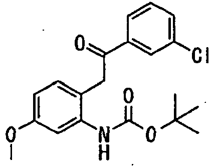 | 1H-NMR (CDCl3) *δ* ppm:8.10-7.95 (2H, m), 7.70-7.35 (4H, m), 7.08 (1H, d, J=8.5Hz), 6.61 (1H, dd, J=8.5, 2.7Hz), 4.17 (2H, s), 3.79 (3H, s), 1.52 (9H, s) |
| 13-15 | 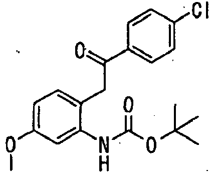 | 1H-NMR (CDCl3) *δ* ppm:8.05-7.95 (2H, m), 7.75-7.55 (1H, br), 7.50-7.40 (3H, m), 7.07 (1H, d, J=8.5Hz), 6.60 (1H, dd, J=8.5. 2.7Hz), 4.16 (2H, s), 3.79 (3H, s), 1.52 (9H, s) |

**[Table 15]**

| Ref. No. | Strc | Physical data |
|---|---|---|
| 13-16 | 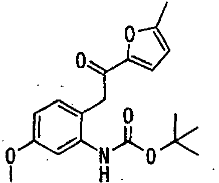 | 1 H-NMR (CDCl3) *δ* ppm:8.40-8.10 (1H, br), 7..60-7.20 (2H, m), 7.11 (1H, d, J=8.5Hz), 6.57 (1H, dd, J=8.5, 2.7Hz), 6.25-6.15 (1H, m), 3.96 (2H, s), 3.79 (3H, s), 2.42 (3H, s), 1.53 (9H, s) |
| 13-17 | 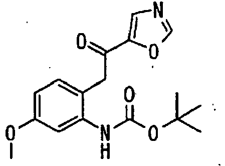 | 1H-NMR (CDCl3) *δ* ppm:8.06 (1H, s), 7.89 (1H, s), 7.65-7.40 (2H, m), 7.12 (1H, d, J=8.5Hz), 6.61 (1H, dd, J=8.5, 2.7Hz), 4.04 (2H, s), 3.80 (3H, s), 1.53 (9H, s) |
| 13-18 | 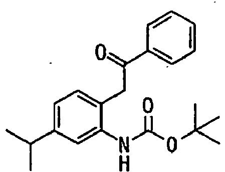 | 1H-NMR (CDCl3) *δ* ppm:8.15-8.00 (2H, m), 7.80-7.40 (5H, m), 7.12 (1H, d, J=8.0Hz), 6.91 (1H, dd, J=8.0, 1.7Hz), 4.24 (2H, s), 3.00-2.80 (1 H, m), 1.52 (9H, s), 1.23 (6H, d, J=6.9Hz) |
| 13-19 | 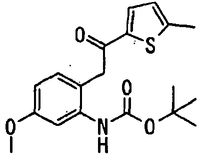 | 1H-NMR (CDCl3) *δ* ppm:8.26 (1H, s), 7.80-7.45 (2H, m), 7.09 (1H, d, J=8.5Hz), 6.90-6.80 (1H, m), 6.57 (1H, dd, J=8.5, 2.7Hz), 4.02 (2H, s), 3.79 (3H, s), 2.55 (3H, s), 1.53 (9H, s) |
| 13-20 | 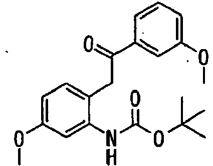 | 1H-NMR (CDCl3) *δ* ppm:7.95-7.75 (1H, br), 7.70-7.65 (1H, m), 7.60-7.35 (3H, m), 7.20-7.10 (1H, m), 7.09 (1H, d, J=8.5Hz), 6.59 (1H, dd, J=8.5, 2.7Hz), 4.17 (2H, s), 3.85 (3H, s), 3.79 (3H, s), 1.53 (9H, s) |

**[Table 16]**

| Ref. No. | Strc | Physical data |
|---|---|---|
| 13-21 | 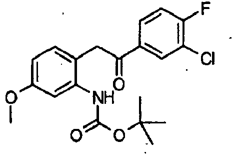 | 1H-NMR (CDCl3) *δ* ppm:8.25-7.80 (2H, m), 7.75-6.90 (4H, m), 6.61 (1H, dd, J=8.5, 2.7Hz), 4.15 (2H, s), 3.80 (3H, s), 1.52 (9H, s) |
| 13-22 | 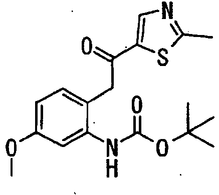 | 1H-NMR (CDCl3) *δ* ppm:8.36 (1H, s), 8.00-7.40 (2H, m), 7.10 (1H, d, J=8.5Hz), 6.60 (1H, dd, J=8.5, 2.7Hz), 4.06 (2H, s), 3.79 (3H, s), 2.76 (3H, s), 1.53 (9H, s) |
| 13-23 | 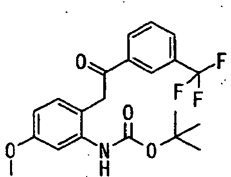 | 1H-NMR (CDCl3) *δ* ppm:8.35-8.15 (2H, m), 7.90-7.80 (1H, m), 7.70-7.35 (3H, m), 7.09 (1H, d, J=8.5Hz), 6.62 (1H, dd, J=8.5, 2.7Hz), 4.22 (2H, s), 3.80 (3H, s), 1.52 (9H, s) |

### (Reference Example 14-1)

### Ethyl 6-[2-(2-tert-butoxycarbonylamino-4-methoxyphenyl)-3-oxo-3-thiophen-3-ylpropyl]pyridine-2-carboxylate

Under ice-cooling, to a solution of *tert*-butyl [5-methoxy-2-(2-oxo-2-thiophen-3-yl-ethyl)phenyl]carbamate (0.387 g) in *N*,*N*-dimethylformamide (5.5 mL) was added sodium hydride (content 55%, 49.0 mg) under an argon gas atmosphere, and the mixture was stirred for 30 minutes under the same condition. Under ice-cooling, to the mixture was added ethyl 6-chloromethylpyridine (0.222 g), and the mixture was stirred for 20 minutes under the same condition and then at room temperature for 6 hours. The reaction mixture was poured into a cold saturated aqueous ammonium chloride solution, followed by extraction with ethyl acetate. The aqueous layer was extracted with ethyl acetate, and the organic layer was combined. The combined organic layer was washed with saturated brine, then dried over anhydrous sodium sulfate, and concentrated under reduced pressure. The obtained crude product was purified by silica gel column chromatography (eluting solvent: hexane-ethyl acetate) to obtain the title compound (0.425 g).

Further, the structural formula and the spectral data of the title compound were shown in Table 17.

In the same manner as in Reference Example 14-1 using the corresponding starting materials, Reference Examples 14-2 to 14-24 below were obtained. The structural formulae and the spectral data thereof were shown in Tables 17 to 22.

**[Table 17]**

| Ref. No. | Strc | Physical data |
|---|---|---|
| 14-1 | 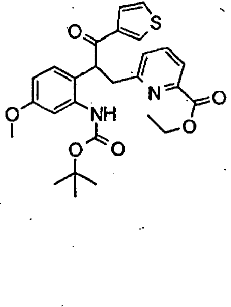 | 1H-NMR (CDCl3) δ ppm:8.19 (1H, s), 8.10 (1H, dd, J=2.9, 1.1Hz), 7.95-7.85 (1H, m), 7.70-7.55 (1H, m), 7.49 (1H, dd, J=5.1, 1.1Hz), 7.25-7.20 (1H, m), 7.18 (1H, dd, J=5:1, 2.9Hz), 7.15-7.05 (2H, m), 6.55 (1H, dd, J=8.7, 2.7Hz, 1H), 5.41 (1H, dd, J=9.6, 5.5Hz), 4.60-4.40 (2H, m), 3.80-3.60 (4H, m), 3.49 (dd, J=15.8, 9.6Hz, 1H), 1.58 (9H, s), 1.44 (3H, t, J=7.2Hz) MS(ESI, m/z) = 511 (M+H)+ |
| 14-2 | 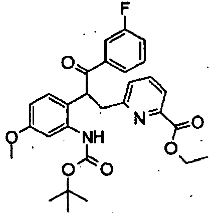 | MS(ESI, m/z) = 523 (M+H)+ |
| 14-3 | 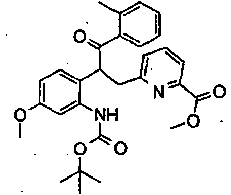 | MS(ESI, m/z) = 505 (M+H)+ |
| 14-4 | 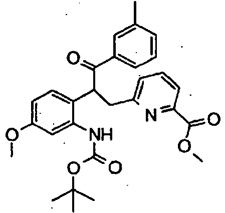 | MS(ESI, m/z) = 505 (M+H)+ |

**[Table 18]**

| Ref. No. | Strc | Physical data |
|---|---|---|
| 14-5 | 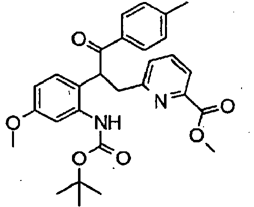 | MS(ESI, m/z) = 505 (M+H)+ |
| 14-6 | 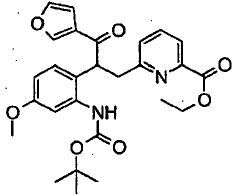 | MS(ESI, m/z) = 495 (M+H)+ |
| 14-7 | 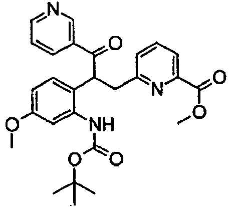 | MS(ESI, m/z) = 492 (M+H)+ |
| 14-8 | 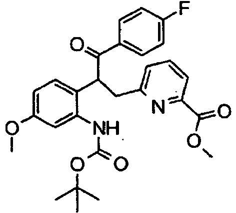 | 1H-NMR (CDCl3) δ ppm:8.25-6.80 (10H, m), 6.53 (1H, dd, J=8.7, 2.7Hz), 5.51 (1H, dd, J=10.1, 5.3Hz), 4.03 (3H, s), 3.75-3.60 (4H, m), 3.54 (1H, dd, J=15.9, 10.1Hz), 1.65-1.55 (9H, m) MS(ESI, m/z) = 509 (M+H)+ |

**[Table 19]**

| Ref. No. | Strc | Physical data |
|---|---|---|
| 14-9 | 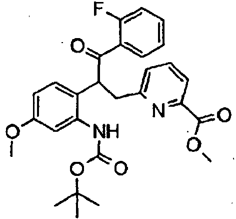 | MS(ESI, m/z) = 509 (M+H)+ |
| 14-10 | 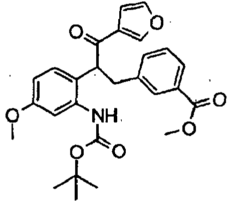 | MS(ESI, m/z) = 478 (M-H)- |
| 14-11 | 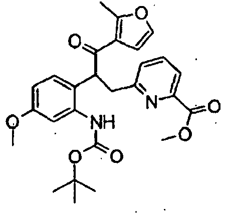 | MS(ESI, m/z) = 495 (M+H)+ |
| 14-12 | 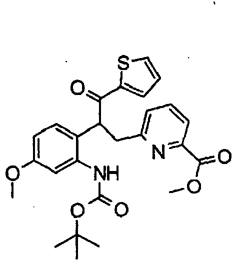 | 1H-NMR (CDCl3) *δ* ppm:8.09 (1H, s), 7.95-7.85 (1H, m), 7.72 (1H, dd, J=3.9, 1.1 Hz), 7.70-7.60 (1H, m), 7.54 (1H, dd, J=4.9, 1.1Hz), 7.25-7.05 (3H, m), 7.00 (1H, dd, J=4.9, 3.9Hz), 6.55 (1H, dd, J=8.7, 2.7Hz), 5.43 (1H, dd, J=10.0, 5.4Hz), 4.03 (3H, s), 3.75-3.65 (4H, m), 3.54 (1H, dd, J=15.8, 10.0Hz), 1.65-1.55 (9H, m) |
| | | MS(ESI, m/z) = 497 (M+H)+ |

**[Table 20]**

| Ref. No. | Strc | Physical data |
|---|---|---|
| 14 - 13 | 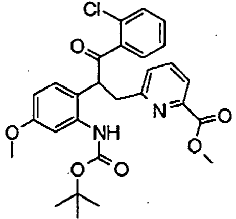 | MS(ESI, m/z) = 525 (M+H)+ |
| 14-14 | 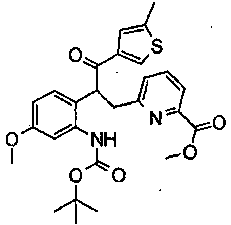 | 1H-NMR (CDCl3) *δ* ppm:8.08 (1H, s), 7.95-7.80 (2H, m), 7.70-7.55 (1H, m), 7.25-7.00 (4H, m), 6.54 (1H, dd, J=8.8, 2.7Hz), 5.34 (1H, dd, J=10.0, 5.4Hz), 4.04 (3H, s), 3.70 (3H, s), 3.65 (1H, dd, J=15.7, 5.4Hz), 3.50 (1H, dd, J=15.7, 10.0Hz), 2.45-2.35 (3H, m), 1.58 (9H, s) |
| | | MS(ESI, m/z) = 511 (M+H)+ |
| 14-15 | 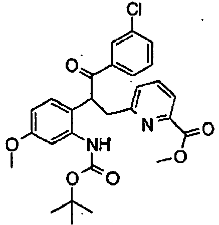 | MS(ESI, m/z) = 525 (M+H)+ |
| 14-16 | 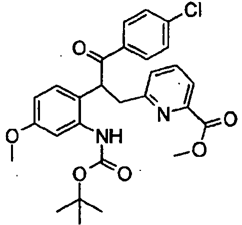 | 1H-NMR (CDCl3) *δ* ppm:8.20-6.90 (10H, m), 6.53 (1H, dd, J=8.7, 2.7Hz), 5.51 (1H, dd, J=9.9, 5.3Hz), 4.10 (3H, s), 3.75-3.65 (4H, m), 3.50 (1 H, dd, J=15.7, 9.9Hz), 1.59 (9H, s) |
| | | MS(ESI, m/z) = 525 (M+H)+ |

**[Table 21]**

| Ref. No. | Strc | Physical data |
|---|---|---|
| 14-17 | 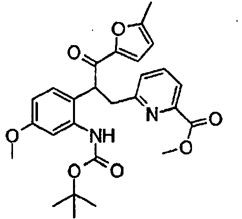 | 1H-NMR (CDCl3) *δ* ppm:8.11 (1H, s), 7.95-7.85 (1H, m), 7.70-7.55 (1H, m), 7.25-7.10 (4H, m), 6.55 (1H, dd, J=8.7, 2.8Hz), 6.15-6.00 (1H, m), 5.24 (1H, dd, J=9.9, 5.8Hz), 4.02 (3H, s), 3.75-3.45 (5H, m), 2.33 (3H, s), 1.57 (9H, s) |
| | | MS(ESI, m/z) = 496 (M+H)+ |
| 14-18 | 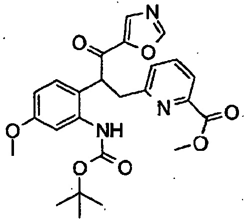 | 1H-NMR (CDCl3) *δ* ppm:8.10-7.85 (3H, m), 7.76 (1H, s), 7.70-7:60 (1H, m), 7.30-7.20 (1H, m), 7.15-7.00 (2H, m), 6.58 (1H, dd, J=8.7, 2.8Hz), 5.38 (1H, dd, J=10.2, 5.2Hz), 4.03 (3H, s), 3.85-3.65 (4H, m), 3.54 (1H, dd, J=16.4, 10.2Hz), 1.59 (9H, s) |
| | | MS(ESI, m/z) = 482 (M+H)+ |
| 14-19 | 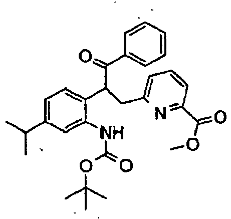 | 1H-NMR (CDCl3) *δ* ppm:8.00-7.85 (4H, m), 7.70-7.55 (1H, m), 7.50-7.20 (5H, m), 7.09 (1H, d, J=8.1Hz), 6.84 (1H, dd, J=8.1, 1.8Hz), 5.60 (1H, dd, J=9.6, 5.5Hz), 4.02 (3H, s), 3.73 (1H, dd, J=15_{.}8_{,} 5.5Hz), 3.55 (1H, dd, J=15.8, 9.6Hz), 2.85-2.70 (1H, m), 1.59 (9H, s), 1.13 (6H, d, J=6.9Hz) |
| | | MS(ESI, m/z) = 503 (M+H)+ |
| 14-20 | 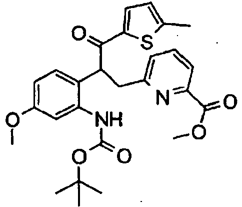 | 1H-NMR (CDCl3) *δ* ppm:8.09 (1H, s), 7.95-7.85 (1H, m), 7.70-7.50 (2H, m), 7.25-7.15 (1H, m), 7.13 (1H, d, J=8.7Hz), 7.10-7.00 (1H, m), 6.75-6.60 (1H, m), 6.54 (1H, dd, J=8.7, 2.2Hz), 5.37 (1H, dd, J=10.3, 5.3Hz), 4.05 (3H, s), 3.70 (3H, s), 3.66 (1H, dd, J=15.9, 5.3Hz), 3.56 (1H, dd, J=15.9, 10.3Hz), 2.45 (3H, s), 1.59 (9H, s) |
| | | MS(ESI, m/z) = 511 (M+H)+ |

**[Table 22]**

| Ref. No. | Strc | Physical data |
|---|---|---|
| 14-21 | 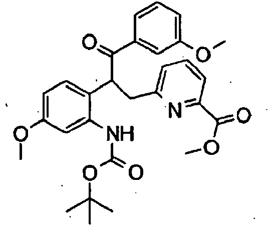 | MS(ESI, m/z) = 519 (M+H)+ |
| 14-22 | 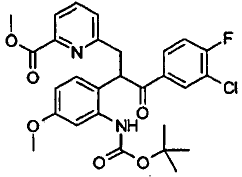 | MS(ESI, m/z) = 543 (M+H)+ |
| 14-23 | 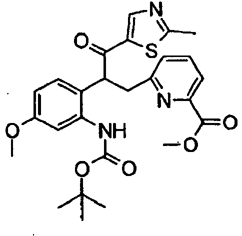 | 1H-NMR (CDCl3) *δ* ppm:8.21 (1H, s), 8.00-7.85 (2H, m), 7.70-7.60 (1H, m), 7.25-7.05 (3H, m), 6.56 (1H, dd, J=8.7, 2.7Hz), 5.32 (1H, dd, J=10.0, 5.4Hz), 4.03 (3H, s), 3.80-3.65 (4H, m), 3.51 (1H, dd, J=15.7, 10.0Hz). 2.67 (3H, s) |
| | | MS(ESI, m/z) = 512 (M+H)+ |
| 14-24 | 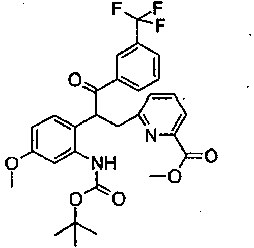 | MS(ESI, m/z) = 559 (M+H)+ |

### (Reference Example 15)

### Ethyl 3-(6-methoxy-2-pyridin-3-yl-1H-indole-3-carbonyl)benzoate

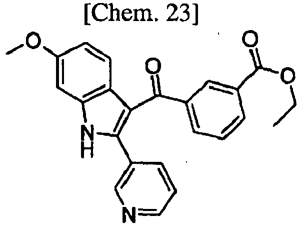

At room temperature, to a solution of 2,2,2-trifluoro-*N*-(2-iodo-5-methoxyphenyl)acetamide (200 mg), 3-ethynylpyridine (65.2 mg) and triethylamine (0.161 mL) in acetonitrile (8.0 mL) were added bis(triphenylphosphine)palladium (II) dichloride (12 mg) and copper iodide (7 mg), and the mixture was stirred overnight under an argon gas atmosphere. To the reaction mixture were added potassium carbonate (240 mg) and ethyl 3-iodobenzoate (0.106 mL), and the mixture was stirred at 50°C overnight under a carbon monooxide gas atmosphere. After cooling to room temperature, to the reaction mixture was added saturated brine, followed by extraction with ethyl acetate. The organic layer was dried over anhydrous magnesium sulfate and then concentrated under reduced pressure. The obtained crude product was purified by silica gel column chromatography (eluting solvent: hexane-ethyl acetate) to obtain the title compound (128 mg).

1H-NMR (CDCl3) δ ppm: 9.00-6.70 (12H, m), 4.32 (2H, q, J=7.2Hz), 3.89 (3H, s), 1.36 (3H, t, J=7.2Hz)
MS (ESI, m/z) = 401 (M+H)+

### (Reference Example 16)

### (2-Phenyl-1H-indol-6-yl)methanol

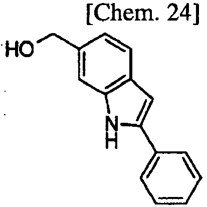

Under ice-cooling, to a suspension of aluminum lithium hydride (content 80%, 96.0 mg) in tetrahydrofuran (5.0 mL) was added a solution of 2-phenyl-1*H*-indole-6-carboxylic acid in tetrahydrofuran (5.0 mL), and the mixture was stirred for 5 minutes under the same condition and then at room temperature for 2 hours. Under ice-cooling, to the reaction mixture was added aluminum lithium hydride (content 80%, 48.0 mg), and the mixture was stirred at room temperature for 2 hours and then at 50°C for 5 hours, and further at room temperature overnight. After cooling to room temperature, the reaction mixture was poured into 1 mol/L cooled hydrochloric acid, followed by extraction with ethyl acetate. The aqueous layer was extracted with ethyl acetate and the organic layer was combined. The combined organic layer was washed with 5% aqueous sodium hydrogen carbonate solution and saturated brine successively, then dried over anhydrous magnesium sulfate, and concentrated under reduced pressure. The obtained crude product was purified by silica gel column chromatography (eluting solvent: hexane-ethyl acetate) to obtain the title compound (121 mg).

1H-NMR (CDCl3) δ ppm: 8.45-8.25 (1H, br), 7.75-7.55 (3H, m), 7.50-7.30 (4H, m), 7.13 (1H, dd, J=8.2, 1.4Hz), 6.85-6.80 (1H, m), 4.80 (2H, s)

### (Reference Example 17)

### 2-Phenyl-1H-indol-6-ylmethyl acetate

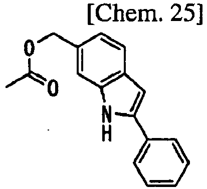

At room temperature, to a solution of (2-phenyl-1*H*-indol-6-yl)methanol (20.0 mg) and pyridine (0.058 mL) in methylene chloride (0.5 mL) was added acetic anhydride (0.034 mL), and the mixture was stirred for 2 hours. To the reaction mixture was added 0.5 mol/L hydrochloric acid, followed by extraction with ethyl acetate. The organic layer was washed with 5% aqueous sodium hydrogen carbonate solution and saturated brine, then dried over anhydrous magnesium sulfate, and concentrated under reduced pressure. The obtained crude product was purified by silica gel column chromatography (eluting solvent: hexane-ethyl acetate) to obtain the title compound (18.7 mg).

1H-NMR (CDCl3) δ ppm: 8.39 (1H, s), 7.75-7.55 (3H, m), 7.55-7.30 (4H, m), 7.13 (1H, dd, J=8.1,1.3Hz), 6.85-6.80 (1H, m), 5.22 (2H, s), 2.11 (3H, s)
MS (ESI, m/z) = 266 (M+H)+

### (Reference Example 18)

### Ethyl 6-(6-acetoxymethyl-2-phenyl-1H-indol-3-ylmethyl)pyridine-2-carboxylate

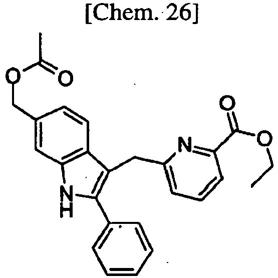

Under ice-cooling, to a suspension of 2-phenyl-1*H*-indol-6-ylmethyl acetate (87.0 mg) and ethyl 6-formylpyridine-2-carboxylate (64.8 mg) in methylene chloride (3.3 mL) were added triethylsilane (0.157 mL) and trifluoroacetic acid (0.038 mL) successively, and the mixture was stirred for 2 hours under the same condition and at room temperature for 4 hours. The reaction mixture was purified by aminopropylated silica gel column chromatography (eluting solvent: hexane-ethyl acetate) to obtain the title compound (66.9 mg).

1H-NMR (CDCl3) δ ppm: 8.24 (1H, s), 8.00-7.85 (1H, m), 7.70-7.55 (3H, m), 7.50-7.30 (5H, m), 7.20-7.10 (1H, m), 7.08 (1H, dd, J=8.2, 1.4Hz), 5.21 (2H, s), 4.57 (2H, s), 4.51 (2H, q, J=7.1Hz), 2.10 (3H, s), 1.47 (3H, t, J=7.1Hz)
MS (ESI, m/z) = 429 (M+H)+

### (Reference Example 19)

### 4-Cyclopropyl-1-iodo-2-nitro benzene

Under ice-cooling, to a suspension of 4-cyclopropyl-2-nitroaniline (1.23 g) in concentrated hydrochloric acid (5.0 mL) was added slowly a solution of sodium nitrite (522 mg) in water (5.0 mL). Under the same condition, the mixture was stirred for 30 minutes. To the mixture was added dropwise a solution of potassium iodide (2.28 g) in water (10 mL). The mixture was stirred at room temperature for 30 minutes and at 60°C for 1.5 hours. After cooling to room temperature, to the reaction mixture was added ethyl acetate, and the organic layer was separated. The layer was washed with 10% aqueous sodium thiosulfate solution and saturated brine successively, dried over anhydrous magnesium sulfate, and then concentrated under reduced pressure. The obtained crude product was purified by silica gel column chromatography (eluting solvent: hexane-ethyl acetate) to obtain the title compound (1.66 g).

Further, the structural formula and the spectral data of the title compound were shown in Table 58.

### (Reference Example 20)

### 5-Cyclopropyl-2-iodoaniline

At room temperature, to a suspension of 4-cyclopropyl-1-iodo-2-nitrobenzene (1.65 g), iron (III) chloride hexahydrate (77.2 mg), and activated carbon (27.4 mg, wet) in methanol (20 mL) was added hydrazine monohydrate (0.83 mL). The mixture was stirred at 70°C overnight. After cooling to room temperature, to the mixture was added hydrazine monohydrate (0.28 mL). The mixture was stirred at 70°C for 2 hours. After cooling to room temperature, the reaction mixture was filtered through Celite (registered trademark) to remove the insoluble materials. The filtrate was concentrated under reduced pressure. To the residue was added water, followed by extraction with ethyl acetate. The organic layer was washed with saturated brine, dried over anhydrous magnesium sulfate, and then concentrated under reduced pressure to obtain the title compound (1.41 g).

Further, the structural formula and the spectral data of the title compound were shown in Table 58.

### (Reference Example 21)

### 3-Ethoxy-4-fluoroaniline

Under ice-cooling, to a solution of 2-ethoxy-1-fluoro-4-nitrobenzene (2.25 g) in tetrahydrofuran (25 mL)-ethanol (25 mL) was added 10% palladium-carbon (675 mg, wet), and the suspension was stirred at room temperature overnight under a hydrogen gas atmosphere. The suspension was filtered through Celite (registered trademark) to remove the insoluble materials. The filtrate was concentrated under reduced pressure to obtain the title compound (2.01 g).

Further, the structural formula and the spectral data of the title compound were shown in Table 58.

### (Reference Example 22-1)

### 2-Bromo-4-methyl-5-trifluoromethylaniline

Under ice-cooling, to a solution of 4-methyl-3-trifluoromethylaniline (701 mg) in methylene chloride (13 mL) was added tetra-n-butylammonium tribromide (1.93 g), and the suspension was stirred at room temperature for 1.5 hours. To the reaction mixture was added a saturated aqueous sodium hydrogen carbonate solution, and the organic layer was separated. The layer was concentrated under reduced pressure to obtain the title compound (2.19 g).

Further, the structural formula and the spectral data of the title compound were shown in Table 58.

In the same manner as in Reference Example 22-1 using the corresponding starting materials, Reference Examples 22-2 to 22-4 below were obtained. The structural formulae and the physical property values thereof were shown in Table 58.

### (Reference Example 23)

In the same manner as in Reference Example 1-1 using the corresponding aniline derivatives, Reference Examples 23-1 to 23-18 below were obtained. The structural formulae and the physical property values thereof were shown in Tables 59 to 61.

### (Reference Example 24)

In the same manner as in Reference Example 2-1 using the corresponding trifluoroacetanilide derivatives, Reference Examples 24-1 to 24-49 below were obtained. The structural formulae and the physical property values thereof were shown in Tables 61 to 68.

### (Reference Example 25-1)

### 5-Methyl-2-phenyl-1H-indol-6-ol

Under ice-cooling, to a suspension of 6-methoxy-5-methyl-2-phenyl-1*H*-indole (197 mg) in methylene chloride (4.2 mL) was added dropwise boron tribromide (1 mol/L methylene chloride solution, 3.3 mL). The mixture was stirred for 20 minutes under ice-cooling and then at room temperature for 1 hour. The reaction mixture was poured slowly into ice. To the mixture were added a saturated aqueous sodium hydrogen carbonate solution and ethyl acetate, and the organic layer was separated. The aqueous layer was extracted with ethyl acetate and the organic layer was combined. The layer was washed with saturated brine, dried over anhydrous sodium sulfate, and then concentrated under reduced pressure. The obtained crude product was purified by silica gel column chromatography (eluting solvent: hexane-ethyl acetate) to obtain the title compound (180 mg).

Further, the structural formula and the spectral data of the title compound were shown in Table 69.

In the same manner as in Reference Example 25-1 using the corresponding starting materials, Reference Examples 25-2 to 25-4 below were obtained. The structural formulae and the physical property values thereof were shown in Table 69.

### (Reference Example 26-1)

### 2-tert-Butyl-6-ethoxy-1H-indole

At room temperature, to a suspension of 2-*tert*-butyl-1H-indol-6-ol (133 mg) and potassium carbonate (117 mg) in *N,N*-dimethylformamide (3.0 mL) was added ethyl iodide (0.085 mL), and the suspension was stirred overnight. To the reaction mixture were added water and ethyl acetate, and the organic layer was separated. The aqueous layer was extracted with ethyl acetate, and the organic layer was combined. The layer was washed with water and saturated brine successively, dried over anhydrous magnesium sulfate, and then the solvent was evaporated under reduced pressure. The obtained crude product was purified by silica gel column chromatography (eluting solvent: hexane-ethyl acetate) to obtain the title compound (74.5 mg).

Further, the structural formula and the spectral data of the title compound were shown in Table 69.

In the same manner as in Reference Example 26-1 using the corresponding starting materials, Reference Examples 26-2 to 26-4 below were obtained. The structural formulae and the physical property values thereof were shown in Table 69.

### (Reference Example 27)

### 6-Difluoromethoxy-2-phenyl-1H-indole

At room temperature, to a solution of 2-phenyl-1*H*-indol-6-ol (250 mg) in *N,N-*dimethylformamide (2.0 mL) were added sodium chlorodifluoroacetate (182 mg), sodium hydroxide (47.8 mg), and water (0.024 mL), and the mixture was stirred at 125°C for 6.5 hours. After leaving to be cooled, to the mixture were added sodium chlorodifluoroacetate (364 mg), sodium hydroxide (95.6 mg), and water (0.047 mL), and the mixture was stirred at 125°C overnight. After cooling to room temperature, to reaction mixture were added water and ethyl acetate, and the organic layer was separated and concentrated under reduced pressure. The obtained crude product was purified by silica gel column chromatography (eluting solvent: hexane-ethyl acetate) to obtain the title compound (7.2 mg).

Further, the structural formula and the spectral data of the title compound were shown in Table 70.

### (Reference Example 28)

### 3-(6-Methoxy-1H-indol-2-yl)benzamide

At room temperature, to a suspension of 6-methoxy-1*H*-indole (500 mg), palladium (II) acetate (76.1 mg) and cesium acetate (1.76 g) in *N,N-*dimethylacetamide (1.7 mL) was added 3-iodobenzamide (1.05 g), and the mixture was stirred at 130°C for 20 hours under an argon gas atmosphere. After cooling to room temperature, the reaction mixture was diluted with ethyl acetate. The mixture was filtered through Celite (registered trademark) to remove the insoluble materials. The filtrate was concentrated under reduced pressure. The residue was purified by silica gel column chromatography (eluting solvent: hexane-ethyl acetate-methanol). To the obtained crude product was added a mixed solvent of hexane-diisopropyl ether, and the suspension was stirred at room temperature for 15 minutes. The precipitate was collected by filtration, washed with the same mixed solvent, and then dried under reduced pressure to obtain the title compound (169 mg).

Further, the structural formula and the spectral data of the title compound were shown in Table 70.

### (Reference Example 29)

### 3-(6-Methoxy-1H-indol-2-yl)benzonitrile

Under ice-cooling, to a suspension of 3-(6-methoxy-1*H*-indol-2-yl)benzamide (100 mg) in methylene chloride (1.0 mL)-tetrahydrofuran (1.0 mL) were added trifluoroacetic anhydride (0.068 mL) and triethylamine (0.117 mL) successively under an argon gas atmosphere. The suspension was stirred at room temperature for 5.5 hours. At room temperature, to the suspension was added trifluoroacetic anhydride (0.016 mL), and the mixture was stirred for 1.5 hours. To the reaction mixture were added ethyl acetate and 2 mol/L hydrochloric acid, and the organic layer was separated. The layer was washed with water and saturated brine successively, dried over anhydrous magnesium sulfate, and then concentrated under reduced pressure. The obtained crude product was purified by silica gel column chromatography (eluting solvent: hexane-ethyl acetate-methanol) to obtain the title compound (44.1 mg).

Further, the structural formula and the spectral data of the title compound were shown in Table 70.

### (Reference Example 30-1)

### 2-tert-Butyl-5,6-dimethyl-1H-indole

Under refluxing, each portion of 1-bromo-3,3-dimethyl-2-butanone (0.405 mL) divided into 4 portions was added to a solution of 3,4-dimethylaniline (1.20 g) in ethanol (3.0 mL) every 5 minutes. The mixture was refluxed overnight. After cooling to room temperature, to the mixture was added 2 mol/L hydrochloric acid, followed by addition of water and ethyl acetate, and the organic layer was separated. The layer was washed with water and saturated brine successively, dried over anhydrous magnesium sulfate, and then concentrated under reduced pressure. The obtained crude product was purified by aminopropylated silica gel column chromatography (eluting solvent: hexane-ethyl acetate) to obtain the title compound (38.1 mg).

Further, the structural formula and the spectral data of the title compound were shown in Table 70.

In the same manner as in Reference Example 30-1 using the corresponding starting materials, Reference Examples 30-2 to 30-3 below were obtained. The structural formulae and the physical property values thereof were shown in Table 70.

### (Reference Example 31)

In the same manner as in Reference Example 10-1 using the corresponding starting materials, Reference Examples 31-1 to 31-40 below were obtained. The structural formulae and the physical property values thereof were shown in Tables 71 to 78.

### (Reference Example 32)

In the same manner as in Reference Example 11-1 using the corresponding starting materials, Reference Examples 32-1 to 32-2 below were obtained. The structural formulae and the physical property values thereof were shown in Table 79.

### (Reference Example 33)

In the same manner as in Reference Example 12-1 using the corresponding starting materials, Reference Examples 33-1 to 33-3 below were obtained. The structural formulae and the physical property values thereof were shown in Table 79.

### (Reference Example 34)

### N-methoxy-N-methyl-3-propylbenzamide

At room temperature, to a solution of 3-allyl-*N*-methoxy-*N*-methylbenzamide (340 mg) in tetrahydrofuran (10.5 mL) was added 10% palladium-carbon (55.3 mg, wet), and the suspension was stirred overnight under a hydrogen gas atmosphere. The suspension was filtered through Celite (registered trademark) to remove the insoluble materials. The filtrate was concentrated under reduced pressure. The obtained crude product was purified by silica gel column chromatography (eluting solvent: hexane-ethyl acetate) to obtain the title compound (355 mg).

Further, the structural formula and the spectral data of the title compound were shown in Table 79.

### (Reference Example 35)

### 5-Cyclopropyl-2-methylaniline

At room temperature, to a suspension of 5-bromo-2-methylaniline (372 mg), cyclopropylboronic acid monohydrate (270 mg), tricyclohexylphosphine (56.0 mg), and potassium phosphate (1.49 g) in toluene (8.0 mL)-water (0.4 mL) was added palladium (II) acetate (22.4 mg), and the mixture was stirred under an argon gas atmosphere at 100°C for 6 hours. To the reaction mixture were added water and ethyl acetate, and the organic layer was separated. The aqueous layer was extracted with ethyl acetate and the organic layer was combined. The layer was washed with saturated brine, dried over anhydrous magnesium sulfate, and then concentrated under reduced pressure. The obtained crude product was purified by silica gel column chromatography (eluting solvent: hexane-ethyl acetate) to obtain the title compound (256 mg).

Further, the structural formula and the spectral data of the title compound were shown in Table 79.

### (Reference Example 36)

In the same manner as in Reference Example 3-1 using the corresponding 2-methylaniline derivatives, Reference Examples 36-1 to 36-4 below were obtained. The structural formulae and the physical property values thereof were shown in Table 80.

### (Reference Example 37)

### tert-Butyl [5-cyclopropyl-2-(2-hydroxy-3,3-dimethylbutyl)phenyl]carbamate

Under an argon gas atmosphere, to a solution of *tert*-butyl (5-cyclopropyl-2-methylphenyl)carbamate (495 mg) in tetrahydrofuran (7.0 mL) was added slowly a *sec-*butyllithium solution (1.03 mol/L *n*-hexane-cyclohexane solution, 4.70 mL) at -40°C. The mixture was stirred at -40°C for 15 minutes. To the mixture was added dropwise a solution of trimethylacetaldehyde (purity 90%, 0.32 mL) in tetrahydrofuran (1.0 mL). The mixture was stirred at -40°C for 15 minutes and at room temperature for 1 hour. To the reaction mixture were added water, then 1 mol/L hydrochloric acid and ethyl acetate, and the organic layer was separated. The layer was washed with water and saturated brine successively, dried over anhydrous magnesium sulfate, and then concentrated under reduced pressure. The obtained crude product was purified by silica gel column chromatography (eluting solvent: hexane-ethyl acetate) to obtain the title compound (544 mg).

Further, the structural formula and the spectral data of the title compound were shown in Table 80.

### (Reference Example 38)

### 1-(2-Amino-4-cyclopropylphenyl)-3,3-dimethylbutan-2-ol

At room temperature, to a solution of *tert*-butyl [5-cyclopropyl-2-(2-hydroxy-3,3-dimethylbutyl)phenyl]carbamate (540 mg) in methylene chloride (5.0 mL) was added trifluoroacetic acid (1.0 mL). The mixture was stirred for 2 hours. The reaction mixture was poured into 5% aqueous sodium hydrogen carbonate solution. To the mixture was added ethyl acetate, and the organic layer was separated. The layer was washed with saturated brine, dried over anhydrous magnesium sulfate, and then concentrated under reduced pressure to obtain the title compound (351 mg).

Further, the structural formula and the spectral data of the title compound were shown in Table 80.

### (Reference Example 39)

### 2-tert-Butyl-6-cyclopropyl-1H-indole

At room temperature, to a suspension of 1-(2-amino-4-cyclopropylphenyl)-3,3-dimethylbutan-2-ol (350 mg), 2-bromo-1,3,5-trimethylbenzene (0.269 mL), and potassium carbonate (413 mg) in *N,N*-dimethylformamide (10 mL) was added tetrakistriphenylphosphine palladium (0) (86.6 mg), and the mixture was stirred at 150°C for 5 hours under an argon gas atmosphere. To the reaction mixture were added water and ethyl acetate, and the organic layer was separated. The layer was washed with saturated brine, dried over anhydrous magnesium sulfate, and then concentrated under reduced pressure. The obtained crude product was purified by silica gel column chromatography (eluting solvent: hexane-ethyl acetate) to obtain the title compound (286 mg).

Further, the structural formula and the spectral data of the title compound were shown in Table 80.

### (Reference Example 40)

In the same manner as in Reference Example 4-1 using the corresponding *tert-*butyl (2-methylphenyl)carbamate derivatives, Reference Examples 40-1 to 40-31 below were obtained. The structural formulae and the physical property values thereof were shown in Tables 81 to 85.

### (Reference Example 41)

In the same manner as in Reference Example 5-1 using the corresponding ketones, Reference Examples 41-1 to 41-5 below were obtained. The structural formulae and the physical property values thereof were shown in Table 86.

### (Reference Example 42)

### Ethyl 2-chloro-3-methylbenzoate

Under ice-cooling, to a solution of 2-chloro-3-methylbenzoic acid (147 mg) in ethanol (4.3 mL) was added concentrated sulfuric acid (0.43 mL) under an argon gas atmosphere. The mixture was refluxed for 6 hours. The reaction mixture was concentrated under reduced pressure, to the residue were added ethyl acetate and a saturated aqueous sodium hydrogen carbonate solution, and the organic layer was separated. The aqueous layer was extracted with ethyl acetate and the organic layer was combined. The layer was washed with saturated brine, dried over anhydrous sodium sulfate, and then evaporated under reduced pressure. The obtained crude product was purified by silica gel column chromatography (eluting solvent: hexane-ethyl acetate) to obtain the title compound (182 mg).

Further, the structural formula and the spectral data of the title compound were shown in Table 86.

### (Reference Example 43)

### Ethyl 3-bromomethyl-2-chlorobenzoate

At room temperature, to a solution of ethyl 2-chloro-3-methylbenzoate (166 mg) in tetrachloromethane (4.2 mL) were added *N*-bromosuccinimide (149 mg) and benzoyl peroxide (content 75%, 13.0 mg), and the mixture was refluxed for 3 hours. After cooling to room temperature, the reaction mixture was concentrated under reduced pressure. The obtained crude product was purified by silica gel column chromatography (eluting solvent: hexane-ethyl acetate) to obtain the title compound (153 mg).

Further, the structural formula and the spectral data of the title compound were shown in Table 86.

### (Reference Example 44)

In the same manner as in Reference Example 6-1 using the corresponding ketones, Reference Examples 44-1 to 44-20 below were obtained. The structural formulae and the physical property values thereof were shown in Tables 87 to 90.

### (Reference Example 45)

In the same manner as in Reference Example 6-1 using the corresponding ketones and also using the corresponding bromides as an alkylating agent, Reference Examples 45-1 to 45-9 below were obtained. The structural formulae and the physical property values thereof were shown in Tables 91 to 92.

### (Reference Example 46)

In the same manner as in Reference Example 6-1 using the corresponding ketones, also using the corresponding bromides as an alkylating agent, and further using sodium iodide as an additive, Reference Examples 46-1 to 46-5 below were obtained. The structural formulae and the physical property values thereof were shown in Table 93.

### (Reference Example 47-1)

### 5-Fluoro-2-isopropyl-6-methoxy-1H-indole

In the same manner as in Reference Example 30-1 using the corresponding starting material, the title compound was obtained.

1H-NMR (CDCl3) δ ppm: 7.81 (1H, s), 7.20 (1H, d, J=11.6Hz), 6.89 (1H, d, J=7.1Hz), 6.14 (1H, s), 3.90 (3H, s), 3.15-2.95 (1H, m), 1.34 (6H, d, J=6.7Hz)

### (Reference Example 47-2)

### 2-Isopropyl-6-methoxy-5-methyl-1H-indole

In the same manner as in Reference Example 30-1 using the corresponding starting material, the title compound was obtained.

1H-NMR (CDCl3) δ ppm: 7.73 (1H, s), 7.25 (1H, s), 6.78 (1H, s), 6.09 (1H, s), 3.84 (3H, s), 3.10-2.95 (1H, m), 2.28 (3H, s), 1.33 (6H, d, J=6.9Hz)

### (Example 1-1)

### Methyl 6-(6-methoxy-2-phenyl-1H-indol-3-ylmethyl)pyridine-2-carboxylate

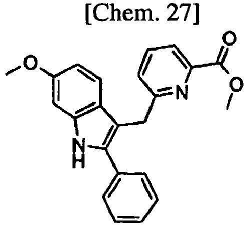

A solution of methyl 6-[2-(2-*tert*-butoxycarbonylamino-4-methoxyphenyl)-3-oxo-3-phenylpropyl]pyridine-2-carboxylate (3.27 g) in trifluoroacetic acid (10 mL)-methylene chloride (25 mL) was stirred at room temperature overnight. The solvent was evaporated under reduced pressure. The residue was diluted with ethyl acetate, and to the mixture was added a saturated aqueous sodium hydrogen carbonate solution. The organic layer was separated, washed with water and saturated brine, then dried over anhydrous magnesium sulfate, and concentrated under reduced pressure. The obtained crude product was purified by silica gel column chromatography (eluting solvent: hexane-ethyl acetate) to obtain the title compound (1.49 g).

1H-NMR (CDCl3) δ ppm: 8.10 (1H, br s), 8.00-7.90 (1H, m) 7.65-7.15 (8H, m), 6.92 (1H, d, J=2.2Hz), 6.74 (1H, dd, J=8.7, 2.2Hz), 4.56 (2H, s), 4.04 (3H, s), 3.86 (3H, s)

In the same manner as in Example 1-1 using the corresponding ketones, Examples 1-2 to 1-6 below were obtained. The structural formulae and the physical property values thereof were shown in Table 23.

**[Table 23]**

| Ex. No. | Strc | Physical data |
|---|---|---|
| 1-2 | 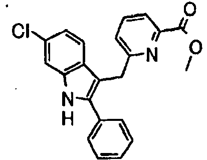 | 1H-NMR (CDCl3) *δ* ppm:8.25 (1H, br s), 8.00-7.90 (1H, m), 7.70-7.25 (8H, m), 7.10-7.00 (1H, m), 7.03 (1H, dd, J=8.5, 1.8Hz), 4.54 (2H, s), 4.04(3H, s) |
| 1-3 | 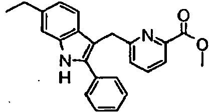 | 1H-NMR (CDCl3) *δ* ppm:8.10 (1H, br s), 8.00-7.90 (1H, m), 7.65-7.15 (9H, m), 7.00-6.90 (1H, m), 4.57 (2H, s), 4.05 (3H, s), 2.76 (2H, q, J=7.6Hz), 1.29 (3H, t, J=7.6Hz) |
| 1-4 | 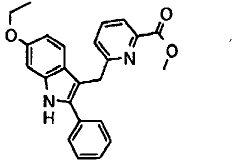 | 1H-NMR (CDCl3) *δ* ppm:8.15 (1H, br s), 8.00-7.90 (1H, m), 7.65-7.10 (8H, m), 6.89 (1H, d, J=2.1Hz), 6.73 (1H, dd, J=8.7, 2.1Hz), 4.54 (2H, s), 4.15-3.95 (5H, m), 1.43 (3H, t, J=7.0Hz) |
| 1-5 | 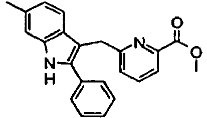 | 1H-NMR (CDCl3) *δ* ppm:8.09 (1H, br s), 8.00-7.90 (1H, m), 7.65-7.50 (3H, m), 7.45-7.15 (6H, m), 6.95-6.85 (1H, m), 4.57 (2H, s), 4.04 (3H, s), 2.47 (3H, s), |
| 1-6 | 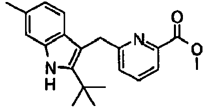 | 1H-NMR (CDCl3) *δ* ppm:7.95-7.85 (2H, m), 7.60-7.50 (1H, m), 7.20-7.00 (3H, m), 6.90-6.80 (1H, m), 4.58 (2H, s), 4.04 (3H, s), 2.44 (3H, s), 1.40 (9H, s) |

### (Example 2-1)

### Methyl 3-(6-methoxy-2-phenyl-1H-indol-3-ylmethyl)benzoate

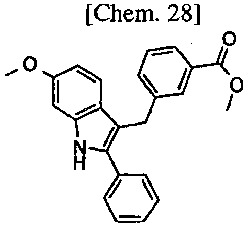

Under ice-cooling, to a solution of triethylsilane (0.204 mL) and trifluoroacetic acid (0.049 mL) in methylene chloride (1.5 mL) was added a solution of 6-methoxy-2-phenyl-1*H*-indole (95.0 mg) and methyl *m*-formylbenzoate (76.9 mg) in methylene chloride (1.8 mL). The solution was stirred for 0.5 hour under the same condition and then at room temperature overnight. To the solution were added 2 mol/L aqueous sodium hydroxide solution and then water. The organic layer was separated and concentrated under reduced pressure. The obtained crude product was purified by aminopropylated silica gel column chromatography (eluting solvent: hexane-ethyl acetate) to obtain the title compound (111 mg).

1H-NMR (CDCl3) δ ppm: 8.05-7.80 (3H, m), 7.55-7.15 (8H, m), 6.90 (1H, d, J=2.2Hz), 6.72 (1H, dd, J=8.7, 2.2Hz), 4.28 (2H, s), 3.88 (3H, s), 3.85 (3H, s)

In the same manner as in Example 2-1 using the corresponding indole derivatives, Examples 2-2 to 2-26 below were obtained. The structural formulae and the physical property values thereof were shown in Tables 24 to 28.

**[Table 24]**

| Ex. No. | Strc | Physical data |
|---|---|---|
| 2-2 | 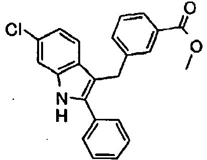 | 1H-NMR (CDCl3) *δ* ppm:8.19 (1H, br s), 7.95-7.80 (2H, m), 7.55-7.20 (9H, m), 7.01 (1H, dd, J=8.5, 1.8Hz), 4.28 (2H, s), 3.88 (3H, s) |
| 2-3 | 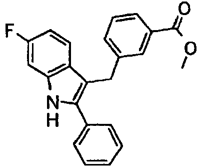 | 1H-NMR (CDCl3) *δ* ppm:8.16 (1H, br s), 8.00-7.80 (2H, m) 7.55-7.20 (8H, m), 7.15-7.05 (1H, m), 6.85-6.75 (1H, m), 4.29 (2H, s), 3.88 (3H, s) |
| 2-4 | 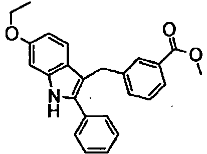 | 1H-NMR (CDCl3) *δ* ppm:8.10-7.80 (3H, m), 7.55-7.15 (8H, m), 6.95-6.85 (1H, m), 6.72 (1H, dd, J=8.6, 2.1 Hz), 4.28 (2H, s), 4.07 (2H, q, J=7.0Hz), 3.88 (3H, s), 1.44 (2H, t, J=7.0Hz) |
| 2-5 | 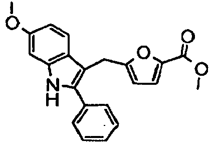 | 1H-NMR (CDCl3) *δ* ppm:8.08 (1H, br s), 7.55-7.30 (6H, m), 7.07 (1H, d, J=3.5Hz), 6.91 (1H, d, J=2.2Hz), 6.79 (1H, dd, J=8.7, 2.2Hz), 6.05-5.95 (1H, m), 4.24 (2H, s), 3.89 (3H, s), 3.86 (3H, s) |
| 2-6 | 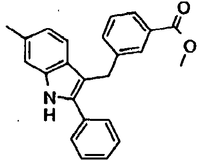 | 1H-NMR (CDCl3) *δ* ppm:8.06 (1H, br s), 8.00-7.80 (2H, m), 7.55-7.15 (9H, m), 6.95-6.85 (1H, m), 4.29 (2H, s), 3.87 (3H, s), 2.45 (3H, s) |

**[Table 25]**

| Ex. No. | Strc | Physical data |
|---|---|---|
| 2-7 | 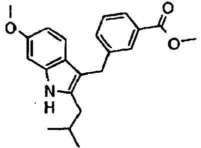 | 1H-NMR (CDCl3) *δ* ppm:8.00-7.60 (3H, m), 7.40-7.05 (3H, m), 6.85-6.75 (1H, m), 6.65 (1H, dd, J=8.6, 2.2Hz), 4.07 (2H, s), 3.88 (3H, s), 3.81 (3H, s), 2.70-2.45 (2H, m), 2.00-1.85 (1H, m), 0.93 (6H, d, J=6.6Hz) |
| 2-8 | 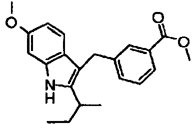 | 1H-NMR (CDCl3) *δ* ppm:8.00-7.75 (3H, m), 7.40-7.10 (3H, m), 6.90-6.60 (2H, m), 4.09 (2H, s), 3.88 (3H, s), 3.82 (3H, s), 3.05-2.85 (1H, m), 1.75-1.50 (2H, m), 1.27 (3H, d, J=7.1Hz), 0.83 (3H, t, J=7.4Hz) |
| 2-9 | 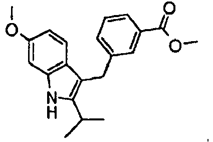 | 1H-NMR (CDCl3) *δ* ppm: ppm:8.00-7.65 3H, m), 7.40-7.10 (3H, m), 6.90-6.80 (1H, m), 6.75-6.60 (1H, m), 4.10 (2H, s), 3.88 (3H, s), 3.82 (3H, s), 3.35-3.15 (1H, m), 1.29 (6H, d, J=7.0Hz) |
| 2-10 | 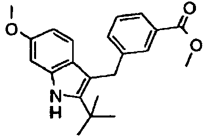 | 1H-NMR (CDCl3) *δ* ppm:8.00-7.75 (3H, m), 7.45-6.60 (5H, m), 4.30 (2H, s), 3.88 (3H, s), 3.82 (3H, s), 1.41 (9H, s) |
| 2-11 | 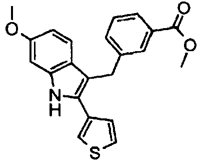 | 1H-NMR (CDCl3) *δ* ppm:8.03 (1H, br s), 8.00-7.80 (2H, m) 7.45-7.20 (6H, m), 6.89 (1H, d, J=2.2Hz), 6.73 (1H, dd, J=8.7, 2.2Hz), 4.29 (2H, s), 3.88 (3H, s), 3.85 (3H, s) |

**[Table 26]**

| Ex. No. | Strc | Physical data |
|---|---|---|
| 2-12 | 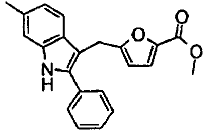 | 1H-NMR (CDCl3) *δ* ppm:8.06 (1H, br s), 7.55-7.40 (6H, m), 7.25-6.90 (3H, m), 6.05-5.90 (1H, m), 4.25 (2H, s), 3.89 (3H, s), 2.47 (3H, s) |
| 2-13 | 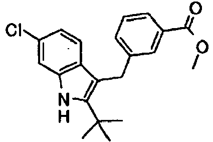 | 1H-NMR (CDCl3) *δ* ppm:8.05-7.80 (3H, m), 7.45-7.20 (3H, m), 7.13 (1H, d, J=8.5Hz), 6.95 (1H, dd, J=8.5, 1.8Hz), 4.31 (2H, s), 3.88 (3H, s), 1.42 (9H, s) |
| 2-14 | 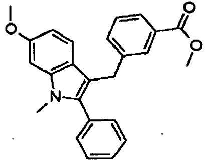 | 1H-NMR (CDCl3) *δ* ppm:7.90-7.75 (2H, m), 7.50-7.20 (8H, m), 6.82 (1H, d, J=2.2Hz), 6.73 (1H, dd, J=8.6, 2.2Hz), 4.07 (2H, s), 3.88 (3H, s), 3.87 (3H, s), 3.59 (3H, s) |
| 2-15 | 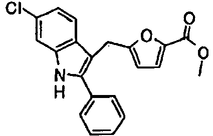 | 1H-NMR (CDCl3) *δ* ppm:8.14 (1H, br s), 7.55-7.35 (7H, m), 7.15-7.00 (2H, m), 5.98 (1H, d, J=3.3Hz), 4.24 (2H, s), 3.89 (3H, s) |
| 2-16 | 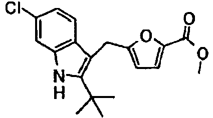 | 1H-NMR (CDCl3) *δ* ppm:7.97(1H.brs), 7.35-7.20 (2H, m), 7.05-6.95 (2H, m), 5.81 (1H, d, J=3.4Hz), 4.28 (2H, s), 3.88 (3H, s), 1.44 (9H, s) |

**[Table 27]**

| Ex. No. | Strc | Physical data |
|---|---|---|
| 2-17 | 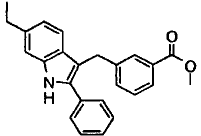 | 1H-NMR (CDCl3) *δ* ppm:8.10-7.80 (3H, m), 7.55-7.20 (9H, m), 6.95-6.90 (1H, m), 4.29 (2H, s), 3.88 (3H, s), 2.76 (2H, q, J=7.6Hz), 1.29 (3H, t, J=7.6Hz) |
| 2-18 | 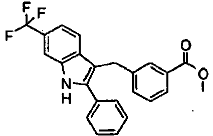 | 1H-NMR (CDCl3) *δ* ppm:8.37 (1H, br s), 8.00-7.65 (3H, m), 7.55-7.20 (9H, m), 4.32 (2H, s), 3.88 (3H, s) |
| 2-19 | 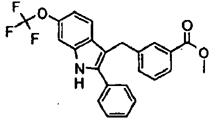 | 1H-NMR (CDCl3) *δ* ppm:8.24 (1H, br s), 8.00-7.80 (2H, m), 7.55-7.20 (9H, m), 7.00-6.90 (1H, m), 4.29 (2H, s), 3.88 (3H, s) |
| 2-20 | 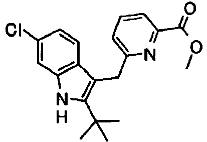 | 1H-NMR (CDCl3) *δ* ppm:8.05-7.90 (2H, m), 7.65-7.50 (1H, m), 7.33 (1H, d, J=1.7Hz), 7.16 (1H, d, J=8.5Hz), 7.05-6.90 (2H, m), 4.57 (2H, s), 4.04 (3H, s), 1.14 (9H ,s) |
| 2-21 | 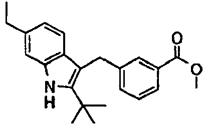 | MS(ESI, m/z) : 350 (M+H)+ |

**[Table 28]**

| Ex. No. | Strc | Physical data |
|---|---|---|
| 2-22 | 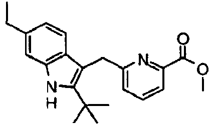 | 1H-NMR (CDCl3) *δ* ppm:8.00-7.85 (2H, m), 7.60-7.50 (1H, m), 7.20-7.15 (2H, m), 7.10-7.00 (1H, m), 6.95-6.85 (1H, m), 4.59 (2H, s), 4.04 (3H, s), 2.73 (2H, q, J=7.6Hz), 1.40 (9H, s), 1.27 (3H, t, J=7.6Hz) |
| 2-23 | 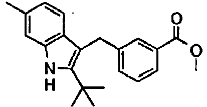 | 1H-NMR (CDCl3) *δ* ppm:7.90-7.75 (3H, m), 7.35-7.20 (1H, m), 7.15-7.10 (2H, m), 6.85-6.80 (1H, m), 4.32 (2H, s), 3.88 (3H, s), 2.43 (3H, s), 1.41 (9H, s) |
| 2-24 | 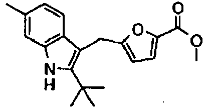 | 1H-NMR (CDCl3) *δ* ppm:7.83 (1H, br s), 7.30-7.20 (1H, m), 7.15-6.85 (3H, m), 5.82 (1H, d, J=3.5Hz), 4.29 (2H, s), 3.88 (3H, s), 2.44 (3H, s), 1.43 (9H, s) |
| 2-25 | 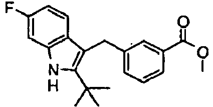 | 1H-NMR (CDCl3) *δ* ppm:8.05-7.75 (3H, m), 7.15-7.10(1H, m), 7.05-6.95 (1H, m), 6.80-6.70 (1H, m), 4.32 (2H, s), 3.88 (3H, s), 1.42 (9H, s) |
| 2-26 | 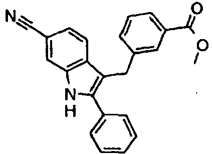 | 1H-NMR (CDCl3) δ ppm:8.44 (1H, brs), 7.95-7.70 (3H, m), 7.55-7.20 (9H, m), 4.31 (2H, s), 3.88 (3H, s) |

### (Example 3-1)

### Methyl 3-(6-chloro-1-methyl-2-phenyl-1H-indol-3-ylmethyl)benzoate

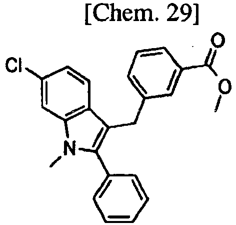

At room temperature, to a solution of methyl 3-(6-chloro-2-phenyl-1*H*-indol-3-ylmethyl)benzoate (112 mg) in *N,N*-dimethylformamide (15 mL) was added sodium hydride (content 55%, 13.6 mg) under an argon gas atmosphere, and the mixture was stirred at room temperature for 0.5 hour under an argon gas atmosphere. At room temperature, to the mixture was added methyl iodide (0.030 mL), and the mixture was stirred at room temperature overnight under an argon gas atmosphere. To the reaction mixture were added water and ethyl acetate, and the organic layer was separated, washed with water and saturated brine successively, then dried over anhydrous magnesium sulfate, and the solvent was evaporated under reduced pressure. The obtained crude product was purified by aminopropylated silica gel column chromatography (eluting solvent: hexane-ethyl acetate) to obtain the title compound (41.2 mg).

1H-NMR (CDCl3) δ ppm: 7.85-7.75 (2H, m), 7.50-7.20 (9H, m), 7.03 (1H, dd, J=8.4, 1.8Hz), 4.07 (2H, s), 3.87 (3H, s), 3.59 (3H, s)

In the same manner as in Example 3-1 using the corresponding indole derivatives, Examples 3-2 to 3-12 below were obtained. The structural formulae and the physical property values thereof were shown in Tables 29 to 31.

**[Table 29]**

| Ex. No. | Strc | Physical data |
|---|---|---|
| 3-2 | 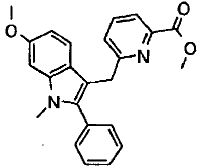 | 1H-NMR (CDCl3) *δ* ppm:7.95-7.85 (1H, m), 7.65-7.55 (1H, m), 7.50-7.25 (6H, m), 7.20-7.15 (1H, m), 6.83 (1H, d, J=2.2Hz), 6.74 (1H, d, J=8.6, 2.2Hz), 4.35 (2H, s), 4.01 (3H, s), 3.89 (3H, s), 3.60 (3H, s) |
| 3-3 | 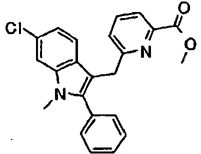 | 1H-NMR (CDCl3) *δ* ppm:7.95-7.85 (1H, m), 7.65-7.55 (1H, m), 7.50-7.30 (7H, m), 7.15-7.05 (1H, m), 7.03 (1H, dd, J=8.5, 1.8Hz), 4.33 (2H, s), 4.01 (3H, s), 3.61 (3H, s) |
| 3-4 | 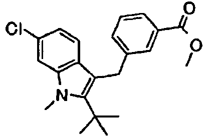 | 1H-NMR (CDCl3) *δ* ppm:7.85-7.75 (2H, m), 7.30-7.15 (4H, m), 6.98 (1H, dd, J=8.4, 1.8Hz), 4.39 (2H, s), 3.89 (3H, s), 3.87 (3H, s), 1.50 (9H, s) |
| 3-5 | 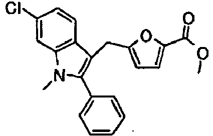 | 1H-NMR (CDCl3) *δ* ppm:7.55-7.30 (7H, m), 7.09 (1H, dd, J=8.5, 1.6Hz), 7.03 (1H, d, J=3.4Hz), 5.94 (1H, d, J=3.4Hz), 4.04 (2H, s), 3.86 (3H, s), 3.59 (3H, s) |

**[Table 30]**

| Ex. No. | Strc | Physical data |
|---|---|---|
| 3-6 | 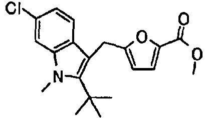 | 1H-NMR (CDCl3) *δ* ppm:7.35-7.20 (1H, m), 7.05-6.95 (2H, m), 5.76 (1H, d, J=3.3Hz), 4.35 (2H, s), 3.88 (3H, s), 3.87 (3H, s), 1.53 (9H, s) |
| 3-7 | 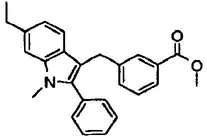 | 1H-NMR (CDCl3) *δ* ppm:7.90-7.75 (2H, m), 7.50-7.15 (9H, m), 7.00-6.90 (1H, m), 4.10-4.05 (2H, m), 3.87 (3H, s), 3.62(3H, s), 2.79 (2H, q, J=7.6Hz), 1.31 (3H, t, J=7.6Hz) |
| 3-8 | 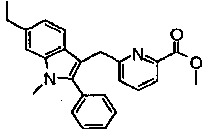 | 1H-NMR (CDCl3) *δ* ppm:7.95-7.85 (1H, m), 7.65-7.55 (1H, m), 7.50-7.30 (6H, m), 7.20-7.10 (2H, m), 7.00-6.90 (1H, m), 4.36 (2H, s), 4.02 (3H, s), 3.63 (3H, s), 2.80 (2H, q, J=7.6Hz), 1.31 (3H, t, J=7.6Hz) |
| 3-9 | 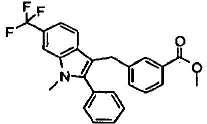 | 1H-NMR (CDCl3) *δ* ppm:7.90-7.60 (3H, m), 7.55-7.20 (9H, m), 4.15-4.05 (2H, s), 3.87 (3H, s), 3.68 (3H, s) |

**[Table 31]**

| Ex. No. | Strc | Physical data |
|---|---|---|
| 3-10 | 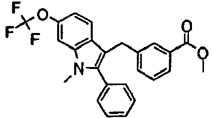 | 1H-NMR (CDCl3) *δ* ppm:7.90-7.75 (2H, m), 7.55-7.15 (9H, m), 7.00-6.90 (1H, m), 4.08 (2H, s), 3.86 (3H, s), 3.62 (3H, s) |
| 3-11 | 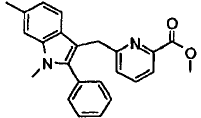 | 1H-NMR (CDCl3) *δ* ppm:7.95-7.85 (1H, m), 7.65-7.55 (1H, m), 7.50-7.25 (6H, m), 7.20-7.10 (2H, m), 6.95-6.85 (1H, m), 4.36 (2H, s), 4.02 (3H, s), 3.62 (3H, s), 2.51 (3H, s) |
| 3-12 | 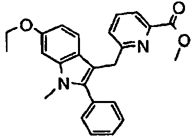 | 1H-NMR (CDCl3) *δ* ppm:7.95-7.85 (1H, m), 7.65-7.55 (1H, m), 7.50-7.10 (7H, m), 6.84 (1H, d, J=2.2Hz), 6.74 (1H, dd, J=8.7, 2.2Hz), 4.35 (2H, s), 4.11 (2H, q, J=7.0Hz), 4.01 (3H, s), 3.59 (3H, s), 1.45 (3H, t, =7.0Hz) |

### (Example 4-1)

### 6-(6-Methoxy-2-phenyl-1H-indol-3-ylmethyl)pyridine-2-carboxylic acid

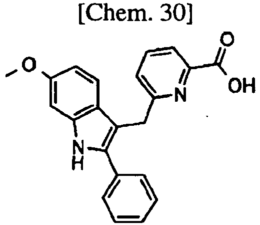

At room temperature, to a solution of methyl 6-(6-methoxy-2-phenyl-1*H*-indol-3-ylmethyl)pyridine-2-carboxylate (0.427 g) in methanol (3.0 mL)-tetrahydrofuran (1.5 mL) was added 2 mol/L aqueous sodium hydroxide solution (1.43 mL), and the solution was stirred at 65°C for 1 hour. After cooling to room temperature, to the mixture was added 2 mol/L hydrochloric acid. The mixture was concentrated under reduced pressure. To the residue was added water, and the mixture was pulverized with ultrasonic wave and then stirred for 15 minutes. The precipitate was collected by filtration to obtain the title compound (0.405 g).

1H-NMR (DMSO-d6) δ ppm: 11.18 (1H, br s), 7.90-7.70 (4H, m), 7.50-7.25 (5H, m), 6.87 (1H, d, J=2.1Hz), 6.62 (1H, dd, J=8.7, 2.1Hz), 4.38 (2H, s), 3.77 (3H, s)
MS (ESI, m/z): 359 (M+H)+

In the same manner as in Example 4-1 using the corresponding esters, Examples 4-2 to 4-44 below were obtained. The structural formulae and the physical property values thereof were shown in Tables 32 to 40.

**[Table 32]**

| Ex. No. | Strc | Physical data |
|---|---|---|
| 4-2 | 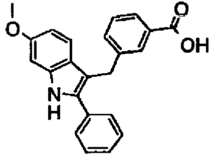 | 1H-NMR (DMSO-d6) *δ* ppm:13.00-12.70 (1H, br), 11.19 (1H, s), 7.80-7.70 (2H, m), 7.60-7.25 (7H, m), 7.21 (1H, d, J=8.7Hz), 6.89 (1H, d, J=2.2Hz), 6.62 (1H, dd, J=8.7, 2.2Hz). 4.26 (2H, s). 3.78 (3H, s) |
| 4-3 | 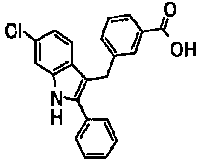 | 1H-NMR (DMSO-d6) *δ* ppm:12.86 (1H, s), 11.57 (1H, s), 7.80-7.30 (11H, m), 6.98 (1H, dd, J=8.5, 1.9Hz), 4.30 (2H, s) |
| 4-4 | 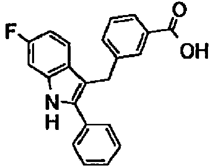 | 1H-NMR (DMSO-d6) *δ* ppm:12.95-12.80 (1H, br), 11.50 (1H, br s), 7.80-7.20 (10H, m), 7.14 (1H, dd, J=10.0, 2.3Hz), 6.90-6.75 (1H, m), 4.29 (2H, s) |
| 4-5 | 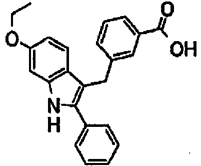 | 1H-NMR (DMSO-d6) *δ* ppm: 12.82 (1H, s), 11.15 (1H, s), 7.80-7.70 (2H, m), 7.60-7.25 (7H, m), 7.19 (1H, d, J=8.6Hz), 6.90-6.85 (1H, m), 6.65-6.55 (1H, m), 4.26 (2H, s), 4.03 (2H, q, J=7.0Hz), 1.35 (3H, t, J=7.0Hz) |
| 4-6 | 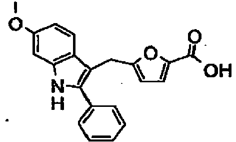 | 1H-NMR (CDCl3) *δ* ppm: 8.08 (1H, br s), 7.55-7.30 (6H, m), 7.21(1H, d, J=3.5Hz), 6.91 (1H, d, J=2.2Hz), 6.80 (1H, dd, J=8.7, 2.2Hz), 6.05 (1H, d, J=3.5Hz), 4.27 (2H, s), 3.87 (3H, s) |

**[Table 33]**

| Ex. No. | Strc | Physical data |
|---|---|---|
| 4-7 | 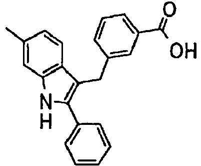 | 1H-NMR (DMSO-d6) *δ* ppm:12.85(1H,brs). 11.20 (1H, s), 7.80-7.65 (2H, m), 7.60-7.30 (7H, m), 7.25-7.15 (2H, m), 6.79 (1H, dd, J=8.1, 1.1Hz), 4.27 (2H, s), 2.39 (3H, s) |
| 4-8 | 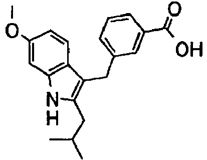 | 1H-NMR (CDCl3) *δ* ppm:8.05-7.85 (2H, m), 7.70 (1H, br s), 7.45-7.25 (2H, m), 7.16 (1H, d, J=8.6Hz), 6.83 (1H, d, J=2.2Hz), 6.68 (1H, dd, J=8.6, 2.2Hz), 4.09 (2H, s), 3.82 (3H, s), 2.60 (2H, d, J=7.4Hz), 2.00-1.85 (1H, m), 0.94 (6H, d, J=6.7Hz) |
| 4-9 | 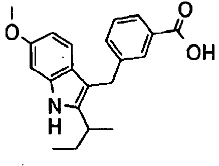 | 1H-NMR (CDCl3) *δ* ppm:8.05-7.85 (2H, m), 7.70 (1H, br s), 7.45-7.25 (2H, m), 7.19 (1H, d, J=8.7Hz), 6.85 (1H, d, J=2.2Hz), 6.69 (1H, dd, J=8.7, 2.2Hz), 4.11 (2H, s), 3.83 (3H, s), 3.05-2.90 (1H, m), 1.80-1.50 (2H, m), 1.28 (3H, d, J=7.0Hz), 0.83 (3H, t, J=7.4Hz) |
| 4-10 | 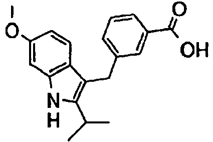 | 1H-NMR (CDCl3) *δ* ppm:8.05-7.85 (2H, m), 7.76 (1H, br s), 7.45-7.25 (2H, m), 7.20 (1H, d, J=8.6Hz), 6.85 (1H, d, J=2.2Hz), 6.69 (1H, dd, J=8.6, 2.2Hz), 4.11 (2H, s), 3.82 (3H, s), 3.30-3.15 (1H, m), 1.30 (6H, d, J=7.0Hz) |
| 4-11 | 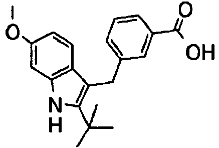 | 1H-NMR (DMSO-d6) *δ* ppm:13.00-12.60 (1H, br), 10.44 (1H, s), 7.75-7.60 (2H, m), 7.40-7.30 (2H, m), 7.07 (1H, d, J=8.6Hz), 6.84 (1H, d, J=2.3Hz), 6.53 (1H, dd, J=8.6, 2.3Hz), 4.24 (2H, s), 3.73 (3H, s), 1.35 (9H, s) |

**[Table 34]**

| Ex. No. | Strc | Physical data |
|---|---|---|
| 4-12 | 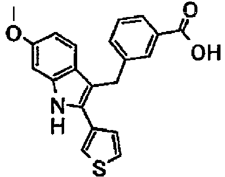 | 1H-NMR (DMSO-d6) *δ* ppm:12.84 (1H, s), 11.14 (1H, s), 7.75-7.55 (4H, m), 7.50-7.30 (3H, m), 7.25 (1H, d, J=8.6Hz), 6.86 (1H, d, J=2.2Hz), 6.62 (1H, dd, J=8.6, 2.2Hz), 4.30 (2H, s), 3.78 (3H, s) |
| 4-13 | 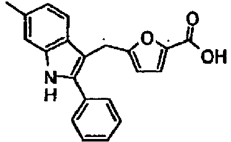 | 1H-NMR (CDCl3) *δ* ppm:8.04 (1H, br s), 7.55-7.30 (6H, m), 7.25-7.15 (2H, m), 7.00-6.90 (1H, m), 6.04 (1H, d, J=3.5Hz), 4.28 (2H, s), 2.48 (3H, s) |
| 4-14 | 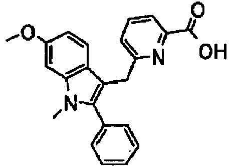 | 1H-NMR (DMSO-d6) *δ* ppm:7.85-7.75 (2H, m), 7.65-7.40 (5H, m), 7.35-7.20 (2H, m), 7.02 (1H, d, J=2.2Hz), 6.64 (1H, dd, J=8.6, 2.2Hz), 4.13 (2H, s), 3.80 (3H, s), 3.58 (3H, s) |
| | | MS(ESI, m/z) : 373 (M+H)+ |
| 4-15 | 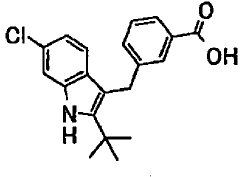 | 1H-NMR (DMSO-d6) *δ* ppm:12.81 (1H, s), 10.78 (1H, s), 7.75-7.60 (2H, m), 7.45-7.25 (3H, m), 7.20 (1H, d, J=8.4Hz), 6.87 (1H, dd, J=8.4, 1.9Hz), 4.28 (2H, s), 1.36 (9H, s) |
| 4-16 | 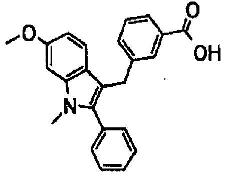 | 1H-NMR(CDCl3) δ ppm:7.95-7.80 (2H, m), 7.50-7.20 (8H, m), 6.83 (1H, d, J=2.2Hz), 6.74 (1H, dd, J=8.7, 2.2Hz), 4.09 (2H, s), 3.89 (3H, s), 3.59 (3H, s) |

**[Table 35]**

| Ex. No. | Strc | Physical data |
|---|---|---|
| 4-17 | 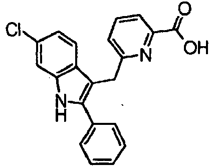 | 1H-NMR (DMSO-d6) *δ* ppm:13.30-12.90 (1H, br), 11.54 (1H, s), 7.90-7.75 (4H, m), 7.55-7.30 (6H, m), 6.98 (1H, dd, J=8.5, 1.9Hz), 4.38 (2H, s) MS(ESI, m/z) : 363 (M+H)+ |
| 4-18 | 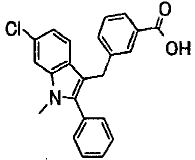 | 1H-NMR (DMSO-d6) *δ* ppm:12.82 (1H, s), 7.75-7.25 (11H, m), 7.02 (1H, dd, J=8.5, 1.8Hz), 4.05 (2H, s), 3.61 (3H, s) |
| 4-19 | 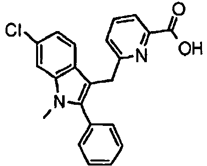 | 1H-NMR (DMSO-d6) *δ* ppm:13.40-12.70 (1H, br), 7.90-7.75 (2H, m), 7.70-7.40 (7H, m), 7.35-7.25 (1H, m), 7.01 (1H, dd, J=8.4, 1.8Hz), 4.15 (2H, s), 3.60 (3H, s) |
| | | MS(ESI, m/z) : 377 (M+H)+ |
| 4-20 | 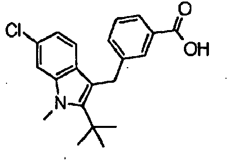 | 1H-NMR (CDCl3) *δ* ppm:7.95-7.80 (2H, m), 7.35-7.20 (4H, m), 6.99 (1H, dd, J=8.4, 1.8Hz), 4.40 (2H, s), 3.90 (3H, s), 1.50 (9H, s) |
| 4-21 | 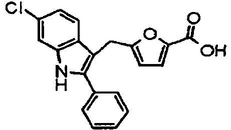 | 1H-NMR (DMSO-d6) *δ* ppm:12.86 (1H, s), 11.57 (1H, s), 7.70-7.35 (7H, m), 7.09 (1H, d, J=3.3Hz), 7.02 (1H, dd, J=8.5, 1.8Hz), 6.23 (1H, d, J=3.3Hz), 4.24 (2H, s) |

**[Table 36]**

| Ex. No. | Strc | Physical data |
|---|---|---|
| 4-22 | 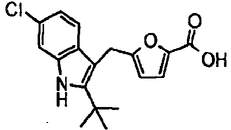 | 1H-NMR (DMSO-d6) *δ* ppm:12.90-12.70 (1H, br), 10.78 (1H, s), 7.37 (1H, d, J=8.5Hz), 7.35-7.25 (1H, m), 7.05 (1H, d, J=3.3Hz), 6.95-6.85 (1H, m), 6.06 (1H, d, J=3.3Hz), 4.24 (2H, s), 1.41 (9H, s) |
| 4-23 | 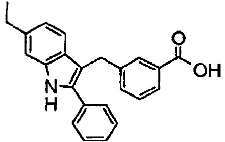 | 1H-NMR (DMSO-d6) *δ* ppm:12.82(1H. br s), 11.19 (1H, s), 7.80-7.70 (2H, m), 7.60-7.15 (9H, m), 6.85-6.75 (1H, m), 4.27 (2H, s), 2.68 (2H, q, J=7.6Hz), 1.22 (3H, t, J=7.6Hz) |
| 4-24 | 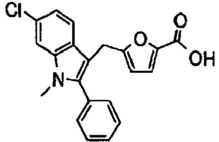 | 1H-NMR (DMSO-d6) *δ* ppm:12.82 (1H, br s), 7.70-7.45 (7H, m), 7.15-7.00 (2H, m), 6.18 (1H, d, J=3.3Hz), 4.02 (2H, s), 3.60 (3H, s) |
| 4-25 | 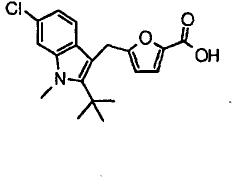 | 1H-NMR (DMSO-d6) *δ* ppm:13:00-12.70 (1H, br), 7.51 (1H, d, J=1.8Hz), 7.48 (1H, d, J=8.5Hz), 7.04 (1H, d, J=3.4Hz), 6.99 (1H, dd, J=8.5, 1.8Hz), 5.99 (1H, d, J=3.4Hz), 4.32 (2H, s), 3.87 (3H, s), 1.52 (9H, s) |
| 4-26 | 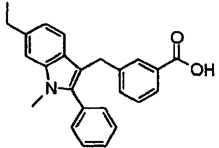 | 1H-NMR(DMSO-d6) *δ* ppm:13.05-12.70 (1H, br), 7.75-7.20 (11H, m), 6.90-6.80 (1H, m), 4.03 (2H, s), 3.59 (3H, s), 2.80-2.65 (2H, m), 1.30-1.20 (3H, m) |
| | | MS(ESI, m/z) : 370 (M+H)+ |

**[Table 37]**

| Ex. No. | Strc | Physical data |
|---|---|---|
| 4-27 | 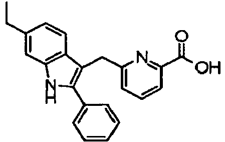 | 1H-NMR (DMSO-d6) *δ* ppm:11.20 (1H, s), 7.90-7.70 (4H, m), 7.55-7.15 (6H, m), 6.90-6.80 (1H, m), 4.36 (2H, s), 2.68 (2H, q, J=7.6Hz), 1.21 (3H, t, J=7.6Hz) |
| | | MS(ESI, m/z) : 357 (M+H)+ |
| 4-28 | 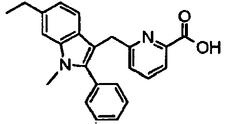 | 1H-NMR (DMSO-d6) *δ* ppm:7.90-7.20 (10H, m), 6.90-6.80 (1H, m), 4.14 (2H, s), 3.59 (3H, s), 2.75-2.65 (2H, m), 1.23 (3H, t, J=7.6Hz) |
| | | MS(ESI, m/z) : 371 (M+H)+ |
| 4-29 | 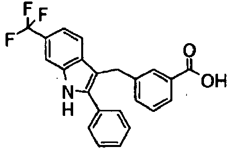 | 1H-NMR (DMSO-d6) *δ* ppm:13.00-12.70 (1H, br), 11.87 (1H, s), 7.85-7.20 (12H, m), 4.34 (2H, s), |
| 4-30 | 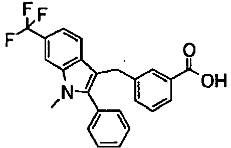 | 1H-NMR (DMSO-d6) *δ* ppm:13.00-12.70 (1H, br), 7.95-7.20(12H, m), 4.10 (2H, s), 3.70 (3H, s) |
| 4-31 | 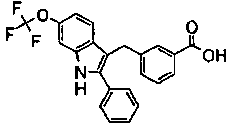 | 1H-NMR (CDCl3) δ ppm:8.25 (1H, br s), 8.05-7.85 (2H, m), 7.60-7.20 (9H, m), 7.00-6.90 (1H, m), 4.31 (2H, s) |

**[Table 38]**

| Ex. No. | Strc | Physical data |
|---|---|---|
| 4-32 | 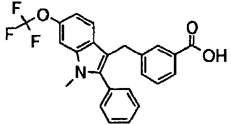 ' | 1H-NMR (CDCl3) *δ* ppm:7.95-7.85 (2H, m), 7.55-7.15 (9H, m), 7.00-6.90 (1H, m), 4.09 (2H, s), 3.62 (3H, s) |
| 4-33 | 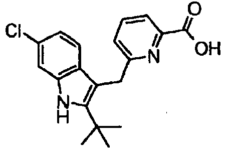 | 1H-NMR (DMSO-d6) *δ* ppm:13.50-12.60 (1H, br), 10.80 (1H, s), 7.90-7.70 (2H, m), 7.40-7.25 (2H, m), 7.10-7.00 (1H, m), 6.90 (1H, dd, J=8.4, 1.8Hz), 4.41 (2H, s), 1.38 (9H, s) |
| | | MS(ESI, m/z): 343 (M+H)+ |
| 4-34 | 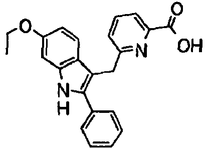 | 1H-NMR (DMSO-d6) *δ* ppm:13.30-12.90 (1H, br), 11.15 (1H, s), 7.90-7.70 (4H, m), 7.50-7.25 (5H, m), 6.86 (1H, d, J=2.1 Hz), 6.61 (1H, dd, J=8.7, 2.1Hz), 4.35 (2H, s), 4.02 (2H, q, J=6.9Hz), 1.34 (3H, t, J=6.9Hz) |
| | | MS(ESI, m/z): 373 (M+H)+ |
| 4-35 | 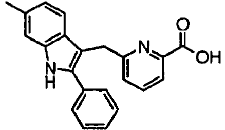 | 1H-NMR (DMSO-d6) *δ* ppm:11.19 (1H, s), 7.90-7.70 (4H, m), 7.55-7.15 (6H, m), 6.85-6.75 (1H, m), 4.36 (2H, s), 2.38 (3H, s) |
| | | MS(ESI, m/z): 343 (M+H)+ |
| 4-36 | 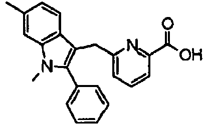 | 1H-NMR (DMSO-d6) *δ* ppm:7.85-7.70 (2H, m), 7.65-7.40 (5H, m), 7.35-7.20 (3H, m), 6.90-6.80 (1H, m), 4.14 (2H, s), 3.58 (3H, s), 2.42 (3H, s) |
| | | MS(ESI, m/z): 357 (M+H)+ |

**[Table 39]**

| Ex. No. | Strc | Physical data |
|---|---|---|
| 4-37 | 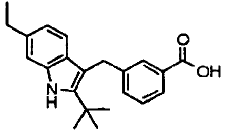 | 1H-NMR (DMSO-d6) *δ* ppm:12.79 (1H, s), 10.44 (1H, s), 7.75-7.65 (2H, m), 7.45-7.30 (2H, m), 7.20-7.05 (2H, m), 6.80-6.70 (1H, m), 4.25 (2H, s), 2.64 (2H, q, J=7.6Hz), 1.36 (9H, s), 1.19 (3H, t, J=7.6Hz) |
| 4-38 | 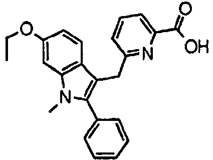 | 1H-NMR (DMSO-d6) *δ* ppm:7.85-7.75 (2H, m), 7.65-7.40 (5H, m), 7.35-7.20 (2H, m), 7.00 (1H, d, J=1.9Hz), 6.64 (1H, dd, J=8.6, 1.9Hz), 4.13 (2H, s), 4.07 (2H, q, J=7.0Hz), 3.56 (3H, s), 1.35 (3H, t, J=7.0Hz) |
| | | MS(ESI, m/z): 387 (M+H)+ |
| 4-39 | 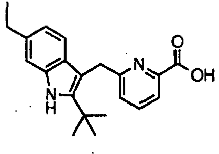 | 1H-NMR (DMSO-d6) *δ* ppm:10.46 (1H, s), 7.85-7.70 (2H, m), 7.20-7.00 (3H, m), 6.80-6.70 (1H, m), 4.38 (2H, s), 2.64 (2H, q, J=7.6Hz), 1.37 (9H, s), 1.19 (3H, t, J=7.6Hz) |
| | | MS(ESI, m/z) : 337 (M+H)+ |
| 4-40 | 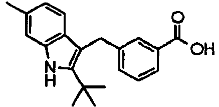 | 1H-NMR (DMSO-d6) *δ* ppm:13.00-12.50 (1H, br), 10.42 (1H, s), 7.75-7.60 (2H, m), 7.40-7.30 (2H, m), 7.15-7.05 (2H, m), 6.75-6.65 (1H, m), 4.25 (2H, s), 2.35 (3H, s), 1.35 (9H, s) |
| 4-41 | 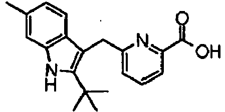 | 1H-NMR(DMSO-d6) *δ* ppm:10.45 (1H, s), 7.85-7.70 (2H, m), 7.20-7.00 (3H, m), 6.75-6.65 (1H,m),4.38 (2H,s),2.35(3H, s), 1.36 (9H, s) |
| | | MS(ESI, m/z) : 323 (M+H)+ |

**[Table 40]**

| Ex. No. | Strc | Physical data |
|---|---|---|
| 4-42 | 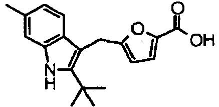 | 1H-NMR (DMSO-d6) *δ* ppm:12.90-12.60 (1H, br), 10.43 (1H, s), 7.22 (1H, d, J=8.1Hz), 7.15-6.95 (2H, m), 6.80-6.70 (1H, m), 6.02 (1H, d, J=3.4Hz), 4.21 (2H, s), 2.35 (3H, s), 1.40 (9H, s) |
| 4-43 | 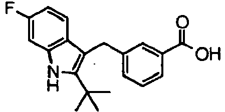 | 1H-NMR (DMSO-d6) *δ* ppm:12.81 (1H, br s), 10.71 (1H, s), 7.80-7.60 (2H, m), 7.45-7.30 (2H, m), 7.25-7.00 (2H, m), 6.80-6.65 (1H, m), 4.27 (2H, s), 1.36 (9H, s) |
| 4-44 | 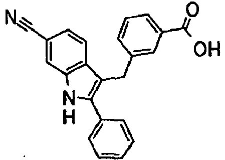 | 1H-NMR (DMSO-d6) *δ* ppm:12.95-12.75 (1H, br), 12.01 (1H, s), 7.90-7.25 (12H, m), 4.34 (2H, s) |

### (Example 5-1)

### Ethyl 6-(6-methoxy-2-thiophen-3-yl-1H-indol-3-ylmethyl)pyridine-2-carboxylate

At room temperature, to a solution of ethyl 6-[2-(2-*tert*-butoxycarbonylamino-4-methoxyphenyl)-3-oxo-3-thiophen-3-ylpropyl]pyridine-2-carboxylate (0.411 g) in methylene chloride (4.0 mL) was added trifluoroacetic acid (0.80 mL), and the solution was stirred for 1 hour. Under ice-cooling, to the reaction mixture was added 1 mol/L aqueous sodium hydroxide solution, and the mixture was stirred. The organic layer was separated, dried over anhydrous sodium sulfate, and then concentrated under reduced pressure. The obtained crude product was purified by aminopropylated silica gel column chromatography (eluting solvent: hexane-ethyl acetate) to obtain the title compound (0.189g).

Further, the structural formula and the spectral data of the title compound were shown in Table 41.

In the same manner as in Example 5-1 using the corresponding ketones, Examples 5-2 to 5-24 below were obtained. The structural formulae and the spectral data thereof were shown in Tables 41 to 46.

**[Table 41]**

| Ex. No. | Strc | Physical data |
|---|---|---|
| 5-1 | 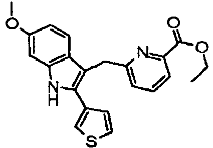 | 1H-NMR (CDCl3) *δ* ppm:8.05 (1H, s), 7.90-7.85 (1H, m), 7.70-7.55 (2H, m), 7.45-7.20 (4H, m), 6.89 (1H, d, J=2.2Hz), 6.76 (1H, dd, J=8.8, 2.2Hz), 4.52 (2H, s), 4.51 (2H, q, J=7.1Hz), 3.85 (3H, s), 1.48 (3H, t, J=7.1Hz) |
| 5-2 | 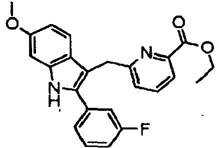 | 1H-NMR (CDCl3) *δ* ppm:8.06 (1H, s), 8.00-7.90 (1H, m), 7.70-7.55 (1H, m), 7.45-7.15 (5H, m), 7.10-6.95 (1H, m), 6.91 (1H, d, J=2.2Hz), 6.76 (1H, dd, J=8.7, 2.2Hz), 4.53 (2H, s), 4.51 (2H, q, J=7.2Hz), 3.86 (3H, s), 1.46 (3H, t, J=7.2Hz) |
| 5-3 | 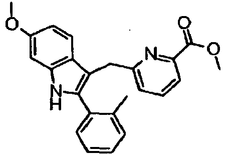 | 1H-NMR (CDCl3) *δ* ppm:8.00-7.80 (2H, m), 7.65-7.50 (1H, m), 7.40-7.05 (6H, m), 6.89 (1H, d, J=2.2Hz), 6.73 (1H, dd, J=8.7, 2.2Hz), 4.29 (2H, s), 4.01 (3H, s), 3.85 (3H, s), 2.26 (3H, s) |
| 5-4 | 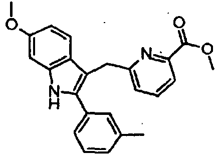 | 1H-NMR (CDCl3) *δ* ppm:8.07 (1H, s), 8.00-7.90 (1H, m), 7.70-7.55 (1H, m), 7.45-7.05 (6H, m), 6.91 (1H, d, J=2.2Hz), 6.73 (1H, dd, J=8.7, 2.2Hz), 4.56 (2H, s), 4.04 (3H, s), 3.85 (3H, s), 2.37 (3H, s) |

**[Table 42]**

| Ex. No. | Strc | Physical data |
|---|---|---|
| 5-5 | 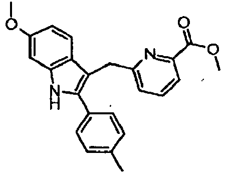 | 1H-NMR (CDCl3) *δ* ppm:8.05 (1H, s), 8.00-7.90 (1H, m), 7.70-7.55 (1H, m), 7.50-7.10 (6H, m), 6.91 (1H, d, J=2.2Hz), 6.73 (1H, dd, J=8.7, 2.2Hz), 4.54 (2H, s), 4.04 (3H, s), 3.85 (3H, s), 2.38 (3H, s) |
| 5-6 | 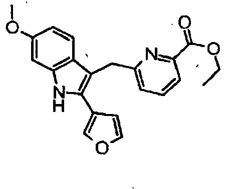 | 1H-NMR (CDCl3) *δ* ppm:8.15-7.85 (3H, m), 7.70-7.45 (2H, m), 7.37 (1H, d, J=8.7Hz), 7.30-7.20 (1H, m), 6.87 (1H, d, J=2.2Hz), 6.76 (1H, dd, J=8.7, 2.2Hz), 6.70-6.65 (1H, m), 4.50 (2H, q, J=7.1Hz), 4.45 (2H, s), 3.84 (3H, s), 1.47 (3H, t, J=7.1 Hz) |
| 5-7 | 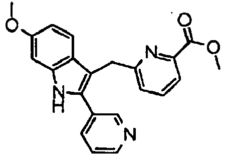 | 1H-NMR (CDCl3) *δ* ppm:8.90-8.75(1H, m), 8.60-8.50 (1H, m), 8.34 (1H, s), 8.05-7.85 (2H, m), 7.70-7.55 (1H, m), 7.40-7.15 (3H, m), 6.92 (1H, d, J=2.2Hz), 6.77 (1H, dd, J=8.7, 2.2Hz), 4.51 (2H, s), 4.03 (3H, s), 3.86 (3H, s) |
| 5-8 | 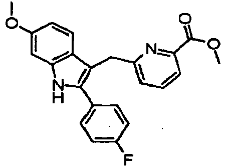 | 1H-NMR (CDCl3) *δ* ppm:8.03 (1H, s), 8.00-7.90 (1H, m), 7.70-7.45 (3H,m), 7.35-7.05 (4H, m), 6.91 (1H ,d, J=2.2Hz), 6.75 (1H, dd, J=8.7, 2.2Hz), 4.50 (2H, s), 4.04 (3H, s), 3.86 (3H, s) |

**[Table 43]**

| Ex. No. | Strc | Physical data |
|---|---|---|
| 5-9 | 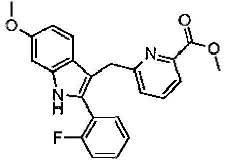 | 1H-NMR (CDCl3) *δ* ppm:8.34 (1H, s), 8.00-7.90 (1H, m), 7.70-7.45 (3H, m), 7.40-7.05 (4H, m), 6.92 (1H, d, J=2.2Hz), 6.73 (1H, dd, J=8.7, 2.2Hz), 4.50 (2H, s), 4.04 (3H, s), 3.86 (3H, s) |
| 5-10 | 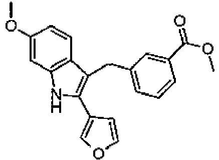 | 1H-NMR(CDCl3) *δ* ppm:8.00-7.80 (3H, m), 7.65-7.45 (2H, m), 7.40-7.20 (3H, m), 6.88 (1H, d, J=2.2Hz), 6.72 (1H,dd,J=8.7, 2.2Hz),6.65-6.55(1H,m),4.23 (2H, s), 3.88 (3H, s), 3.84 (3H, s) |
| 5-11 | 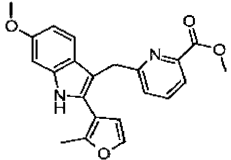 | 1H-NMR(CDCl3) *δ* ppm:8.00-7.80 (2H, m), 7.65-7.55 (1H, m), 7.33 (1H, d, J=1.9Hz), 7.21 (1H, d, J=8.7Hz), 7.20-7.10 (1H, m), 6.88 (1H, d, J=2.2Hz), 6.71 (1H, dd, J=8.7, 2.3Hz), 6.44 (1H, d, J=1.9Hz), 4.37 (2H, s), 4.03 (3H, s), 3.84 (3H, s), 2.33 (3H, s) |
| 5-12 | 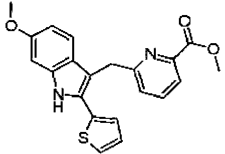 | 1H-NMR(CDCl3) *δ* ppm:8.09 (1H, s), 8.00-7.90 (1H, m), 7.70-7.55 (1H, m), 7.35-7.05 (5H, m), 6.89 (1H,d,J=2.2Hz), 6.74 (1H, dd, J=8.7, 2.2Hz), 4.61 (2H, s), 4.05 (3H, s), 3.85 (3H, s) |

**[Table 44]**

| Ex. No. | Strc | Physical data |
|---|---|---|
| 5-13 | 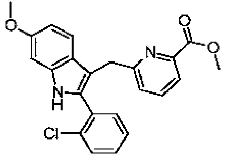 | 1H-NMR (CDCl3) *δ* ppm:8.16 (1H, s), 7.95-7.85 (1H, m), 7.65-7.40 (3H, m), 7.35-7.15 (4H, m), 6.91 (1H, d, J=2.2Hz), 6.74 (1H, dd, J=8.7, 2.2Hz), 4.38 (2H, s), 4.02 (3H, s), 3.85 (3H, s) |
| 5-14 | 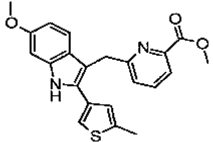 | 1H-NMR (CDCl3) *δ* ppm:8.03 (1H, s), 8.00-7.90 (1H, m), 7.70-7.55 (1H, m), 7.35-7.15 (3H, m), 7.05-6.95 (1H, m), 6.88 (1H, d, J=2.2Hz), 6.74 (1H, dd, J=8.7, 2.2Hz), 4.54 (2H, s), 4.05 (3H, s), 3.85 (3H, s), 2.50 (3H, d, J=0.9Hz) |
| 5-15 | 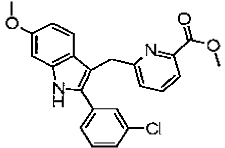 | 1H-NMR (CDCl3) *δ* ppm:8.10 (1H, s), 8.00-7.90 (1H, m), 7.70-7.50 (2H, m), 7.45-7.10 (5H, m), 6.90 (1H, d, J=2.2Hz), 6.75 (1H, dd, J=5.7, 2.2Hz), 4.53 (2H, s), 4.04 (3H, s), 3.86 (3H, s) |
| 5-16 | 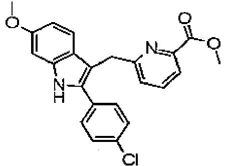 | 1H-NMR(CDCl3) *δ* ppm:8.08 (1H, s), 8.00-7.90 (1H, m), 7.70-7.35 (5H, m), 7.29 (1H, d, J=8.8Hz), 7.25-7.15 (1H, m), 6.90 (1H, d, J=2.2Hz), 6.75 (1H, dd, J=8.8, 2.2Hz), 4.51 (2H, s), 4.04 (3H, s), 3.85 (3H, s) |

**[Table 45]**

| Ex. No. | Strc | Physical data |
|---|---|---|
| 5-17 | 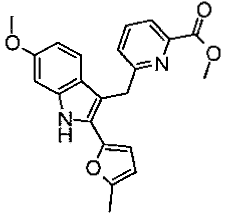 | 1H-NMR(CDCl3) *δ* ppm:8.39 (1H, s), 8.00-7.90 (1H, m), 7.65-7.50 (1H, m), 7.34(1H, d, J=8.7Hz), 7.20-7.10 (1H, m), 6.88 (1H, d, J=2.2Hz), 6.75 (1H, dd, J=8.7, 2.2Hz), 6.39 (1H, d, J=3.3Hz), 6.03 (1H, dd, J=3.3, 1.0Hz), 4.57 (2H, s), 4.05 (3H, s), 3.85 (3H, s), 2.34 (3H, s) |
| 5-18 | 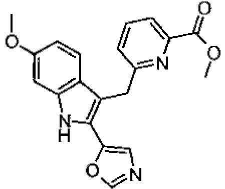 | ¹H-NMR(CDCl3) *δ* ppm:8.32 (1H, s), 8.00-7.85 (2H, m), 7.70-7.55 (1H, m), 7.42 (1H, d, J=8.8Hz), 7.34 (1H, s), 7.20-7.10 (1H, m), 6.90 (1H, d, J=2.2Hz), 6.80 (1H, dd, J=8.8, 2.2Hz), 4.57 (2H, s), 4.06 (3H, s), 3.87 (3H, s) |
| 5-19 | 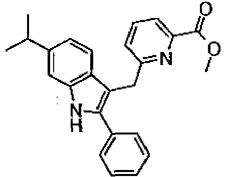 | 1H-NMR (CDCl3) *δ* ppm:8.12(1H, s), 8.00-7.90 (1H, m), 7.70-7.15 (9H, m), 6.98 (1H, dd, J=8.2, 1.3Hz), 4.57 (2H, s), 4.05 (3H, s), 3.10-2.90 (1H, m), 1.31 (6H, d, J=6.9Hz) |
| 5-20 | 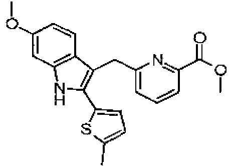 | 1H-NMR (CDCl3) *δ* ppm:8.03 (1H, s), 8.00-7.90 (1H, m), 7.65-7.55 (1H, m), 7.35-7.10 (2H, m), 6.97 (1H, d, J=3.4Hz), 6.87 (1H, d, J=2.2Hz), 6.85-6.65 (2H, m), 4.59 (2H, s), 4.05 (3H, s), 3.85 (3H, s), 2.49 (3H, s) |

**[Table 46]**

| Ex. No. | Strc | Physical data |
|---|---|---|
| 5-21 | 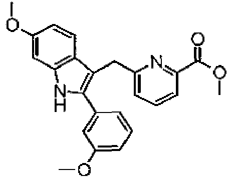 | 1H-NMR(CDCl3) *δ* ppm:8.10(1H, s), 8.00-7.90 (1H, m), 7.70-7.55 (1H, m), 7.40-7.00 (5H, m), 6.92 (1H, d, J=2.2Hz), 6.90-6.80 (1H, m), 6.74 (1H, dd, J=8.7, 2.2Hz), 4.56 (2H, s), 4.04 (3H, s), 3.86 (3H, s), 3.77 (3H, s) |
| 5-22 | 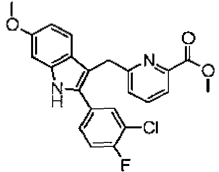 | 1H-NMR (CDCl3) *δ* ppm:8.01 (1H, s), 8.00-7.90 (1H, m), 7.75-7.60 (2H, m), 7.50-7.40 (1H, m), 7.31 (1H, d, J=8.7Hz), 7.25-7.15 (2H, m), 6.95 (1H, d, J=2.2Hz), 6.76 (1H, dd, J=8.7, 2.2Hz), 4.48 (2H, s), 4.04 (3H, s), 3.86 (3H, s) |
| 5-23 | 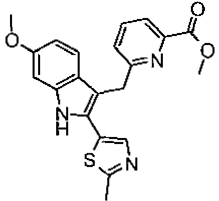 | 1H-NMR(CDCl₃) *δ* ppm:8.28 (1H, s), 8.00-7.90 (1H, m), 7.74 (1H, s), 7.65-7.55 (1H, m), 7.30 (1H, d, J=8.7Hz), 7.15-7.05 (1H, m), 6.86 (1H, d, J=2.2Hz), 6.74 (1H, dd, J=8.7, 2.2Hz), 4.53 (2H, s), 4.04 (3H, s), 3.84 (3H, s), 2.71 (3H, s) |
| 5-24 | 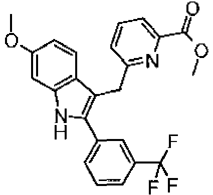 | 1H-NMR(CDCl3) *δ* ppm:8.23 (1H, s), 8.00-7.90 (1H, m), 7.85-7.45 (5H, m), 7.31 (1H, d, J=8.7Hz), 7.25-7.15 (1H, m), 6.91 (1H, d, J=2.2Hz), 6.76 (1H, dd, J=8.7, 2.2Hz), 4.52 (2H, s), 4.03 (3H, s), 3.85 (3H, s) |

### (Example 6-1)

### Methyl 6-(6-fluoro-2-phenyl-1H-indol-3-ylmethyl)pyridine-2-carboxylate

Under ice-cooling, to a solution of 6-fluoro-2-phenyl-1*H*-indole (88.9 mg), methyl 6-formylpyridine-2-carboxylate (76.5 mg), and triethylsilane (0.200 mL) in methylene chloride (2.0 mL) was added dropwise trifluoroacetic acid (0.049 mL). The mixture was stirred at the same temperature for 5 minutes and then at room temperature for 14 hours. Under ice-cooling, to the reaction mixture was added 1 mol/L aqueous sodium hydroxide solution, and the mixture was stirred. The organic layer was separated, dried over anhydrous sodium sulfate, and then concentrated under reduced pressure. The obtained crude product was purified by aminopropylated silica gel column chromatography (eluting solvent: hexane-ethyl acetate) to obtain the title compound (73.8 mg).

Further, the structural formula and the spectral data of the title compound were shown in Table 47.

In the same manner as in Example 6-1 using the corresponding indole derivatives, Examples 6-2 to 6-10 below were obtained. The structural formulae and the spectral data thereof were shown in Tables 47 and 48.

**[Table 47]**

| Ex. No. | Strc | Physical data |
|---|---|---|
| 6-1 | 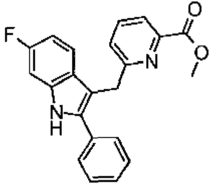 | 1H-NMR(CDCl3) *δ* ppm:8.20 (1H, s), 8.00-7.90 (1H, m), 7.70-7.25 (7H, m), 7.20-7.05 (2H, m), 6.90-6.75 (1H, m), 4.55 (2H, s), 4.04 (3H, s) |
| 6-2 | 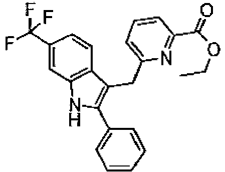 | 1H-NMR (CDCl3) *δ* ppm:8.41 (1H, s), 8.00-7.90 (1H, m), 7.75-7.10 (10H, m), 4.57 (2H, s), 4.51 (2H, q, J=7.2Hz), 1.47 (3H, t, J=7.2Hz) |
| 6-3 | 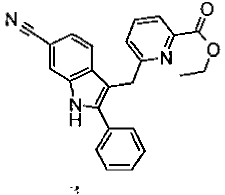 | 1H-NMR (CDCl3) δ ppm:8.49 (1H, s), 8.00-7.90 (1H, m), 7.80-7.10 (10H, m), 4.54 (2H, s), 4.50 (2H, q, J=7.2Hz), 1.46 (3H, t, J=7.2Hz) |
| 6-4 | 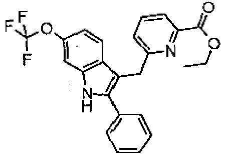 | 1H-NMR (CDCl3) *δ* ppm:8.28 (1H, s), 8.00-7.90 (1H, m), 7.70-7.55 (3H, m), 7.50-7.25 (5H, m), 7.20-7.10 (1H, m), 7.00-6.90 (1H, m), 4.55 (2H, s), 4.51 (2H, q, J=7.2Hz), 1.47 (3H, t, J=7.2Hz) |
| 6-5 | 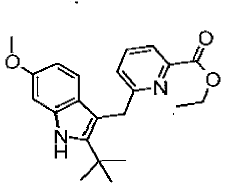 | 1H-NMR(CDCl3) *δ* ppm:8.00-7.85 (2H, m), 7.65-7.50 (1H, m), 7.15 (1H, d, J=8.7Hz), 7.10-7.00 (1H, m), 8.86 (1H, d, J=2.2Hz), 6.68 (1H, dd, J=8.7, 2.2Hz), 4.57 (2H, s), 4.51 (2H, q, J=7.2Hz), 3.83 (3H, s), 1.46 (3H, t, J=7.2Hz), 1.40 (9H, s) |

**[Table 48]**

| Ex. No. | Strc | Physical data |
|---|---|---|
| 6-6 | 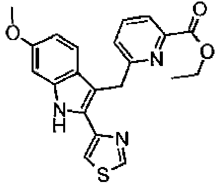 | 1H-NMR (CDCl3) *δ* ppm:9.08 (1H, s), 8.83 (1 H, d, J=1.9Hz), 8.04 (1 H, d, J=1.9Hz), 8.00-7.90 (1H, m), 7.70-7.55 (1H, m), 7.52 (1H, d, J=8.7Hz), 7.40-7.25 (1H, m), 6.90 (1H, d, J=2.2Hz), 6.82 (1H, dd, J=8.7, 2.2Hz), 4.61 (2H, s), 4.51 (2H, q, J=7.2Hz), 3.87 (3H, s), 1.48 (3H, t, J=7.2Hz) |
| 6-7 | 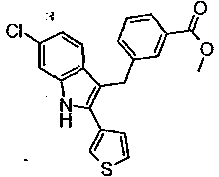 | 1H-NMR (CDCl3) *δ* ppm:8.19 (1H, s), 7.95-7.80 (2H, m), 7.42 (1H, dd, J=5.0, 2.9Hz), 7.36 (1H, d, J=1.8Hz), 7.35-7.20 (5H, m), 7.02 (1H, dd, J=8.5, 1.8H), 4.29 (2H, s), 3.88 (3H, s) |
| 6-8 | 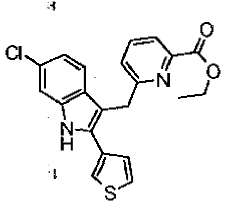 | 1H-NMR (CDCl3) *δ* ppm:8.30 (1H, s), 8.00-7.90 (1H, m), 7.73 (1H, dd, J=3.0, 1.4Hz), 7.70-7.55 (1H, m), 7.42 (1H, dd, J=5.1, 3.0Hz), 7.45-7.30 (3H, m), 7.25-7.15 (1H, m), 7.05 (1H, dd, J=8.4, 1.8Hz), 4.55-4.45 (4H, m), 1.47 (3H, t, J=7.1Hz) |
| 6-9 | 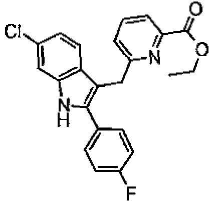 | 1H-NMR (CDCl3) *δ* ppm:8.26 (1H, s), 8.00-7.90 (1H, m), 7.70-7.50 (3H, m), 7.34 (1H, d, J=1.8Hz), 7.34 (1H, d, J=8.5Hz), 7.25-7.05 (3H, m), 7.04 (1H, dd, J=8.5, 1.8Hz), 4.55-4.40 (4H, m), 1.46 (3H, t, J=7.1Hz) |
| 6-10 | 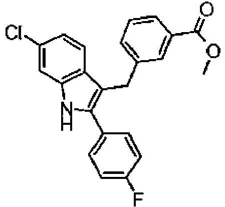 | 1H-NMR(CDCl3) *δ* ppm:8.18(1H, s), 7.95-7.80 (2H, m), 7.50-7.40 (2H, m), 7.37 (1H, d, J=1.8Hz), 7.35-7.05 (5H, m), 7.02 (1H, dd, J=8.5, 1.8Hz), 4.23 (2H, s), 3.88 (3H, s) |

### (Example 7)

### Ethyl 3-(6-methoxy-2-pyridin-3-yl-1H-indol-3-ylmethyl)benzoate

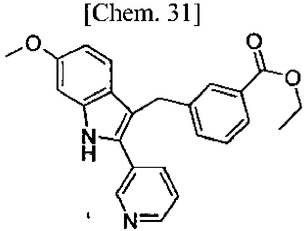

Under ice-cooling, to a solution of ethyl 3-(6-methoxy-2-pyridin-3-yl-1*H*-indole-3-carbonyl)benzoate (0.12 g) in tetrahydrofuran (1.5 mL)-ethanol (1.5 mL) was added 10% palladium-carbon (125 mg, wet), and the mixture was stirred overnight at room temperature under a hydrogen atmosphere, and then at 40°C for 8 hours. The reaction mixture was filtered through Celite (registered trademark) to remove the insoluble materials. The filtrate was concentrated under reduced pressure. To the residue was added a solution of methylene chloride (3 mL), then under ice-cooling, triethylsilane (0.105 mL) and trifluoroacetic acid (0.277 mL) were added successively thereto, and the mixture was stirred at room temperature overnight. The reaction mixture was purified by aminopropylated silica gel column chromatography (eluting solvent: hexane-ethyl acetate) to obtain the title compound (41.8 mg).

1H-NMR (CDCl3) δ ppm: 8.85-8.70 (1H, m), 8.65-8.5 0 (1H, m), 8.10 (1H, s), 8.00-7.70 (3H, m), 7.40-7.20 (4H, m), 6.93 (1H, d, J=2.2Hz), 6.75 (1H, dd, J=8.7, 2.2Hz), 4.35 (2H, q, J=7.1Hz), 4.28 (2H, s), 3.86 (3H, s), 1.37 (3H, t, J=7.1Hz)
MS (ESI, m/z)=387 (M+H)+

### (Example 8-1)

### Ethyl 6-(6-chloro-2-pyridin-3-yl-1H-indol-3-ylmethyl)pyridine-2-carboxylate

At room temperature, to a solution of ethyl 6-[(6-chloro-2-pyridin-3-yl-1*H*-indol-3-yl)hydroxymethyl]pyridine-2-carboxylate (72.0 mg) in methylene chloride (1.5 mL) were added triethylsilane (0.034 mL) and trifluoroacetic acid (0.163 mL) successively, and the mixture was stirred for 7 hours. The reaction mixture was purified by aminopropylated silica gel column chromatography (eluting solvent: hexane-ethyl acetate) to obtain the title compound (49.5 mg).

Further, the structural formula and the spectral data of the title compound were shown in Table 49.

In the same manner as in Example 8-1 using the corresponding alcohol, Example 8-2 below was obtained. The structural formula and the spectral data thereof were shown in Table 49.

**[Table 49]**

| Ex. No. | Strc | Physical data |
|---|---|---|
| 8-1 | 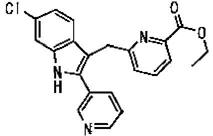 | 1H-NMR(DMSO-d6) *δ* ppm:11.68(1H, s), 9.10-9.00 (1H, m), 8.65-8.55 (1H, m), 8.50-8.40 (1H, m), 7.95-7.80 (2H, m), 7.65-7.50 (3H, m), 7.41 (1H, d, J=1.9Hz), 7.02 (1H, dd, J=8.6, 1.9Hz), 4.40-4.25 (4H, m), 1.33 (3H, t, J=7.1Hz) MS(ESI, m/z) = 392 (M+H)+ |
| 8-2 | 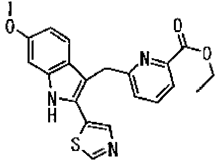 | 1H-NMR (CDCl3) *δ* ppm:8.78 (1H, s), 8.14 (1H, s), 8.11 (1H, s), 8.00-7.90 (1H, m), 7.70-7.55 (1H, m), 7.38 (1H, d, J=8.7Hz), 7.20-7.10 (1H, m), 6.88 (1H, d, J=2.2Hz), 6.77 (1H, dd, J=8.7, 2.2Hz), 4.54 (2H, s), 4.51 (2H, q, J=7.1Hz), 3.85 (3H, s), 1.47 (3H, t, J=7.1 Hz) MS(ESI, m/z) = 394 (M+H)+ |

### (Example 9)

### Ethyl 2-(6-methoxy-2-phenyl-1H-indol-3-ylmethyl)thiazole-4-carboxylate

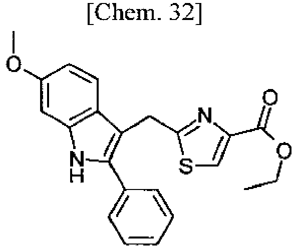

At room temperature, to a solution of ethyl 2-[hydroxy-(6-methoxy-2-phenyl-1*H-*indol-3-yl)methyl]thiazole-4-carboxylate (69.0 mg) in methylene chloride (1.7 mL) was added triethylsilane (0.135 mL) under an argon gas atmosphere. Under ice-cooling, to the mixture was added dropwise slowly a borontrifluoride ethyl ether complex (0.107 mL). The mixture was stirred for 10 minutes under the same condition and at room temperature for 15 minutes. To the reaction mixture was added a saturated aqueous sodium hydrogen carbonate solution. The organic layer was separated, and the layer was dried over anhydrous sodium sulfate and then concentrated under reduced pressure. The obtained crude product was purified by aminopropylated silica gel column chromatography (eluting solvent: hexane-ethyl acetate) to obtain the title compound (17.0 mg).

1H-NMR (CDCl3) ppm: 8.13 (1H, br s), 7.99 (1H, s), 7.60-7.30 (6H, m), 6.93 (1H, d, J=2.1Hz), 6.79 (1H, dd, J=8.7, 2.1Hz), 4.61 (2H, s), 4.45 (2H, q, J=7.2Hz), 3.87 (3H, s), 1.43 (3H, t, J=7.2Hz)
MS (ESI, m/z)=393 (M+H)+

### (Example 10)

### 6-(6-Hydroxymethyl-2-phenyl-1H-indol-3-ylmethyl)pyridine-2-carboxylic acid

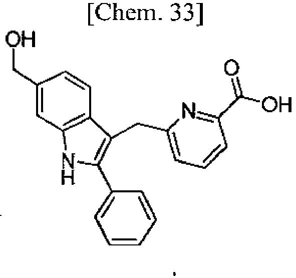

At room temperature, to a solution of ethyl 6-(6-acetoxymethyl-2-phenyl-1*H-*indol-3-ylmethyl)pyridine-2-carboxylate (66.0 mg) in tetrahydrofuran (1 mL)-ethanol (0.5 mL) was added 2 mol/L aqueous sodium hydroxide solution (0.31 mL), and the mixture was stirred at 30°C overnight. The reaction mixture was concentrated under reduced pressure. To the residue was added water, followed by dropwise addition of 2 mol/L hydrochloric acid (0.31 mL), and the mixture was stirred for 20 minutes. The precipitate was collected by filtration to obtain the title compound (41.4 mg).

1H-NMR (CDCl3) δ ppm: 11.29 (1H, s), 7.90-7.70 (411, m), 7.55-7.25 (6H, m), 6.95-6.85 (1H, m), 5.15-5.10 (111, m), 4.60-4.50 (2H, m), 4.38 (2H, s)
MS (ESI, m/z)=359(M+H)+

### (Example 11-1)

### 6-(6-Methoxy-2-thiophen-3-yl-1H-indol-3-ylmethyl)pyridine-2-carboxylic acid

To a solution of ethyl 6-(6-methoxy-2-thiophen-3-yl-1*H*-indol-3-ylmethyl)pyridine-2-carboxylate (0.187 g) in tetrahydrofuran (3.6 mL)-ethanol (1.8 mL) was added 1 mol/L aqueous sodium hydroxide solution (1.4 mL), and the mixture was stirred at 60°C for 2 hours. After cooling to room temperature, the reaction mixture was evaporated under reduced pressure. The residue was diluted with water, and then under ice-cooling, to the mixture was added 1 mol/L hydrochloric acid (1.4 mL). The precipitate was collected by filtration to obtain the title compound (0.171 g).

Further, the structural formula and the spectral data of the title compound were shown in Table 50.

In the same manner as in Example 11-1 using the corresponding esters, Examples 11-2 to 11-38 below were obtained. The structural formulae and the spectral data thereof were shown in Tables 50 to 57.

**[Table 50]**

| Ex. No. | Strc | Physical data |
|---|---|---|
| 11-1 | 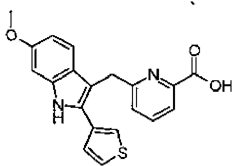 | 1H-NMR (DMSO-d6) *δ* ppm:13.40-12.80 (1H, br), 11.12 (1H, s), 8.08 (1H, dd, J=2.9, 1.1Hz), 7.90-7.75 (2H, m), 7.66 (1H, dd, J= 5.0, 2.9Hz), 7.57 : (1H, dd, J=5.0, 1.1Hz), 7.41 (1H, d, J=8.6Hz), 7.40-7.30 (1H, m), 6.85 (1H, d, J=2.2Hz), 6.64 (1H, dd, J=8.6, 2.2Hz), 4.37 (2H, s), 3.77 (3H, s) |
| | | MS (ESI, m/z) = 365 (M+H)+ |
| 11-2 | 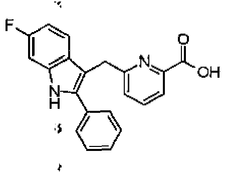 | 1H-NMR (DMSO-d6) *δ* ppm:13.50-12.50 (1H, br), 11.47 (1H, s), 7.90-7.75 (4H, m), 7.55-7.30 (5H, m), 7.12 (1H, dd, J=10.0, 2.2Hz), 6.90-6.75 (1H, m), 4.38 (2H, s) |
| | | MS (ESI, m/z) = 347 (M+H)+ |
| 11-3 | 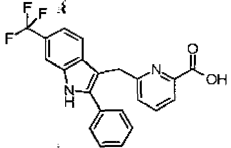 | 1H-NMR(DMSO-d6) *δ* ppm:11.86 (1H, s), 7.95-7.30 (10H, m), 7.26 (1H, dd, J=8.5, 1.2Hz), 4.42 (2H, s) |
| | | MS(ESI, m/z) = 397 (M+H)+ |
| 11-4 | 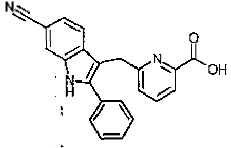 | 1H-NMR (DMSO-d6) *δ* ppm:11.99 (1H, s), 7.95-7.75 (5H, m), 7.64 (1H, d, J=8.3 Hz), 7.60-7.35 (4H, m), 7.31 (1H, dd, J=8.3, 1.2Hz), 4.41 (2H, s) |
| | | MS(ESI, m/z) = 354 (M+H)+ |
| 11-5 | 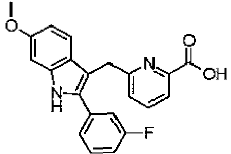 | 1H-NMR DMSO-d6) *δ* ppm:11.26 (1H, s), 7.90-7.30 (7H, m), 7.25-7.10 (1H, m), 6.86 (1H, d, J=2.2Hz), 6.65 (1H, dd, J=8.7, 2.2Hz), 4.36 (2H, s), 3.77 (3H, s) |
| | | MS (ESI, m/z) = 377 (M+H)+ |

**[Table 51]**

| Ex. No. | Strc | Physical data |
|---|---|---|
| 11-6 | 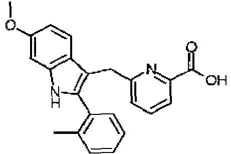 | 1H-NMR(DMSO-d6) *δ* ppm:10.99(1H, s), 7.90-7.70 (2H, m), 7.50-7.10 (6H, m), 6.82 (1H, d, J=2.2Hz), 6.59 (1H, dd, J=8.7, 2.2Hz), 4.07 (2H, s), 3.75 (3H, s), 2.22 (3H, s) |
| | | MS(ESI, m/z) = 373 (M+H)+ |
| 11-7 | 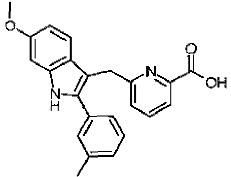 | 1H-NMR DMSO-d6) *δ* ppm:11.15 (1H, s), 7.90-7.75 (2H, m), 7.70-7.60 (1H, m), 7.55-7.45 (1H, m), 7.40-7.25 (3H, m), 7.20-7.10 (1H, m), 6.86 (1H, d, J=2.2Hz), 6.63 (1H, dd, J=8.6, 2.2Hz) 4.35 (2H, s), 3.77 (3H, s), 2.36 (3H, s) |
| | | MS(ESI, m/z) = 373(M+H)+ |
| 11-8 | 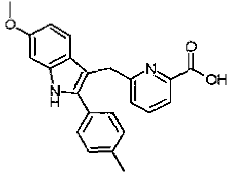 | 1H-NMR (DMSO-d6) *δ* ppm:11.13 (1H, s), 7.90-7.75 (2H, m), 7.70-7.60 (2H, m), 7.35-7.20 (4H, m), 6.86 (1H, d, J=2.2Hz), 6.61 (1H, dd, J=8.8, 2.2Hz), 4.34 (2H, s), 3.76 (3H, s), 2.34 (3H, s) |
| | | MS(ESI, m/z) = 373(M+H)+ |
| 11-9 | 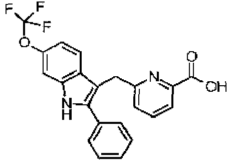 | 1H-NMR (DMSO-d6) *δ* ppm::14.00-12.50 (1H, br), 11.66 (1 H, s), 7.90-7.75 (4H, m), 7.60-7.30 (6H, m), 7.00-6.90 (1H, m), 4.39 (2H, s) |
| | | MS (ESI, m/z) = 413 (M+H)+ |
| 11-10 | 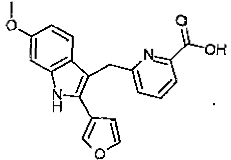 | 1H-NMR(DMSO-d6) *δ* ppm:11.04 (1H, s), 8.45-8.35 (1H, m), 7.90-7.70 (3H, m), 7.50-7.35 (2H, m), 7.05-6.90 (1H, m), 6.83 (1H, d, J=2.2Hz), 6.65 (1H, dd, J=8.7, 2.2Hz), 4.32 (2H, s), 3.77 (3H, s) |
| | | MS(ESI, m/z) = 349(M+H)+ |

**[Table 52]**

| Ex. No. | Strc | Physical data |
|---|---|---|
| 11-11 | 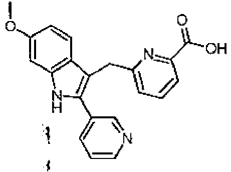 | 1H-NMR(DMSO-d6) *δ* ppm:11.34(1H, s), 9.05-8.25 (3H, m), 7.90-7.80 (2H, m), 7.55-7.35 (3H, m), 6.88 (1H, d, J=2.2Hz), 6.65 (1H, dd, J=8.7, 2.2Hz), 4.35 (2H, s), 3.78 (3H,s) |
| | | MS(ESI, m/z) = 360 (M+H)+ |
| 11-12 | 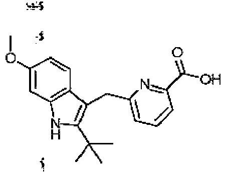 | 1H-NMR(DMSO-d6) *δ* ppm:10.45 (1H, s), 7.90-7.70 (2H, m), 7.15 (1H, d, J=8.7Hz), 7.10-7.00 (1H, m), 6.83 (1H, d, J=2.2Hz), 6.55 (1H, dd, J=8.7, 2.2Hz), 4.37 (2H, s), 3.73 (3H, s), 1.36 (9H, s) |
| | | MS(ESI, m/z) = 339 (M+H)+ |
| 11-13 | 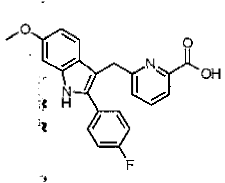 | 1H-NMR (DMSO-d6) *δ* ppm:11.18 (1H, s), 7.90-7.75 (4H, m), 7.40-7-25 (4H, m), 6.86 (1H, d, J=2.2Hz), 6.62 (1H, dd, J=8.7, 2.2Hz), 4.31 (2H, s), 3.76 (3H, s) |
| | | MS (ESI, m/z) = 377 (M+H)+ |
| 11-14 | 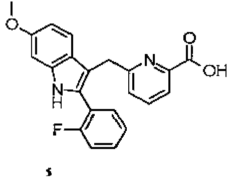 | 1H-NMR (DMSO-d6) *δ* ppm:11.15(1H, s), 7.85-7.75 (3H, m), 7.50-7.20 (5H, m), 6.87 (1H, d, J=2.2Hz), 6.61 (1H, dd, J=8.7, 2.2Hz), 4.20 (2H, s), 3.76 (3H, s) |
| | | MS (ESI, m/z) = 377 (M+H)+ |
| 11-15 | 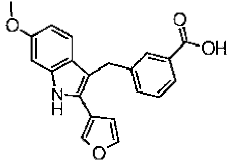 | 1H-NMR (DMSO-d6) *δ* ppm:13.00-12.60 (1H, br), 11.03(1H,s), 8.00-7.90 (1H, m), 7.80-7.65 (3H, m), 7.50-7.30 (2H, m), 7.25 (1H, d, J=8.7Hz), 6.90-6.85 (1H, m), 6.84 (1H, d, J=2.2 Hz), 6.61 (1H, dd, J=8.7, 2.2Hz), 4.24 (2H, s), 3.77 (3H, s) |
| | | MS(ESI, m/z) = 348 (M+H)+ |

**[Table 53]**

| Ex. No. | Strc | Physical data |
|---|---|---|
| 11-16 | 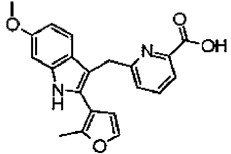 | 1H-NMR (DMSO-d6) *δ* ppm:10.85(1H, s), 7.95-7.75 (2H, m), 7.61 (1H, d, J=1.9Hz), 7.35-7.25 (1H, m), 7.21 (1H, d, J=8.7Hz), 6.84 (1H, d, J=2.2Hz), 6.77 (1H, d, J=1.9Hz), 6.58 (1H, dd, J=8.7, 2.2Hz), 4.18 (2H, s), 3.75 (3H, s), 2.34 (3H, s) |
| | | MS(ESI, m/z) = 36 |
| 11-17 | 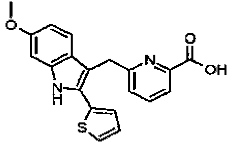 | 1H-NMR(DMSO-d6) *δ* ppm.11.24(1H, s), 7.85-7.70 (2H, m), 7.60-7.50 (2H, m), 7.32 (1H, d, J=8.7Hz), 7.25-7.10 (2H, m), 6.86 (1H, d, J=2.2Hz), 6.63 (1H, dd, J=8.7, 2.2Hz), 4.43 (2H, s), 3.77 (3H, s) |
| | | MS (ESI, m/z) = 365 (M+H)+ |
| 11-18 | 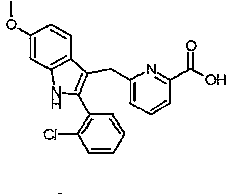 | 1H-NMR (DMSDO-d6) *δ* ppm:11.11 (1H, s), 7.85-7.70 (3H, m), 7.65-7.55 (1H, m), 7.50-7.35 (2H, m), 7.30-7.15 (2H, m), 6.84 (1H, d, J=2.2Hz), 6.61 (1H, dd, J=8.7, 2.2Hz), 4.10 (2H, s), 3.75 (3H, s) |
| | | MS(ESI, m/z) = 393(M+H)+ |
| 11-19 | 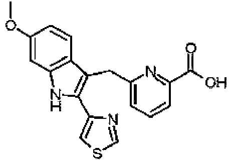 | 1H-NMR (DMSO-d6) *δ* ppm:11.28(1H, s), 9.25 (1H, d, J=1.9Hz), 8.26 (1H, d, J=1.9Hz), 7.85-7.70 (2H, m), 7.43 (1H, d, J=8.6Hz), 7.40-7.30 (1H, m), 6.89 (1H, d, J=2.2Hz), 6.64 (1H, dd, J=8.6, 2.2Hz), 4.63 (2H, s), 3.77 (3H, s) |
| | | MS(ESI, m/z) = 366(M+H)+ |
| 11-20 | 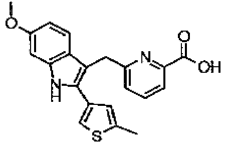 | 1H-NMR (DMSO-d6) *δ* ppm:11.04 (1H, s), 7.90-7.70 (3H, m), 7.41 (1H, d, J=8.7Hz), 7.40-7.30 (2H, m), 6.83 (1H, d, J=2.2Hz), 6.64 (1H, dd, J=8.7, 2.2Hz), 4.35 (2H, s), 3.76 (3H, s) |
| | | MS (ESI, m/z) = 379 (M+H)+ |

**[Table 54]**

| Ex. No. | Strc | Physical data |
|---|---|---|
| 11-21 | 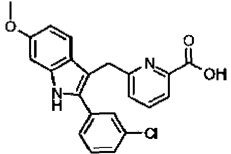 | 1H-NMR (DMSO-d6) *δ* ppm:11.27 (1H, s), 7.90-7.75 (4H, m), 7.55-7.30 (4H, m), 6.86 (1H, d, J=2.2Hz), 6.65 (1H, dd, J=8.7, 2.2Hz), 4.35 (2H, s), 3.77 (3H, s) |
| | | MS (ESI, m/z) = 393 (M+H)+ |
| 11-22 | 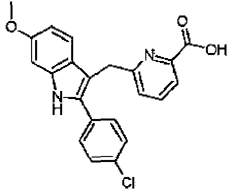 | 1H-NMR (DMSO-d6) *δ* ppm:11.25 (1H, s), 7.95-7.80 (4H, m), 7.60-7.30 (4H, m), 6.87 (1H, d, J=2.2Hz), 6.64 (1H, dd, J=8.7, 2.2Hz), 4.35 (2H, s), 3.77 (3H, s) |
| | | MS(ESI, m/z) = 393 (M+H)+ |
| 11-23 | 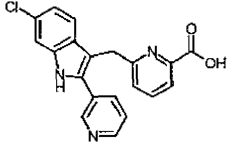 | 1H-NMR DMSO-d6) *δ* ppm:11.69 (1H, s), 9.05-8.95 (1H, m), 8.65-8.55 (1H, m), 8.45-8.30 (1H, m), 7.90-7.80 (2H, m), 7.60-7.35 (4H, m), 7.01 (1H, dd, J=8.5, 1.9Hz), 4.37 (2H, s) |
| | | MS(ESI, m/z) = 364(M+H)+ |
| 11-24 | 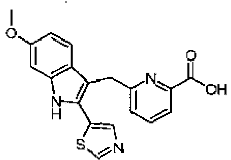 | 1H-NMR (DMSO-d6) *δ* ppm:11.39 (1H, s), 9.09 (1H, d, J=0.6Hz), 8.40 (1H, d, J=0.6Hz), 7.90-7.75 (2H, m), 7.41 (1H, d, J=8.7Hz), 7.35-7.20 (1H, m), 6.86 (1H, d, J=2.2Hz), 6.66 (1H, dd, J=8.7, 2.2Hz), 4.39 (2H, s), 3.78 (3H, s) |
| | | MS(ESI, m/z) = 366(M+H)+ |
| 11-25 | 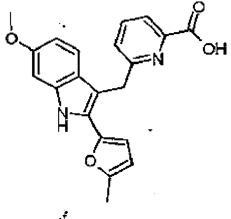 | 1H-NMR (bMSQ-d8) *δ* ppm:11.15 (1H, s), 7.90-7.70 (2H, m), 7.36 (1H, d, J=8.7Hz), 7.30-7.20 (1H, m), 6.84 (1H, d, J=2.2Hz), 6.71 (1H, d, J=3.3Hz), 6.62 (1H, dd, J=8.7, 2.2Hz), 6.20 (1H, dd, J=3.3. 1.0Hz). 4.43 (2H, s), 3.76 (3H, s), 2.33 (3H, s) |
| | | MS(ESI, m/z) = 363 (M+H)+ |

**[Table 55]**

| Ex. No. | Strc | Physical data |
|---|---|---|
| 11-26 | 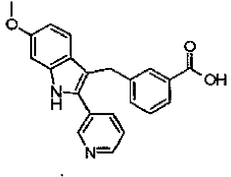 | 1H-NMR (DMSO-d6) *δ* ppm: 12.86 (1H, s), 11.35 (1H, s), 8.80-8.70 (1H, m), 8.55-8.45 (1H, m), 8.00-7.85 (1H, m), 7.80-7.65 (2H, m), 7.55-7.30 (3H, m), 7.28 (1H, d, J=8.8Hz), 6.90 (1H, d, J=2.2Hz), 6.65 (1H, dd, J=8.8, 2.2Hz), 4.29 (2H, s), 3.79 (3H, s) |
| | | MS(ESI, m/z) = 359(M+H)+ |
| 11-27 | 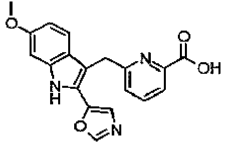 | 1H-NMR (DMSO-d6) *δ* ppm: 11.43 (1H, s), 8.49 (1H, s), 7.90-7.75 (2H, m), 7.72 (1H, s), 7.45 (1H, d, J=8.6Hz), 7.40-7.25 (1H, m), 6.95-6.60 (2H, m), 4.42 (2H, s), 3.77 (3H, s) |
| | | MS (ESI, m/z) = 350 (M+H)+ |
| 11-28 | 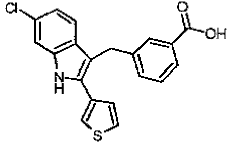 | 1H-NMR (DMSO-d6) *δ* ppm:12.85 (1H, s), 11.51 (1H, s), 7.80-7.65 (4H, m), 7.50-7.30 (5H, m), 6.97 (1H, dd, J=8^{.}5, 1.9Hz), 4.34 (2H, s) |
| | | MS(ESI, m/z) = 366 (M-H)- |
| 11-29 | 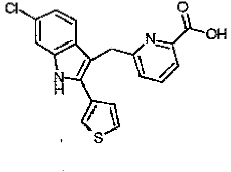 | 1H-NMR (DMSO-d6) *δ* ppm:11.50(1H, s), 8.23 (1H, dd, J=3.0, 1.3Hz), 7.95-7.75 (2H, m), 7.71 (1H, dd, J=5.50, 3.0Hz), 7.61 (1H, dd, J=5.0, 1.3Hz), 7.57 (1H, d, J=8.5Hz), 7.45-7.30 (2H, m), 7.00 (1H, dd, J=8.5, 1.9Hz), 4.40 (2H, s) |
| | | MS(ESI, m/z) = 369 (M+H)+ |
| 11-30 | 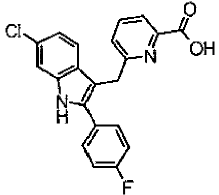 | 1H-NMR (DMSO-d6) *δ* ppm::13.30-12.70 (1H, br), 11.55 (1H, s), 8.00-7.75 (4H, m), 7.55-7.25 (5H, m), 6.98 (1H, dd, J=8.5, 1.9Hz), 4.34 (2H, s) |
| | | MS(ESI, m/z) = 381 (M+H)+ |

**[Table 56]**

| Ex. No. | Strc | Physical data |
|---|---|---|
| 11-31 | 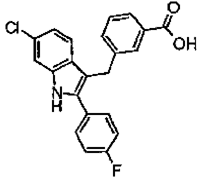 | 1H-NMR (DMSO-d6) *δ* ppm:12.86 (1H, m), 11.58 (1H, m), 7.80-7.55 (4H, m), 7.50-7.25 (6H, m), 7.05-6.95 (1H, m), 4.27 (2H, s) |
| | | MS(ESI, m/z) = 378 (M-H)- |
| 11-32 | 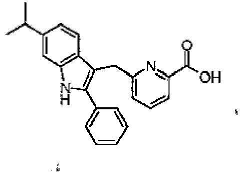 | 1H-NMR (DMSO-d6) *δ* ppm:11.21(1H, s), 7.90-7.70 (4H, m), 7.55-7.15 (6H, m), 6.88 (1H, dd, J=8.4, 1.4Hz), 4.36 (2H, s), 3.05-2.85 (1H, m), 1.24 (6H, d, J=6.9Hz) |
| | | MS(ESI, m/z) = 371(M+H)+ |
| 11-33 | 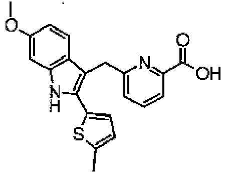 | 1H-NMR (DMSO-d6) *δ* ppm: 13.50-12.50 (1H, br), 11.16 (1H, s), 7.90-7.75 (2H, m), 7.31 (1H, d, J=3.6Hz), 7.28 (1H, d, J=8.7Hz), 7.25-7.15 (1H, m), 6.90-6.80 (2H, m), 6.61 (1H, dd, J=8.7, 2.2Hz,), 4.41 (2H, s), 3.76 (3H, s), 2.45 (3H, s) |
| | | MS (ESI, m/z) = 379 (M+H)+ |
| 11-34 | 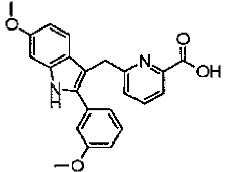 | 1H-NMR (DMSO-d6) *δ* ppm: 11.19 (1H, s), 7.90-7.75 (2H, m), 7.40-7.25 (5H, m), 6.95-6.85 (2H, m), 6.63 (1H, dd, J=8.7, 2.2Hz), 4.37 (2H, s), 3.77 (3H, s), 3.76 (3H, s) |
| | | MS (ESI, m/z) = 389 (M+H)+ |
| 11-35 | 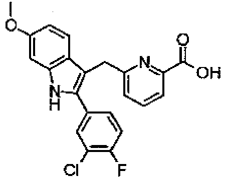 | 1H-NMR (DMSO-d6) *δ* ppm:13.50-12.50 (1H, br), 11.27 (1H, s), 8.15 (1H, dd, J=2.2, 7.0Hz), 7.90-7.80 (3H, m), 7.55-7.45 (2H, m), 7.40 (1H, d, J=8.6Hz), 6.85 (1H, d, J=2.2Hz), 6.65 (1H, dd, J=2.2, 8.6Hz), 4.31 (2H, s), 3.77 (3H, s) |
| | | MS(ESI, m/z) = 411 (M+H)+ |

**[Table 57]**

| Ex. No. | Strc | Physical data |
|---|---|---|
| 11-36 | 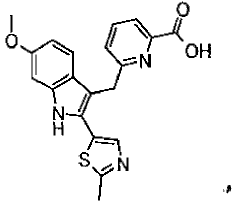 | 1H-NMR (DMSO-d6) *δ* ppm:11.32 (1H, s), 8.12 (1H, s), 7.90-7.70 (2H, m), 7.37 (1H, d, J=8.8Hz), 7.30-7.20 (1H, m), 6.84 (1H, d, J=2.2Hz), 6.64 (1H, dd, J=8.8, 2.2Hz), 4.37 (2H, s), 3.77 (3H, s), 2.67 (3H, s) |
| | | MS(ESI, m/z) = 380 (M+H)+ |
| 11-37 | 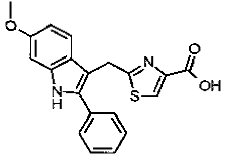 | 1H-NMR (DMSO-d6) *δ* ppm:13,20-12,80 (1H, br), 11.32 (1H, s), 8.16 (1H, s), 7.65-7.55 (2H, m), 7.55-7.45 (2H, m), 7.40-7.30 (2H, m), 6.90 (1H, d, J=2.2Hz), 6.68 (1H, dd, J=2.2, 8.8Hz), 4.51 (2H,s), 3.79(3H,s) |
| | | MS(ESI, m/z) = 365 (M+H)+ |
| 11-38 | 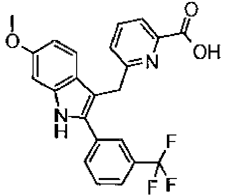 | 1H-NMR (DMSO-d6) *δ* ppm:11.39 (1H, s), 8.25-8.05 (2H, m), 7.90-7.80 (2H, m), 7.75-7.60 (2H, m), 7.50-7.35 (2H, m), 6.89 (1H, d, J=2.2Hz), 6.67 (1H, dd, J=8.7, 2.2Hz), 4.37 (2H, s), 3.78 (3H, s) |
| | | MS(ESI, m/z) = 427 (M+H)+ |

### (Example 12)

In the same manner as in Example 1-1 using the corresponding ketones, Examples 12-1 to 12-34 below were obtained. The structural formulae and the physical property values thereof were shown in Tables 94 to 99.

### (Example 13)

In the same manner as in Example 2-1 using the corresponding indole derivatives, Examples 13-1 to 13-35 below were obtained. The structural formulae and the physical property values thereof were shown in Tables 100 to 106.

### (Example 14-1)

### Ethyl 6-(5-fluoro-6-methoxy-2-pyridin-3-yl-1H-indol-3-ylmethyl)pyridine-2-carboxylate

Under ice-cooling, to a solution of ethyl 6-[(5-fluoro-6-methoxy-2-pyridin-3-yl-1*H*-indol-3-yl)hydroxymethyl]pyridine-2-carboxylate (81.0 mg) and triethylsilane (0.120 mL) in methylene chloride (1.9 mL) was added slowly trifluoroacetic acid (0.059 mL). The mixture was stirred at room temperature for 14 hours. To the reaction mixture was added a saturated aqueous sodium hydrogen carbonate solution, and the organic layer was separated. The layer was dried over anhydrous sodium sulfate and then concentrated under reduced pressure. The crude product was purified by aminopropylated silica gel column chromatography (eluting solvent: methylene chloride-methanol) to obtain the title compound (70.5 mg).

Further, the structural formula and the spectral data of the title compound were shown in Table 106.

In the same manner as in Example 14-1 using the corresponding starting materials, Examples 14-2 to 14-15 below were obtained. The structural formulae and the physical property values thereof were shown in Tables 106 to 108.

### (Example 15-1)

### Methyl 6-(6-methoxy-5-methyl-2-phenyl-1H-indol-3-ylmethyl)pyridine-2-carboxylate

Under ice-cooling, to a suspension of sodium iodide in acetonitrile (0.30 mL) was added triethylsilane (0.063 mL) under an argon gas atmosphere, and the mixture was stirred for 5 minutes under the same condition. To the mixture was added a suspension of methyl 6-[hydroxy-(6-methoxy-5-methyl-2-phenyl-1*H*-indol-3-yl)methyl]pyridine-2-carboxylate (50.0 mg) in acetonitrile (1 mL), and the mixture was stirred for 1 hour under the same condition. Under ice-cooling, to the mixture were added ethyl acetate and water, then sodium hydrogen carbonate. The organic layer was separated and the layer was washed with 9% aqueous sodium thiosulfate solution and saturated brine successively, dried over anhydrous magnesium sulfate, and then concentrated under reduced pressure. The obtained crude product was purified by silica gel column chromatography (eluting solvent: hexane-ethyl acetate) to obtain the title compound (40.9 mg).

Further, the structural formula and the spectral data of the title compound were shown in Table 109.

In the same manner as in Example 15-1 using the corresponding starting materials, Examples 15-2 to 15-25 below were obtained. The structural formulae and the physical property values thereof were shown in Tables 109 to 113.

### (Example 16)

In the same manner as in Example 4-1 using the corresponding esters, Examples 16-1 to 16-109 below were obtained. The structural formulae and the physical property values thereof were shown in Tables 114 to 135.

### (Example 17)

In the same manner as in Example 2-1 using the corresponding starting materials, Examples 17-1 to 17-2 below were obtained. The structural formulae and the physical property values thereof were shown in Table 136.

### (Example 18)

In the same manner as in Example 4-1 using the corresponding starting materials, Examples 18-1 to 18-2 below were obtained. The structural formulae and the physical property values thereof were shown in Table 136.

**[Table 58]**

| Ref.No. | Strc | physical data |
|---|---|---|
| 19 | 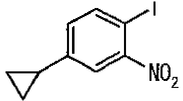 | 1H-NMR (CDCl3) *δ* ppm:7.86 (1H, d, J=8.2Hz), 7.54 (1H, d, J=2.1 Hz), 6.96 (1H, dd, J=8.2, 2.1 Hz), 2.00-1.85 (1H, m), 1.15-1.00 (2H, m), 0.85-0.65 (2H, m) |
| 20 | 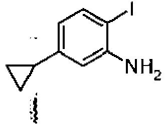 | 1H-N MR (CDCl3) *δ* ppm:7.47 (1H, d, J=8.2Hz), 6.48 (1H, d, J=2.1 Hz), 6.21 (1H, dd, J=8.2, 2.1 Hz), 4.15-3.85 (2H, br), 1.85-1.70 (1H, m), 1.00-0.85 (2H, m), 0.70-0.55 (2H, m) |
| 21 | 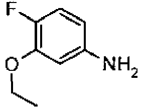 | 1H-NMR (CDCl3) *δ* ppm:6.85 (1H, dd, J=11.5, 8.6Hz), 6.31 (1H, dd, J=7.1, 2.7Hz), 6.25-6.10 (1H, m), 4.05 (2H, q, J=7.0Hz), 3.75-3.30 (2H, br), 1.43 (3H, t, J=7.0Hz) |
| 22-1 | 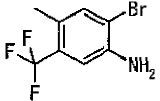 | 1H-NMR (CDCl3) *δ* ppm:7.33 (1H, s), 7,01 (1H, s), 4.60-3.80 (2H, br), 2.48 (3H, d, J=1.5Hz) |
| 22-2 | 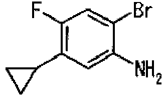 | 1H-NMR (CDCl3) δ ppm: 7.08 (1H, d, J = 9.4Hz), 6.28 (1H, d, J = 7.0Hz), 4.40-3.20 (2H, br), 2.05-1.90 (1H, m), 1.00-0.90 (2H, m), 0.70-0.60 (2H, m) |
| 22-3 | 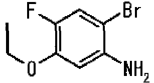 | 1H-N MR (CDCl3) *δ* ppm: 7.14 (1H, d, J = 10.6 Hz), 6.39 (1H, d, J = 7.7Hz), 4.03 (2H, q, J = 7.0Hz), 4.10-3.65 (2H, br), 1.43 (3H, t, J =7.0Hz) |
| 22-4 | 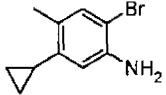 | 1H-NMR (CDCl3) *δ* ppm:7.17 (1H, d, J=0.5Hz), 6.41 (1H, s), 4.00-3.90 (2H, br), 2.28 (3H, s), 1.85-1.70 (1H, m), 0.95-0.80 (2H, m), 0.60-0.50 (2H, m) |

**[Table 59]**

| Ref.No. | Strc | physical data |
|---|---|---|
| 23-1 | 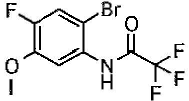 | 1H-NMR (CDCl3) *δ* ppm:8.45-8.25 (1H, br), 8.10 (1H, d, J=8.3Hz), 7.34 (1H, d, J=10.0Hz), 3.93 (3H, s) |
| 23-2 | 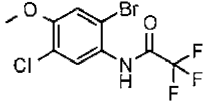 | 1H-N MR (CDCl3) *δ* ppm:8.33 (1H, s), 8.25-8.10 (1H, br), 7.14 (1 H, s), 3.92 (3H, s) |
| 23-3 | 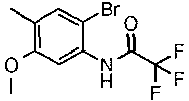 | 1H-NMR (CDCl3) *δ* ppm:8.50-8.30 (1H, br), 7.91 (1 H, s), 7.32 (1H, d, J=0.7Hz), 3.86 (3H, s), 2.19 (3H, s) |
| 23-4 | 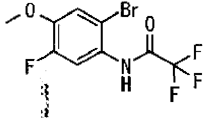 | 1H-NMR (CDCl3) *δ* ppm:8.40-8.20 (1H, br), 8.15 (1H, d, J=12.5Hz). 7.18 (1H, d, J=8.2Hz), 3.90 (3H, s) |
| 23-5 | 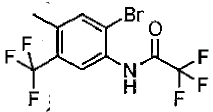 | 1H-NMR (CDCl3) *δ* ppm:8.58 (1H, s), 8.45-8.30 (1H, br), 7.57 (1H, s), 2.48 (3H, d, J=1.5Hz) |
| 23-6 | 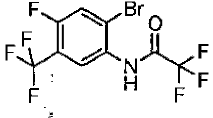 | 1H-N MR (CDCl3) *δ* ppm:8.62 (1H, d, J=6.8Hz), 8.50-8.25 (1H, br), 7.54 (1H, d, J=9.0Hz) |
| 23-7 | 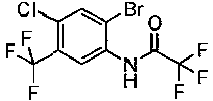 | 1H-N MR (CDCl3) *δ* ppm:8.72 (1H, s), 8.50-8.35 (1H, br), 7.81 (1H,d, J=0.5Hz) |
| 23-8 | 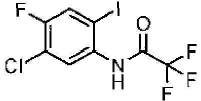 | 1H-N MR (CDCl3) *δ* ppm:8.46 (1H, d, J=7.1Hz), 8.40-8.20 (1H, br), 7.45 (1H, d, J=7.9Hz) |

**[Table 60]**

| Ref.No. | Strc | physical data |
|---|---|---|
| 23-9 | 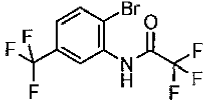 | 1H-NMR (CDCl3) *δ* ppm:8.66 (1H, dd, J=1.6Hz), 8.60-8.40 (1H, br), 7.76 (1H, d, J=8.4Hz), 7.39 (1H, dd, J=8.4, 1.6Hz) |
| 23-10 | 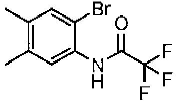 | 1H-NMR (CDCl3) *δ* ppm:8.25-8.05 (1H, br), 7.95 (1H, s), 7.59 (1H, s), 2.26 (3H, s), 2.22 (3H, s) |
| 23-11 | 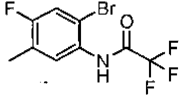 | 1H-NMR (CDCl3) *δ* ppm:8.40-8.20 (1H, br), 8.14 (1H, d, J=7.3Hz), 7.29 (1H, d, J=8.6Hz), 2.28 (3H, d, J=2.0Hz) |
| 23-12 | 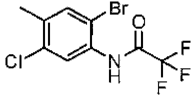 | 1H-NMR (CDCl3) *δ* ppm:8.45-8.25 (2H, m), 7.48 (1 H, s), 2.36 (3H, s) |
| 23-13 | 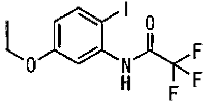 | 1H-NMR (CDCl3) *δ* ppm: 8.35-8.15 (1H, br), 7.91 (1H, d, J = 2.9Hz), 7.66 (1H, d, J = 8.8Hz), 6.59 (1H, dd, J = 8.8, 2.9Hz), 4.05 (2H, q, J = 7.0Hz), 1.42 (3H, t, J = 7.0Hz) |
| 23-14 | 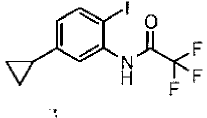 | 1H-NMR (CDCl3) *δ* ppm: 8.35-8.10 (1H, br), 7.95 (1H, d, J=2.1Hz), 7.67 (1H, d, J=8.3Hz), 6.69 (1H, dd, J = 8.3, 2.1Hz), 1.95-1.80 (1H, m), 1.10-0.95 (2H, m), 0.80-0.65 (2H, m) |
| 23-15 | 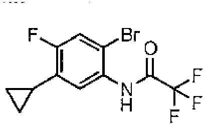 | 1H-NMR (CDCl3) *δ* ppm: 8.40-8.10 (1H, br), 7.84 (1H, d, J = 7.2Hz), 7.28 (1H, d, J = 9.1Hz), 2.15-2.00 (1H, m), 1.10-0.95 (2H, m), 0.85-0.70 (2H, m) |

**[Table 61]**

| Ref.No. | Strc | physical data |
|---|---|---|
| 23-16 | 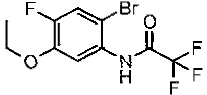 | 1H-NMR (CDCl3) *δ* ppm: 8.45-8.20 (1H, br), 8.07 (1H, d, J = 8.2Hz), 7.33 (1H, d, J = 10.0Hz), 4.14 (2H, q, J = 7.0Hz), 1.47 (3H, t, J = 7.0Hz) |
| 23-17 | 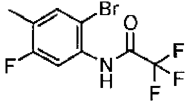 | 1H-NMR (CDCl3) *δ* ppm:8.50-8.30 (1H, br), 8.10 (1H, d, J=11.0Hz), 7.43 (1H, d, J=7.4Hz), 2.27 (1H, d, J=1.8Hz) |
| 23-18 | 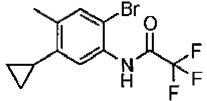 | 1H-NMR (CDCl3) *δ* ppm:8.40-8.25 (1H, br), 7.89 (1H, s), 7.36 (1H, d, J=0.5Hz), 2.39 (3H, s), 1.90-1.75 (1H, m), 1.05-0.90 (2H, m), 0.75-0.60 (2H, m) |
| 24-1 | 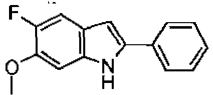 | 1H-NMR (CDCl3) *δ* ppm:8.30-8.15 (1H, br, 7.65-7.55 (2H, m), 7.50-7.40 (2H, m), 7.35-7.20 (2H, m), 6.97 (1H, d, J=7.3 Hz), 6.73 (1H, dd, J=2.1, 0.7Hz), 3.94 (3H, s) |
| 24-2 | 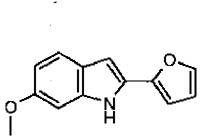 | 1H-NMR (CDCl3) *δ* ppm:8.45-8.25 (1H, br), 7.47 (1H, d, J=8.7Hz), 7.44 (1H, d, J=1.8, 0.5Hz), 6.87 (1H. d, J=2.2Hz), 6.77 (1H, dd, J=8.7, 2.2Hz), 6.67 (1H, dd, J=2.1, 0.8Hz), 6.56 (1H, dd, J=3.4, 0.5Hz), 6.49 (1H, dd, J=3.4, 1.8Hz), 3.86 (3H, s) |
| 24-3 | 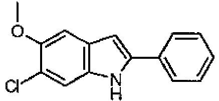 | 1H-NMR (CDCl3) *δ* ppm:8.35-8.15 (1H, br), 7.70-7.60 (2H, m), 7.50-7.25 (4H, m), 7.12 (1H, s), 6.74 (1H, dd, J=2.0, 0.8Hz), 3.94 (3H, s) |
| 24-4 | 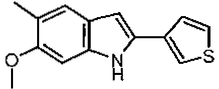 | 1H-NMR (CDCl3) *δ* ppm:8.15-7.95 (1H, br), 7.45-7.25 (4H, m), 6.83 (1H, s), 6.57 (1H, d, J=1.8Hz), 3.87 (3H, s), 2.30 (3H, s) |

**[Table 62]**

| Ref.No. | Strc | physical data |
|---|---|---|
| 24-5 | 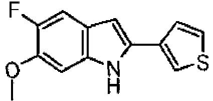 | 1H-NMR (CDCl3) *δ* ppm:8.25-8.05 (1H, br), 7.45-7.30 (3H, m), 7.28 (1H, d, J=11.4Hz), 6.94 (1H, d, J=7.0Hz), 6.60 (1H, d, J=1.9Hz), 3.93 (3H, s) |
| 24-6 | 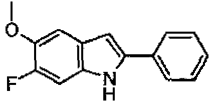 | 1H-NMR (CDCl3) *δ* ppm:8.35-8.15 (1H, br), 7.70-7.55 (2H, m), 7.50-7.25 (3H, m), 7.20-7.10 (2H, m), 6.74 (1H, dd, J=2.1, 0.6Hz), 3.94 (3H, s) |
| 24-7 | 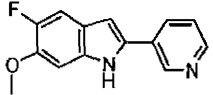 | 1H-NMR (CDCl3) *δ* ppm:8.95-8.90 (1H, m), 8.89-8.50 (1H, m), 8.45-8.30 (1H, br), 7.95-7.85 (1H, m), 7.40-7.25 (2H, m), 6.99 (1H, d, J=7.1Hz), 6.80 (1H, dd, J=.2, 0.7Hz), 3.95 (3H, s) |
| 24-8 | 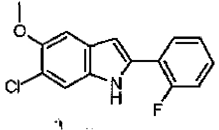 | 1H-NMR (CDCl3) *δ* ppm:8.90-8.60 (1H, br), 7.85-7.70 (1 H, m), 7.45 (1H, d, J=0.7Hz), 7.35-7.10 (4H, m), 6.86 (1H, d, J=2.1Hz), 3.95 (3H, s) |
| 24-9 | 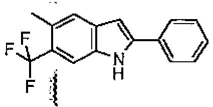 | 1H-NMR (CDCl3) *δ* ppm:8.55-8.30 (1H, br), 7.75-7.60 (3H, m), 7.55-7.30 (4H, m), 6.80 (1H, d, J=2.1, 0.8Hz), 2.56 (3H, d, J=0.9Hz) |
| 24-10 | 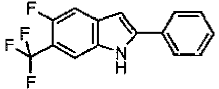 | 1H-NMR (CDCl3) *δ* ppm:8.70-8.45 (1H, br), 7.80-6.75 (8H, m) |
| 24-11 | 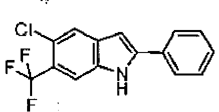 | 1H-NMR (CDCl3) *δ* ppm:8.80-8.45 (1H, br), 7.75-7.60 (4H, m), 7.55-7.35 (3H, m), 6.86 (1H, dd, J=2.1, 0.8Hz) |
| 24-12 | 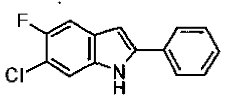 | 1H-NMR (CDCl3) *δ* ppm:8.40-8.20 (1H, br), 7.70-7.60 (2H, m), 7.50-7.30 (5H, m), 6.76 (1H, dd, J=2.2, 0.9Hz) |

**[Table 63]**

| Ref.No. | Strc | physical data |
|---|---|---|
| 24-13 | 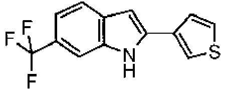 | 1H-NMR (CDCl3) *δ* ppm:8.60-8.40 (1H, br), 7.70-7.60 (2H, m), 7.51 (1H, dd, J=2.8, 1.5Hz), 7.50-7.40 (2H, m), 7.34 (1H, dd, J=8.4, 1.2Hz), 6.75 (1H, dd, J=2.1, 0.8Hz) |
| 24-14 | 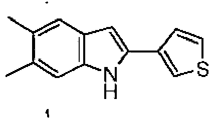 | 1H-NMR (CDCl3) *δ* ppm::8.10-7.90 (1H, br), 7.45-7.30 (4H, m), 7.15 (1H, s), 6.59 (1H, dd, J=2.1, 0.8Hz), 2.36 (3H, s), 2.34 (3H, s) |
| 24-15 | 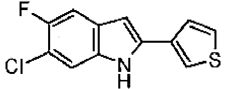 | 1H-NMR (CDCl3) *δ* ppm: 8.30-8.10 (1H, br), 7.50-7.35 (4H, m), 7.32 (1H, d, J=9.6Hz), 6.64 (1H, dd, J=2.1, 0.9Hz) |
| 24-16 | 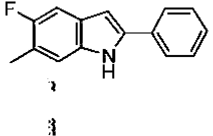 | 1H-NMR (CDCl3) *δ* ppm: 8.35-8.05 (1H, br), 7.70-7.60 (2H, m), 7.50-7.40 (2H, m), 7.35-7.25 (1H, m), 7.21 (1H, d, J=10.3Hz), 7.16 (1H, d, J=6.3Hz), 6.74 (1H, dd, J=2.1, 0.8Hz), 2.38 (3H, d,J=2.1Hz) |
| 24-17 | 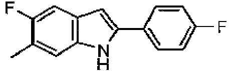 | 1H-NMR (CDCl3) *δ* ppm: 8.25-8.00 (1H, br), 7.65-7.55 (2H, m), 7.25-7.05 (4H, m), 6.67 (1H, dd, J=2.1, 0.7Hz), 2.38 (3H, d, J=2.0Hz) |
| 24-18 | 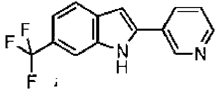 | 1H-NMR (CDCl3) *δ* ppm:9.10-8.75 (2H, m), 8.70-8.55 (1H, m), 8.05-7.90 (1H, m), 7.80-7.65 (2H, m), 7.50-7.35 (2H, m), 6.95 (1H, dd, J=2.2, 0.8Hz) |
| 24-19 | 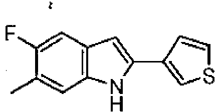 | 1H-NMR (CDCl3) *δ* ppm: 8.25-7.90 (1H, br), 7.45-7.35 (3H, m), 7.19 (1H, d, J=18.5Hz), 7.13 (1H, d, J=6.3Hz), 6.62 (1H, dd, J=2.2, 0.8Hz), 2.38 (3H, d, J=1.9Hz) |

**[Table 64]**

| Ref.No. | Strc | physical data |
|---|---|---|
| 24-20 | 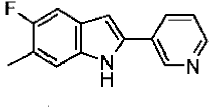 | 1H-NMR (CDCl3) δ ppm: 8.95-8.90 (1H, m), 8.60-8.50 (1H, m), 8.45-8.20 (1H, br), 7.95-7.85 (1H, m), 7.40-7.30 (1H, m), 7.24 (1H, d, J=10.2Hz), 7.19 (1H, d, J=6.4Hz), 6.81 (1H, dd, J=2.1, 0.7Hz), 2.39 (3H, d, J=2.0HZ) |
| 24-21 | 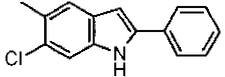 | 1H-NMR (CDCl3) δ ppm::8.30-8.15 (1H, br), 7.70-7.60 (2H, m), 7.50-7.30 (5H, m), 6.73 (1H, dd, J=2.1, 0.9Hz), 2.45 (3H, s) |
| 24-22 | 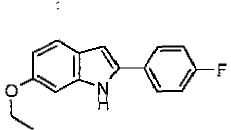 | 1H-NMR (CDCl3) δ ppm: 8.20-8.00 (1H, br), 7.65-7.50 (2H, m), 7.48 (1H, d, J=8.7Hz), 7.20-7.15 (2H, m), 6.89 (1H, d, J=2.2Hz), 6.79 (1H, dd, J=8.7, 2.2Hz), 6.68 (1H, d, J=2.0Hz), 4.08 (2H, q, J=7.0Hz), 1.45 (3H, t, J=7.0Hz) |
| 24-23 | 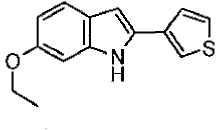 | 1H-NMR (CDCl3) δ ppm: 8.20-7.95 (1H, br), 7.50-7.30 (4H, m), 6.87 (1H, d, J=2.2Hz), 6.78 (1H, dd, J=8.8, 2.2Hz), 6.62 (1H, dd, J=2.0, 0.9Hz), 4.08 (2H, q, J=7.0Hz), 1.45 (3H, t, J=7.0Hz) |
| 24-24 | 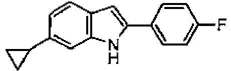 | 1H-NMR (CDCl3) δ ppm: 8.25-8.00 (1H, br), 7.65-7.55 (2H, m), 7.49 (1H, d, J=8.2Hz), 7.20-7.05 (3H, m), 6.88 (1H, dd, J=8.2, 1.5 Hz), 6.69 (1H, dd, J=2.1, 0.9Hz), 2.10-1.95 (1H, m), 1.05-0.90 (2H, m), 0.80-0.65 (2H, m) |
| 24-25 | 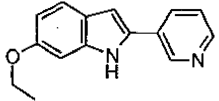 | 1H-NMR (CDCl3) δ ppm: 8.95-8.85 (1H, m), 8.60-8.45 (1H, m), 8.40-8.20 (1H, br), 7.95-7.85 (1H, m), 7.51 (1H, d, J=8.7Hz), 7.40-7.30 (1H, m), 6.91 (1H,d, J=2.1 Hz), 6.85-6.75 (2H, m), 4.09 (2H, q, J=7.0Hz). 1.46 (3H, t, J=7.0Hz) |

**[Table 65]**

| Ref.No. | Strc | Physical data |
|---|---|---|
| 24-26 | 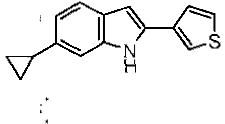 | 1H-NMR (CDCl3) δ ppm: 8.20-7.95 (1H, br), 7.48 (1H, d, J=8.2Hz), 7.45-7.30 (3H, m), 7.15-7.05 (1H, m), 6.87 (1H, dd, J=8.2, 1.5Hz), 6 70-6.60 (1H, m), 2.10-1.95 (1H, m), 1.05-0.90 (2H, m), 0.80-0.65 (2H, m) |
| 24-27 | 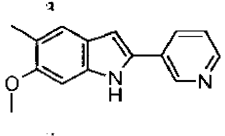 | 1H-NMR (CDCl3) δ ppm:8.95-8.85 (1H, m), 8.55-8.45 (1H, m), 8.30-8.15 (1H, br), 7.95-7.85 (1H, m), 7.40-7.30 (2H, m), 6.87 (1H, s), 6.77 (1H, dd, J=2.1, 0.8Hz), 3.89 (3H, s), 2.31 (3H, d, J=0.5Hz) |
| 24-28 | 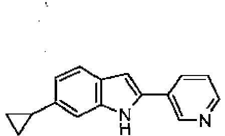 | 1H-NMR (CDCl3) δ ppm: 9.00-8.90 (1H,m), 8.60-8.55 (1H, m), 8.45-8.25 (1H, br), 7.95-7.85 (1H, m), 7.53 (1H, d, J=8.3Hz), 7.40-7.30 (1H, m), 7.20-7.10 (1H, m), 6.91 (1H, dd, J=8.3, 1.5Hz), 6.84 (1H, dd, J=2.1, 0.8Hz), 2.10-1.95 (1H, m), 1.05-0.90 (2H, m), |
| 24-29 | 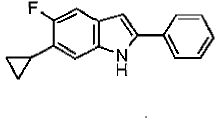 | 1H-NMR (CDCl3) δ ppm: 8.30-8.10 (1H, br), 7.70-7.55 (2H, m), 7.50-7.25(3H, m), 7.22 (1H, d, J=10.9Hz), 6.94 (1H, d, J=6.3Hz), 6.74 (1H, dd, J=2.2, 0.9Hz), 2.25-2.10 (1H, m), 1.05-0.90 (2H, m), 0.80-0.65 (2H, m) |
| 24-30 | 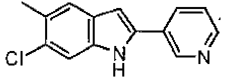 | 1H-NMR (CDCl3) δ ppm:9.00-8.90 (1H, m), 8.60-8.50 (1H, m), 8.40-8.30 (1H, br), 8.00-7.85 (1H, m), 7.55-7.35 (3H, m), 6.85-6.75 (1H, m), 2.46 (3H, s) |
| 24-31 | 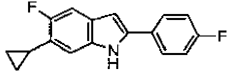 | 1H-NMR (CDCl3) δ ppm: 8.25-8.00 (1H, br), 7.65-7.50 (2H, m), 7.25-7.05 (3H, m), 6.93 (1H, d, J=6.2Hz), 6.63 (1H, d, J=2.2, 0.8Hz), 2.25-2.10 (1H, m), 1.05-0.90 (2H, m), 0.80-0.65 (2H, m) |

**[Table 66]**

| Ref.No. | Strc | physical data |
|---|---|---|
| 24-32 | 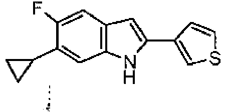 | 1H-NMR (CDCl3) δ ppm: 8.20-7.95 (1H, br), 7.45-7.35 (3H, m), 7.20 (1H, d, J=10.8Hz), 6.91 (1H, d, J=6.2Hz), 6.65-6.55 (1H, m), 2.25-2.10 (1H, m), 1.05-0.90 (2H, m), 0.80-0.65 (2H, m) |
| 24-33 | 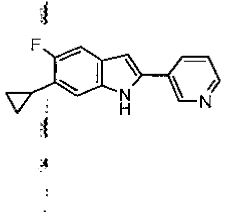 | 1H-NMR (CDCl3) δ ppm: 8.95-8.90 (1H, m), 8.60-8.50 (1H, m), 8.45-8.25 (1H, br), 7.95-7.85 (1H, m), 7.40-7.30 (1H, m), 7.24 (1H, d, J=10.7Hz). 6.96 (1H, d, J=6.2Hz), 6.81 (1 H, dd, J=2.2, 0.8Hz), 2.25-2.10 (1H, m), 1.10-0.90 (2H, m), 0.80-0.65 (2H, m) |
| 24-34 | 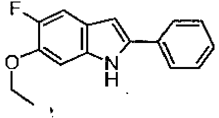 | 1H-NMR (CDCl3) δ ppm: 8.35-8.10 (1H, br), 7.65-7.55 (2H, m), 7.50-7.40 (2H, m), 7.35-7.25 (2H, m), 6.97 (1H, d, J=7.0Hz), 6.72 (1H, dd, J=2.2, 0.7Hz), 4.14 (2H, q, J=7.0Hz), 1.50 (3H, t, J =7.0 Hz) |
| 24-35 | 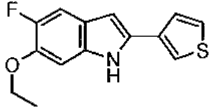 | 1H-NMR (CDCl3) δ ppm: 8.20-8.00 (1H, br), 7.45-7.30 (3H, m), 7.26 (1H, d, J=11.6Hz), 6.94 (1H, d, J=7.1 Hz), 6.60 (1H, d, J=2.1 Hz), 4.1 3 (2H, q, J=7.0Hz), 1.49 (3H, t, J=7.0Hz) |
| 24-36 | 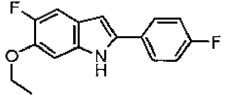 | 1H-NMR (CDCl3) δ ppm: 8.25-8.05 (1H, br), 7.65-7.50 (2H, m), 7.35-7.25 (1H, m), 7.20-7.05 (2H, m), 6.95 (1H, d, J=7.1Hz), 6.65 (1H, d, J=2.2Hz), 4.13 (2H, q, J=7.0Hz), 1.49 (3H, t, J=7.0Hz) |
| 24-37 | 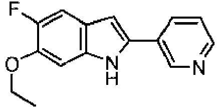 | 1H-NMR (CDCl3) δ ppm: 8.95-8.85 (1H, m), 8.60-8.50 (1H, m), 8.45-8.30 (1H, br), 7.95-7.85 (1H, m), 740-7.25 (2H, m), 6.98 (1H, d, J=7.0Hz), 6.79 (1H, d, J=2.2Hz), 4.14 (2H, q, J=7.0Hz), 1.50 (3H, t, J=7.0Hz) |

**[Table 67]**

| Ref. No. | Strc | physical data |
|---|---|---|
| 24-38 | 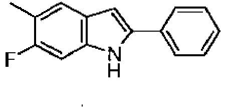 | 1H-NMR (CDCl3) δ ppm:8.35-8.15 (1H, br), 7.70-7.55 (2H, m), 7.50-7.40 (2H, m), 7.37 (1H, d, J=7.6Hz), 7.35-7.25 (1H, m), 7.05 (1H, d, J=10.5Hz), 6.73 (1H, dd, J=2.3, 0.9Hz), 2.36 (3H, dd, J=2.2, 0.6Hz) |
| 24-39 | 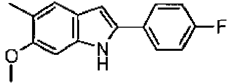 | 1H-NMR (CDCl3) δ ppm:8.20-8.00 (1H, br), 7.65-7.50 (2H, m), 7.34 (1H, s), 7.20-7.05 (2H, m), 6.84 (1H, s), 6.63 (1H, d, J=2.0Hz), 3.88 (3H, s), 2.30 (3H, s) |
| 24-40 | 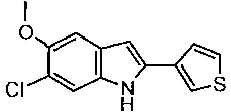 | 1H-NMR (CDCl3) δ ppm:8.20-8.05 (1H, br), 7.45-7.35 (4H, m), 7.10 (1H, s), 6.62 (1H, dd, J=2.2, 0.9Hz), 3.94 (3H, s) |
| 24-41 | 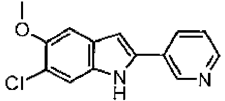 | MS(ESI, m/z) = 259 (M+H)+ |
| 24-42 | 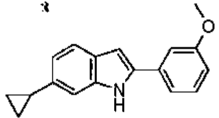 | 1H-NMR (CDCl₃) δ ppm:8.30-8.10 (1H, br), 7.55-7.45 (1H, m), 7.40-7.30 (1H, m), 7.25-7.15 (2H, m), 7.12 (1H, s), 6.95-6.80 (2H, m), 6.77 (1H, dd, J=2.2, 0.9Hz), 3.88 (3H, s), 2.10-1.95 (1H, m), 1.05-0.90 (2H, m), 0.80-0.75 (2H, m) |
| 24-43 | 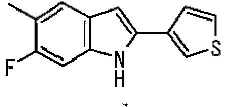 | 1H-NMR (CDCl3) δ ppm:8.20-8.05 (1H, br), 7.50-7.30 (4H, m), 7.03 (1H, d, J=10.2Hz), 6.60 (1H, dd, J=2.2, 0.9 Hz), 2.35 (3H, dd, J=2.2, 0.6Hz) |
| 24-44 | 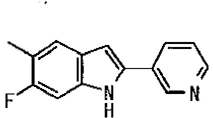 | 1H-NMR (CDCl3) δ ppm:8.95-8.90 (1H, m), 8.60-8.50 (1H, m), 8.45-8.25 (1H, br), 7.95-7.85 (1H, m), 7.45-7.30 (2H, m), 7.08 (1H, d, J=10.1Hz), 6.80 (1H, dd, J=2.2, 0.9Hz), 2.37 (3H, dd, J=2.2, 0.7Hz) |

**[Table 68]**

| Ref.No. | Strc | physical data |
|---|---|---|
| 24-45 | 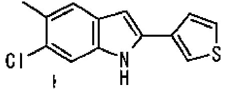 | 1H-NMR (CDCl3) δ ppm:8.20-8.05 (1H, br), 7.45-7.35 (5H, m), 6.60 (1H, dd, J =2.1, 0.8,Hz), 2.45 (3H, d, J=0.5Hz) |
| 24-46 | 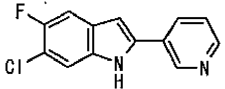 | 1H-NMR (CDCl3) δ ppm:8.95 (1H, s), 8.65-8.40 (2H. m), 8.00-7.85 (1H, m), 7.50-7.30 (3H, m), 6.84 (1H, d, J=1.6Hz) |
| 24-47 | 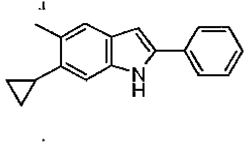 | 1H-NMR (CDCl3) δ ppm:8.25-8.05 (1H, br), 7.70-7.55 (2H, m), 7.50-7.20 (4H, m), 7.08 (1H, s), 6.72 (1H, dd, J=2.1, 0.9 Hz), 2.51 (3H, s), 2.05-1.90 (1H, m), 1.05-0.90 (2H, m), 0.75-0.60 (2H, m) |
| 24-48 | 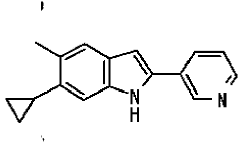 | 1H-NMR (CDCl3) δ ppm:8.95-8.85 (1H, m), 8.60-8.45 (1H, m), 8.30-8.15 (1H, br), 7.95-7.85 (1H, m), 7.41 (1H, s), 7.40-7.30 (1H, m), 7.10 (1H,s). 6.79 (1H, dd, J =2.1, 0.9Hz), 2.52 (3H, dd, J=0.5Hz), 2.05-1.90 (1H, m), 1.05-0.90 (2H, m), 0.75-0.60 (2H, m) |
| 24-49 | 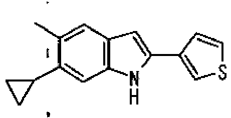 | 1H-NMR (CDCl3) δ ppm:8.20-8.05 (1H, br), 7.45-7.30 (4H, m), 7.06 (1H, s), 6.59 (1H, dd, J=2.1, 0.9Hz), 2.50 (3H, d, J=0.5Hz), 2.05-1.90 (1 H, m), 1.00-0.90 (2H, m), 0.70-0.60 (2H, m) |

**[Table 69]**

| Ref.No. | Strc | physical data |
|---|---|---|
| 25-1 | 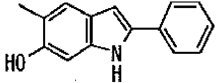 | 1H-NMR (CDCl3) δ ppm:8.12 (1H, s), 7.65-7.50 (2H, m), 7.50-7.20 (4H, m), 6.84 (1H, s), 6.70 (1H, dd, J=2.1, 0.8Hz), 4.61 (1H, s), 2.35 (3H, s) |
| 25-2 | 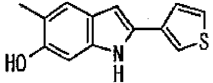 | 1H-NMR (CDCl3) δ ppm:8.05-7.95 (1H, br), 7.45-7.25 (4H, m), 6.82 (1H, s), 6.57 (1H, dd, J=2.0, 0.8Hz), 4.60 (1H, s), 2.34 (3H, s) |
| 25-3 | 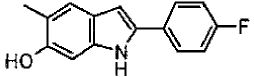 | 1H-NMR (CDCl3) δ ppm:8.10-8.00 (1H, s), 7.65-7.50 (2H, m), 7.33 (1H, s), 7.20-7.05 (2H, m), 6.84 (1H, s), 6.62 (0H, dd, J=2.1, 0.7Hz), 4.63 (1H, s), 2.35 (3H, s) |
| 25-4 | 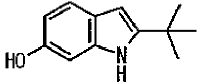 | 1H-NMR (CDCl3) δ ppm:7.90-7.70 (1H, br), 7.36 (1H, d, J=8.4Hz), 6.79 (1H, d, J=2.2Hz), 6.62 (1H, dd, J=8.4, 2.2Hz), 6.16 (1H, dd, J=2.3, 0.9Hz), 4.54 (1H, s), 1.37 (9H, s) |
| 26-1 | 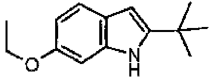 | 1H-NMR (CDCl3) δ ppm:7.81 (1H, s), 7.39 (1H, d, J=8.6 Hz), 6.84 (1H, d, J=2.2Hz), 6.74 (1H, dd, J=8.6, 2.2Hz), 6.16 (1H, dd, J=2.2, 0.9Hz), 4.06 (2H, q, J=7.0Hz). 1.43 (3H, t, J=7.0Hz), 1.37 (9H, s) |
| 26-2 | 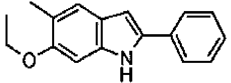 | 1H-NMR (CDCl3) δ ppm:8.14 (1H, s), 7.65-7.55 (2H, m), 7.45-7.20 (4H, m), 6.84 (1H, s), 6.70 (1H, dd, J=2.1, 0.8Hz), 4.07 (2H, q, J=7.0Hz), 2.31 (3H, s), 1.47 (3H, t, J=7.0Hz) |
| 26-3 | 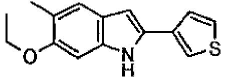 | 1H-NMR (CDCl3) δ ppm:8.03 (1H, s), 7.45-7.20 (4H, m), 6.81 (1H, s), 6.56 (1H, dd, J=2.1, 0.6Hz), 4.06 (2H, q, J=7.0Hz), 2.30 (3H, d, J=0.7Hz), 1.47 (3H, t, J=7.0Hz) |
| 26-4 | 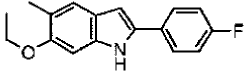 | 1H-NMR (CDCl3) δ ppm:8.06 (1H, s), 7.60-7.50 (2H, m), 7.34 (1H, s), 7.15-7.10 (2H, m), 6.83 (1H, s), 6.62 (1H, d, J=2.0 Hz), 4.06 (2H, q, J=7.0Hz), 2.30 (3H, d, J=0.5Hz), 1.47 (3H, t, J=7.0Hz) |

**[Table 70]**

| Ref.No. | Strc | physical data |
|---|---|---|
| 27 | 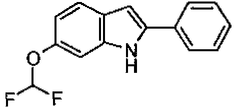 | 1H-NMR (CDCl3) δ ppm:8.37 (1H, s), 7.70-7.60 (2H, m), 7.57 (1H, d, J=8.5Hz), 7.50-7.30 (3H, m), 7.25-7.15 (1H, m), 6.94 (1H, dd, J=8.5, 2.1 Hz), 6.81 (1H, dd, J=2.2, 0.9Hz), 6.52 (1H, t, J=74.8Hz) |
| 28 | 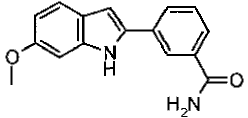 | 1H-NMR (DMSO-d6) δ ppm:11.46 (1H, s), 8.35-8.25 (1H, m), 8.04 (1H, s), 7.95-7.90 (1H, m), 7.80-7.70 (1H, m), 7.55-7.35 (3H, m), 6.95-6.85 (2H, m), 6.67 (1H, dd, J=8.7, 2.3Hz), 3.79 (3H, s) |
| 29 | 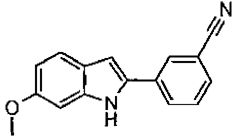 | 1H-NMR (DMSO-d6) δ ppm:11.51 (1H, s), 8.30-8.05 (2H, m), 7.75-7.55 (2H, m), 7.44 (1H, d, J=8.7Hz), 7.02(1H, d, J=2.0Hz), 6.88 (1H, d, J=2.2Hz), 6.69 (1H, dd, J=8.7, 2.2Hz), 3.80 (3H, s) |
| 30-1 | 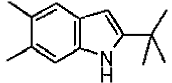 | 1H-NMR (CDCl3) δ ppm:7.74 (1H, s), 7.28 (1H, s), 7.09 (1H, s), 6.13 (1H, dd, J=2.2, 0.9Hz), 2.34 (3H, s), 2.32 (3H, s), 1.36 (9H, s) |
| 30-2 | 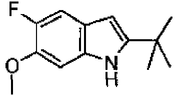 | 1H-NMR (CDCl3) δ ppm:7.84 (1H, s), 7.20 (1H, d, J= 11.8Hz), 6.90 (1H, d, J=7.2Hz), 8.35 (1H, dd, J=2.2, 0.7Hz), 3.90 (3H, s), 1.37 (9H, s) |
| 30-3 | 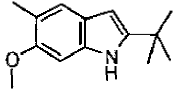 | 1H-NMR (CDCl3) δ ppm:7.77 (1H, s), 7.25 (1H, s), 6.79 (1H, s), 6.10 (1H, d, J=2.1 Hz), 3.84 (3H, s), 2.28 (3H, s), 1.36(9H,s) |

**[Table 71]**

| Ref.No. | Strc | physical data |
|---|---|---|
| 31-1 | 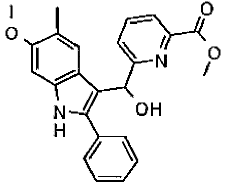 | 1H-NMR (CDCl3) δ ppm:8.12 (1H, s), 8.05-7.95 (1H, m), 7.75-7.60 (3H, m), 7.50-7.20 (4H, m), 6.87 (1H, s), 6.78 (1H, s), 6.15 (1H, d, J=2.6Hz), 5.27 (1H, d, J =2.6Hz), 4.04 (3H, s), 3.81 (3H, s), 2.15-2.05 (3H, d, J=0.5Hz) |
| 31-2 | 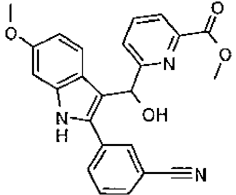 | 1H-NMR (CDCl3) δ ppm:8.34 (1H, s), 8.10-7.90 (3H, m), 7.80-7.35 (4H, m), 7.02 (1H, d, J=8.8Hz), 6.81 (1H, d, J=2.2Hz), 6.62 (1H, dd, J=8.8, 2.2Hz), 6.10 (1H, d, J=2.5Hz), 5.00 (1H, d, J=2.5Hz), 4.04 (3H, s), 3.80 (3H, s) |
| 31-3 | 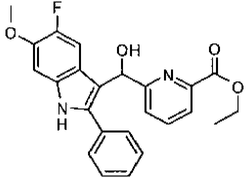 | 1H-N M R (CDCl3) δ ppm:8.22 (1H, s), 8.10-7.95 (1 H, m), 7.75-7.65 (3H, m), 7.55-7.35 (3H, m), 7.30-7.20 (1H, m), 6.89 (1H, d, J=7.0Hz), 6.82 (1H, d, J=11.8Hz), 6.12 (1H, d, J=2.6Hz), 5.34 (1H, d, J=2.6Hz), 4.50 (2H, q, J=7.1 Hz), 3.86 (3H, s), 1.48 (3H, t, J=7.1Hz) |
| 31-4 | 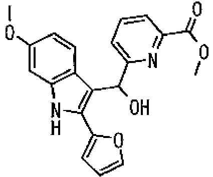 | 1H-NMR (CDCl3) δ ppm:8.45 (1H, s), 8.10-7.95 (1H, m), 7.75-7.65 (1H, m), 7.49 (1H, dd, J=1.8, 0.7Hz), 7.40-7.30 (1H, m), 7.13 (1H, d, J=8.8Hz), 6.79 (1H, d, J=2.3Hz), 6.73 (1H, dd, J=3.5, 0.7Hz), 6.61 (1H, dd, J=8.8, 2.3Hz), 6.51 (1H, dd, J=3.5, 1.8Hz), 6.44 (1H, d, J=2.8Hz), 5.30 (1H, d, J=2.8Hz), 4.05 (3H, s), 3.79 (3H, s) |
| 31-5 | 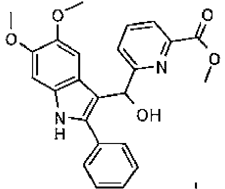 | 1H-NMR (CDCl3) δ ppm:8.35 (1H, s), 8.05-7.95 (1H, m), 7.75-7.60 (3H, m), 7.50-7.20 (4H, m), 6.80 (1H, s), 6.59 (1H, s), 6.16(1H, d, J=3.0Hz), 5.37 (1H, d, J=3.0Hz), 4.02 (3H, s), 3.81 (3H, s), 3.64 (3H, s) |

**[Table 72]**

| Ref. No. | Strc | physical data |
|---|---|---|
| 31-6 | 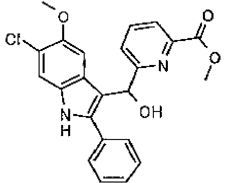 | 1H-NMR (CDCl3) δ ppm:8.12 (1H, s), 8.05-7.95 (1H, m), 7.80-7.65 (3H, m), 7.55-7.45 (3H, m), 7.37 (1H, s), 7.25-7.20 (1H, m), 6.68 (1H, s), 6.16 (1H, d, J=3.1Hz), 5.36 (1H, d, J=3.1Hz), 4.04 (3H, s), 3.67 (3H, s) |
| 31-7 | 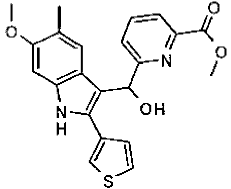 | 1H-NMR (CDDl3) δ ppm:8.12 (1H, s), 8.05-7.95 (1H, m), 7.75-7.65 (2H, m), 7.45-7.35 (3H, m), 6.92 (1H, s), 6.73 (1H, s), 6.21 (1H, d, J=2.6Hz), 4.99 (1H, d, J=2.6Hz), 4.03 (3H, s), 3.79 (3H, s), 2.13 (3H, d,J=0.8Hz) |
| 31-8 | 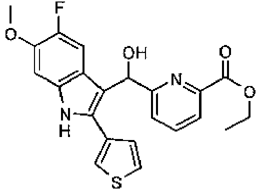 | 1H-NMR (CDCl3) δ ppm:8.15-8.00 (2H, m), 7.82 (1H, dd, J=3.0, 1.2Hz), 7.80-7.70 (1H, m), 7.50-7.35 (3H, m), 6.95-6.85 (2H, m), 6.20 (1H, d, J=2.8Hz), 4.98 (1H, d, J=2.8Hz), 4.50 (2H, q, J=7.3Hz), 3.88 (3H, s), 1.48 (3H, t, J=7.3Hz) |
| 31-9 | 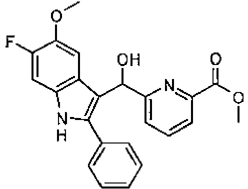 | 1H-NMR (DMSO-d6) δ ppm:11.27 (1H, s), 8.25-8.00 (4H, m), 7.95-7.85 (1H, m), 7.60-7.35 [3H, m), 7.12 (1H, d, J=11.6Hz), 7.08 (1H, d, J=8.6Hz), 6.10-6.00 (2H, m), 3.83 (3H, s), 3.71 (3H, s), |
| 31-10 | 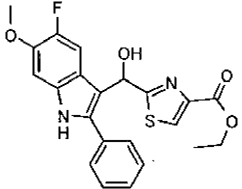 | 1H-NMR (CDCl3) δ ppm:8.70 (1H, s), 8.19 (1H, s), 7.55-7.45 (2H, m), 7.40-7-25 (3H, m), 7.05 (1H, d, J=11.7Hz), 6.62 (1H, d, J=7.3Hz), 6.31 (1H, d, J=3.0Hz), 4.45-4.30 (2H, m), 3.79 (3H, s), 3.22 (1H, d, J=3.0Hz), 1.34 (3H, t, J=7.3Hz) |

**[Table 73]**

| Ref.No. | Strc | physical data |
|---|---|---|
| 31-11 | 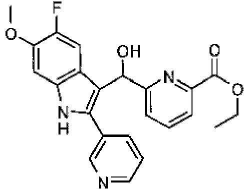 | 1H-NMR (CDCl3) δ ppm:8.95-8.85 (1H, m), 8.65-8.55 (1H, m), 8.32 (1H, s), 8.20-8.00 (2H, m), 7.80-7.70 (1H, m), 7.45-7.30 (2H, m), 6.95-6.80 (2H, m), 6.06 (1H, s), 5.50-4.90 (1H, br), 4.50 (2H, q, J=7.1Hz), 3.88 (3H, s), 1.47 (3H, t, J=7.1Hz) |
| 31-12 | 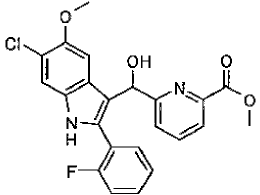 | 1H-NMR (CDCl3) δ ppm:8.28 (1H, s), 8.05-7.95 (1H, m), 7.85-7.65 (2H, m), 7.50-7.15 (5H, m), 6.70 (1H, s), 6.05 (1H, d, J=3.0Hz), 5.36 (1 H, d, J=3.0Hz), 4.04 (3H, s), 3.68 (3H, s), |
| 31-13 | 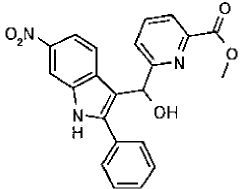 | 1H-NMR (CDCl3) δ ppm:9.17 (1H, s), 8.20-8.00 (2H, m), 7.85-7.60 (4H, m), 7.45-7.30 (4H, m), 7.12 (1H, d, J=8.9Hz), 6.20 (1H, s), 5.45-5.05 (1 H, br), 4.05 (3H, s) |
| 31-74 | 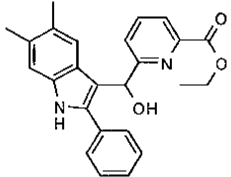 | 1H-NMR (CDCl3) δ ppm:8.10-7.95 (2H, m), 7.80-7.60 (3H, m), 7.55-7.35 (3H, m), 7.35-7.25 (1H, m), 7.14 (1H, s), 6.91 (1H, s), 6.15 (1H, s), 5.35-5.20 (1H, br), 4.51 (2H, q, J=7.2Hz), 2.30 (3H, s), 2.17 (3H, s), 1.48 (3H, t, J=7.2Hz) |
| 31-15 | 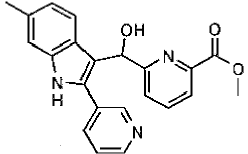 | 1H-NMR (CDCl3) δ ppm:9-00-8-90 (1H, m), 8.63 (1H, dd, J=4.9, 1.6Hz), 8-30-8.10 (2H, m), 8.05-7.95 (1H, m), 7.75-7.65 (1H, m), 7.45-7.30 (2H, m), 7.20-7.15 (1H, m), 7.02 (1H, d, J=8.1Hz), 6.80 (1H, dd, J=8,9, 1.0Hz). 6.15-6.05 (1H, m), 5.23 (1H, d, J=2.4 Hz), 4.04 (3H, s), 2.41 (3H, s) |

**[Table 74]**

| Ref.No. | Strc | physical data |
|---|---|---|
| 31-16 | 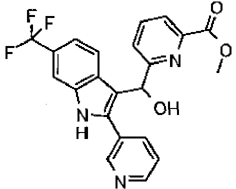 | 1H-NMR (CDCl3) δ ppm:9.04 (1H, s), 8.95-8.85 (1H, m), 8.65-8.55 (1H, m), 8.20-8.00 (2H, m), 7.85-7.70 (1H, m), 7.60-7.20 (4H, m), 7.16 (1H, dd, J=8.5, 1.3Hz), 6.12 (1H, s), 5.40-5.00 (1H, br), 4.03 (3H, s) |
| 31-17 | 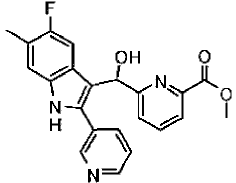 | 1H-NMR (CDCl3) δ ppm:8.95-8.90 (1H, m), 8.70-8.60 (1H, m), 8.28 (1H, s), 8-20-8.00 (2H, m), 7.80-7.70 (1H, m), 7.45-7.30 (2H, m), 7.20-7.10 (1H, m), 6.77 (1H, d, J=10.5Hz), 6.06 (1H, s), 5.30-5.05 (1H, br), 4.04 (3H,s), 2.32 (3H, d, J=1.8Hz) |
| 31-18 | 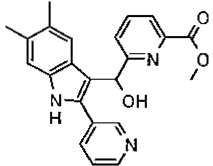 | 1H-NMR (CDCl3) δ ppm:9.05-8.95 (1H, m), 8.65-8.55 (1H, m), 8.18 (1H, s), 8.15-8.10 (1H, m), 8.05-7.95 (1H, m), 7.75-7.65 (1H, m), 7.45-7.30 (2H, m), 7.17 (1H, s), 6.96 (1H, s), 6.09 (1H, s), 5.40-4.90 (1H, br), 4.04 (3H, s), 2.31 (3H, s), 2.19 (3H, s) |
| 31-19 | 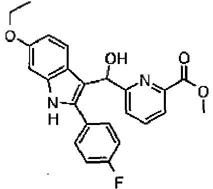 | 1H-NMR (CDCl3) δ ppm: 8.10-7.9 5 (2H, m), 7.80-7.65 (3H, m), 7.35-7.10 (3H, m), 6.95 (1H, d, J=8.7Hz), 6.84 (1H, d, J=2.2Hz), 6.60 (1H, dd, J=8.7, 2.2Hz), 6.08 (1H, s), 5.30-5.15 (1H, br), 4.10-3.95 (5H, m), 1.40 (3H, t, J=7.0Hz) |
| 31-20 | 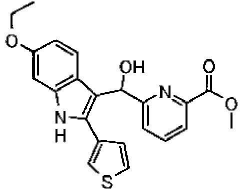 | 1H-NMR (CDCl3) δ ppm:8.10-7.95 (2H, m), 7.76 (1H, dd, J=2.8, 1.6Hz), 7.75-7.65 (1H, m), 7.50-7.35 (3H, m), 7.00 (1H, d, J=8.7Hz), 6.82 (1H, d, J=2.3Hz), 6.60 (1H, dd, J=8.7, 2.3Hz). 6.22 (1H, d, J =2.7Hz), 5.05 (1H, d, J=2.7Hz), 4.10-3.95 (5H, m), 1.40 (3H, t, J=7.0Hz) |

**[Table 75]**

| Ref.No. | Strc | physical data |
|---|---|---|
| 31-21 | 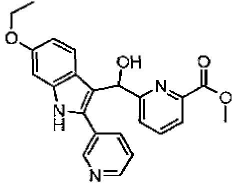 | 1H-NMR (CDCl3) δ ppm:8.95-8.90 (1H, m), 8.65-8.60 (1H, m), 8.20-8.10 (2H, m), 8.05-7.95 (1H, m), 7.75-7.65 (1H, m), 7.45-7.30 (2H, m), 7.01 (1H, d, J=8.8Hz), 6.86 (1H, d, J=2.2Hz), 6.63 (1H, dd, J=8.8, 2.2Hz), 6.08 (1H, s), 5.35-5.10 (1H, br), 4.10-3.95(5H, m), 1.41 (3H, t, J=7.0Hz) |
| 31-22 | 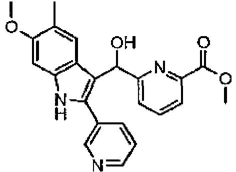 | 1H-NMH (CDCl3) δ ppm:8.95-8.85 (1H, m), 8.65-8.55 (1H, m), 8.17 (1H, s), 8.15-8.05 (1H, m), 8.05-8.00 (1H, m), 7.75-7.65 (1H, m), 7.45-7.30 (2H, m), 6.93 (1H, s), 6.81 (1H, s), 6.08 (1H, s), 5.60-4.80 (1H, br), 4.05 (3H, s), 3.84 (3H, s), 2.14 (3H, d, J=0.5Hz) |
| 31-23 | 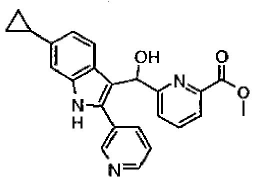 | 1H-NMR (CDCl3) δ ppm.8.95-8.90 (1H, m), 8.70-8.60 (1H, m), 8.22 (1H, s), 8.20-8.10 (1H, m), 8.05-7.95 (1H, m), 7.75-7.65 (1H, m), 7.45-7.35 (1H, m), 7.34 (1H, d, J=8.3Hz), 7.15-7.05 (1H, m), 7.31 (1H, d, J=8.3Hz), 6.71 (1H, dd, J=8.3,1.4Hz), 6.09 (1H, s), 5.35-5.15 (1H, br), 4.04 (3H, s), 2.00-1.90 (1H, m), 1.00-0.85 (2H, m), 0.75-0.60 (2H, m) |
| 31-24 | 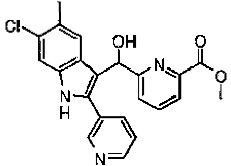 | 1H-NMR (CDCl3) δ ppm:8.90-8.85 (1H, m), 8.65-8.50 (2H, m), 8.15-8.00 (2H, m), 7.80-7.70 (1H, m), 7.40-7.30 (3H, m), 7.04 (1H, s), 6.07 (1H, s), 5.60-4.70 (1H, br), 4.04 (3H, s), 2.28 (3H, s) |
| 31-25 | 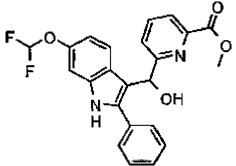 | 1H-NMR (CDCl3) δ ppm:8.40-8.25 (1H, s), 8.10-7.95 (1H, m), 7.80-7.65 (3H, m), 7.55-7.35 (3H, m), 7.30-7.20 (1H, m), 7.13 (1H, d, J=2.1Hz). 7.05 (1H, d, J=8.6Hz), 6.73 (1H, dd, J=8.6, 2.1Hz), 6.44 (1H, t, J=74.8Hz), 6.16 (1H, d, J=2.6Hz), 5.39 (1H, d, J=2.6Hz), 4.05 (3H, s) |

**[Table 76]**

| Ref.No. | Strc | physical data |
|---|---|---|
| 31-26 | 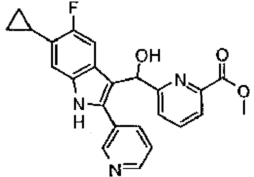 | 1H-NMR (CDCl3) δ ppm:9.00-8.85 (1H, m), 8.70-8.60 (1H, m), 8.30-8.00 (3H, m), 7.80-7.70 (1H, m), 7.45-7.30 (2H, m), 6.90 (1H, d, J=6.2Hz), 6.77 (1H, d, J=10.9Hz), 6.05 (1H, s), 5.30-5.05 (1H, br), 4.05 (3H, s), 2.15-2.05 (1H, m), 1.05-0.90 (2H, m), 0.75-0.60 (2H, m) |
| 31-27 | 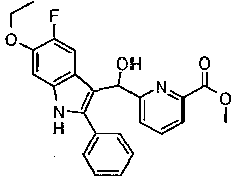 | 1H-NMR (CDCl3) δ ppm:8.15-8.00 (2H, m), 7.75-7.65 (3H, m), 7.55-7.35 (3H, m), 7.30-7.25 (1H, m), 6.32 (1H, d, J=7.0Hz), 6.80 (1H, d, J-11.8Hz). 6.13 (1H, d, J=2.5Hz), 5.32 (1H, d, J=2.5Hz), 4.15-4.00 (5H, m), 1.45 (3H, t, J=71Hz) |
| 31-28 | 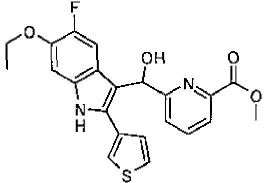 | 1H-NMR (CDCl3) δ ppm:8.15-8.00 (2H, m), 7.80-7.70 (2H, m), 7.47 (1H, dd, J=5.1, 3.0Hz), 7.43 (1H, dd, J=5.0, 1.3Hz), 7.40-7.35 (1H, m), 6.89 (1H, d, J=7.1Hz), 6.84 (1H, d, J=11.8Hz), 6.19 (1H, d, J=2.6Hz), 5.03 (1H, d, J=2.6Hz), 4.15-4.00 (5H, m), 1.44 (3H, t, J=7.0Hz) |
| 31-29 | 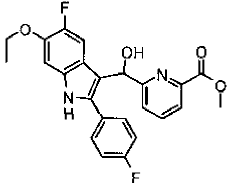 | 1H-NMR (CDCl3) δ ppm :8.10-8.00 (2H, m), 7.75-7.65 (3H, m), 7.35-7.10 (3H, m), 6.91 (1H, d, J=7.1Hz), 6.80 (1H, d, J=11.8Hz), 6.06 (1H, d, J=2.5Hz), 5.21 (1H, d, J=2.5H z), 4.15-4.00 (5H, m), 1.48 (3H, t, J=7.0Hz) |
| 31-30 | 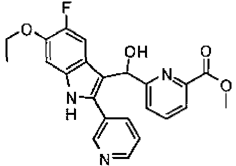 | 1H-NMR (CDCl3) δ ppm:8.95-8.85 (1H, m), 8.65-8.60 (1H, m), 8.28 (1H, s), 8.15-8.00 (2H, m), 7.80-7.70 (1H, m), 7.45-7.30 (2H, m), 6.91 (1H, d, J=7.0Hz), 6.85 (1 H, d, J=11.7Hz), 6.06 (1H, s), 5.35-5.05 (1H, br), 4.15-4.00 (5H, m), 1.46 (3H, t, J=7.0Hz) |

**[Table 77]**

| Ref.No. | Strc | physical data |
|---|---|---|
| 31-31 | 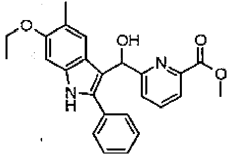 | 1H-NMR (CDCl3) δ ppm:8.05-7.95 (2H, m), 7.75-7.60 (3H, m), 7.55-7.25 (4H, m), 6.86 (1H, s), 6.79 (1H, s), 6.15 (1H, s), 5.35-5.15 (1H, br), 4.10-3.95 (5H, m), 2.12 (3H, s), 1.43 (3H, t, J=7.0Hz) |
| 31-32 | 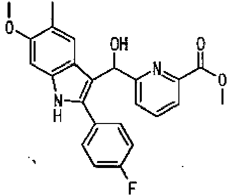 | 1H-NMR (CDCl3) δ ppm:8.05-7.95 (2H, m), 7.75-7.60 (3H, m), 7.35-7.25 (1H, m), 7.20-7.10 (2H, m), 6.88 (1H, s), 6.79 (1H, s), 6.08 (1H, s), 5.16 (1H, s), 4.05 (3H, s), 3.83 (3H, s), 2.12 (3H, s) |
| 31-33 | 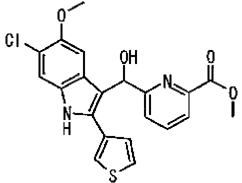 | 1H-NMR (CDCl3) δ ppm:8.14 (1H, s), 8.05-8.00 (1H, m), 7.80-7.65 (2H, m), 7.46 (1H, dd, J=5.0, 3.0Hz), 7.42 (1H, dd, J=5.0, 1.3Hz), 7.40-7.30 (2H, m), 6.68 (1H, s), 6.22 (1H, s), 5.13 (1H, s), 4.04 (3H, s), 3.68 (3H, s) |
| 31-34 | 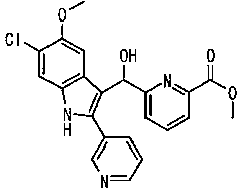 | 1H-NMR (CDCl3) δ ppm:8.90-8.85 (1H, m), 8.70-8.50 (2H, m), 8.15-8.00 (2H, m), 7.80-7.70 (1H, m), 7.45-7.30 (3H, m), 6.69 (1H, s), 6.08 (1H, s), 5.70-5.00 (1H, br), 4.03 (3H, s), 3.68 (3H, s) |
| 31-35 | 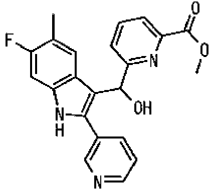 | 1H-NMR (CDCl3) δ ppm:8.90-8.70 (2H, m), 8.60-8.50 (1H, m), 8.15-7.95 (2H, m), 7.80-7.70 (1H, m), 7.39 (1H, d, J=7.8Hz), 7.35-7.25 (1H, m), 7.00-6.90 (2H, m), 6.07 (1H, s), 5.50-5.10 (1H, br), 4.03 (3H, s), 2.18 (3H, d, J=1.9Hz) |

**[Table 78]**

| Ref.No. | Strc | physical data |
|---|---|---|
| 31-36 | 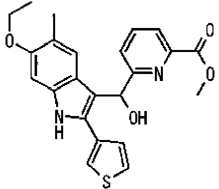 | 1H-NMR (CDCl3) δ ppm:8.05-7.95 (2H, m), 7.75-7.65 (2H, m), 7.50-7.35 (3H, m), 6.92 (1H, s), 6.75 (1H, s), 6.21 (1H,s), 5.10-4.90 (1H, br), 4.10-3.90 (5H, m), 2.14 (3H, s), 1.42 (3H, t, J=7.0Hz) |
| 31-37 | 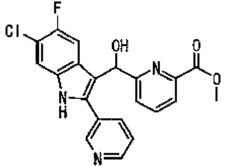 | 1H-NMR (CDCl3) δ ppm:8.99 (1H, s), 8.90-8.80 (1H, m), 8.65-8.50 (1H, m), 8.10-8.00 (2H, m), 7.85-7.75 (1H, m), 7.50-7.40 (1H, m), 7.35-7.25 (2H, m), 6.90 (1H, d, J=9.8Hz), 6.05 (1H, s), 5.80-4.60 (1H, br), 4.03 (3H, s) |
| 31-38 | 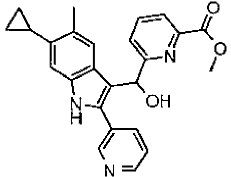 | 1H-NMR (CDCl3) δ ppm:8.95-8.85 (1H, m), 8.65-8.55 (1H, m), 8.20-7.95 (3H, m), 7.75-7.65 (1H, m), 7.45-7.30 (2H, m), 7.06 (1H, s), 6.98 (1H, s), 6.09 (1H, d, J=2.5Hz), 5.12 (1H, d, J=2.5Hz), 4.51 (2H, s), 4.05 (3H, s), 2.35 (3H, s), 2.00-1.85 (1H, m), 1.00-0.85 (2H, m), 0.70-0.50 (2H, m) |
| 31-39 | 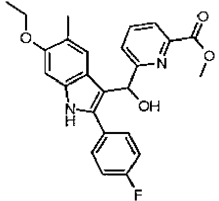 | 1H-NMR (CDCl3) δ ppm:8.10-7.90 (2H, m), 7.75-7.60 (3H, m), 7.35-7.25 (1H, m), 7.20-7.10 (2H, m), 6.87 (1H, s), 6.78 (1H, s), 6.08 (1H, s), 5.16 (1H, s), 4.10-3.95 (5H, m), 2.13 (3H, s), 1.43 (3H, t, J=7.0Hz) |
| 31-40 | 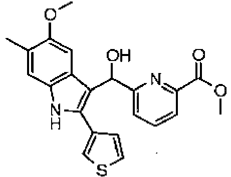 | 1H-NMR (CDCl3) δ ppm:8.05-7.90 (2H, m), 7.80-7.65 (2H, m), 7.50-7.30 (3H, m), 7.09 (1H, s), 6.56 (1H, s), 6.23 (1H, d, J=3.1Hz), 5.08 (1H, d, J=3.1 Hz), 4.03 (3H, s), 3.62 (3H, s), 2.25 (3H, s) |

**[Table 79]**

| Ref.No. | Strc | physical data |
|---|---|---|
| 32-1 | 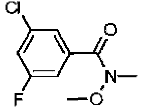 | 1H-NMR (CDCl3) δ ppm:7.50 (1H, s), 7.40-7.10 (2H, m), 3.56 (3H, s), 3.37 (3H, s) |
| 32-2 | 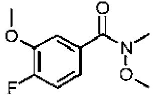 | 1H-NMR (CDCl3) δ ppm:7.37 (1H, dd, J=8.2, 2.0Hz), 7.35-7.25 (1H, m), 7.09 (1H, dd, J=10.9. 8.0Hz), 3.92 (3H, s), 3.56 (3H, s), 3.37 (3H, s) |
| 33-1 | 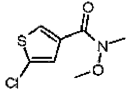 | 1H-NMR (CDCl3) δ ppm:7.85 (1H, d, J=1.6Hz), 7.39 (1H, d, J=1.6Hz). 3.67 (3H, s), 3.35 (3H, s) |
| 33-2 | 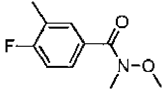 | 1H-NMR (CDCl3) δ ppm:7.65-7.45 (2H, m), 7.10-6.95 (1H, m), 3.55 (3H, s), 3.36 (3H, s), 2.30 (3H, s) |
| 33-3 | 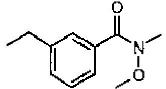 | 1H-NMR (CDCl3) δ ppm:7.60-7.40 (2H, m), 7.35-7.25 (2H, m), 3.58 (3H, s), 3.35 (3H, s), 2.69 (2H, q, J=7.6Hz), 1.25 (3H, t, J=7.6Hz) |
| 34 | 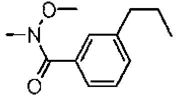 | 1H-NMR(CDCl3) δ ppm:7.50-7.40 (2H, m), 7.35-7.20 (2H, m), 3.57 (3H, s), 3.35 (3H, s), 2.62 (2H, t, J=7.6Hz), 1.75-1.55 (2H, m), 0.94 (2H, t, J=7.4Hz) |
| 35 | 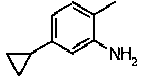 | 1H-NMR(CDCl3) δ ppm:6.93 (1H, d, J=7.5Hz), 6.44 (1H, dd, J=7.5, 1.5Hz), 6.41 (1H, d, J=1.5Hz), 3.70-3.40 (2H, br), 2.12 (3H, s), 1.85-1.70 (1H, m), 0.95-0.80 (2H, m), 0.70-0.55 (2H, m) |

**[Table 80]**

| Ref.No. | Strc | physical data |
|---|---|---|
| 36-1 | 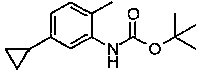 | 1H-NMR (CDCl3) δ ppm:7.70-7.50 (1 H, br), 7.01 (1H, d, J=7.8Hz), 6.69 (1H, dd, J=7.8, 1.7Hz), 6.30-6.10 (1H, br), 2.19 (3H, s), 1.95-1.80 (1H, m), 1.52 (9H, s), 0.95-0.85 (2H, m), 0.75-0.60 (2H, m) |
| 36-2 | 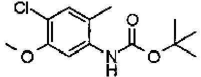 | 1H-NMR (CDCl3) δ ppm:7.80-7.60 (1H, br), 7.11 (1H, s), 6.35-6.15 (1H, br), 3.91 (3H, s), 2.16 (3H, s), 1.53(9H, s) |
| 36-3 | 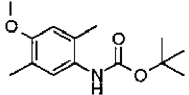 | 1 H-NMR (CDCl3) δ ppm:7.50-7.20 (1 H, br), 6.62 (1H, s), 6.15-5.85 (1 H, br), 3.79 (3H, s), 2.22 (3H, s), 2.17 (3H, s), 1.51 (9H, s) |
| 36-4 | 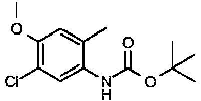 | 1H-NMR (CDCl3) δ ppm:7.90-7.60 (1H, br), 6.72 (1 H, s), 6.20-5.90 (1H, br), 3.86 (3H, s), 1.51 (9H, s) |
| 37 | 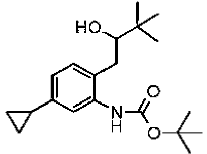 | 1H-NMR (CDCl3) δ ppm:7.72 (1 H, s), 7.48 (1H, s), 7.00 (1H, d, J=7.9Hz), 6.74 (1H, dd, J=7.9, 1.8Hz), 3.45-3.30 (1H, m), 2.63 (2H, d, J=6.3Hz), 1.99 (1 H, d, J=3.8Hz), 1.95-1.80 (1H, m), 1.51 (9H, s), 1.00 (9H, s), 0.95-0.85 (2H, m), 0.75-0.60 (2H, m) |
| 38 | 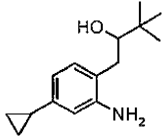 | 1H-NMR (CDCl3) δ ppm:6.92 (1H, d, J=7.8Hz), 6.49 (1H, dd, J=7.8, 1.8Hz), 6.43 (1H, d, J=1.8Hz), 3.43 (1 H, dd, J=10.2. 2.2Hz), 2.64 (1H, dd, J=14.3, 2.2Hz), 2.55 (1H, dd, J=14.3, 10.2Hz), 1.85-1.70 (1 H, m), 1.00 (9H, s), 0.95-0.80 (2H, m), 0.70-0.55 (2H, m) |
| 39 | 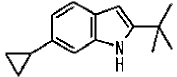 | 1H-NMR(CDCl3) δ ppm:7.81 (1H, s), 7.40 (1H, d, J=8.1Hz). 7.10-7.00 (1H, m), 6.84 (1H, dd, J=8.1, 1.5Hz), 6.18 (1H, dd, J=2.3, 0.9Hz), 2.05-1.95 (1 H, m), 1.37 (9H, s), 1.00-0.85 (2H, m), 0.75-0.65 (2H, m) |

**[Table 81]**

| Ref.No. | Strc | physical data |
|---|---|---|
| 40-1 | 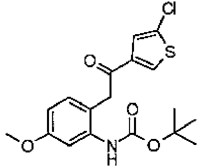 | 1H-NMR (CDCl3) δ ppm:7.97 (1H, d, J=1.7Hz), 7.80-7.65 (1H, br), 7.50-7.40 (1H, m), 7.39 (1H, d, J=1.7Hz), 7.05 (1H, d, J=8.5Hz), 6.60 (1H, dd, J=8.5, 2.7Hz), 4.01 (2H, s), 3.79 (3H, s), 1.53 (9H, s) |
| 40-2 | 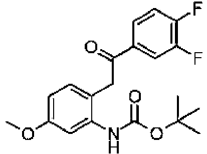 | 1H-NMR (CDCl3) δ ppm:7.95-7.80 (2H, m), 7.60-7.20 (3H, m), 7.07 (1H, d, J=8.6Hz), 6.61 (1H, dd, J=8.6, 2.7Hz), 4.14 (2H, s), 3.79 (3H, s), 1.52 (9H, s) |
| 40-3 | 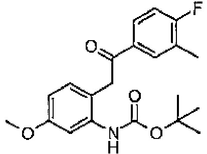 | 1H-NMR (CDCl3) δ ppm:8.20-7.65 (3H, m), 7.55-7.40 (1H, s), 7.20-7.00 (2H, m), 6.59 (1H, dd, J=8.5, 2.6Hz), 4.15 (2H, s), 3.79 (3H, s), 2.33 (3H, s), 1.53 (9H, s) |
| 40-4 | 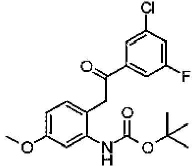 | MS(ESI, m/z) = 394 (M+H)+ |
| 40-5 | 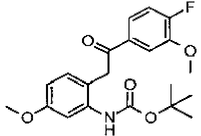 | 1H-NMR (CDCl3) δ ppm:7.90-7.60 (3H, m), 7.48 (1H, s), 7.18 (1H, dd, J=10.5, 8.5Hz), 7.07 (1H, d, J=8.5Hz), 6.59 (1H, dd, J=8.5, 2.7Hz), 4.15 (2H, s), 3.93 (3H, s), 3.79 (3H, s), 1.53 (9H, s) |
| 40-6 | 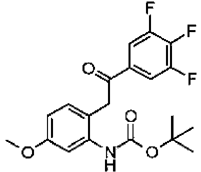 | 1H-NMR (CDCl3) δ ppm:7.75-7.60 (2H, m), 7.45-7.25 (2H, m), 7.06 (1H, d, J=8.5Hz), 6.63 (1H, dd, J=8.5, 2.7Hz), 4.12 (2H, s), 3.80 (3H, s), 1.52 (9H, s) |

**[Table 82]**

| Ref.No. | Strc | physical data |
|---|---|---|
| 4t7-7 | 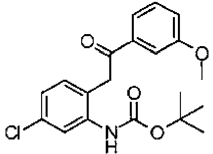 | 1H-NMR (CDCl3) δ ppm:8.00-7.35 (5H, m), 7.20-7.05 (2H, m), 7.00 (1H, dd, J=8.3, 2.2Hz), 4.21 (2H, s), 3.86 (3H, s), 1.53 (9H, s) |
| 40-8 | 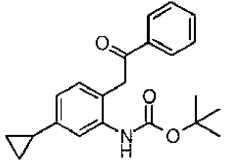 | 1H-NMR (CDCl3) δ ppm: 8.10-8.00 (2H, m), 7.80-7.45 (5H, m), 7.08 (1H, d, J=7.9Hz), 6.73 (1H, dd, J=7.9, 1.6Hz), 4.22 (2H, s), 1.95-1.80 (1H, m), 1.52 (9H, s), 1.00-0.85 (2H, m), 0.75-0.60 (2H, m) |
| 40-9 | 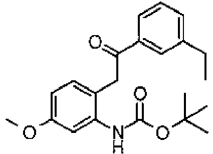 | 1H-NMR (CDCl3) δ ppm:8.00-7.70 (3H, m), 7.55-7.35 (3H, m), 7.09 (1H, d, J=8.4Hz), 6.59 (1H, dd, J=8.4, 2.7Hz), 4.19 (2H, s), 3.79 (3H, s), 2.72 (2H, q, J=7.6Hz), 1.53 (9H, s), 1.27 (3H, t, J=7.6Hz) |
| 40-10 | 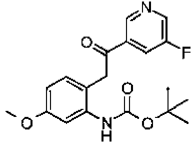 | MS(ESI, m/z) = 359 (M-H)- |
| 40-11 | 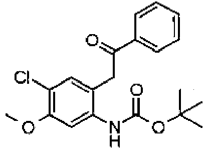 | 1H-NMR (CDCl3) δ ppm: 8.10-8.00 (2H, m), 7.95-7.45 (5H, m), 7.18 (1H, s), 4.17 (2H, s), 3.91 (3H, s), 1.53(9H,s) |
| 40-12 | 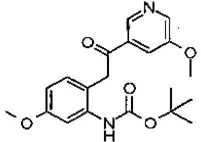 | 1H-NMR (CDCl3) δ ppm:900-8.90 (1H, m), 8.55-8.45 (1H, m), 7.80-7.70 (1H, m), 7.65-7.40 (2H, m), 7.11 (1H, d, J=8.5Hz), 6.61 (1H, dd, J=8.5, 2.7Hz), 4.20 (2H, s), 3.90 (3H, s), 3.79 (3H, s), 1.53(9H,s) |

**[Table 83]**

| Ref.No. | Strc | physical data |
|---|---|---|
| 40-13 | 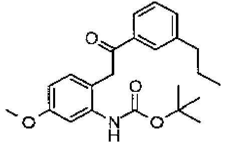 | 1H-NMR (CDCl3) δ ppm:7.95-7.70 (3H, m), 7.55-7.35 (3H, m), 7.09 (1H, d, J=8.5Hz), 6.59 (1H, dd, J=8.5, 2.6Hz), 4.18 (2H, s), 3.79 (3H, s), 2.66 (2H, t, J=7.7Hz), 1.75-1.60 (2H, m), 1.53 (9H, s), 0.95 (3H, t, J=7.4Hz) |
| 40-14 | 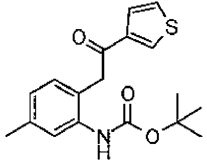 | 1H-NMR (CDCl3) δ ppm:8.23 (1H, dd, J=2.9, 1.2Hz), 7.95-7.75 (1H, br), 7.70-7.50 (2H, m), 7.35 (1H, dd, J=5.1, 2.9Hz), 7.08 (1H, d, J=7.7Hz), 6.85 (1H, dd, J=7.7, 1.2Hz), 4.12 (2H, s), 2.32 (3H, s), 1.53 (9H, s) |
| 40-15 | 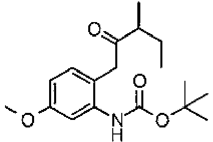 | 1H-NMR (CDCl3) δ ppm:7.80-7.65 (1H, m), 7.55-7.40 (1H, br), 7.01 (1H, d, J=8.5Hz), 6.59 (1H, dd, J=8.5, 2.7Hz), 3.80 (3H, s), 3.65 (2H, s), 2.70-2.55 (1H, m), 1.80-1.65 (1H, m), 1.52 (9H, s), 1.50-1.30 (1H, m), 1.09 (3H, d, J=7.0Hz), 0.82 (3H, t, J=7.5Hz) |
| 40-16 | 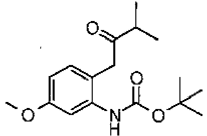 | 1H-NMR (CDCl3) δ ppm:7.90-7.65 (1H, br), 7.55-7.40 (1H, m), 7.02 (1H, d, J=8.5Hz), 6.59 (1 H, dd, J=8.5, 2.7Hz), 3.80 (3H, s), 3.66 (2H, s), 2.90-2.70(1H, m),1.52 (9H, s), 1.13 (6H, d, J=7.0Hz) |
| 40-17 | 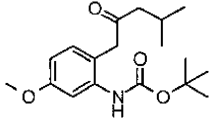 | 1H-NMR (CDCl3) δ ppm:7.70-7.40 (2H, m), 7.01 (1H, d, J=8.5Hz), 6.59 (1H, dd, J=8.5. 2.7Hz), 3.80 (3H, s), 3.58 (2H, s), 2.40 (2H, d, J=6.9Hz), 2.25-2.05 (1H, m), 1.52 (9H, s), 0.88 (6H, d, J=6.7Hz) |
| 40-18 | 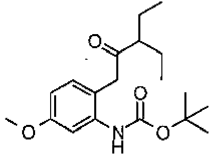 | 1H-NMR (CDCl3) δ ppm:7.75-7.60 (1H, br), 7.55-7.40 (1H, br), 7.00 (1H, d, J=8.4Hz), 6.59 (1H, dd, J=8.4, 2.7Hz), 3.80 (3H, s), 3.63 (2H, s), 2.60-2.45 (1H, m), 1.75-1.40 (13H, m), 0.78 (6H, t, J=7.4Hz) |

**[Table 84]**

| Ref.No. | Strc | physical data |
|---|---|---|
| 40-19 | 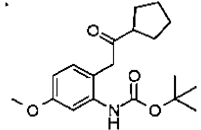 | 1H-NMR (CDCl3) δ ppm:7.90-7.65 (1H, br), 7.55-7.40 (1H, br), 7.02 (1H, d, J=8.5Hz), 6.58 (1H, dd, J=8.5, 2.6Hz), 3.80 (3H, s), 3.66 (2H, s), 3.15-2.95 (1H, m), 2.00-1.40 (17H, m) |
| 40-20 | 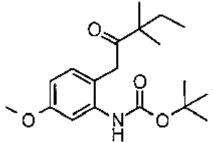 | 1H-NMR (CDCl3) δ ppm:8.20-7.90 (1H, br), 7.55-7.30 (1H, br), 6.97 (1H, d, J=8.5Hz), 6.57 (1H, dd, J=8.5, 2.7Hz), 3.80 (3H, s), 3.69 (2H, s), 1.67 (2H, q, J=7.6Hz), 1.52 (9H, s), 1.17 (6H, s), 0.78 (3H, t, J=7.6Hz) |
| 40-21 | 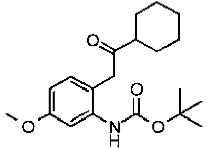 | 1H-NMR (CDCl3) δ ppm:7.90-7.75 (1H, br), 7.55-7.40 (1H, br), 7.02 (1H, d, J=8.4Hz), 6.56 (1H, dd, J=8.4, 2.7Hz), 3.80 (3H, s), 3.64 (2H, s), 2.60-2.45 (1H, m), 1.95-1.10 (19H, m) |
| 40-22 | 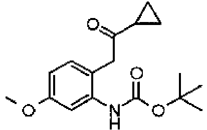 | 1H-NMR (CDCl3) δ ppm:7.65-7.40 (2H, m), 7.08 (1 H, d, J=8.4Hz), 6.61 (1H, dd, J=8.4, 2.7Hz), 3.81 (3H, s), 3.73 (2H, s), 2.10-2.00 (1H, m), 1.52 (9H, s), 1.15-0.85 (4H, m) |
| 40-23 | 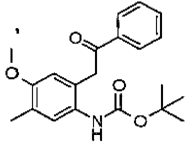 | 1H-NMR (CDCl3) δ ppm:8.10-8.00 (2H, m), 7.70-7.30 (4H, m), 7.10-6.85 (1H, br), 6.61 (1H, s), 4.27 (2H, s), 3.76 (3H, s), 2.18 (3H, s), 1.48 (9H, s) |
| 40-24 | 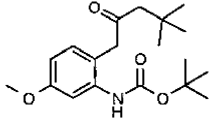 | 1H-NMR (CDCl3) δ ppm:7.60-7.40 (2H, m), 7.00 (1H, d, J=8.4Hz), 6.60 (1H, dd, J=8.4, 2.7Hz), 3.80 (3H, s), 3.59 (2H, s), 2.42 (2H, s), 1.52 (9H, s), 1.01 (9H, s) |
| 40-25 | 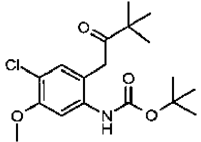 | MS(ESI, m/z) = 354 (M-H)- |

**[Table 85]**

| Ref.No. | Strc | physical data |
|---|---|---|
| 40-26 | 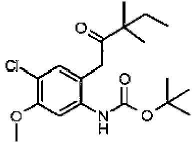 | MS (ESI, m/z) = 368 (M-H)- |
| 40-27 | 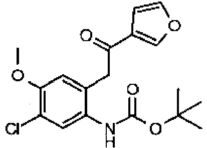 | 1H-NMR (CDCl3) δ ppm:8.20-8.15 (1H, m), 7.80-7.60 (1H, br), 7.49 (1H, dd, J=2.0, 1.5Hz), 7.40-7.20 (1H, br), 6.81 (1H, dd, J=2.0, 0.8 Hz), 6.70 (1H, s), 4.01 (2H, s), 3.85 (3H, s), 1.50 (9H, s) |
| 40-28 | 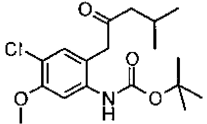 | 1H-NMR (CDCl3) δ ppm:7.80-7.50 (2H, m), 7.11 (1H, s), 3.92 (3H, s), 3.56 (2H, s), 2.43 (2H, d, J=6.9Hz), 2.25-2.05 (1H, m), 1.52 (9H, s), 0.90 (6H, d, J=6.7Hz) |
| 40-29 | 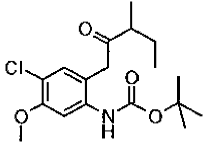 | 1H-NMR (CDCl3) δ ppm:7.90-7.70 (1H, br), 7.65-7.50 (1H, br), 7.11 (1H, s), 3.92 (3H, s), 3.62 (2H, s), 2.75-2.55 (1H, m), 1.80-1.60 (1H, m), 1.52 (9H, s), 1.50-1.35 (1H, m), 1.10 (3H, d, J=7.0Hz), 0.84 (3H, t, J=7.4Hz) |
| 40-30 | 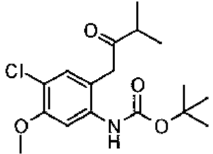 | 1H-NMR (CDCl3) δ ppm:7.95-7.70 (1H, br), 7.65-7.50 (1H, br), 7.11 (1H, s), 3.91 (3H, s), 3.63 (2H, s), 2.90-2.70 (1H, m), 1.52 (9H, s), 1.14 (6H, d, J=7.0Hz) |
| 40-31 | 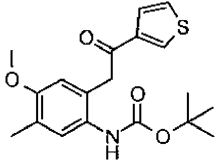 | 1H-NMR (CDCl3) δ ppm:8.20 (1H, dd, J=2.9, 1.2Hz), 7.60 (1H, dd, J=5.1, 1.2Hz), 7.45-7.30 (2H, m), 7.25-7.00 (1H, br), 6.61 (1H, s), 4.15 (2H, s), 3.77 (3H, s), 2.18 (3H, s), 1.49 (9H, s) |

**[Table 86]**

| Ref.No. | Strc | physical data |
|---|---|---|
| 41-1 | 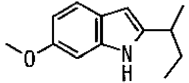 | 1H-NMR (CDCl3) *δ* ppm:7.90-7.75 (1H, br), 7.40 (1H, d, J=8.6Hz), 6.84 (1H, d, J=2.2Hz), 6.74 (1H, dd, J=8.6, 2.2Hz), 6.16 (1H, d, J=2.1Hz), 3.84 (3H, s), 2.90-2.70 (1H, m), 1.80-1.55 (2H, m), 1.32 (3H, d, J=7.0Hz), 0.91 (3H, t, J=7.4Hz) |
| 41-2 | 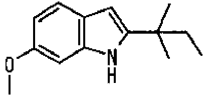 | 1H-NMR (CDCl3) *δ* ppm:7.90-7.70 (1H, br), 7.40 (1H, d, J=8.6Hz), 6.84 (1H, d, J=2.2Hz), 6.74 (1H, dd, J=8.6, 2.2Hz), 6.16 (1H, dd, J=2.3, 0.8Hz), 3.84 (3H, s), 1.65 (2H, q, J=7.5Hz), 1.33 (6H, s), 0.78 (3H, t, J=7.5Hz) |
| 41-3 | 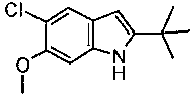 | 1H-NMR (CDCl3) *δ* ppm:8.00-7.80 (1H, br), 7.50 (1H, s), 6.89 (1H, s), 6.12 (1H, dd, J=2.3, 0.8 Hz), 3.90 (3H, s), 1.37 (9H, s) |
| 41-4 | 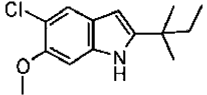 | 1H-NMR (CDCl3) *δ* ppm:7.95-7.75 (1H, br), 7.50 (1H, s), 6.89 (1H, s), 6.12 (1H, dd, J=2.3, 0.7Hz), 3.91 (3H, s), 1.64 (2H, q, J=7.4Hz), 1.32 (6H, s), 0.77 (3H, t, J=7.4Hz) |
| 41-5 | 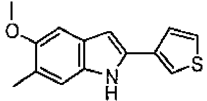 | 1H-NMR (CDCl3) *δ* ppm:8.15-7.95 (1H, br), 7.45-7.30 (3H, m), 7.13 (1H, s), 7.00 (1H, s), 6.61 (1 H, dd, J=2.2, 0.8Hz), 3.88 (3H, s), 2.33 (3H, d, J=0.5Hz) |
| 42 | 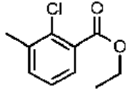 | 1H-NMR (CDCl3) *δ* ppm:7.60-7.50 (1H, m), 7.40-7.30 (1H, m), 7.25-7.15 (1H, m), 4.40 (2H, q, J=7.2Hz), 2.43 (3H, s), 1.40 (3H, t, J=7.2Hz) |
| 43 | 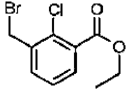 | 1H-NMR (CDCl3) *δ* ppm:7.70 (1H, dd, J=7.8, 1.6Hz), 7.57 (1H, dd, J=7.7, 1.6Hz), 7.35-7.25 (1H, m), 4.64 (2H, s), 4.41 (2H, q, J=7.2Hz), 1.41 (3H, t, J=7.2Hz) |

**[Table 87]**

| Ref.No. | Strc | physical data |
|---|---|---|
| 44-1 | 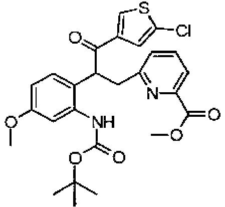 | MS(ESI, m/z) = 531 (M+H)+ |
| 44-2 | 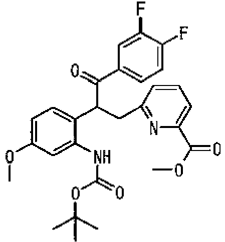 | MS(ESI, m/z) = 527 (M+H)+ |
| 44-3 | 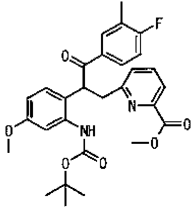 | MS(ESI, m/z) = 523 (M+H)+ |
| 44-4 | 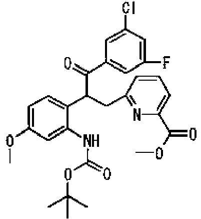 | MS(ESI, m/z) = 543 (M+H)+ |
| 44-5 | 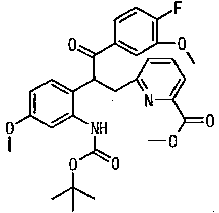 | 1H-NMR (CDCl3) δ ppm:8.00-7.85 (1H, m), 7.80 (1H, s), 7.70-7.55 (3H, m), 7.25-6.90 (4H, m), 6.54 (1H, dd, J=8.8, 2.6Hz), 5.46 (1H, dd, J=10.1, 5.2Hz), 4.03 (3H, s), 3.87 (3H, s), 3.75-3.60 (4H, m), 3.52 (1H, dd, J=15.5, 10.1Hz), 1.58 (9H, s) |

**[Table 88]**

| Ref.No. | Strc | physical data |
|---|---|---|
| 44-6 | 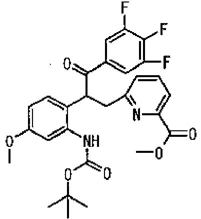 | MS(ESI, m/z) = 545 (M+H)+ |
| 44-7 | 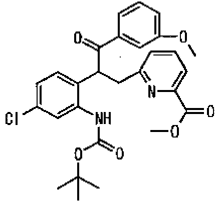 | MS(ESI, m/z) = 525 (M+H)+ |
| 44-8 | 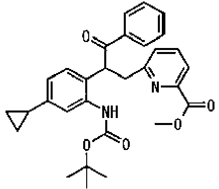 | MS(ESI, m/z) = 501 (M+H)+ |
| 44-9 | 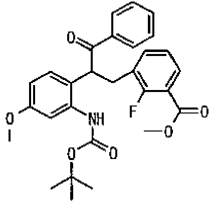 | MS(ESI, m/z) = 506 (M-H)- |
| 44-10 | 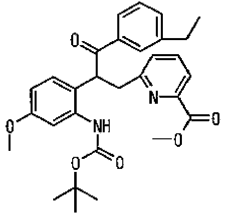 | MS(ESI, m/z) = 519 (M+H)+ |

**[Table 89]**

| Ref. No. | Strc | physical data |
|---|---|---|
| 44-11 | 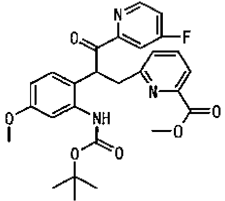 | MS(ESI, m/z) = 510 (M+H)+ |
| 44-12 | 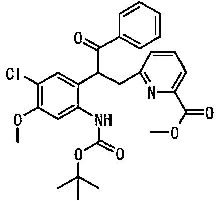 | MS(ESI, m/z) = 525 (M+H)+ |
| 44-13 | 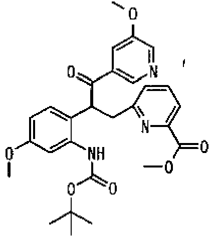 | 1H-NMR (CDCl3) δ ppm:8.80-8.70 (1H, m), 8.40-8.30 (1H, m), 8.00-7.90 (1H, m), 7.70-7.60 (3H, m), 7.25-7.15 (2H, m), 7.03 (1H, d, J=8.5Hz), 6.54 (1H, dd, J=8.5, 2.8Hz), 5.43 (1H, dd, J=9.3, 5.8Hz), 3.98 (3H, s), 3.82 (3H, s), 3.77 (1H, dd, J=15.1, 5.8Hz), 3.71 (3H, s), 3.48 (1H, dd, J=15.1, 9.3Hz), 1.56 (9H, s) |
| 44-14 | 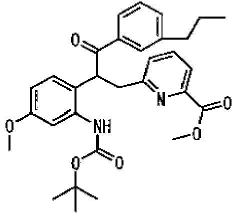 | 1H-NMR (CDCl3) δ ppm:8.00-6.90 (10H, m), 6.50 (1H, dd, J=8.6, 2.7Hz), 5.46 (1H, dd, J=10.2, 5.3Hz), 4.01 (3H, s), 3.80-3.40 (6H, m), 2.57 (2H, t, J=7.6Hz), 1.70-1.55 (10H, m), 0.88 (3H, t, J=7.4Hz) |
| 44-15 | 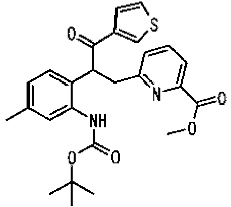 | 1H-NMR (CDCl3) δ ppm:8.09 (1H, dd, J=2.9, 1.2Hz), 8.04 (1H, s), 7.95-7.85 (1H, m), 7.70-7.55 (1H, m), 7.50 (1H, dd, J=5.2, 1.2Hz), 7.25-7.20 (1H, m), 7.18 (1H, dd, J=5.2, 2.9Hz), 7.08 (1H, d, J=8.0Hz), 6.81 (1H, dd, J=8.0, 1.3Hz), 5.46 (1H, dd, J=10.2, 5.1 Hz), 4.05 (3H, s), 3.69 (1H, dd, J =16.0, 5.1 Hz), 3.56 (1H, dd, J=16.0, 10.2Hz), 2.21 (3H, s), 1.59 (9H, s) |

**[Table 90]**

| Ref. No. | Strc | physical data |
|---|---|---|
| 44-16 | 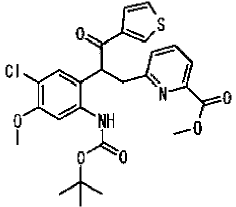 | 1H-NMR (CDCl3) δ ppm:8.23 (1H, s), 8.10 (1H, dd, J=2.9, 1.1Hz), 8.00-7.85 (1H, m), 7.70-7.60 (1H, m), 7.51 (1H, dd, J=5.1, 1.1Hz), 7.30-7.10 (3H, m), 7.12 (1H, s), 5.43 (1H, dd, J=10.5, 5.0Hz), 4.06 (3H, s), 3.81 (3H, s), 3.66 (1H, dd, J=16.3, 5.0Hz), 3.55 (1H, d, J=16.3, 10.5Hz), 1.60(9H,s) |
| 44-17 | 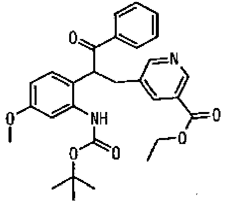 | MS(ESI, m/z) = 505 (M+H)+ |
| 44-18 | 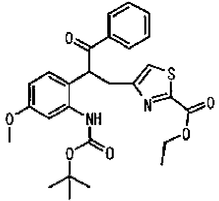 | 1H-NMR (CDCl3) δ ppm:7.95-7.85 (2H, m), 7.65-7.55 (1H, m), 7.50-7.30 (4H, m), 7.16 (1H, s), 7.06 (1H, s), 7.01 (1H, d, J=8.7Hz), 5.34 (1H, dd, J=10.3, 5.0Hz), 4.51 (2H, q, J=7.2Hz), 3.71 (3H, s), 3.64 (1H, dd, J=15.0, 5.0Hz), 3.44 (1H, dd, J=15.0, 10.3Hz), 1.59 (9H, s), 1.45 (3H, t, J=7.2Hz) |
| 44-19 | 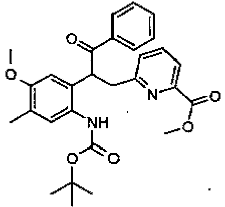 | 1H-NMR (CDCl3) δ ppm:8.00-7.85 (3H, m), 7.76 (1H, s), 7.70-7.55 (1H, m), 7.50-7.20 (4H, m), 7.11 (1H, s), 6.58 (1H, s), 5.66 (1H, dd, J=10.0, 5.1Hz), 4.05 (3H, s), 3.75-3.55 (5H, m), 2.05 (3H, s), 1.60 (9H, s), |
| 44-20 | 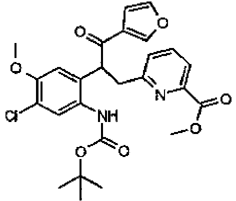 | 1H-NMR (CDCl3) δ ppm:8.15-7.90 (3H, m), 7.75-7.60 (1H, m), 7.45-7.20 (3H, m), 6.73 (2H, s), 5.39 (1H, dd, J=10.7, 4.8Hz), 4.07 (3H, s), 3.79 (3H, s), 3.68 (1H, dd, J=16.3, 4.8Hz), 3.57 (1H, dd, J=16.3, 10.7Hz), 1.65-1.50 (9H, m) |

**[Table 91]**

| Ref.No. | Strc | physical data |
|---|---|---|
| 45-1 | 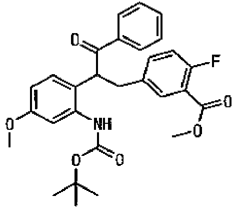 | 1H-NMR (CDCl3) δ ppm:8.00-6.90 (11H, m), 6.59 (1H, dd, J=8.6, 2.6Hz), 4.74 (1H, dd, J=8.1, 6.7Hz), 3.90 (3H, s), 3.75 (3H, s), 3.48 (1H, dd, J=13.7, 6.7Hz), 3.11 (1H, dd, J=13.7, 8.1 Hz), 1.52 (9H, s) |
| 45-2 | 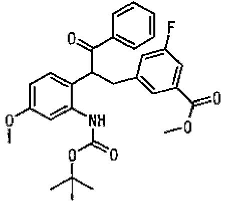 | MS(ESI, m/z) = 506 (M-H)- |
| 45-3 | 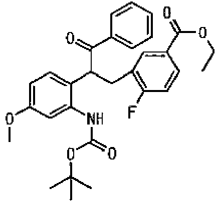 | 1H-NMR (CDCl3) δ ppm:8.00-6.80 (11H, m), 6.55 (1H, dd, J=8.5, 2.6Hz), 4.95-4.80 (1H, m), 4.45-4.25 (2H, m), 3.75 (3H, s), 3.53 (1H, dd, J=13.9 7.7Hz), 3.14 (1H, dd, J=13.9, 6.6Hz), 1.60-1.50 (9H, m), 1.45-1.30 (3H, m) |
| 45-4 | 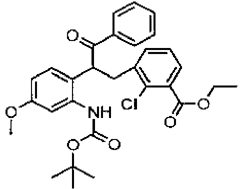 | 1H-NMR (CDCl3) δ ppm:7.90-6.90 (11H, m), 6.53 (1H, dd, J=8.7, 2.6Hz), 5.05-4.90 (1H, m), 4.40 (2H, q, J=7.1Hz), 3.76 (3H, s), 3.66 (1H, dd, J=14.0, 8.1Hz), 3.18 (1H, dd, J=14.0, 6.5Hz), 1.65-1.50 (9H, m), 1.40 (3H, t, J=7.1Hz) |
| 45-5 | 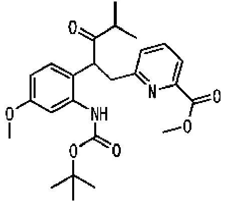 | 1H-NMR (CDCl3) δ ppm:8.00-7.85 (2H, m), 7.70-7.55 (1H, m), 7.25-7.15 (1H, m), 7.05 (1H, s), 6.94 (1H, d, J=8.6Hz), 6.60 (1H, dd, J=8.6, 2.7Hz), 4.95 (1H, dd, J=10.5, 4.9Hz), 4.06 (3H, s), 3.74 (3H, s), 3.58 (1H, dd, J=16.3, 4.9Hz), 3.36 (1H, dd, J=16.3, 10.5Hz), 2.75-2.60 (1H, m), 1.56 (9H, s), 1.10-0.80 (6H, m) |

**[Table 92]**

| Ref.No. | Strc | physical data |
|---|---|---|
| 45-6 | 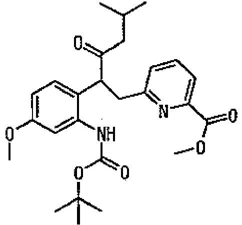 | 1H-NMR (CDCl3) δ ppm:7.95-7.80 (2H, m), 7.70-7.55 (1H, m), 7.25-7.15 (1H, m), 7.06 (1H, s), 6.96 (1H, d, J=8.7Hz), 6.60 (1H, dd, J=8.7, 2.7Hz), 4.85-4.70 (1H, m), 4.06 (3H, s), 3.74 (3H, s), 3.61 (1H, dd, J=16.4, 5.0Hz), 3.35 (1H, dd, J=16.4, 10.4Hz), 2.32 (1 H, dd, J=16.5, 6.5Hz), 2.21 (1H, dd, J=16.5, 7.3Hz), 2.15-2.00 (1H, m), 1.56 (9H, s), 0.83 (3H, d, J=6.7Hz), 0.71 (3H, d, J=6.7Hz) |
| 45-7 | 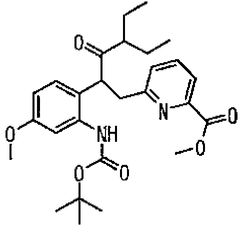 | 1H-NMR (CDCl3) δ ppm:8.02 (1H, s), 7.95-7.85 (1H, m), 7.70-7.55 (1H, m), 7.30-7.20 (1H, m), 7.10-6.90 (2H, m), 6.59 (1H, dd, J=8.7, 2.7Hz), 5.05-4.90 (1H, m), 4.06 (3H, s), 3.73 (3H, s), 3.54 (1H, dd, J=18.5, 4.9Hz), 3.43 (1H, dd, J=16.5, 10.6Hz), 2.45-2.30 (1H, m), 1.70-1.35 (13H, m), 0.82 (3H, t, J=7.5Hz), 0.45 (3H, t, J=7.5Hz) |
| 45-8 | 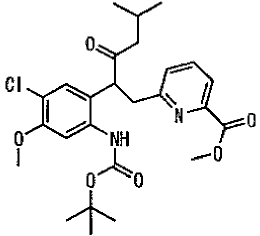 | 1H-NMR (CDCl3)δ ppm:8.05 (1H, s), 8.05-7.95 (1H, m), 7.75-7.60 (1H, m), 7.30-7.00 (3H, m), 4.85-4.70 (1H, m), 4.06 (3H, s), 3.85 (3H, s), 3.60 (1H, dd, J=16.7, 4.8Hz), 3.34 (1H, dd, J=16.7, 10.4Hz), 2.32 (1H, dd, J=16.5, 6.4Hz), 2.21 (1H, dd, J=16.5, 7.3Hz), 2.15-2.00 (1H, m), 1.57 (9H, s), 0.84 (3H, d, J=6.6Hz), 0.73 (3H, d, J=6.7Hz) |
| 45-9 | 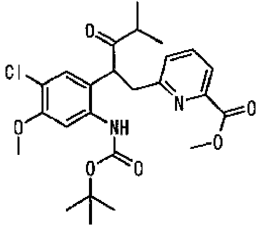 | 1H-NMR (CDCl3) δ ppm:8.12 (1H, s), 7.95-7.85 (1H, m), 7.70-7.60 (1H, m), 7.30-7.20 (1H, m), 7.13 (1H, s), 7.07 (1 H, d, J=2.2Hz), 5.05-4.90 (1H, m), 4.06 (3H, s), 3.85 (3H, s), 3.56 (1H, dd, J=16.5_{.} 6.3Hz), 3.45-3.25 (1H, m), 2.75-2.55 (1H, m), 1.56 (9H, s), 1.07 (3H, d, J=7.1Hz), 0.90 (3H, d, J=7.1Hz) |

**[Table 93]**

| Ref.No. | Strc | physical data |
|---|---|---|
| 46-1 | 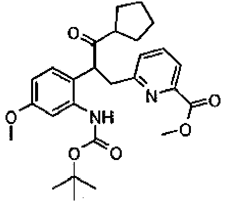 | 1H-NMR (CDCl3) δ ppm:8.00-7.75 (2H, m), 7.70-7.55 (1H, m), 7.25-7.15 (1H, m), 7.04 (1H, s), 6.95 (1H, d, J=8.7Hz), 6.60 (1H, dd, J=8.7, 2.7Hz), 4.90 (1H, dd, J=10.6, 4.8Hz), 4.06 (3H, s), 3.74 (3H, s), 3.60 (1H, dd, J=16.2, 4.8Hz), 3.36 (1H, dd, J=16.2, 10.6Hz), 2.95-2.75 (1H, m), 2.00-1.30 (17H, m) |
| 46-2 | 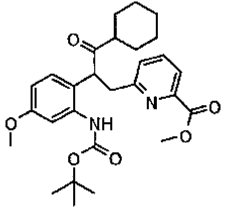 | 1H-NMR (CDCl3) δ ppm:8.00-7.85 (2H, m), 7.70-7.55 (1H, m), 7.25-7.15 (1H, m), 7.03 (1H, s), 6.94 (1H, d, J=8.6Hz), 6.59 (1H, dd, J=8.6, 2.7Hz), 4.91 (1H, dd, J=10.7, 4.9Hz), 4.06 (3H, s), 3.74 (3H, s), 3.55 (1H, dd, J=16.3, 4.9Hz), 3.36 (1H, dd, J=16.3, 10.7Hz), 2.50-2.30 (1H, m), 2.00-1.30 (19H, m) |
| 46-3 | 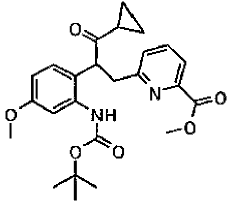 | 1H-NMR (CDCl3) δ ppm:7.00-7.75 (2H, m), 7.65-7.55 (1H, m), 7.20-7.19 (1H, m), 7.09 (1H, s), 6.95 (1H, d, J=8.6Hz), 6.62 (1H, dd, J=8.6, 2.7Hz), 4.91 (1H, dd, J=10,7, 4.5Hz), 4.06 (3H, s), 3.74 (3H, s), 3.66 (1H, dd, J=16.1, 4.5Hz), 3.31 (1H, dd, J=16,1, 10.7Hz), 1.95-1.80 (1H, m), 1.05-0.60 (4H, m) |
| 46-4 | 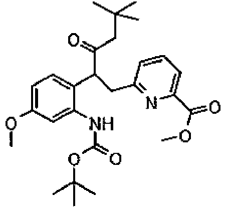 | 1H-NMR (CDCl3) δ ppm:7.95-7.80 (2H, m), 7.70-7.55 (1H, m), 7.25-7.15 (1H, m), 7.04 (1H, s), 6.95 (1H, dd, J=8.6Hz), 6.60 (1H, dd, J=8.6, 2.7Hz), 4.77 (1H, dd, J=10.7, 4.7Hz), 4.06 (3H, s), 3.74 (3H, s), 3.56 (1H, dd, J=16.5, 4.7Hz), 3.35 (1H, dd, J=16.5, 10.7Hz), 2.36 (1H, d, J=15.7Hz), 2.18(1H, d, J=15.7Hz), 1.57 (9H, s), 0.92 (9H, s) |
| 46-5 | 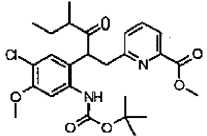 | MS(ESI, m/z) = 505 (M+H)+ |

**[Table 94]**

| Ex. No. | Strc | Physical data |
|---|---|---|
| 12-1 | 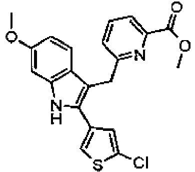 | 1H-NMR (CDCl3) δ ppm:8.07 (1H, s), 8.00-7.90 (1H, m), 7.70-7.60 (1H, m), 7.40-7.20 (4H, m), 6.85 (1H, d, J=2.2Hz), 6.76 (1H, dd, J=8.7, 2.2Hz), 4.48 (2H, s), 4.04 (3H, s), 3.84 (3H, s) |
| 12-2 | 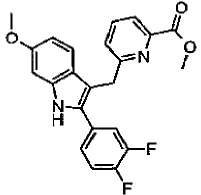 | 1H-NMR (DMSO-d6) δ ppm:11.23 (1H, s), 8.35-8.20 (1H, m), 7.95-7.85 (2H, m), 7.75-7.50 (3H, m), 7.44 (1H, d, J=8.7Hz), 6.85 (1H, d, J=2.2Hz), 6.65 (1H, dd, J=8.7, 2.2Hz), 4.31 (2H, s), 3.89 (3H, s), 3.76 (3H, s) |
| 12-3 | 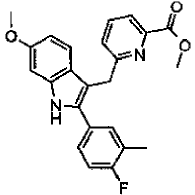 | 1H-NMR (CDCl3) δ ppm:8.06 (1H, s), 8.00-7.90 (1H, m), 7.70-7.55 (1H, m), 7.45-7.15 (4H, m), 7.10-7.00 (1H, m), 6.90 (1H, d, J=2.2Hz), 6.74 (1H, dd, J=8.6, 2.2Hz), 4.50 (2H, s), 4.04 (3H, s), 3.85 (3H, s), 2.29 (3H, d, J=1.8Hz) |
| 12-4 | 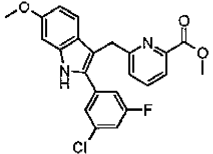 | 1H-NMR (CDCl3) δ ppm:8.06 (1H, 5), 8.00-7.91 (1H, m), 7.70-7.60(1H, m), 7.45-7.20 (4H, m), 7.10-7.00 (1H, m), 6.88 (1H, d, J=2.2Hz), 6.77 (1H, dd, J=8.8, 2.2Hz), 4.50 (2H, s), 4.04 (3H, s), 3.85 (3H, s) |
| 12-5 | 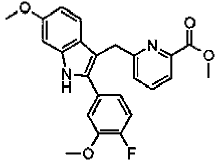 | 1H-NMR (CDCl3) δ ppm:8.07 (1H, s), 8.00-7.90 (1H, m), 7.70-7.60 (1H, m), 7.31 (1H, d, J=8.8Hz), 7.30-7.00 (4H, m), 6.91 (1H, d, J=2.2Hz), 6.77 (1H, dd, J=8.8, 2.2Hz), 4.51 (2H, s), 4.03 (3H, s), 3.86 (3H, s), 3.81 (3H, s) |
| 12-6 | 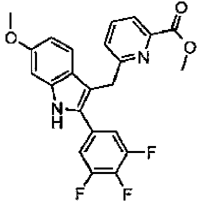 | 1H-NMR (CDCl3) δ ppm:8.12 (1H, s), 8.05-7.90 (1H, m), 7.75-7.65 (1H, m), 7.50-7.25 (4H, m), 6.83 (1H, d, J=2.2Hz), 6.76 (1H, dd, J=8.7, 2.2Hz), 4.43 (2H, s), 4.02 (3H, s), 3.83 (3H, s) |

**[Table 95]**

| Ex. No. | Strc | Physical data |
|---|---|---|
| 12-7 | 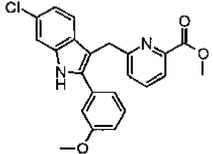 | 1H-NMR (CDCl3) δ ppm:8.26 (1H, s), 8.00-7.90 (1H, m), 7.70-7.60 (1H, m), 7.45-7.25 (3H, m), 7.20-7.00 (4H, m), 6.95-6.85 (1H, m), 4.55 (2H, s), 4.03 (3H, s), 3.78 (3H, s) |
| 12-8 | 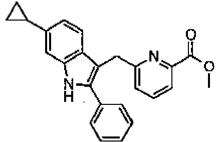 | 1H-NMR (CDCl3) δ ppm: 8.09 (1H, s), 8.00-7.90 (1H, m), 7.65-7.10 (9H, m), 6.83 (1H, dd, J=8.2, 1.4Hz), 4.56 (2H, s), 4.05 (3H, s), 2.10-1.95 (1H, m), 1.05-0.90 (2H, m), 0.80-0.65 (2H, m) |
| 12-9 | 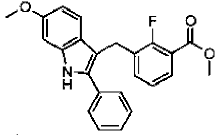 | 1H-NMR (CDCl3) δ ppm:8.08 (1H, s), 7.80-7.70 (1H, m), 7.50-7.30 (5H, m), 7.23 (1H, d, J=8.7Hz), 7.20-7.10 (1H, m), 7.05-6.90 (1H, m), 6.92 (1H, d, J=2.2Hz), 6.75 (1H, dd, J=8.7, 2.2Hz), 4.27 (2H, s), 3.95 (3H, s), 3.86 (3H, s) |
| 12-10 | 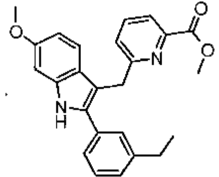 | 1H-NMR (CDCl3) δ ppm:8.14 (1H, s), 8.00-7.90 (1H, m), 7.65-7.55 (1H, m), 7.40-7.10 (6H, m), 6.92 (1H, d, J=2.2Hz), 6.73 (1H, dd, J=8.7, 2.2Hz), 4.56 (2H, s), 4.04 (3H, s), 3.85 (3H, s), 2.66 (2H, q, J=7.6Hz), 1.22 (3H, t, J=7.6Hz) |
| 12-11 | 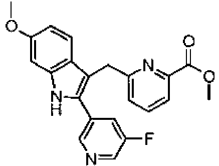 | 1H-NMR (CDCl3) δ ppm:8.70-8.60 (1H, m), 8.50-8.40 (1H, m), 8.11 (1H, s), 8.10-7.95 (2H, m), 7.75-7.60 (1H, m), 7.39 (1H, d, J=8.7Hz), 7.35-7.20 (1H, m), 6.92 (1H, d, J=2.2 Hz), 6.80 (1H, dd, J=8.7, 2.2Hz), 4.48 (2H, s), 4.04 (3H, s), 3.86 (3H, s) |
| 12-12 | 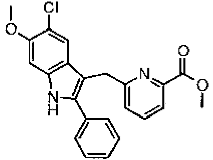 | 1H-NMR (CDCl3) δ ppm: 8.15 (1H, s), 8.05-7.90 (1H, m), 7.70-7.60 (1H, m), 7.55-7.30 (6H, m), 7.20-7.10 (1H, m), 6.96 (1H, s), 4.50 (2H, s), 4.05 (3H, s), 3.94 (3H, s) |

**[Table 96]**

| Ex. No. | Strc | Physical data |
|---|---|---|
| 12-13 | 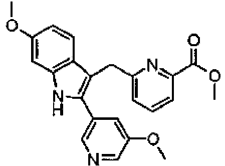 | 1H-NMR (CDCl3) δ ppm:8.45-8.35 (1H, m), 8.30-8.25 (1H, m), 8.17 (1H, s), 8.00-7.90 (1H, m), 7.75-7.60 (1H, m), 7.50-7.45 (1H, m), 7.35 (1H, d, J=8.8Hz), 7.30-7.20 (1H, m), 6.92 (1H, d, J=2.2Hz), 6.78 (1H, dd, J=8.8, 2.2Hz), 4.52 (2H, s), 4.03 (3H, s), 3.87 (3H, s), 3.84 (3H, s) |
| 12-14 | 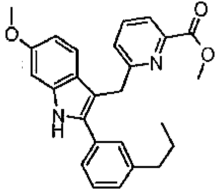 | 1H-NMR (CDCl3) δ ppm:8.13 (1H, s), 8.00-7.90 (1H, m), 7.65-7.55 (1H, m), 7.35-7.10 (6H, m), 6.91 (1H, d, J=2.2Hz), 6.73 (1H, dd, J=8.7, 2.2Hz), 4.56 (2H, s), 4.04 (3H, s), 3.85 (3H, s), 2.59 (2H, t, J=7.6Hz), 1.70-1.55 (2H, m), 0.92 (3H, t, J=7.3Hz) |
| 12-15 | 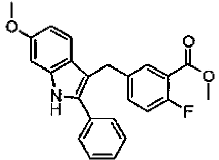 | 1H-NMR (CDCl3) δ ppm:8.06 (1H, s), 7.85-7.75 (1H, m), 7.50-7.25 (6H, m), 7.21 (1H, d, J=8.7Hz), 6.98 (1H, dd, J=10.5, 8.5Hz), 6.91 (1H, d, J=2.3Hz), 6.73 (1H, dd, J=8.7, 2.3Hz), 4.23 (2H, s), 3.89 (3H, s), 3.86 (3H, s) |
| 12-16 | 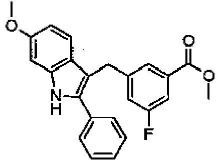 | 1H-NMR (CDCl3) δ ppm:8.07 (1H, s), 7.77 (1H, s), 7.55-7.30 (6H, m), 7.21 (1H, d, J=8.7Hz), 7.10-7.00 (1H, m), 6.92 (1H, d, J=2.2Hz), 6.74 (1H, dd, J=8.7, 2.2Hz), 4.27 (2H, s), 3.89 (3H, s), 3.86 (3H, s) |
| 12-17 | 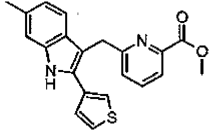 | 1H-NMR (CDCl3) δ ppm:8.06 (1H, s), 8.00-7.90 (1H, m), 7.65-7.50 (2H, m), 7.41 (1H, dd, J=5.1, 2.9Hz), 7.40-7.30 (2H, m), 7.25-7.15 (2H, m), 6.93 (1H, dd, J=8.2, 1.0Hz), 4.56 (2H, s), 4.05 (3H, s), 2.47 (3H, s) |
| 12-18 | 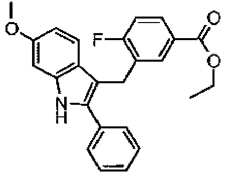 | 1H-NMR (CDCl3) δ ppm:8.05 (1H, s), 7.90-7.70 (2H, m), 7.55-7.30 (5H, m), 7.30-7.20 (1H, m), 7.15-7.00 (1H, m), 6.90 (1H, d, J=2.2Hz), 6.73 (1H, dd, J=8.7, 2.2Hz), 4.30-4.15 (4H, m), 3.85 (3H, s), 1.28 (3H, t, J=7.1Hz) |

**[Table 97]**

| Ex. No. | Strc | Physical data |
|---|---|---|
| 12-19 | 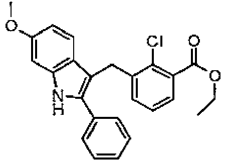 | 1H-NMR (CDCl3) δ ppm:8.11 (1H, s), 7.60-7.50 (1H, m), 7.45-7.29 (5H, m), 7.20 (1H, d, J=8.7Hz), 7.16-7.05 (2H, m), 6.94 (1H, d, J=2.2Hz), 6.75 (1H, dd, J=8.7, 2.2Hz), 4.44 (2H, q, J=7.0Hz), 4.32 (2H, s), 3.87 (3H, s), 1.43 (3H, t, J=7.0Hz) |
| 12-20 | 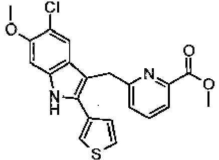 | 1H-NMR (CDCl3) δ ppm: 8.11 (1H, s), 8.00-7.90 (1H, m), 7.70-7.55 (2H, m), 7.48 (1H, s), 7.42 (1H, dd, J=5.1, 3.0Hz), 7.31 (1H, dd, J=5.1, 1.2Hz), 7.25-7.15 (1H, m), 6.94 (1H, s), 4.49 (2H, s), 4.05 (3H, s), 3.94 (3H, s) |
| 12-21 | 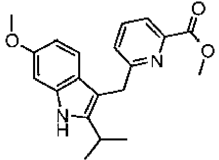 | 1H-NMR (CDCl3) δ ppm:8.00-7.90 (1H, m), 7.83 (1H, s), 7.65-7.55 (1H, m), 7.22 (1H, d, J=8.6Hz), 7.20-7.05 (1H, m), 6.86 (1H, d, J=2.2Hz), 6.70 (1H, dd, J=8.6, 2.2Hz), 4.36 (2H, s), 4.04 (3H, s), 3.82 (3H, s), 3.35-3.15 (1H, m), 1.27 (6H, d, J=7.1Hz) |
| 12-22 | 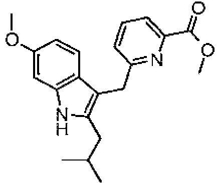 | 1H-NMR (CDCl3) δ ppm:8.00-7.90 (1H, m), 7.80 (1H, s), 7.65-7.50 (1H, m), 7.18 (1H, d, J=8.6Hz), 7.15-7.05 (1H, m), 6.84 (1H, d, J=2.2Hz), 6.69 (1H, dd, J=8.6, 2.2Hz), 4.35 (2H, s), 4.04 (3H, s), 3.82 (3H, s), 2.59 (2H, d, J=7.4Hz), 2.00-1.80 (1H, m), 0.90 (6H, d, J=6.6Hz) |
| 12-23 | 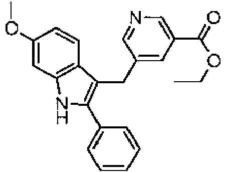 | 1H-NMR (CDCl3) δ ppm:9.10-9.00 (1H, m), 8.70-8.60 (1H, m), 8.20-8.05 (2H, m), 7.50-7.30 (5H, m), 7.21 (1H, d, J=8.7Hz), 6.90 (1H, d, J=2.2Hz), 6.74 (1H, dd, J=8.7, 2.2Hz), 4.35 (2H, q, J=7.2Hz), 4.28 (2H, s), 3.85 (3H, s), 1.36 (3H, t, J=7.2Hz) |
| 12-24 | 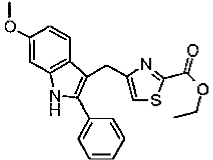 | 1H-NMR (CDCl3) δ ppm:8.08 (1H, s), 7.55-7.25 (6H, m), 7.00-6.95 (1H, m), 6.93 (1H, d, J=2.2Hz), 6.77 (1H, dd, J=8.7, 2.2Hz), 4.51 (2H, q, J=7.2Hz), 4.50 (2H, s), 3.87 (3H, s), 1.46 (3H, t, J=7.2Hz) |

**[Table 98]**

| Ex. No. | Strc | Physical data |
|---|---|---|
| 12-25 | 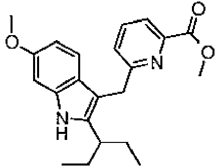 | 1H-NMR (CDCl3) δ ppm:8.00-7.90 (1H, m), 7.70 (1H, s), 7.65-7.50 (1H, m), 7.25-7.10 (2H, m), 6.86 (1H, d, J=2.2Hz), 6.70 (1H, dd, J=8.6, 2.2Hz), 4.37 (2H, s), 4.04 (3H, s), 3.83 (3H, s), 2.80-2.65 (1H, m), 1.80-1.40 (4H, m), 0.75 (6H, t, J=7.4Hz) |
| 12-26 | 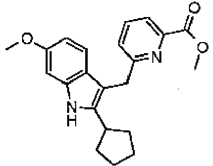 | 1H-NMR (CDCl3) δ ppm:8.00-7.90 (1H, m), 7.85 (1H, s), 7.65-7.50 (1H, m), 7.22 (1H, d, J=8.6Hz), 7.20-7.05 (1H, m), 6.84 (1H, d, J=2.2Hz), 6.70 (1H, dd, J=8.6, 2.2Hz), 4.37 (2H, s), 4.04 (3H, s), 3.82 (3H, s), 3.40-3.20 (1H, m), 2.10-1.50 (8H, m) |
| 12-27 | 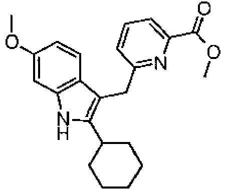 | 1H-NMR (CDCl3) δ ppm:8.00-7.80 (2H, m), 7.65-7.55 (1H, m), 7.21 (1H, d, J=8.7Hz), 7.15-7.05 (1H, m), 6.84 (1H, d, J=2.2Hz), 6.70 (1H, dd, J=8.7, 2.2Hz), 4.37 (2H, s), 4.04 (3H, s), 3.82 (3H, s), 2.95-2.75 (1H, m), 1.95-1.15 (10H, m) |
| 12-28 | 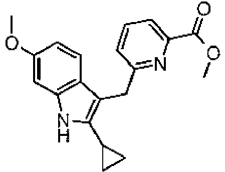 | 1H-NMR (CDCl3) δ ppm:8.00-7.90 (1H, m), 7.75-7.55 (2H, m), 7.25-7.05 (2H, m), 6.80 (1H, d, J=2.2Hz), 6.69 (1H, dd, J=8.6, 2.2Hz), 4.48 (2H, s), 4.04 (3H, s), 3.81 (3H, s), 2.05-1.90 (1H, m), 1.05-0.85 (2H, m), 0.75-0.60 (2H, m) |
| 12-29 | 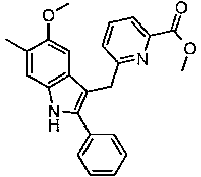 | 1H-NMR (CDCl3) δ ppm:8.10-7.90 (2H, m), 7.70-7.10 (8H, m), 6.78 (1H, s), 4.57 (2H, s), 4.05 (3H, s), 3.74 (3H, s), 2.33 (3H, s) |

**[Table 99]**

| Ex. No. | Strc | Physical data |
|---|---|---|
| 12-30 | 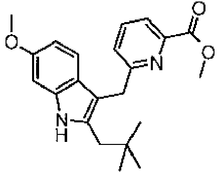 | 1H-NMR (CDCl3) δ ppm:8.00-7.90 (1H, m), 7.78 (1H, s), 7.65-7.50 (1H, m), 7.20-7.05 (2H, m), 6.85 (1 H, dd, J=2.2Hz), 6.68 (1H, dd, J=8.6, 2.2Hz), 4.37 (2H, s), 4.04 (3H, s), 3.83 (3H, s), 2.62 (2H, s), 0.96 (9H, s) |
| 12-31 | 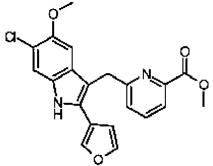 | 1H-NMR (CDCl3) δ ppm:8.01 (1H, s), 8.00-7.90 (1H, m), 7.79 (1H, dd, J=1.5, 1.0Hz), 7.70-7.60 (1H, m), 7.55-7.45 (1H, m), 7.39 (1H, s), 7.20-7.10(1H, m), 6.98 (1H, s), 6.65 (1H, dd, J=1.9, 1.0Hz), 4.48 (2H, s), 4.05 (3H, s), 3.86 (3H, s) |
| 12-32 | 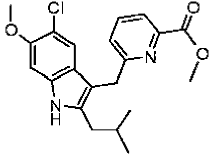 | 1H-NMR (CDCl3) δ ppm:8.00-7.80 (1H, m), 7.79 (1H, s), 7.70-7.55 (1H, m), 7.36 (1H, s), 7.15-7.05 (1H, m), 6.88 (1H, s), 4.31 (2H, s), 4.05 (3H, s), 3.91 (3H, s), 2.59 (2H, d, J=7.4Hz), 2.00-1.80 (1H, m), 0.90 (6H, d, J=6.6Hz) |
| 12-33 | 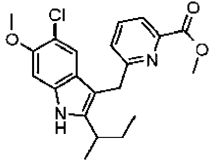 | 1H-NMR (CDCl3) δ ppm:8.00-7.90 (1H,m), 7.79 (1H, s), 7.70-7.55 (1H, m), 7.39 (1H, s), 7.15-7.05 (1H, m), 6.90 (1H, s), 4.32 (2H, s), 4.04 (3H, s), 3.91 (3H, s), 3.10-2.90 (1H, m), 1.75-1.50 (2H, m), 1.25 (3H, d, J=7.0Hz), 0.79 (3H, t, J=7.4Hz) |
| 12-34 | 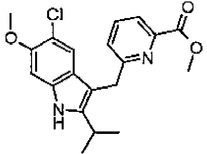 | 1H-NMR (CDCl3) δ ppm:8.00-7.85 (2H, m), 7.70-7.55 (1H, m), 7.39 (1H, s), 7.15-7.05 (1H, m), 6.89 (1H, s), 4.31 (2H, s), 4.04 (3H, s), 3.89 (3H, s), 3.35-3.15 (1H, m), 1.27 (6H, d, J=6.8Hz) |

**[Table 100]**

| Ex. No. | Strc | Physical data |
|---|---|---|
| 13-1 | 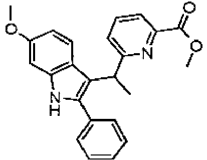 | 1H-NMR (CDCl3) δ ppm:8.01 (1H, s), 8.00-7.85 (1H, m), 7.75-7.30 (8H, m), 6.90 (1H, d, J=2.2Hz), 6.73 (1H, dd, d=8.8, 2.2Hz), 4.90-4.75 (1H, m), 4.01 (3H, s), 3.85 (3H, s), 1.92 (3H, d, J=6.7Hz) |
| 13-2 | 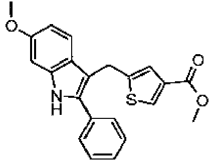 | 1H-NMR (CDCl3) δ ppm:8.05 (1H, s), 7.89 (1H, d, J=1.3Hz), 7.55-7.30 (6H, m), 7.25 (1H, d, J=1.3Hz), 6.91 (1H, d, J=2.3Hz), 6.78 (1H, dd, J=8.8, 2.3Hz), 4.33 (2H, d, J=0.8Hz), 3.86 (3H, s), 3.81 (3H, s) |
| 13-3 | 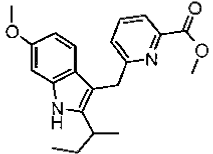 | 1H-NMR (CDCl3) δ ppm:7.90-7.80 (1H, m), 7.75 (1H, s), 7.65-7.55 (1H, m), 7.22 (1H, d, J=8.6Hz), 7.20-7.10 (1H, m), 6.86 (1H, d, J=2.2Hz), 6.70 (1H, dd, J=8.6, 2.2Hz), 4.36 (2H, s), 4.04 (3H, s), 3.83 (3H, s), 3.05-2.90 (1H, m), 1.75-1.50 (2H, m), 1.25 (3H, d, J=7.0Hz), 0.79 (3H, t, J=7.4Hz) |
| 13-4 | 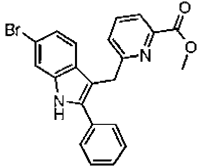 | 1H-NMR (CDCl3) δ ppm:8.28 (1H, s), 8.00-7.90 (1H, m), 7.70-7.10 (10H, m), 4.54 (2H, s), 4.04 (3H, s) |
| 13-5 | 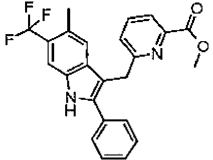 | 1H-NMR (CDCl3) δ ppm:8.33 (1H, s), 8.05-7.95 (1H, m), 7.75-7.35 (7H, m), 7.31 (1H, s), 7.20-7.10 (1H, m), 4.55 (2H, s), 4.05 (3H, s), 2.49 (3H, d, J=1.1Hz) |
| 13-6 | 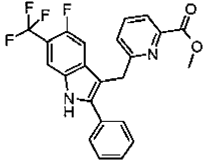 | 1H-NMR (CDCl3) δ ppm:8.40 (1H, s), 8.05-7.95 (1H, m), 7.75-7.35 (7H, m), 7.30-7.10 (2H, m), 4.50 (2H, s), 4.04 (3H, s) |

**[Table 101]**

| Ex. No. | Strc | Physical data |
|---|---|---|
| 13-7 | 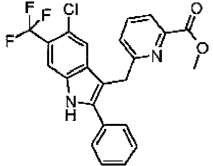 | 1H-NMR (CDCl3) δ ppm:8.43 (1H, s), 8.05-7.95 (1H, m), 7.76 (1H, s), 7.75-7.35 (7H, m), 7.20-7.10 (1H, m), 4.51 (2H, s), 4.05 (3H ,s) |
| 13-8 | 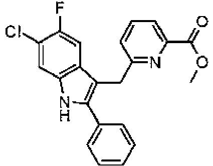 | 1H-NMR (CDCl3) δ ppm:8.17 (1H, s), 7.97 (1H, m), 7.75-7.35 (7H, m), 7.25-7.10 (2H, m), 4.49 (2H, s), 4.04 (3H, s) |
| 13-9 | 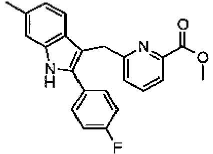 | 1H-NMR (CDCl3) δ ppm:8.03 (1H, s), 8.00-7.90 (1H, m), 7.70-7.45 (3H, m), 7.29 (1H, d, J=8.1 Hz), 7.25-7.05 (4H, m), 6.92 (1H, dd, J=8.1, 1.0Hz), 4.51 (2H, s), 4.04 (3H, s), 2.47 (3H, s) |
| 13-10 | 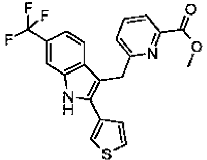 | 1H-NMR (CDCl3) δ ppm:8.41 (1H, s), 8.05-7.95 (1H, m), 7.72 (1H, dd, J=2.9, 1.3Hz), 7.70-7.50 (3H, m), 7.46 (1H, dd, J=5.1, 2.9Hz), 7.37 (1H, dd, J=5.1, 1.3Hz), 7.35-7.30 (1H, m), 7.25-7.15 (1H, m), 4.57 (2H, s), 4.04 (3H, s) |
| 13-11 | 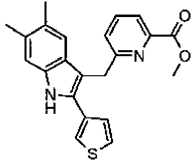 | 1H-NMR (CDCl3) δ ppm:8.05-7.90 (2H, m), 7.65-7.55 (1H, m), 7.51 (1H, dd, J=3.0, 1.3Hz), 7.40 (1H, dd, J=5.1, 3.0Hz), 7.31 (1H, dd, J=5.1, 1.3Hz), 7.25-7.15 (3H, m), 4.54 (2H, s), 4.05 (3H, s), 2.37 (3H, s), 2.30 (3H, s) |
| 13-12 | 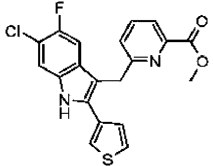 | 1H-NMR (CDCl3) δ ppm:8.13 (1H, s), 8.05-7.90 (1H, m), 7.70-7.60 (2H, m), 7.50-7.35 (2H, m), 7.34 (1H, dd, J=5.0, 1.3Hz), 7.5-7.15 (2H, m), 4.48 (2H, s), 4.04 (3H, s) |

**[Table 102]**

| Ex. No. | Strc | Physical data |
|---|---|---|
| 13-13 | 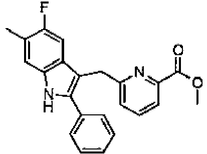 | 1H-NMR (CDCl3) δ ppm:8.06 (1H, m), 8.00-7.90 (1H, m), 7.70-7.30 (6H, m), 7.20-7.10 (2H, m), 7.01 (1H, d, J=10.3Hz), 4.51 (2H, s), 4.05 (3H, s), 2.37 (3H, d, J=2.0Hz) |
| 13-14 | 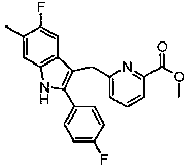 | 1H-NMR (CDCl3) δ ppm:8.10-7.90 (2H, m), 7.70-7.50 (3H, m), 7.25-7.10 (4H, m), 7.03 (7H, d, J=10.3Hz), 4.45 (2H, s), 4.04 (3H, s), 2.37 (3H, d, J=2.0Hz) |
| 13-15 | 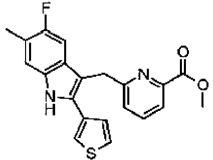 | 1H-NMR (CDCl3) δ ppm:8.10-7.90 (2H, m), 7.70-7.55 (2H, m), 7.42 (1H, dd, J=5.1, 2.9Hz), 7.33 (1H, dd, J=5.1, 1.3Hz), 7.25-7.10 (2H, m), 7.06 (1H, d, J=10.3 Hz), 4.50 (2H, s), 4.05 (3H, s), 2.37 (3H, d, J=1.9Hz) |
| 13-16 | 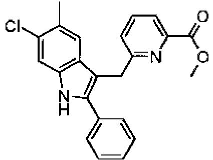 | 1H-NMR (CDCl3) δ ppm:8.09 (1H, s), 8.05-7.90(1H, m), 7.70-7.10 (9H, m), 4.53 (2H, s), 4.05 (3H, s), 2.38 (3H, s) |
| 13-17 | 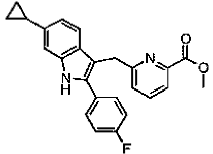 | 1H-NMR (CDCl3) δ ppm:8.02 (1H, s), 8.00-7.90 (1H, m), 7.70-7.45 (3H, m), 7.29 (1H, d, J=8.3Hz), 7.25-7.05 (4H, m), 6.84 (1H, dd, J=8.3, 1.4Hz), 4.50 (2H, s), 4.04 (3H, s), 2.10-1.95 (1H, m), 1.05-0.90 (2H, m), 0.85-0.65 (2H, m) |
| 13-18 | 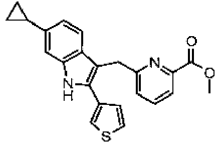 | 1H-NMR (CDCl3) δ ppm:8.05 (1H, s), 8.00-7.90 (1H, m), 7.65-7.55 (1H, m), 7.54 (1H, dd, J=3.0, 1.3Hz), 7.41 (1H, dd, J=5.1, 3.0Hz), 7.35-7.05 (4H, m), 6.85 (1H, dd, J=8.2, 1.5Hz), 4.55 (2H, s), 4.05 (3H, s), 2.10-1.95 (1H, m), 1.05-0.90 (2H, m), 0.80-0.65 (2H, m) |

**[Table 103]**

| Ex. No. | Strc | Physical data |
|---|---|---|
| 13-19 | 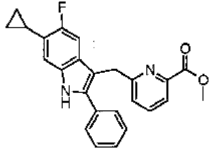 | 1H-NMR (CDCl3) δ ppm:8.06 (1H, s), 8.00-7.90 (1H, m), 7.70-7.30 (6H, m), 7.20-7.10 (1H, m), 7.02 (1H, d, J=10.8Hz), 6.95 (1H, d, J=6.2Hz), 4.51 (2H, s), 4.05 (3H, s), 2.25-2.10 (1H, m), 1.05-0.90 (2H, m), 0.80-0.65 (2H, m) |
| 13-20 | 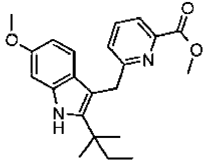 | 1H-NMR (CDCl3) δ ppm:8.00-7.90 (1H, m), 7.87 (1H, s), 7.65-7.50 (1H, m), 7.14 (1H, d, J=8.6Hz), 7.10-7.00 (1H, m), 6.87 (1H, d, J=2.3Hz), 6.69 (1H, dd, J=8.6, 2.3Hz), 4.55 (2H, s), 4.05 (3H, s), 3.84 (3H, s), 1.69 (2H, q, J=7.5Hz), 1.36 (6H, s), 0.71 (3H, t, J=7.5Hz) |
| 13-21 | 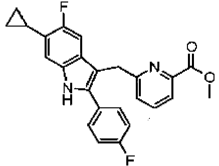 | 1H-NMR (CDCl3) δ ppm:8.10-7.90 (2H, m), 7.70-7.45 (3H, m), 7.25-7.05 (3H, m), 7.03 (1H, d, J=10.8Hz), 6.94 (1H, d, J=6.2Hz), 4.45 (2H, s), 4.04 (3H, s), 2.25-2.10 (1H, m), 1.05-0.90 (2H, m), 0.75-0.65 (2H, m) |
| 13-22 | 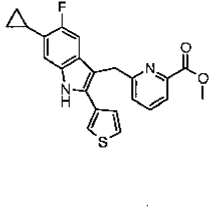 | 1H-NMR (CDCl3) δ ppm:8.03 (1H, s), 8.00-7.90 (1H, m), 7.70-7.60 (1H, m), 7.59 (1H, dd, J=3.0, 1.3Hz), 7.42 (1H, dd, J=5.1, 3.0Hz), 7.31 (1H, dd, J=5.0, 1.3Hz), 7.25-7.15 (1H, m), 7.07 (1H, d, J=10.7Hz), 6.92 (1H, d, J=6.1Hz), 4.49 (2H, s), 4.05 (3H, s), 2.25-2.10 (1H, m), 1.05-0.90 (2H, m), 0.80-0.65 (2H, m) |
| 13-23 | 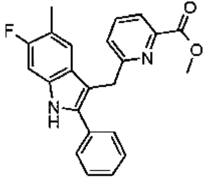 | 1H-NMR (CDCl3) δ ppm:8.16 (1H, s), 8.00-7.90 (1H, m), 7.70-7.25 (6H, m), 7.20-7.10 (2H, m), 7.06 (1H, d, J=10.7Hz), 4.53 (2H, s), 4.05 (3H, s), 2.29 (3H, d, J=1.9Hz) |

**[Table 104]**

| Ex. No. | Strc | Physical data |
|---|---|---|
| 13-24 | 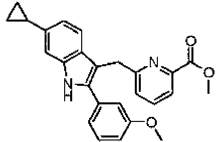 | 1H-NMR (CDCl3) δ ppm:8.08 (1H, s), 8.00-7.90 (1H, m), 7.65-7.55 (1H, m), 7.40-7.30 (1H, m), 7.28 (1H, d, J=8.2Hz), 7.25-7.00 (4H, m), 6.95-6.85 (1H, m), 6.83 (1H, dd, J=8.2, 1.4Hz), 4.57 (2H, s), 4.04 (3H, s), 3.77 (3H, s), 2.10-1.95 (1H, m), 1.05-0.90 (2H, m), 0.80-0.65 (2H, m) |
| 13-25 | 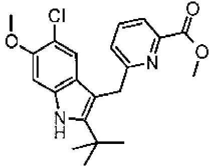 | 1H-NMR (CDCl3) δ ppm:8.00-7.90 (2H, m), 7.65-7.55 (1H, m), 7.28 (1H, s), 7.05-6.95 (1H, m), 6.92 (1H, s), 4.52 (2H, s), 4.05 (3H, s), 3.91 (3H, s), 1.39 (9H, s) |
| 13-26 | 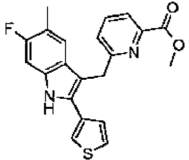 | 1H-NMR (CDCl3) δ ppm:8.09 (1H, s), 8.00-7.90 (1H, m), 7.70-7.60 (1H, m), 7.53 (1H, dd, J=3.0, 1.3Hz), 7.41 (1H, dd, J=5.0, 3.0Hz), 7.40 (1H, dd, J=5.0, 1.3Hz), 7.25-7.15 (2H, m), 7.04 (1H, d, J=10.2Hz). 4.52 (2H, s), 4.05 (3H, s), 2.31 (3H, d, J=2.0Hz) |
| 13-27 | 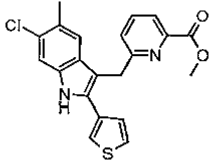 | 1H-NMR (CDCl3) δ ppm:8.11 (1H, s), 8.00-7.90 (1H, m), 7.70-7.60 (1H, m), 7.57 (1H, dd, J=3.0, 1.3 Hz), 7.42 (1H, dd, J=5.1, 3.0Hz), 7.40 (1H, s), 7.31 (1H, d, J=5.1, 1.3Hz), 7.29 (1H, s), 7.25-7.15 (1H, m), 4.52 (2H, s), 4.05 (3H, s), 2.40 (3H, s) |
| 13-28 | 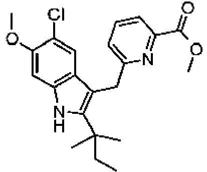 | 1H-NMR (CDCl3) δ ppm:8.00-7.85 (2H, m), 7.65-7.55 (1H, m), 7.28 (1H, s), 7.05-6.95 (1H, m), 6.92 (1H,s), 4.50 (2H, s), 4.05 (3H, s), 3.92 (3H, s), 1.69 (2H, q, J=7.4Hz), 1.36 (6H, s), 0.70 (3H, t, J=7.4Hz) |

**[Table 105]**

| Ex. No. | Strc | Physical data |
|---|---|---|
| 13-29 | 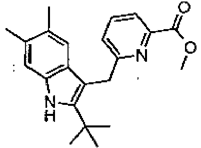 | 1H-NMR (CDCl3) δ ppm:8.00-7.90 (1H, m), 7.85 (1H,s), 7.65-7.50 (1H,m), 7.13 (1H,s), 7.10-7.00 (2H, m), 4.57 (2H, s), 4.05 (3H, s), 2.34 (3H, s), 2.25 (3H, s), 1.38 (9H, s) |
| 13-30 | 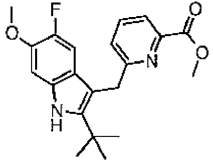 | 1H-NMR (CDCl3) δ ppm:8.00-7.85 (2H, m), 7.65-7.55 (1H, m), 7.05-6.95 (1H, m), 6.94 (1H, d, J=11.6Hz), 6.93 (1H, d, J=7.1 Hz), 4.52 (2H, s), 4.05 (3H, s), 3.90 (3H, s), 1.40 (9H, s) |
| 13-31 | 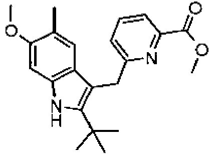 | 1H-NMR (CDCl3) δ ppm:8.00-7.90 (1H, m), 7.85 (1H, s), 7.65-7.50 (1H, m), 7.10-6.95 (2H, m), 6.82 (1H, s), 4.55 (2H, s), 4.05 (3H, s), 3.85 (3H, s), 2.20 (3H, s), 1.38 (9H, s) |
| 13-32 | 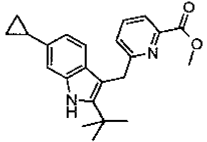 | 1H-NMR (CDCl3) δ ppm:8.00-7.80 (2H, m), 7.60-7.50 (1H, m), 7.14 (1H, d, J=8.2Hz), 7.10-7.00 (2H, m), 6.79 (1H, dd, J=8.2, 1.5Hz), 4.57 (2H, s), 4.04 (3H, s), 2.05-1.90 (1H, m), 1.39 (9H, s), 1.00-0.85 (2H, m), 0.75-0.65 (2H, m) |
| 13-33 | 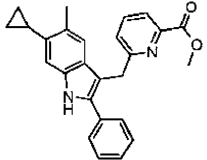 | 1H-NMR (CDCl3) δ ppm:8.03 (1H, s), 8.00-7.90 (1H, m), 7.70-7.15 (8H, m), 7.10 (1H, s), 4.55 (2H, s), 4.05 (3H, s), 2.44 (3H, s), 2.05-1.90 (1H, m), 1.00-0.90 (2H, m), 0.70-0.60 (2H, m) |
| 13-34 | 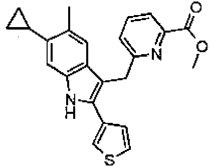 | 1H-NMR (CDCl3) δ ppm:8.01 (1H, s), 8.00-7.90 (1H, m), 7.65-7.55 (1H, m), 7.51 (1H, dd, J=2.9, 1.3Hz), 7.40 (1H, dd, J=5.1, 2.9Hz), 7.30 (1H, dd, J=5.1, 1.3Hz), 7.25-7.15 (2H, m), 7.07 (1H, s), 4.54 (2H, s), 4.05 (3H, s), 2.46 (3H, s), 2.05-1.90 (1H, m), 1.00-0.90 (2H, m), 0.70-0.60 (2H, m) |

**[Table 106]**

| Ex. No. | Strc | Physical data |
|---|---|---|
| 13-35 | 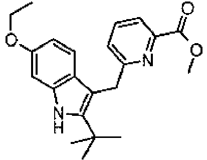 | 1H-NMR (CDCl3) δ ppm::8.00-7.80 (2H, m), 7.65-7.50 (1H, m), 7.12 (1H, d, J=8.6Hz), 7.10-7.00 (1H, m), 6.86 (1H, d, J=2.2Hz), 6.68 (1H, dd, J=8.6, 2.2Hz), 4.56 (2H, s), 4.10-4.00 (5H, m), 1.42 (3H, t, J=7.1 Hz), 1.39 (9H, s) |
| 14-1 | 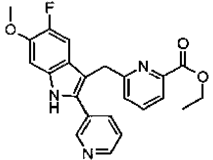 | 1H-NMR (CDCl3) δ ppm:8.90-8.80 (1H, m), 8.65-8.55 (1H, m), 8.22 (1H, s), 8.10-7.90 (2H, m), 7.75-7.60 (1H, m), 7.40-7.30 (1H, m), 7.30-7.15 (2H, m), 7.00-6.90 (1H, m), 4.49 (2H, q, J=7.2Hz), 4.43 (2H, s), 3.93 (3H, s), 1.46 (3H, t, J=7.2Hz) |
| 14-2 | 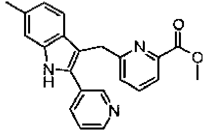 | 1H-NMR (CDCl3) δ ppm:8.85-8.75 (1H, m), 8.65-8.55 (1H, m), 8.16 (1H, s), 8.00-7.90 (2H, m), 7.70-7.55 (1H, m), 7.40-7.15 (4H, m), 6.95 (1H, dd, J=8.1, 1.1Hz), 4.52 (2H, s), 4.04 (3H, s), 2.48 (3H, s) |
| 14-3 | 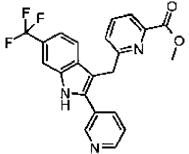 | 1H-NMR (CDCl3) δ ppm:8.95-8.85 (1H, m), 8.78 (1H, s), 8.70-8.55 (1H, m), 8.10-7.90 (2H, m), 7.75-7.50 (3H, m), 7.45-7.15 (3H, m), 4.51 (2H, s), 4.02 (3H, s) |
| 14-4 | 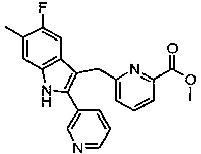 | 1H-NMR (CDCl3) δ ppm:8.85-8.80 (1H, m), 8.65-8.55 (1H, m), 8.20 (1H, s), 8.05-7.90 (2H, m), 1.70-7.60 (1H, m), 7.40-7.30 (1H, m), 7.25-7.15 (2H, m), 7.07 (1H, d, J=10.2Hz), 4.46 (2H, s), 4.04 (3H, s), 2.38 (3H, d, J=1.8Hz) |
| 14-5 | 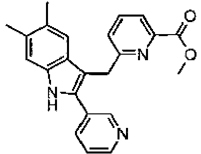 | 1H-NMR (DMSO-d6) δ ppm:11.22 (1H, s), 8.95-8.90 (1H, m), 8.60-8.50 (1H, m), 8.35-8.25 (1H, m), 7.90-7.80 (2H, m), 7.55-7.40 (2H, m), 7.29 (1H, s), 7.18 (1H, s), 4.33 (2H, s), 3.89 (3H, s), 2.30 (3H, s), 2.25 (3H, s) |

**[Table 107]**

| Ex. No. | Strc | Physical data |
|---|---|---|
| 14-6 | 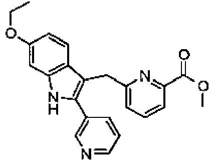 | 1H-NMR (CDCl3) δ ppm:8.85-8.75 (1H, m), 8.60-8.50 (1H, m), 8.15 (1H, s), 8.00-7.85 (2H, m), 7.70-7.60 (1H, m), 7.40-7.15 (3H, m), 6.92 (1H, d, J=2.2Hz), 6.77 (1H, dd, J=8.7, 2.2Hz), 4.51 (2H, s), 4.08 (2H, q, J=7.0Hz), 4.04 (3H, s), 1.45 (3H, t, J=7.0Hz) |
| 14-7 | 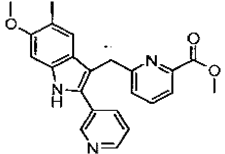 | 1H-NMR (CDCl3) δ ppm:8.85-8.75 (1H, m), 8.60-8.50 (1H, m), 8.12 (1H, s), 8.00-7.85 (2H, m), 7.70-7.60 (1H, m), 7.40-7.30 (1H, m), 7.25-7.15 (2H, m), 6.88 (1H, s), 4.50 (2H, s), 4.05 (3H, s), 3.89 (3H, s), 2.25 (3H, s) |
| 14-8 | 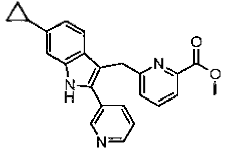 | 1H-NMR (CDCl3) δ ppm:8.85-8.75 (1H, m), 8.65-8.50 (1H, m), 8.17 (1H, s), 8.00-7.90 (2H, m), 7.70-7.60 (1H, m), 7.40-7.30 (2H, m), 7.25-7.10 (2H, m), 6.86 (1H, dd, J=8.3, 1.4Hz), 4.52 (2H, s), 4.04 (3H, s), 2.10-1.95 (1H, m), 1.05-0.90 (2H, m), 0.80-0.65 (2H, m) |
| 14-9 | 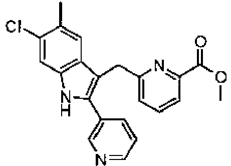 | 1H-NMR (DMSO-d6) δ ppm:11.56 (1H, s), 9.00-8.90(1H, m), 8.65-8.55 (1H, m), 8.45-8.30 (1H, m), 7.95-7.80 (2H, m), 7.60-7.45 (3H, m), 7.42 (1H, s), 4.35 (2H, s), 3.89 (3H, s), 2.36 (3H, s) |
| 14-10 | 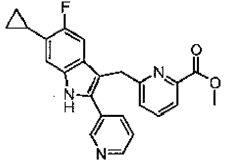 | 1H-NMR (CDCl3) δ ppm:8.85-8.75 (1H, m), 8.65-8.55 (1H, m), 8.14 (1H, s), 8.05-7.90 (2H, m), 7.70-7.60 (1H, m), 7.45-7.30 (1H, m), 7.25-7.15 (1H, m), 7.08 (1H, d, J=10.7Hz), 6.97 (1H, d, J=6.1Hz), 4.46 (2H, s), 4.04 (3H, s), 2.25-2.10 (1H, m), 1.05-0.95 (2H, m), 0.80-0.65 (2H, m) |

**[Table 108]**

| Ex. No. | Strc | Physical data |
|---|---|---|
| 14-11 | 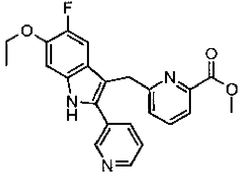 | 1H-NMR (CDCl3) δ ppm::8.85-8.75 (1H, m), 8.65-8.55 (1H, m), 8.17 (1H, s), 8.05-7.90 (2H, m), 7.75-7.60 (1H, m), 7.40-7.30 (1H, m), 7.25-7.10 (2H, m), 6.98 (1H, d, J=6.9Hz), 4.45 (2H, s), 4.13 (2H, q, J=7.0Hz), 4.04 (3H, s), 1.49 (3H, t, J=7.0Hz) |
| 14-12 | 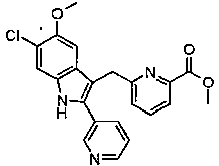 | 1H-NMR (CDCl3) δ ppm:8.90-8.75 (1H, m), 8.65-8.55 (1H, m), 8.31 (1H, s), 8.05-7.85 (2H, m), 7.75-7.60 (1H, m), 7.50-7.30 (2H, m), 7.20-7.15 (1H, m), 6.97 (1H, s), 4.50 (2H, s), 4.03 (3H, s), 3.84 (3H, s) |
| 14-13 | 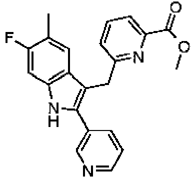 | 1H-NMR (CDCl3) δ ppm:8.85-8.75 (1H, m), 8.75-8.55 (2H, m), 8.15-7.95 (2H, m), 7.75-7.60 (1H, m), 7.40-7.30 (1H, m), 7.25-7.15 (2H, m), 7.05 (1H, d, J=10.1Hz), 4.48 (2H, s), 4.03 (3H, s), 2.30 (3H, d, J=2.0Hz) |
| 14-14 | 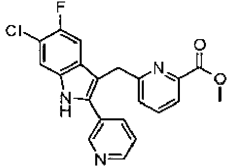 | 1H-NMR (DMSO-d6) δ ppm:11.78 (1H, s), 9.05-8.95 (1H, m), 8.65-8.55 (1H, m), 8.50-8.40 (1H, m), 7.95-7.85 (2H, m), 7.63 (1H, d, J=10.3Hz), 7.60-7.50 (3H, m), 4.34 (2H, s), 3.88 (3H, s) |
| 14-15 | 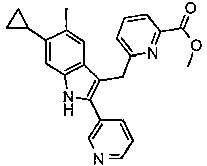 | 1H-NMR (CDCl3) δ ppm:8.85-8.75 (1H, m), 8.60-8.50 (1H, m), 8.09 (1H, s), 8.00-7.85 (2H, m), 7.70-7.60 (1H, m), 7.40-7.30 (1H, m), 7.25-7.05 (3H, m), 4.51 (2H, s), 4.05 (3H, s), 2.46 (3H, s), 2.05-1.90 (1H, m), 1.05-0.90 (2H, m), 0.75-0.60 (2H, m) |

**[Table 109]**

| Ex. No. | Strc | Physical data |
|---|---|---|
| 15-1 | 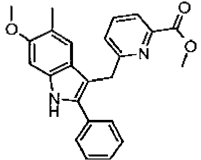 | 1H-NMR (CDCl3) δ ppm::8.06 (1H, s), 8.00-7.90 (1H, m), 7.70-7.15 (7H, m), 7.13 (1H, s), 6.87 (1H, s), 4.54 (2H, s), 4.05 (3H, s), 3.88 (3H, s), 2.23 (3H, s) |
| 15-2 | 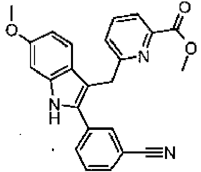 | 1H-NMR (CDCl3) δ ppm:8.21 (1H, s), 8.05-7.95 (2H, m), 7.90-7.80 (1H, m), 7.75-7.45 (3H, m), 7.33 (1H, d, J=8.7Hz), 7.30-7.20 (1H, m), 6.88 (1H, d, J=2.2Hz), 6.77 (1H, dd, J=8.7, 2.2Hz), 4.48 (2H, s), 4.04 (3H, s), 3.85 (3H,s) |
| 15-3 | 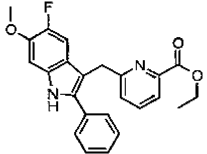 | 1H-NMR (CDCl3) δ ppm:8.10 (1H, s), 8.00-7.90 (1H, m), 7.70-7.30 (6H, m), 7.25-7.10 (2H, m), 6.97 (1H, d, J=7.0Hz), 4.51 (2H, q, J=7.1Hz), 4.49 (2H, s), 3.93 (3H, s), 1.47 (3H, t, J=7.1Hz) |
| 15-4 | 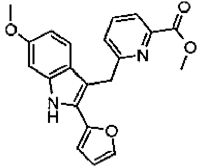 | 1H-NMR (CDCl3) δ ppm:8.43 (1H, s), 8.00-7.90 (1H, m), 7.65-7.55 (1H, m), 7.44 (1H, d, J=1.7Hz), 7.36 (1H, d, J=8.7Hz), 7.20-7.10 (1H, m), 6.89 (1H, d, J=2.2Hz), 6.76 (1H, dd, J=8.7, 2.2Hz), 6.52 (1H, d, J=3.4Hz), 6.46 (1H, dd, J=3.4, 1.7Hz), 4.59 (2H, s), 4.05 (3H, s), 3.86 (3H, s) |
| 15-5 | 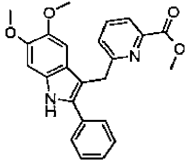 | 1H-NMR (CDCl3) δ ppm:8.08 (1H, s), 8.00-7.90 (1H, m), 7.70-7.10 (7H, m), 6.93 (1H, s), 6.86 (1H, s), 4.56 (2H, s), 4.04 (3H, s), 3.93 (3H, s), 3.81 (3H, s) |

**[Table 110]**

| Ex. No. | Strc | Physical data |
|---|---|---|
| 15-6 | 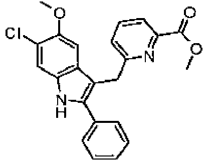 | 1H-NMR (CDCl3) δ ppm:8.88 (1H, s), 8.00-7.90 (1H, m), 7.70-7.30 (7H, m), 7.20-7.10 (1H, m), 6.93 (1H, s), 4.54 (2H, s), 4.04 (3H, s), 3.82 (3H, s) |
| 15-7 | 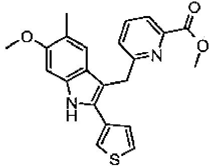 | 1H-NMR (CDCl3) δ ppm:8.06 (1H, s), 8.00-7.90 (1H, m), 7.70-7.55 (1H, m), 7.46 (1H, dd, J=3.0, 1.2Hz), 7.39 (1H, dd, J=5.0, 3.0Hz), 7.29 (1H, dd, J=5.0, 1.2Hz), 7.25-7.15 (2H, m), 6.84 (1H, s), 4.53 (2H, s), 4.05 (3H, s), 3.87 (3H, s), 2.25 (3H, s) |
| 15-8 | 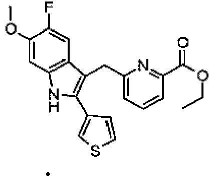 | 1H-NMR (CDCl3) δ ppm:8.07 (1H, s), 8.00-7.90 (1H, m), 7.75-7.60 (2H, m), 7.43 (1H, d, J=5.0, 3.0Hz), 7.35 (1H, dd, J=5.0, 1.2Hz), 7.30-7.15 (2H, m), 6.94 (1H, d, J=7.0Hz), 4.50 (2H, q, J=7.0Hz), 4.47 (2H, s), 3.92 (3H, s), 1.48 (3H, t, J=7.0Hz) |
| 15-9 | 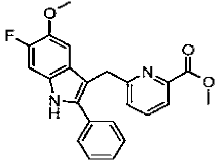 | 1H-NMR (CDCl3) δ ppm:8.10 (1H, s), 8.00-7.90 (1H, m), 7.70-7.60 (1H, m), 7.55-7.30 (5H, m), 7.20-7.10 (2H, m), 6.95 (1H, d, J=8.3 Hz), 4.54 (2H, s), 4.04 (3H, s), 3.82(3H,s) |
| 15-10 | 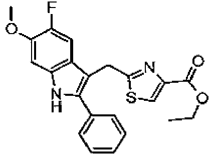 | 1H-NMR (CDCl3) δ ppm:8.18 (1H, s), 8.01 (1H, s), 7.55-7.30 (5H, m), 7.14 (1H, d, J=11.3Hz), 6.98 (1H, d, J=7.0Hz), 4.56 (2H, s), 4.45 (2H, q, J=7.1Hz), 3.94 (3H, s), 1.43 (3H, t, J=7.1Hz) |

**[Table 111]**

| Ex. No. | Strc | Physical data |
|---|---|---|
| 15-11 | 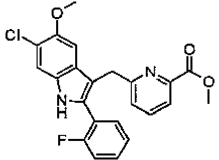 | 1H-NMR (CDCl3) δ ppm:8.30 (1H, s), 7.90-8.05 (1H, m), 7.70-7.60 (1H, m), 7.55-7.30 (3H, m), 7.25-7.10 (3H, m), 6.93 (1H, s), 4.48 (2H, s), 4.03 (3H, s), 3.81 (3H, s) |
| 15-12 | 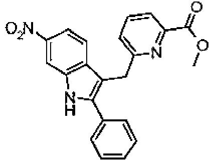 | 1H-NMR (CDCl3) δ ppm:8.67 (1H, s), 8.40-8.30 (1H, m), 8.05-7.90 (2H, m), 7.75-7.40 (7H, m), 7.20-7.10 (1H, m), 4.57 (2H, s), 4.04 (3H, s) |
| 15-13 | 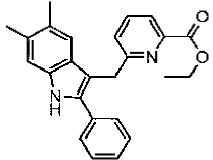 | 1H-NMR (CDCl3) δ ppm:8.01 (1H, s), 8.00-7.90 (1H, m), 7.65-7.10 (9H, m), 4.55 (2H, s), 4.52 (2H, q, J=7.2Hz), 2.37 (3H, s), 2.29 (3H, s), 1.47 (3H, t, J=7.2Hz) |
| 15-14 | 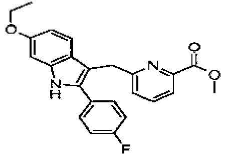 | 1H-NMR (CDCl3) δ ppm:8.01 (1H, s), 8.00-7.90 (1H, m), 7.70-7.45 (3H, m), 7.30-7.05 (4H, m), 5.89 (1H, d, J=2.2Hz), 6.74 (1H, dd, J=8.7, 2.2Hz), 4.49 (2H, s), 4.15-4.00 (5H, m), 1.44 (3H, t, J=7.1Hz) |
| 15-15 | 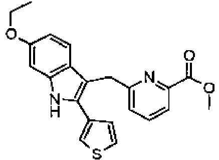 | 1H-NMR (CDCl3) δ ppm:8.05 (1H, s), 8.00-7.90 (1H, m), 7.70-7.55 (1H, m), 7.52 (1H, dd, J=3.0, 1.3Hz), 7.40 (1H, dd, J=5.1, 3.0Hz), 7.35-7.15 (3H, m), 6.88 (1H, d, J=2.2Hz), 6.75 (1H, dd J=8.7, 2.2Hz), 4.53 (2H, s), 4.15-4.00 (5H, m), 1.44 (3H, t, J=7.0Hz) |

**[Table 112]**

| Ex. No. | Strc | Physical data |
|---|---|---|
| 15-16 | 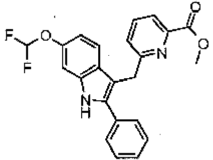 | 1H-NMR (CDCl3) δ ppm:8.33 (1H, s), 8.05-7.90 (1H, m), 7.70-7.35 (6H, m), 7.34 (1H, d, J=8.6Hz), 7.25-7.10 (2H, m), 6.88 (1H, dd, J=8.6, 2.1Hz), 6.50 (1H, t, J=74.8Hz), 4.55 (2H, s), 4.04 (3H, s) |
| 15-17 | 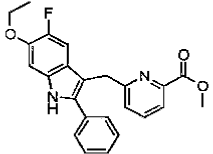 | 1H-NMR (CDCl3) δ ppm:8.08 (1H, s), 8.00-7.90 (1H, m), 7.70-7.30 (6H, m), 7.20-7.15 (1H, m), 7.09 (1H, d, J=11.5Hz), 6.97 (1H, d, J=6.9Hz), 4.50 (2H, s), 4.13 (2H, q, J=7.0Hz), 4.05 (3H, s), 1.48 (3H, t, J=7.0Hz) |
| 15-18 | 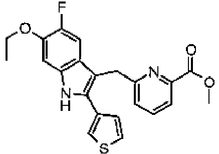 | 1H-NMR (CDCl3) δ ppm:8.06 (1H, s), 8.00-7.90 (1H, m), 7.70-7.60 (1H, m), 7.56 (1H, dd, J=3.0, 1.3Hz), 7.42 (1H, dd, J=5.1, 3.0Hz), 7.31 (1H, dd, J=5.1, 1.3Hz), 7.25-7.15 (1H, m), 7.14 (1H, d, J=11.6Hz), 6.95 (1H, d, J=7.0Hz), 4.48 (2H, s), 4.12 (2H, q, J=7.0Hz), 4.05 (3H, s), 1.48 (3H, t, J=7.0Hz) |
| 15-19 | 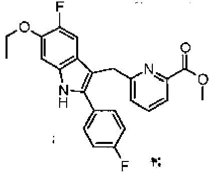 | 1H-NMR (CDCl3) δ ppm:8.02 (1H, s), 8.00-7.90 (1H, m), 7.70-7.45 (3H, m), 7.25-7.05 (4H, m), 6.96 (1H, d, J=7.0Hz), 4.44 (2H, s), 4.13 (2H, q, J=7.0Hz), 4.04 (3H, s), 1.48 (3H, t, J=7.0Hz) |
| 15-20 | 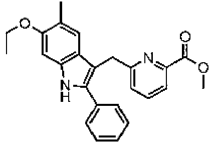 | 1H-NMR (CDCl3) δ ppm:8.02 (1H, s), 8.00-7.90 (1H, m), 7.65-7.15 (7H, m), 7.13 (1H, s), 6.85 (1H, s), 4.54 (2H, s), 4.15-4.00 (5H, m), 2.24 (3H, s), 1.46 (3H, t, J=7.0Hz) |

**[Table 113]**

| Ex. No. | Strc | Physical data |
|---|---|---|
| 15-21 | 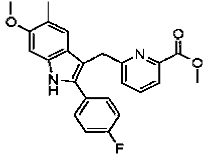 | 1H-NMR (CDCl3) δ ppm:8.05-7.90 (2H, m), 7.70-7.60 (1H, m), 7.55-7.40 (2H, m), 7.25-7.05 (4H, m), 6.86 (1H, s), 4.49 (2H, s), 4.05 (3H, s), 3.88 (3H, s), 2.24 (3H, s) |
| 15-22 | 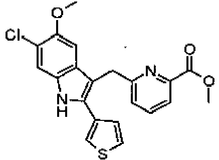 | 1H-NMR (CDCl3) δ ppm:8.09 (1H, s), 8.05-7.90 (1H, m), 7.70-7.60 (1H, m), 7.51 (1H, dd, J=2.9, 1.3Hz), 7.43 (1H, dd, J=5.0, 2.9Hz), 7.41 (1H, s), 7.30 (1H, dd, J=5.0, 1.3Hz), 7.20-7.10 (1H, m), 6.95 (1H, s), 4.54 (2H, s), 4.05 (3H, s), 3.84 (3H, s) |
| 15-23 | 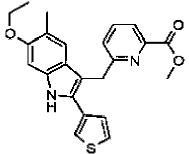 | 1H-NMR (CDCl3) δ ppm:8.05-7.90 (2H, m), 7.70-7.55 (1H, m), 7.46 (1H, dd, J=3.0, 1.2Hz), 7.39 (1H, dd, J=5.0, 3.0Hz), 7.29 (1H, dd, J=5.0, 1.2Hz), 7.25-7.15 (2H, m), 6.83 (1H, s), 4.53 (2H, s), 4.15-3.95 (5H, m), 2.25 (3H, s), 1.46 (3H, t, J=7.0Hz) |
| 15-24 | 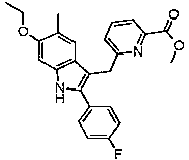 | 1H-NMR (CDCl3) δ ppm:8.00-7.90 (2H, m), 7.70-7.60 (1H, m), 7.55-7.40 (2H, m), 7.25-7.05 (4H, m), 6.84 (1H, s),4.48 (2H, s), 4.06 (2H, q, J=7.0Hz), 4.05 (3H, s), 2.24 (3H, s), 1.46 (3H, t, J=7.0Hz) |
| 15-25 | 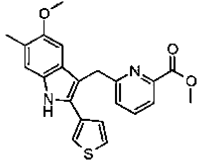 | 1H-NMR (CDCl3) δ ppm:7.96 (1H, s), 7.70-7.55 (1H, m), 7.50-7.10 (6H, m), 6.83 (1H, s), 4.56 (2H, s), 4.05 (3H, s), 3.77 (3H, s), 2.32 (3H, s) |

**[Table 114]**

| Ex. No. | Strc | Physical data |
|---|---|---|
| 16-1 | 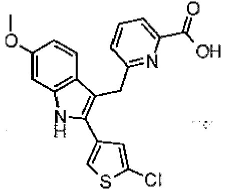 | 1H-NMR (DMSO-d₆) δ ppm:11.17 (1H, s), 8.04 (1H, d, J=1.7Hz), 7.90-7.70 (2H, m), 7.72 (1H, d, J=1.7Hz), 7.55-7.40 (2H, m), 6.83 (1H, d, J=2.3Hz), 6.66 (1H, dd, J=8.7, 2.3 Hz), 4.35 (2H, s), 3.77 (3H, s) |
| | | MS(ESI, m/z) = 399 (M+H)+ |
| 16-2 | 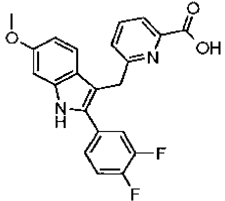 | 1H-NMR (DMSO-d6) δ ppm:13.90-12.40 (1H, br), 11.24 (1H, s), 8.25-8.10 (1H, m), 7.90-7.80 (2H, m), 7.75-7.45 (3H, m), 7.41 (1H, d, J=8.7Hz), 6.85 (1H, d, J=2.2Hz), 6.65 (1H, dd, J=8.7, 2.2Hz), 4.32 (2H, s), 3.77 (3H, s) |
| | | MS(ESI, m/z) = 395 (M+H)+ |
| 16-3 | 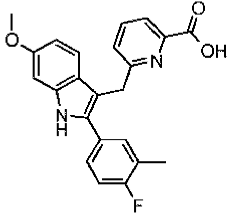 | 1H-NMR (DMSO-d6) δ ppm:11.15 (1H, s), 7.95-7.75 (3H, m), 7.65-7.55 (1H, m), 7.50-7.10 (3H, m), 6.90-6.80 (1H, m), 6.70-6.55 (1H, m), 4.31 (2H, s), 3.76 (3H, s), 2.23 (3H, s) |
| | | MS(ESI, m/z) = 391 (M+H)+ |
| 16-4 | 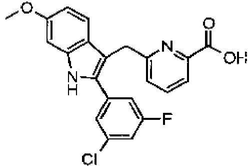 | 1H-NMR (DMSO-d6) δ ppm:11.33 (1H, s), 7.95-7.70 (4H, m), 7.60-7.35 (3H, m), 6.85 (1H, d, J=2.3Hz), 6.67 (1H, dd, J=8.7, 2.3Hz), 4.35 (2H, s), 3.78 (3H, s) |
| | | MS(ESI, m/z) = 411 (M+H)+ |
| 16-5 | 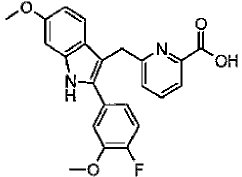 | 1H-NMR (DMSO-d6) δ ppm:13.50-12.50 (1H, br), 11.19 (1H, s), 7.90-7.80 (2H, m), 7.75-7.65 (1H, m), 7.55-7.40 (1H, m), 7.39 (1H, d, J=8.8Hz), 7.35-7.25 (2H, m), 6.86 (1H, d, J=2.3Hz), 6.64 (1H, dd, J=8.8, 2.3Hz), 4.35 (2H, s), 3.85 (3H, s), 3.77 (3H, s) |
| | | MS(ESI, m/z) = 407 (M+H)+ |

**[Table 115]**

| Ex. No. | Strc | Physical data |
|---|---|---|
| 16-6 | 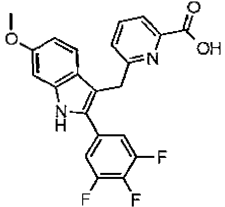 | 1H-NMR (DMSO-d6) δ ppm:11.28 (1H, s), 8.15-8.00 (2H, m), 7.95-7.80 (2H, m), 7.70-7.60 (1H, m), 7.50 (1H, d, J=8.7Hz), 6.84 (1H, d, J=2.2Hz), 6.67 (1H, dd, J=8.7, 2.2Hz), 4.32 (2H, s), 3.77 (3H, s) |
| | | MS(ESI, m/z) = 413 (M+H)+ |
| 16-7 | 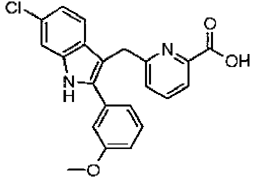 | 1H-NMR (DMSO-d6) δ ppm:13.60-12.70 (1H, br), 11.55 (1H, s), 7.90-7.75 (2H, m), 7.48 (1H, d, J=8.5Hz), 7.45-7.30 (5H, m), 7.05-6.90 (2H, m), 4.40 (2H, s), 3.77 (3H, s) |
| | | MS(ESI, m/z) = 393 (M+H)+ |
| 16-8 | 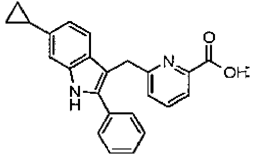 | 1H-NMR (DMSO-d6) δ ppm:11.18 (1H, s), 7.90-7.70 (4H, m), 7.55-7.25 (5H, m), 7.15-7.05 (1H, m), 6.70 (1H, dd, J=8.3, 1.4Hz), 4.35 (2H, s), 2.05-1.90 (1H, m), 1.00-0.85 (2H, m), 0.70-0.60 (2H, m) |
| | | MS(ESI, m/z) = 369(M+H)+ |
| 16-9 | 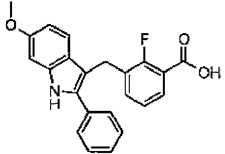 | 1H-NMR (DMSO-d5) δ ppm:11.19 (1H, s), 7.60-7.25 (6H, m), 7.19 (1H, d, J=8.6Hz), 7.00-6.85 (3H, m), 6.62 (1H, dd, J=8.6, 2.3Hz), 4.17 (2H, s), 3.77 (3H, s) |
| | | MS(ESI, m/z) = 376 (M+H)+ |
| 16-10 | 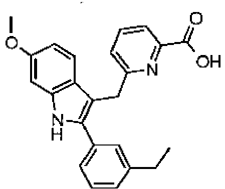 | 1H-NMR (DMSO-d6) δ ppm:11.16 (1H, s), 7.90-7.75 (2H, m), 7.65-7.45 (2H, m), 7.40-7.30 (3H, m), 7.25-7.15 (1H, m), 6.87 (1H, d, J=2.2Hz), 6.63 (1H, dd, J=8.7, 2.2Hz), 4.35 (2H, s), 3.77 (3H, s), 2.64 (2H, q, J=7.7Hz), 1.18 (3H, t, J=7.7Hz) |
| | | MS(ESI, m/z) = 387 (M+H)+ |

**[Table 116]**

| Ex. No. | Strc | Physical data |
|---|---|---|
| 16-11 | 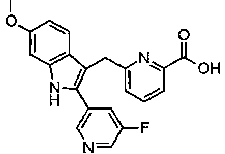 | 1H-NMR (DMSO-d6) δ ppm:13.30-13.00 (1H, br), 11.39 (1H, s), 8.90-8.80 (1H, m), 8.60-8.50 (2H, m), 7.90-7.80 (2H, m), 7.60-7.52 (1H, m), 7.49 (1H, d, J=8.7Hz), 6.87 (1H, d, J=2.2Hz), 6.68 (1H, dd, J=8.7, 2.2Hz), 4.35 (2H, s), 3.78 (3H, s) |
| | | MS(ESI, m/z) = 378 (M+H)+ |
| 16-12 | 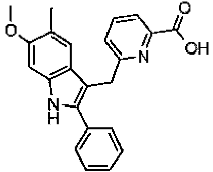 | 1H-NMR (DMSO-d6) δ ppm:13.50-12.70 (1H, br), 11.10 (1H, s), 7.90-7.65 (4H, m), 7.50-7.25 (4H, m), 7.20 (1H, s), 6.86 (1H, s), 4.34 (2H, s), 3.81 (3H, s), 2.16 (3H, s) |
| | | MS(ESI, m/z) = 373 (M+H)+ |
| 16-13 | 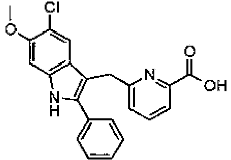 | 1H-NMR (DMSO-d6) δ ppm:11.39 (1H, s), 7.90-7.70 (4H, m), 7.55-7.30 (5H, m), 7.02 (1H, s), 4.34 (2H, s), 3.86 (3H, s) |
| | | MS(ESI, m/z) = 393(M+H)+ |
| 16-14 | 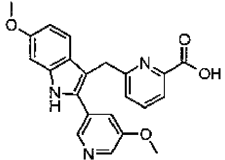 | 1H-NMR (DMSO-d6) δ ppm:11.34 (1H, s), 8.55-8.50 (1H, m), 8.40-8.20 (1H, m), 8.00-7.95 (1H,m), 7.90-7.80 (2H, m), 7.55-7.45 (1H, m), 7.43 (1H, d, J=8.8Hz), 6.88 (1H, d, J=2.3Hz), 6.67 (1H, dd, J=8.8, 2.3Hz), 4.37 (2H, s), 3.87 (3H, s), 3.78 (3H, s) |
| | | MS(ESI, m/z) = 390 (M+H)+ |
| 16-15 | 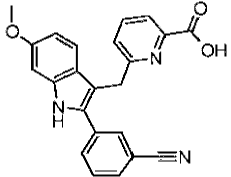 | 1H-NMR (DMSO-d6) δ ppm:13.30-13.00 (1H, br), 11.33 (1H, s), 8.45-8.35 (1H, m), 8.25-8.15 (1H, m), 7.90-7.75 (3H, m), 7.70-7.60 (1H, m), 7.55-7.45 (1H, m), 7.42 (1N, d, J=8.7Hz), 6.87 (1H, d, J=2.3Hz), 6.66 (1H, dd, J=8.7, 2.3Hz), 4.35 (2H, s), 3.77 (3H, s) |
| | | MS(ESI, m/z) = 384 (M+H)+ |

**[Table 117]**

| Ex. No. | Strc | Physical data |
|---|---|---|
| 16-16 | 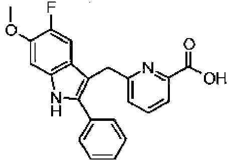 | 1H-NMR (DMSO-d6) δ ppm:13.50-12.80 (1H, br), 11.30 (1H,s), 7.90-7.70 (4H, m), 7.50-7.30 (4H, m), 7.28 (1H, d, J=12.1Hz), 7.02 (1H, d, J=7.3Hz), 4.33 (2H, s), 3.85 (3H, s) |
| | | MS(ESI, m/z) = 377 (M+H)+ |
| 16-17 | 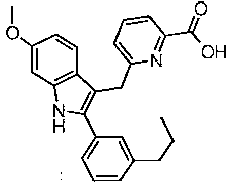 | 1H-NMR (DMSO-d6) δ ppm:11.16 (1H, s), 7.90-7.75 (2H, m), 7.65-7.45 (2H, m), 7.40-7.25 (3H, m), 7.20-7.10 (1H, m), 6.87 (1H, d, J=2.2Hz), 6.64 (1H, dd, J=8.7, 2.2Hz), 4.35 (2H, s), 3.77 (3H, s), 2.58 (2H, t, J=7.6Hz), 1.65-1.50 (2H, m), 0.87 (3H, t, J=7.4Hz) |
| | | MS(ESI, m/z) = 401 (M+H)+ |
| 16-18 | 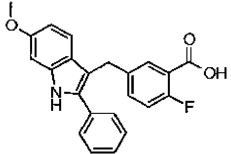 | 1H-NMR (DMSO-d6) δ ppm:13.20-13.05 (1H, br), 11.17 (1H, s), 7.65-7.10 (9H, m), 6.88 (1H, d, J=2.2Hz), 6.62 (1H, dd, J=8.5, 2.2Hz), 4.21 (2H, s), 3.78 (3H, s) |
| | | MS(ESI, m/z) = 376 (M+H)+ |
| 16-19 | 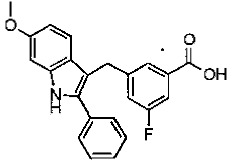 | 1H-NMR (DMSO-d6) δ ppm:11.19 (1H, s), 7.60-7.25 (7H, m), 7.22 (1H, d, J=8.7Hz), 7.15-7.00 (1H, br), 6.89 (1H, d, J=2.1Hz), 6.63 (1H, dd, J=8.7, 2.1Hz), 4.25 (2H, s), 3.78 (3H, s) |
| | | MS(ESI, m/z) = 376 (M+H)+ |
| 16-20 | 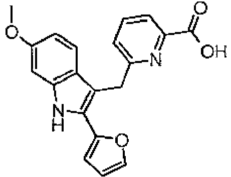 | 1H-NMR (DMSO-d6) δ ppm:11.28 (1H, s), 7.90-7.70 (3H, m), 7.38 (1H, d, J=8.7Hz), 7.30-7.20 (1H, m), 6.87 (1H, dd, J=3.4, 0.5Hz), 6.85 (1H, d, J=2.2Hz), 6.70-6.55 (2H, m), 4.46 (2H, s), 3.77 (3H, s) |
| | | MS(ESI, m/z) = 349(M+H)+ |

**[Table 118]**

| Ex. No. | Strc | Physical data |
|---|---|---|
| 16-21 | 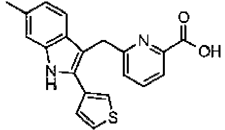 | 1H-NMR (DMSO-d6) δ ppm:13.35-12.90 (1H, br), 11.14 (1H, s), 8.14 (1H, dd, J=3.0, 1.3Hz), 7.90-7.75 (2H, m), 7.68 (1H, dd, J=5.1, 3.0Hz), 7.59 (1H, dd, J=5.1, 1.3 Hz), 7.42 (1H, d, J=8.1Hz), 7.40-7.30 (1H, m), 7.15 (1H, s), 6.81 (1H, dd, J=8.1, 1.1Hz), 4.38 (2H, s), 2.39 (3H, s) |
| | | MS(ESI, m/z) = 349 (M+H)+ |
| 16-22 | 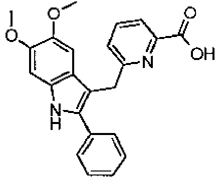 | 1H-NMR (DMSO-d6) δ ppm:11.06 (1H, s), 7.90-7.65 (4H, m), 7.55-7.25 (4H, m), 7.02 (1 H, s), 6.89 (1H, s), 4.36 (2H, s), 3.78 (3H, s), 3.69 (3H, s) |
| | | MS(ESI, m/z) = 389 (M+H)+ |
| 16-23 | 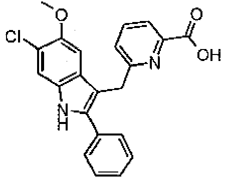 | 1H-NMR (DMSO-d6) δ ppm:11.33 (1H, s), 7.90-7.70 (4H, m), 7.55-7.30 (5H, m), 7.24 (1H, s), 4.38 (2H, s), 3.78 (3H, s) |
| | | MS(ESI, m/z) = 393 (M+H)+ |
| 16-24 | 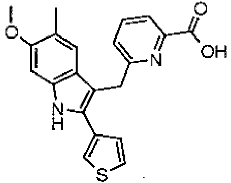 | 1H-NMR (DMSO-d6) δ ppm:11.04 (1H, s), 8.02 (1H, dd, J=3.0, 1.2Hz), 7.90-7.75 (2H, m), 7.65 (1H, dd, J=5.0, 3.0Hz), 7.54 (1H, dd, J=5.0, 1.2Hz), 740-7.25 (2H, m), 6.83 (1H, s), 4.35 (2H, s), 3.81 (3H, s), 2.19 (3H, s) |
| | | MS(ESI, m/z) = 379 (M+H)+ |
| 16-25 | 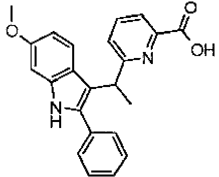 | 1H-NMR (DMSO-d6) δ ppm:13.20-12.90 (1H, s), 11.07 (1H, s), 7.90-7.60 (4H, m), 7.55-7.30 (5H, m), 6.82 (1H, d, J=2.3Hz), 6.54 (1H, dd, J=8.8, 2.3Hz), 4.62 (1H, q, J=7.3Hz), 3.74 (3H, s), 1.78 (3H, d, J=7.3Hz) |
| | | MS(ESI, m/z) = 373 (M+H)+ |

**[Table 119]**

| Ex. No. | Strc | Physical data |
|---|---|---|
| 16-26 | 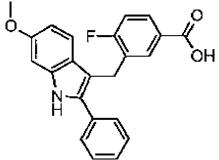 | 1H-NMR (DMSO-d6) δ ppm:12.95-12.80 (1H, br), 11.26 (1H,s), 7.85-7.75 (1H, m), 7.60-7.25 (7H, m), 7.21 (1H, d, J=8.7Hz), 6.90 (1H, d, J=2.2Hz), 6.63 (1H, dd, J=8.7, 2.2Hz), 4.21 (2H, s), 3.78 (3H, s) |
| | | MS(ESI, m/z) = 376 (M+H)+ |
| 16-27 | 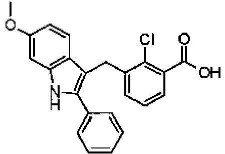 | 1H-NMR (DMSO-d6) δ ppm:13.60-13.20 (1H, br), 11.28 (1H, s), 7.60-7.15 (7H, m), 7.14 (1H, d, J=8.6Hz), 7.10-6.95 (1H, m), 6.91 (1H, d, J=2.1Hz), 6.62 (1H, dd, J=8.6, 2.1Hz), 4.25 (2H, s), 3.78 (3H, s) |
| | | MS(ESI, m/z) = 392 (M+H)+ |
| 16-28 | 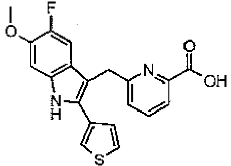 | 1H-NMR (DMSO-d6) δ ppm:13.40-12.90 (1H, br), 11.25 (1H, s), 8.20-8.10 (1H, m), 7.90-7.80 (2H, m), 7.68 (1H, dd, J=5.0, 3.0Hz), 7.60-7.55 (1H, m), 7.45-7.35 (2H, m), 6.99 (1H, d, J=7.3Hz), 4.34 (2H, s), 3.85 (3H, s) |
| | | MS(ESI, m/z) = 383 (M+H)+ |
| 16-29 | 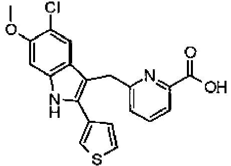 | 1H-NMR (DMSO-d6) δ ppm:11.33(1H, s), 8.15 (1H, dd, J=2.9, 1.2Hz), 7.90-7.80 (2H, m), 7.69 (1H, dd, J=5.0, 2.9Hz), 7.63 (1H, s), 7.57 (1H, dd, J=5.0, 1.2Hz), 7.45-7.35 (1H, m), 6.99(1H, s), 4.36 (2H, s), 3.86 (3H, s) |
| | | MS(ESI, m/z) = 399(M+H)+ |
| 16-30 | 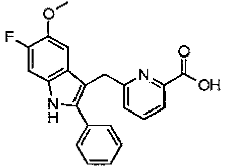 | 1H-NMR (CDCl3) δ ppm:13.40-12.70 (1H, br), 11.29 (1H, s), 7.90-7.65 (4H, m), 7.55-7.30 (4H, m), 7.24 (1H, d, J=8.5Hz), 7.16 (1H, d, J=11.6Hz), 4.37 (2H, s), 3.78 (3H, s), |
| | | MS(ESI, m/z) = 377 (M+H)+ |

**[Table 120]**

| Ex. No. | Strc | Physical data |
|---|---|---|
| 16-31 | 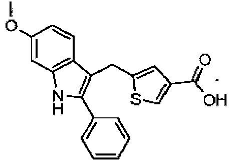 | 1H-NMR (DMSO-d6) δ ppm:11.17 (1H, s), 7.65-7.25 (7H, m), 6.98 (1H, s), 6.88 (1H, d, J=2.2Hz), 6.65 (1H, dd, J=8.6, 2.2Hz), 4.27 (2H, s), 3.78 (3H, s) |
| | | MS(ESI, m/z) = 364 (M+H)+ |
| 16-32 | 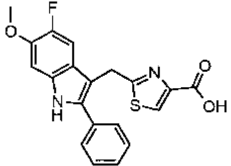 | 1H-NMR (DMSO-d6) δ ppm:13.00-12.85 (1H, s), 11.43 (1H, s), 8.19 (1H,s), 7.65-7.25 (6H, m), 7.05 (1H, d, J=7.4Hz), 4.51 (2H, s), 3.87 (3H, s) |
| | | MS(ESI, m/z) = 383 (M+H)+ |
| 16-33 | 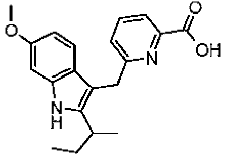 | 1H-NMR (DMSO-d6) δ ppm:10.56 (1H, s), 7.85-7.70 (2H, m), 7.30-7.15 (2H, m), 6.78 (1H, d, J=2.3Hz), 6.55 (1H, dd, J=8.6, 2.3Hz), 4.17 (2H, s), 3.73 (3H, s), 3.10-2.95 (1H, m), 1.75-1.50 (2H, m), 1.22 (3H, d, J=7.0Hz), 0.70 (3H, t, J=7.4Hz) |
| | | MS(ESI, m/z) = 339 (M+H)+ |
| 16-34 | 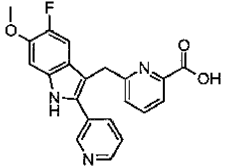 | 1H-NMR (DMSO-d6) δ ppm:11.43 (1H, s), 9.00-8.90 (1H, m), 8.60-8.50 (1H, m), 8.40-8.25 (1H, m), 7.95-7.80 (2H, m), 7.60-7.40 (2H, m), 7.37 (1H, d, J=11.8Hz), 7.03 (1H, d, J=7.3Hz), 4.32 (2H, s), 3.86 (3H, s) |
| | | MS(ESI, m/z) = 378 (M+H)+ |
| 16-35 | 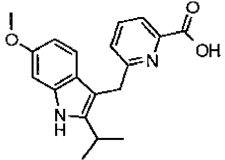 | 1H-NMR (DMSO-d6) δ ppm:10.60 (1H, s), 7.85-7.70 (2H, m), 7.30-7.15 (2H, m), 6.78 (1H, d, J=2.3Hz), 6.54 (1H, dd, J=8.6, 2.3Hz), 4.17 (2H, s), 3.72 (3H, s), 3.40-3.20 (1H, m), 1.24 (6H, d, J=7.0Hz) |
| | | MS(ESI, m/z) = 325 (M+H)+ |

**[Table 121]**

| Ex. No. | Strc | Physical data |
|---|---|---|
| 16-36 | 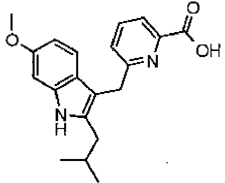 | 1H-NMR (DMSO-d6) δ ppm:10.62 (1H, s), 7.90-7.75 (2H, m), 7.30-7.20 (1H, m), 7.18 (1H, d, J=8.6Hz), 6.77 (1H, d, J=2.2Hz), 6.54 (1H, dd, J=8.6, 2.2Hz), 4.18 (2H, s), 3.73 (3H, s), 2.59 (2H, d, J=7.3Hz), 2.05-1.85 (1H, m), 0.84 (6H, d, J=6.6Hz) |
| | | MS(ESI, m/z) = 339 (M+H)+ |
| 16-37 | 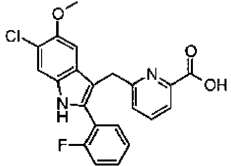 | 1H-NMR (DMSO-d6) δ ppm:13.60-12.80 (1H, br), 11.34 (1H, s), 7.90-7.70 (3H, m), 7.55-7.20 (6H, m), 4.22 (2H, s), 3.77 (3H, s) |
| | | MS(ESI, m/z) = 411 (M+H)+ |
| 16-38 | 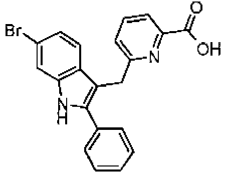 | 1H-NMR (DMSO-d6) δ ppm:13.70-12.70 (1H, br), 11.56 (1H, s), 7.90-7.75 (4H, m), 7.60-7.30 (6H, m), 7.10 (1H, dd, J=8.5, 1.8Hz), 4.38 (2H, s) |
| 16-39 | 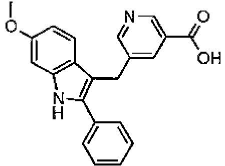 | 1H-NMR (DMSO-d6) δ ppm:11.22 (1H, s), 8.77 (1H, s), 8.46 (1H, s), 7.82 (1H, s), 7.60-7.50 (2H, m), 7.50-7.40 (2H, m), 7.40-7.30 (1H, m), 7.23 (1H, d, J=8.7Hz), 6.89 (1H, d, J=2.3Hz), 6.62 (1H, dd, J=8.7, 2.3Hz), 4.24 (2H, s), 3.77 (3H, s) |
| | | MS(ESI, m/z) = 359 (M+H)+ |
| 16-40 | 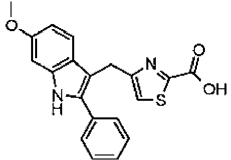 | 1H-NMR (DMSO-d6) δ ppm:14.20-13.70 (1H, hr), 11.17 (1H, s), 7.75-7.65 (2H, m), 7.55-7.40 (3H, m), 7.40-7.25 (2H, m), 6.87 (1H, d, J=2.2Hz), 6.63 (1H, dd, J=8.5, 2.2Hz), 4.31 (2H, s), 3.77 (3H, s) |

**[Table 122]**

| Ex. No. | Strc | Physical data |
|---|---|---|
| 16-41 | 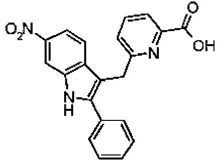 | 1H-NMR (DMSO-d6) δ ppm:13.50-12.80 (1H, br), 12.21 (1H, s), 8.30-8.25 (1H, m), 7.95-7.80 (5H, m), 7.75-7.35 (5H, m), 4.44 (2H, s) |
| | | MS(ESI, m/z) = 374 (M+H)+ |
| 16-42 | 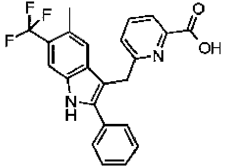 | 1H-NMR (DMSO-d6) δ ppm:13.40-12.70 (1H, br), 11.70 (1H, s), 7.90-7.75 (4H, m), 7.67 (1H, s), 7.60-7.30 (5H, m), 4.39 (2H, s), 2.45 (3H, d, J=1.0Hz) |
| | | MS(ESI, m/z) = 411 (M+H)+ |
| 16-43 | 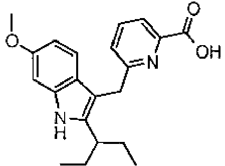 | 1H-NMR (DMSO-d6) δ ppm:10.51 (1H, s), 7.90-7.70 (2H, m), 7.30-7.15 (2H, m), 6.78 (1H, d, J=2.2Hz), 6.56 (1H, dd, J=8.6, 2.2Hz), 4.18 (2H, s), 3.73 (3H, s), 2.85-2.60 (1H, m), 1.75-1.45 (4H, m), 0.65 (6H, t, J=7.4Hz) |
| | | MS(ESI, m/z) = 353 (M+H)+ |
| 16-44 | 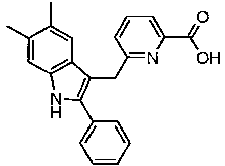 | 1H-NMR (DMSO-d6) δ ppm:13.50-12.60 (1H, br), 11.08 (1H, s), 7.90-7.65 (4H, m), 7.50-7.25 (4H, m), 7.20 (1H, s), 7.16 (1H, s), 4.35 (2H, s), 2.29 (3H, s), 2.23 (3H, s) |
| | | MS(ESI, m/z) = 357 (M+H)+ |
| 16-45 | 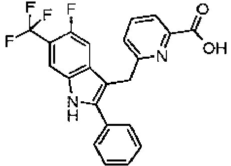 | 1H-NMR (DMSO-d6) δ ppm:13.40-12.70 (1H, br), 11.93 (1H, s), 7.95-7.75 (4H,m), 7.70-7.35 (6H, m), 4.38 (2H, s) |
| | | MS(ESI, m/z) = 415 (M+H)+ |

**[Table 123]**

| Ex. No. | Strc | Physical data |
|---|---|---|
| 16-46 | 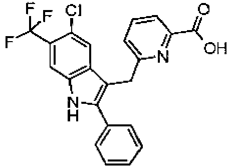 | 1H-NMR (DMSO-d6) δ ppm:13.40-12.70 (1H, br), 12.03 (1H, s), 7.95-7.80 (5H, m), 7.79 (1H, s), 7.60-7.35 (4H, m), 4.40 (2H, s) |
| | | MS(ESI, m/z) = 431 (M+H)+ |
| 16-47 | 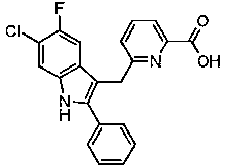 | 1H-NMR (DMSO-d6) δ ppm:13.40-12.80 (1H, br), 11.61 (1H, s), 7.90-7.75 (4H, m), 7.55-7.35 (6H, m), 4.35 (2H, s) |
| | | MS(ESI, m/z) = 381 (M+H)+ |
| 16-48 | 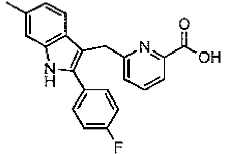 | 1H-NMR (DMSO-d6) δ ppm:11.19 (1H, s), 7.95-7.75 (4H, m), 7.45-7.25 (4H, m), 7.16 (1H, s), 6.79 (1H, dd, J=8.2, 1.0Hz), 4.33 (2H, s), 2.38 (3H, s) |
| | | MS(ESI, m/z) = 361(M+H)+ |
| 16-49 | 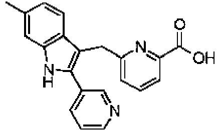 | 1H-NMR (DMSO-d6) δ ppm:11.34 (1H, s), 9.05-8.90 (1H, m), 8.60-8.50 (1H, m), 8.40-8.25 (1H, m), 7.90-7.75 (2H, m), 7.55-7.30 (3H, m), 7.25-7.15 (1H, m), 6.83 (1H, dd, J=8.2, 1.1 Hz), 4.35 (2H, s), 2.39 (3H, s) |
| | | MS(ESI, m/z) = 344(M+H)+ |
| 16-50 | 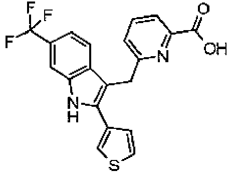 | 1H-NMR (DMSO-d6) δ ppm:13.50-12.75 (1H, br), 11.80 (1H, s), 8.32 (1H, dd, J=2.9, 1.3Hz), 7.95-7.80 (2H, m), 7.77 (1H, d, J=8.4Hz), 7.74 (1H, dd, J=5.1, 2.9Hz), 7.70-7.60 (2H, m), 7.50-7.40 (1H, m), 7.28 (1H, dd, J=8.4, 1.3Hz), 4.45 (2H, s) |
| | | MS(ESI, m/z) = 403 (M+H)+ |

**[Table 124]**

| Ex. No. | Strc | Physical data |
|---|---|---|
| 16-51 | 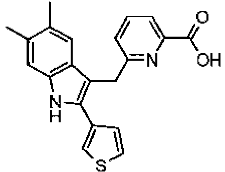 | 1H-NMR (DMSO-d6) δ ppm:13.50-12.50 (1H, br), 11.02 (1H, s), 8.07 (1H, dd, J=3.0, 1.2Hz), 7.90-7.75 (2H, m), 7.66 (1H, dd, J=5.0, 3.0Hz), 7.56 (1H, dd, J=5.0, 1.2Hz), 7.40-7.25 (2H, m), 7.13 (1H, s), 4.37 (2H, s), 2.29 (3H, s), 2.25 (3H, s) |
| | | MS(ESI, m/z) = 363 (M+H)+ |
| 16-52 | 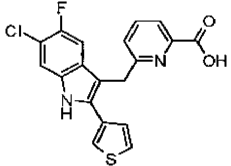 | 1H-NMR (DMSO-d6) δ ppm:13.35-12.95 (1H, br), 11.55 (1H, s), 8.35-8.25 (1H, m), 7.90-7.80 (2H, m), 7.71 (1H, dd, J=5.0, 3.1 Hz), 7.65-7.55 (2H, m), 7.50-7.40 (2H, m), 4.38 (2H, s) |
| | | MS(ESI, m/z) = 387 (M+H)+ |
| 16-53 | 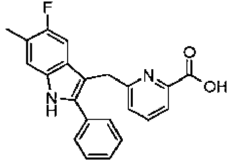 | 1H-NMR (DMSO-d6) δ ppm:11.29 (1H, s), 7.90-7.70 (4H, m), 7.55-7.30 (4H, m), 7.22 (1H, d, J=6.5 Hz), 7.17 (1H, d, J=10.8Hz), 4.33 (2H, s), 2.31 (3H, d, J=1.4 Hz) |
| | | MS(ESI, m/z) = 361(M+H)+ |
| 16-54 | 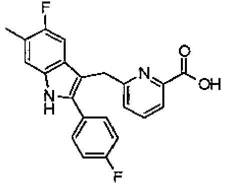 | 1H-NMR (DMSO-d6) δ ppm:11.29 (1H, s), 7.95-7.75 (4H, m), 7.45-7.15 (5H, m), 4.30 (2H, s), 2.30(3H, d, J=1.4Hz) |
| | | MS(ESI, m/z) = 379(M+H)+ |
| 16-55 | 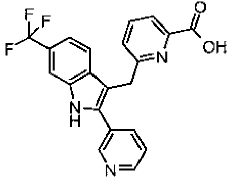 | 1H-NMR (DMSO-d6) δ ppm:13.40-12.70 (1H, br), 12.00 (1H, s), 9.10-9.00 (1H, m), 8.70-8.60 (1H, m), 8.45-8.35 (1H, m), 7.90-7.65 (4H, m), 7.60-7.45 (2H, m), 7.29 (1H, dd, J=8.6, 1.3Hz), 4.41 (2H, s) |
| | | MS(ESI, m/z) = 398 (M+H)+ |

**[Table 125]**

| Ex. No. | Strc | Physical data |
|---|---|---|
| 16-56 | 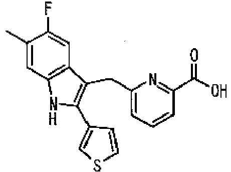 | 1H-NMR (DMSO-d6) δ ppm:11.23 (1H, s), 8.19 (1H, dd, J=3.0, 1.3Hz), 7.90-7.75 (2H, m), 7.68 (1H, dd, J=5.0, 3.0Hz), 7.60 (1H, dd, J=5.0, 1.3Hz), 7.45-7.35 (1H, m), 7.29 (1H, d, J=10.7Hz), 7.19 (1H, d, J=6.6Hz), 4.35 (2H, s), 2.31 (3H, d, J=1.4Hz) |
| | | MS(ESI, m/z) = 367(M+H)+ |
| 16-57 | 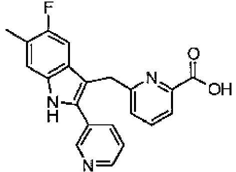 | 1H-NMR (DMSO-d6) δ ppm:11.44 (1H, s), 9.00-8.95 (1H, m), 8.65-8.50 (1H, m), 8.40-8.30 (1H, m), 7.90-7.75 (2H, m), 7.55-7.40 (2H, m), 7.30-7.20 (2H, m), 4.32 (2H, s), 2.31 (3H, d, J=1.5Hz) |
| | | MS(ESI, m/z) = 362(M+H)+ |
| 16-58 | 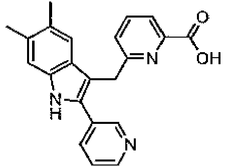 | 1H-NMR (DMSO-d6) δ ppm:13.40-12.80 (1H, br), 11.23 (1H, s), 9.00-8.90 (1H, m), 8.60-8.50 (1H, m), 8.35-8.20 (1H, m), 7.90-7.75 (2H, m), 7.55-7.35 (2H, m), 7.28 (1H, s), 7.18 (1H, s), 4.34 (2H, s), 2.30 (3H, s), 2.24 (3H, s) |
| | | MS(ESI, m/z) = 358 (M+H)+ |
| 16-59 | 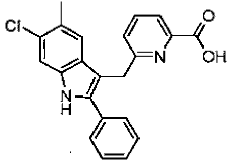 | 1H-NMR (DMSO-d6) δ ppm:13.40-12.80 (1H, br), 11.39 (1H, s), 7.90-7.70 (4H, m), 7.55-7.25 (6H, m), 4.36 (2H, s), 2.34 (3H, s) |
| | | MS(ESI, m/z) = 377 (M+H)+ |
| 16-60 | 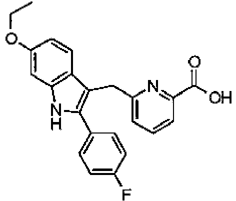 | 1H-NMR (DMSO-d6) δ ppm:11.15 (1H, s), 7.90-7.75 (4H, m), 7.45-7.25 (4H, m), 6.84 (1H, d, J=2.2Hz), 6.61 (1H, dd, J=8.7, 2.2Hz), 4.31 (2H, s), 4.02 (2H, q, J=7.0Hz), 1.34 (3H, t, J=7.0 Hz) |
| | | MS(ESI, m/z) = 391(M+H)+ |

**[Table 126]**

| Ex. No. | Strc | Physical data |
|---|---|---|
| 16-61 | 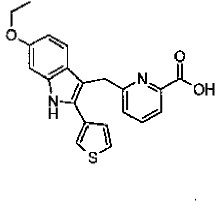 | 1H-NMR (DMSO-d6) δ ppm:11.10 (1H, s), 8.08 (1H, dd, J=3.0, 1.2Hz), 7.90-7.75 (2H, m), 7.66 (1H, dd, J=5.1, 3.0Hz), 7.57 (1H, dd, J=5.1, 1.2Hz), 7.45-7.30 (2H, m), 6.83 (1H, d, J=2.2Hz), 6.63 (1H, dd, J=8.8, 2.2Hz), 4.37 (2H, s), 4.02 (2H, q, J=7.0Hz), 1.35 (3H, t, J=7.0Hz) |
| | | MS(ESI, m/z) = 379(M+H)+ |
| 16-62 | 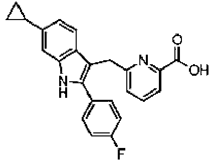 | 1H-NMR (DMSO-d6) δ ppm:11.17 (1H, s), 7.95-7.75 (4H, m), 7.40-7.25 (4H, m), 7.07 (1H, s), 6.70 (1H, dd, J=8.3, 1.5Hz), 4.32 (2H, s), 2.05-1.90 (1H, m), 1.00-0.85 (2H, m), 0.70-0.55 (2H, m) |
| | | MS(ESI, m/z) = 387(M+H)+ |
| 16-63 | 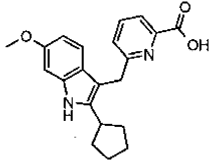 | 1H-NMR (DMSO-d6) δ ppm:10.57 (1H, s), 7.90-7.70 (2H, m), 7.30-7.15 (2H, m), 6.77 (1H, d, J=2.2Hz), 6.54 (1H, dd, J=8.6, 2.2Hz), 4.17 (2H, s), 3.72 (3H, s), 2.05-1.55 (8H, m) |
| | | MS(ESI, m/z) = 351 (M+H)+ |
| 16-64 | 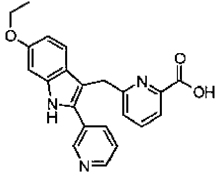 | 1H-NMR (DMSO-d6) δ ppm:13.50-12.80 (1H, br), 11.32 (1H, s), 9.00-8.90 (1H, m), 8.60-8.50 (1H, m), 8.35-8.25 (1H, m), 7.90-7.75 (2H, m), 7.55-7.40 (2H, m), 7.37 (1H, d, J=8.6Hz), 6.86 (1H, d, J=2.2Hz), 6.64 (1H, dd, J=8.7, 2.2Hz), 4.34 (2H, s), 4.03 (2H, q, J=6.9Hz), 1.35 (3H, t, J=6.9Hz) |
| | | MS(ESI, m/z) = 374(M+H)+ |
| 16-65 | 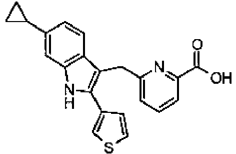 | 1H-NMR (DMSO-d6) δ ppm:11.13 (1H, s), 8.13 (1H, dd, J=3.0, 1.2Hz), 7.95-7.75 (2H, m), 7.68 (1H, dd, J=5.1, 3.0Hz), 7.58 (1H, dd, J=5.1, 1.2Hz), 7.45-7.30 (2H, m), 7.05 (1H, s), 6.72 (1H, dd, J=8.4, 1.3Hz), 4.37 (2H, s), 2.05-1.90 (1H, m), 1.00-0.85 (2H, m), 0.70-0.55 (2H, m) |
| | | MS(ESI, m/z) = 375(M+H)+ |

**[Table 127]**

| Ex. No. | Strc | Physical data |
|---|---|---|
| 16-66 | 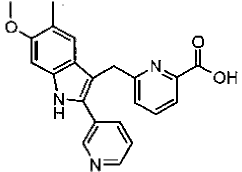 | 1H-NMR (DMSO-d6) δ ppm:13.50-12.80 (1H, br), 11.27 (1H, s), 9.05-8.95 (1H, m), 8.60-8.50 (1H, m), 8.40-8.25 (1H, m), 7.90-7.80 (2H, m), 7.60-7.35 (2H, m), 7.30 (1H, s), 6.86 (1H, s), 4.34 (2H, s), 3.82 (3H, s), 2.18 (3H, s) |
| | | MS(ESI, m/z) = 374 (M+H)+ |
| 16-67 | 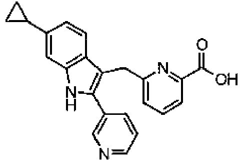 | 1H-NMR (DMSO-d6) δ ppm:11.33 (1H, s), 9.00-8.90 (1H, m), 8.60-8.50 (1H, m), 8.40-8.25 (1H, m), 7.90-7.75 (2H, m), 7.55-7.30 (3H, m), 7.15-7.05 (1H, m), 6.73 (1H, dd, J=8.3, 1.4Hz), 4.34 (2H, s), 2.05-1.95 (1H, m), 1.00-0.85 (2H, m), 0.70-0.60 (2H, m) |
| | | MS(ESI, m/z) = 370(M+H)+ |
| 16-68 | 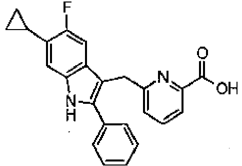 | 1H-NMR (DMSO-d6) δ ppm:13.40-12.90 (1H, br), 11.27 (1H, s), 7.95-7.70 (4H, m), 7.55-7.30 (4H, m), 7.18 (1H, d, J =11.4Hz), 6.93 (1H, d, J=6.5 Hz), 4.33 (2H, s), 2.15-2.00 (1H, m), 1.05-0.90 (2H, m), 0.75-0.60 (2H, m) |
| | | MS(ESI, m/z) = 387(M+H)+ |
| 16-69 | 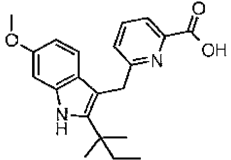 | 1H-NMR (DMSO-d6) δ ppm:13.20-12.70 (1H, br), 10.40 (1H, s), 7.90-7.70 (2H, m), 7.17 (1 H, d, J=8.6Hz), 7.10-7.00 (1H, m), 6.84 (1H, d, J=2.3Hz), 6.56 (1H, dd, J=8.6, 2.3Hz), 4.35 (2H, s), 3.74 (3H, s), 1.65 (2H, q, J=7.4Hz), 1.32 (6H, s), 0.59 (3H, t, J=7.4Hz) |
| | | MS(ESI, m/z) = 353 (M+H)+ |
| 16-70 | 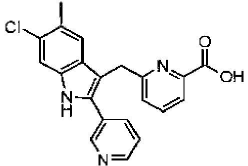 | 1H-NMR (DMSO-d6) δ ppm:13.40-12.90 (1H, br), 11.56 (1H, s), 9.00-8.95 (1H, m), 8.65-8.55 (1H, m), 8.40-8.30 (1H, m), 7.90-7.80 (2H, m), 7.60-7.35 (4H, m), 4.35 (2H, s), 2.35 (3H, s) |
| | | MS(ESI, m/z) = 378 (M+H)+ |

**[Table 128]**

| Ex. No. | Strc | Physical data |
|---|---|---|
| 16-71 | 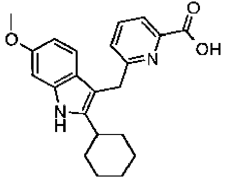 | 1H-NMR (DMSO-d6) δ ppm:10.59 (1H, s), 7.85-7.70 (2H, m), 7.30-7.15 (2H, m), 6.76 (1H, d, J=2.2Hz), 6.54 (1H, dd, J=8.6, 2.2Hz), 4.17 (2H, s), 3.72 (3H, s), 3.00-2.85 (1H, m), 1.85-1.10 (10H, m) |
| | | MS(ESI, m/z) = 365 (M+H)+ |
| 16-72 | 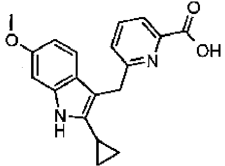 | 1H-NMR (DMSO-d6) δ ppm:10.33 (1H, s), 7.90-7.70 (2H, m), 7.30-7.20 (1H, m), 7.17 (1H, d, J=8.6Hz), 6.72 (1H, d, J=2.2Hz), 6.53 (1H, dd, J=8.6, 2.2Hz), 4.25 (2H, s), 3.71 (3H, s), 2.25-2.05 (1H, m), 1.00-0.70 (4H, m) |
| | | MS(ESI, m/z) = 323 (M+H)+ |
| 16-73 | 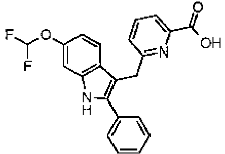 | 1H-NMR (DMSO-d6) δ ppm:13.40-12.80 (1H, br), 11.51 (1H, s), 7.95-7.75 (4H, m), 7.60-6.95 (7H, m), 6.82 (1H, dd, J=8.6, 2.1 Hz), 4.38 (2H, s) |
| | | MS(ESI, m/z) = 395 (M+H)+ |
| 16-74 | 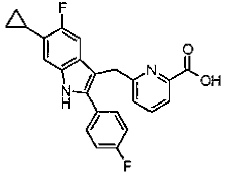 | 1H-NMR (DMSO-d6) δ ppm:13.50-12.80 (1H, br), 11.28 (1H, s), 7.95-7.80 (4H, m), 7.50-7.25 (3H, m), 7.20 (1H, d, J=11.3Hz), 6.92 (1H, d, J=6.5Hz), 4.29 (2H, s), 2.15-2.00 (1H, m), 1.05-0.90 (2H, m), 0.75-0.60 (2H, m) |
| | | MS(ESI, m/z) = 405(M+H)+ |
| 16-75 | 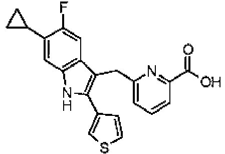 | 1H-NMR (DMSO-d6) δ ppm:13.40-12.90 (1H, br), 11.22 (1H, s), 8.20 (1H, dd, J=3.0, 1.2Hz), 7.90-7.75 (2H, m), 7.69 (1H, dd, J=5.1, 3.0Hz), 7.58 (1H, dd, J=5.1, 1.2Hz), 7.50-7.35 (1H, m), 7.31 (1H, d, J=11.2Hz), 6.90 (1H, d, J=6.4Hz), 4.35 (2H, s), 2.15-2.00 (1H, m), 1.05-0.90 (2H, m), 0.75-0.60 (2H, m) |
| | | MS(ESI, m/z)=393(M+H)+ |

**[Table 129]**

| Ex. No. | Strc | Physical data |
|---|---|---|
| 16-76 | 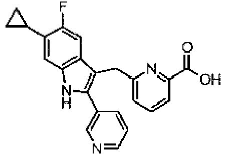 | ¹H-NMR (DMSO-d6) δ ppm:13.40-12.90 (1H, br), 11.42 (1H, s), 9.00-8.90 (1H, m), 8.60-8.50 (1H, m), 8.40-8.30 (1H, m), 7.90-7.80 (2H, m), 7.55-7.40 (2H, m), 7.27 (1H, d, J=11.3Hz), 6.95 (1H, d, J=6.5 Hz), 4.32 (2H, s), 2.15-2.00 (1H, m), 1.05-0.90 (2H, m), 0.75-0.60 (2H, m) |
| | | MS(ESI, m/z) = 388(M+H)+ |
| 16-77 | 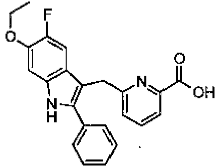 | 1H-NMR (DMSO-d6) δ ppm:13.50-12.70 (1H, br), 11.27 (1H, s), 7.90-7.70 (4H, m), 7.55-7.30 (4H, m), 7.27 (1H, d, J=12.0Hz), 7.00 (1H, d, J=7.4Hz), 4.32 (2H, s), 4.09 (2H, q, J=6.9Hz), 1.38 (3H, t, J=6.9Hz) |
| | | MS(ESI, m/z) = 391 (M+H)+ |
| 16-78 | 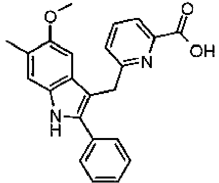 | 1H-NMR (DMSO-d6) δ ppm:13.50-12.70 (1H, br), 11.05 (1H, s), 7.90-7.65 (4H, m), 7.55-7.25 (4H, m), 7.14 (1H,s), 6.96 (1H, s), 4.37 (2H, s), 3.71 (3H, s), 2.23 (3H, s) |
| | | MS(ESI, m/z) = 373 (M+H)+ |
| 16-79 | 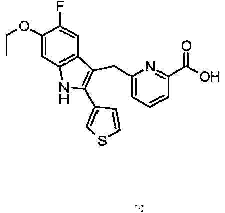 | 1H-NMR (DMSO-d6) δ ppm:13.50-12.80 (1H, br), 11.22 (1H, s), 8.15 (1H, dd, J=3.0, 1.3Hz), 7.90-7.75 (2H, m), 7.68 (1H, dd, J=5.1, 3.0Hz), 7.57 (1H, dd, J=5.1, 1.3Hz), 7.50-7.35 (2H, m), 6.98 (1H, d, J=7.3Hz), 4.34 (2H, s), 4.09 (2H, q, J=7.0Hz), 1.38 (3H, t, J=7.0Hz) |
| | | MS(ESI, m/z) = 397(M+H)+ |
| 16-80 | 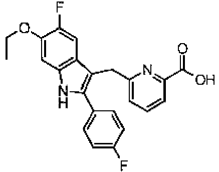 | 1H-NMR (DMSO-d6) δ ppm:11.28 (1H, s), 7.95-7.80 (4H, m), 7.45-7.25 (4H, m), 6.99 (1H, d, J=7.5Hz), 4.28 (2H, s), 4.08 (2H, q, J=7.0Hz), 1.37 (3H, t, J=7.0Hz) |
| | | MS(ESI, m/z) = 409(M+H)+ |

**[Table 130]**

| Ex. No. | Strc | Physical data |
|---|---|---|
| 16-81 | 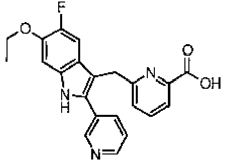 | 1H-NMR (DMSO-d6) δ ppm:11.43 (1H, s), 9.00-8.90 (1H, m), 8.60-8.50 (1H, m), 8.40-8.30 (1H, m), 7.90-7.80 (2H, m), 7.55-7.40 (2H, m), 7.37 (1H, d, J=12.0Hz), 7.01 (1H, d, J=7.4Hz), 4.31 (2H, s), 4.10 (2H, q, J=7.0Hz), 1.38 (3H, t, J=7.0Hz) |
| | | MS(ESI, m/z) = 392(M+H)+ |
| 16-82 | 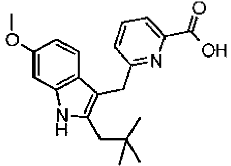 | 1H-NMR (DMSO-d6) δ ppm:10.49 (1H, s), 7.90-7.70 (2H, m), 7.25-7.15 (1H, m), 7.08 (1H, d, J=8.6Hz), 6.81 (1H, d, J=2.3Hz), 6.52 (1H, dd, J=8.6, 2.3Hz), 4.18 (2H, s), 3.72 (3H, s), 2.63 (2H, s), 0.93 (9H, s) |
| | | MS(ESI, m/z) = 353 (M+H)+ |
| 16-83 | 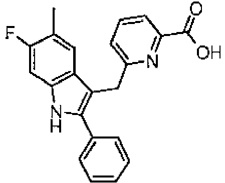 | 1H-NMR (DMSO-d6) δ ppm:13.40-12.80 (1H, br), 11.35 (1H, s), 7.95-7.70 (4H, m), 7.55-7.25 (5H, m), 7.09 (1H, d, J=10.5Hz), 4.35 (2H, s), 2.25 (3H, d, J=1.6Hz) |
| | | MS(ESI, m/z) = 361(M+H)+ |
| 16-84 | 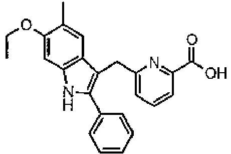 | 1H-NMR (DMSO-d6) δ ppm:13.40-12.90 (1H, br), 11.07(1H, s), 7.90-7.65 (4H, m), 7.50-7.40 (2H, m), 7.35-7.25 (2H, m), 7.19 (1H, s), 6.84 (1H, s), 4.33 (2H, s), 4.03 (2H, q, J=7.0Hz), 2.16 (3H, s), 1.38 (3H, t, J=7.0Hz) |
| | | MS(ESI, m/z) = 387 (M+H)+ |
| 16-85 | 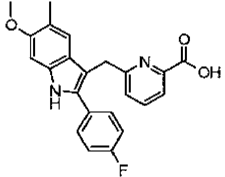 | 1H-NMR (DMSO-d6) δ ppm:13.40-12.90 (1H, br), 11.10 (1H, s), 7.90-7.75 (4H, m), 7.40-7.25 (3H, m), 7.21 (1H, s), 6.84 (1H, s), 4.30 (2H, s), 3.80 (3H, s), 2.16 (3H, s) |
| | | MS(ESI, m/z) = 391 (M+H)+ |

**[Table 131]**

| Ex. No. | Strc | Physical data |
|---|---|---|
| 16-86 | 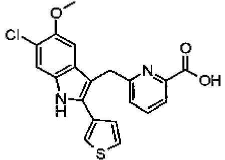 | 1H-NMR (DMSO-d6) δ ppm:13.40-13.00 (1H, br), 11.29 (1H, s), 8.14 (1H, dd, J=2.9, 1.3Hz), 7.90-7.75 (2H, m), 7.70 (1H, dd, J=5.1, 2.9Hz), 7.57 (1H, dd, J=5.1, 1.3Hz), 7.45-7.35 (1H, m), 7.36 (1H, s), 7.32 (1H, s), 4.41 (2H, s), 3.83 (3H, s) |
| | | MS(ESI, m/z) = 399 (M+H)+ |
| 16-87 | 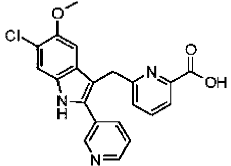 | 1H-NMR (DMSO-d6) δ ppm:13.50-12.80 (1H, br), 11.54 (1H, s), 9.10-9.00 (1H, m), 8.65-8.55 (1H, m), 8.45-8.30 (1H, m), 7.90-7.80 (2H, m), 7.65-7.45 (2H, m), 7.43 (1H, s), 7.31 (1H, s), 4.38 (2H, s), 3.81 (3H, s) |
| | | MS(ESI, m/z) = 394 (M+H)+ |
| 16-88 | 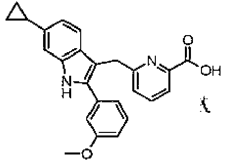 | 1H-NMR (DMSO-d6) δ ppm:13.40-12.80 (1H, br), 11.18 (1H, s), 7.90-7.75 (2H, m), 7.45-7.25 (5H, m), 7.08 (1H, s), 6.95-6.85 (1H, m), 6.71 (1H, dd, J=8.2, 1.5Hz), 4.37 (2H, s), 3.76 (3H, s), 2.05-1.90 (1H, m), 1.00-0.85 (2H, m), 0.70-0.60 (2H, m) |
| | | MS(ESI, m/z) = 399 (M+H)+ |
| 16-89 | 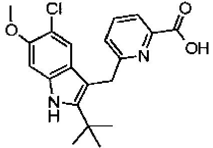 | 1H-NMR (DMSO-d6) δ ppm:13.40-12.80 (1H, br), 10.66 (1H, s), 7.90-7.70 (2H, m), 7.35 (1H, s), 7.10-7.00 (1H, m), 6.98 (1H, s), 4.36 (2H, s), 3.82 (3H, s), 1.35 (9H, s) |
| | | MS(ESI, m/z) = 373 (M+H)+ |
| 16-90 | 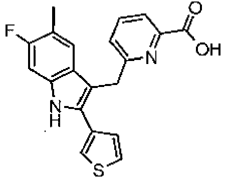 | 1H-NMR (DMSO-d6) δ ppm:13.40-12.90 (1H, br), 11.29 (1H, s), 8.14 (1H, dd, J=2.9, 1.3Hz), 7.90-7.75 (2H, m), 7.68 (1H, dd, J=5.0, 2.9Hz), 7.57 (1H, dd, J=5.0, 1.3Hz), 7.50-7.35 (2H, m), 7.06 (1H, d, J=10.6Hz), 4.37 (2H, s), 2.27 (3H, d, J=1.5Hz) |
| | | MS(ESI, m/z) = 367 (M+H)+ |

**[Table 132]**

| Ex. No. | Strc | Physical data |
|---|---|---|
| 16-91 | 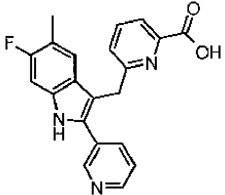 | 1H-NMR (DMSO-d6) δ ppm:13.40-12.80 (1H, br), 11.53 (1H, s), 9.10-8.95 (1H, m), 8.65-8.55 (1H, m), 8.45-8.35 (1H, m), 7.90-7.80 (2H, m), 7.60-7.40 (3H, m), 7.13 (1H, d, J=10.5Hz), 4.35 (2H, s), 2.27 (3H, d, J=1.5Hz) |
| | | MS(ESI, m/z) = 362 (M+H)+ |
| 16-92 | 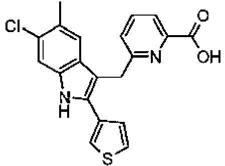 | 1H-NMR (DMSO-d6) δ ppm:13.40-12.90 (1H, br), 11.35 (1H, s), 8.19 (1H, dd, J=2.9, 1.3Hz), 7.90-7.80 (2H, m), 7.70 (1H, dd, J=5.1, 3.0 Hz), 7.58 (1H, dd, J=5.1, 1.3Hz), 7.56 (1H, s), 7.45-7.35 (2H, m), 4.38 (2H, s), 2.36 (3H, s) |
| | | MS(ESI, m/z) = 383 (M+H)+ |
| 16-93 | 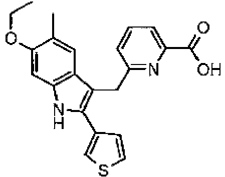 | 1H-NMR (DMSO-d6) δ ppm:13.40-12.80 (1H, br), 11.01 (1H, s), 8.02 (1H, dd, J=3.0, 1.2Hz), 7.90-7.75 (2H, m), 7.65 (1H, dd, J=5.1, 3.0Hz), 7.54 (1H, dd, J=5.1, 1.2Hz), 7.40-7.25 (2H, m), 6.81 (1H, s), 4.35 (2H, s), 4.03 (2H, q, J=7.0Hz), 2.19 (3H, s), 1.38 (3H, t, J=7.0Hz) |
| | | MS(ESI, *m*/*z*) = 393 (M+H)+ |
| 16-94 | 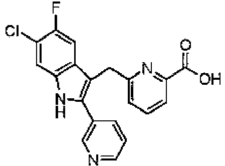 | 1H-NMR (DMSO-d6) δ ppm:13.60-12.60 (1H, br), 11.80 (1H, s), 9.10-9.00 (1H, m), 8.70-8.55 (1H, m), 8.50-8.35 (1H, m), 7.95-7.80 (2H, m), 7.62 (1H, d, J=10.3Hz), 7.60-7.45 (3H, m), 4.34 (2H, s) |
| | | MS(ESI, m/z) = 382 (M+H)+ |
| 16-95 | 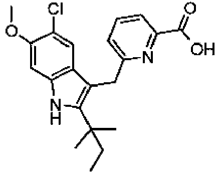 | 1H-NMR (DMSO-d6) δ ppm:10.61 (1H, s), 7.95-7.85 (2H, m), 7.37 (1H, s), 7.15-7.00 (1H, m), 6.99 (1H, s), 4.36 (2H, s), 3.83 (3H, s), 1.65 (2H, q, J=7.5Hz), 1.32 (6H, s), 0.58 (3H, t, J=7.5Hz) |
| | | MS(ESI, m/z) = 387 (M+H)+ |

**[Table 133]**

| Ex. No. | Strc | Physical data |
|---|---|---|
| 16-96 | 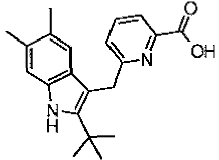 | 1H-NMR (DMSO-d6) δ ppm:13.40-12.80 (1H, br), 10.35 (1H, s), 7.90-7.65 (2H, m), 7.15-6.95 (3H, m), 4.36 (2H, s), 2.26 (3H, s), 2.19 (3H, s), 1.34 (9H, s) |
| | | MS(ESI, m/z) = 337 (M+H)+ |
| 16-97 | 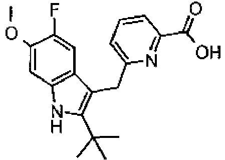 | 1H-NMR (DMSO-d6) δ ppm:10.57 (1H, s), 7.90-7.70 (2H, m), 7.20-7.05 (2H, m), 6.98 (1H, d, J=7.5Hz), 4.36 (2H, s), 3.81 (3H, s), 1.35 (9H, s) |
| | | MS(ESI, m/z) = 357 (M+H)+ |
| 16-98 | 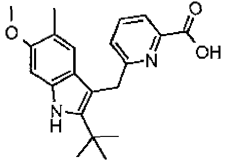 | 1H-NMR (DMSO-d6) δ ppm:10.36 (1H, s), 7.90-7.70 (2H, m), 7.10-6.95 (2H, m), 6.82 (1H, s), 4.34 (2H, s), 3.77 (3H, s), 2.13 (3H, s), 1.34 (9H, s) |
| | | MS(ESI, m/z) = 353 (M+H)+ |
| 16-99 | 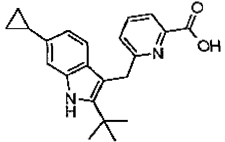 | 1H-NMR (DMSO-d6) δ ppm:10.44 (1H, s), 7.85-7.65 (2H, m), 7.14 (1H, d, J=8.2Hz), 7.05-6.95 (2H, m), 6.64 (1H, dd, J=8.2, 1.5Hz), 4.37 (2H, s), 2.00-1.85 (1H, m), 1.36 (9H, s), 0.95-0.85 (2H, m), 0.65-0.55 (2H, m) |
| | | MS(ESI, m/z) = 349 (M+H)+ |
| 16-100 | 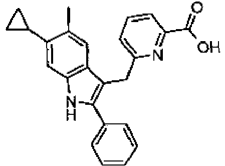 | 1H-NMR (DMSO-d6) δ ppm:11.08 (1H, s), 7.90-7.65 (4H, m), 7.55-7.25 (4H, m), 7.21 (1H, s), 7.00 (1H, s), 4.34 (2H, s), 2.39 (3H, s), 2.00-1.85 (1H, m), 1.00-0.85 (2H, m), 0.60-0.50 (2H, m) |
| | | MS(ESI, m/z)=383(M+H)+ |

**[Table 134]**

| Ex. No. | Strc | Physical data |
|---|---|---|
| 16-101 | 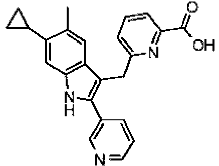 | 1H-NMR (DMSO-d6) δ ppm:11.24 (1H, s), 8.95-8.90 (1H, m), 8.60-8.50 (1H, m), 8.35-8.20 (1H, m), 7.90-7.80 (2H, m), 7.55-7.35 (2H, m), 7.29 (1H, s), 7.01 (1H, s), 4.33 (2H, s), 2.40 (3H, s), 2.05-1.85 (1H, m), 1.00-0.85 (2H, m), 0.65-0.50 (2H, m) |
| | | MS(ESI, m/z) = 384 (M+H)+ |
| 16-102 | 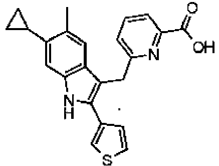 | 1H-NMR (D MSO-d6) δ ppm:11.03 (1H, s), 8.08 (1H, dd, J=3.0, 1.2Hz), 7.90-7.75 (2H, m), 7.66 (1H, dd, J=5.1, 3.0Hz), 7.55 (1H, dd, J=5.1, 1.2Hz), 7.40-7.25 (2H, m), 6.97 (1H, s), 4.36 (2H, s), 2.41 (3H, s), 2.00-1.85 (1H, m), 1.00-0.85 (2H, m), 0.60-0.50 (2H, m) |
| | | MS(ESI, m/z) = 389 (M+H)+ |
| 16-103 | 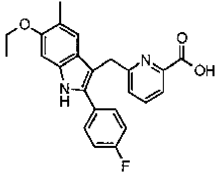 | 1H-NMR (DMSO-d6) δ ppm:13.50-12.70 (1H, br), 11.07 (1H, s), 7.90-7.75 (4H, m), 7.40-7.25 (3H, m), 7.21 (1H, s), 6.83 (1H, s), 4.29 (2H, s), 4.02 (2H, q, J=7.0Hz), 2.16 (3H, s), 1.38 (3H, t, J=7.0Hz) |
| | | MS(ESI, m/z) = 405 (M+H)+ |
| 16-104 | 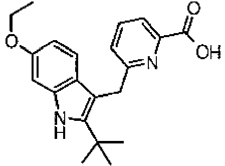 | 1H-NMR (DMSO-d6) δ ppm:10.43 (1H, s), 7.90-7.70 (2H, m), 7.14 (1H, d, J=8.6Hz), 7.10-7.00 (1H, m), 6.82 (1H, d, J=2.2Hz), 6.53 (1H, dd, J=8.6, 2.2Hz), 4.36 (2H, s), 3.98 (2H, q, J=7.0Hz), 1.36 (9H, s), 1.33 (3H, t, J=7.0Hz) |
| | | MS(ESI, m/z) = 353 (M+H)+ |
| 16-105 | 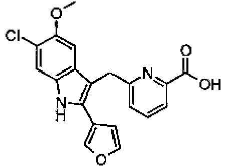 | 1H-NMR (DMSO-d6) δ ppm:11.21 (1H,s), 8.50-8.40 (1H, m), 7.90-7.75 (3H, m), 7.45-7.30 (3H, m), 6.99 (1H, dd, J=1.9, 0.7Hz), 4.35 (2H, s), 3.84 (3H, s) |
| | | MS(ESI, m/z) = 383 (M+H)+ |

**[Table 135]**

| Ex. No. | Strc | Physical data |
|---|---|---|
| 16-106 | 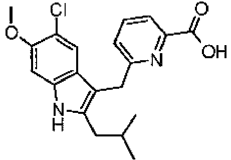 | 1H-NMR (DMSO-d6) δ ppm:10.80 (1H, s), 7.90-7.75 (2H, m), 7.42 (1H, s), 7.30-7.20 (1H, m), 6.93 (1H, s), 4.16 (2H, s), 3.81 (3H, s), 2.60 (2H, d, J=7.3Hz), 2.05-1.85 (1H, m), 0.84 (6H, d, J=6.6Hz) |
| | | MS(ESI, m/z) = 373 (M+H)+ |
| 16-107 | 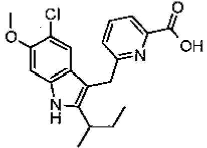 | 1H-NMR (DMSO-d6) δ ppm:10.76 (1H, s), 7.90-7.75 (2H, m), 7.46 (1H, s), 7.35-7.20 (1H, m), 6.92 (1H, s), 4.17 (2H, s), 3.81 (3H, s), 3.15-2.95 (1H, m), 1.70-1.50 (2H, m), 1.21 (3H, d, J=6.9Hz), 0.69 (3H, t, J=7.4Hz) |
| | | MS(ESI, m/z) = 373 (M+H)+ |
| 16-108 | 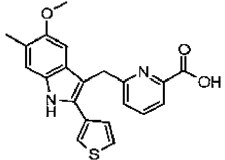 | 1H-NMR (DMSO-d6) δ ppm:13.30-12.80 (1H, br), 10.98 (1H, s), 8.03 (1H, dd, J=3.0, 1.3Hz), 7.90-7.75 (2H, m), 7.66 (1H, dd, J=5.0, 3.0Hz), 7.56 (1H, dd, J=5.0, 1.3Hz), 7.45-7.30 (1H, m), 7.11 (1H, s), 7.05 (1H, s), 4.39 (2H, s), 3.76 (3H, s), 2.23 (3H, s) |
| | | MS(ESI, m/z) = 379 (M+H)+ |
| 16-109 | 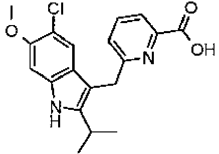 | 1H-NMR (DMSO-d6) δ ppm:10.81 (1H, s), 7.85-7.75 (2H, m), 7.45 (1H, s), 7.35-7.25 (1H, m), 6.92 (1H, s), 4.17 (2H, s), 3.81 (3H, s), 3.40-3.25 (1H,m), 1.24 (6H, d, J=6.9Hz) |
| | | MS(ESI, m/z) = 359 (M+H)+ |

**[Table 136]**

| Ex. No. | Strc | Physical data |
|---|---|---|
| 17-1 | 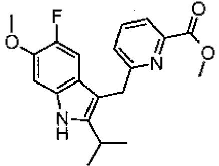 | 1H-NMR (CDCl3) δ ppm:8.00-7.90 (1H, m), 7.84 (1H, s), 7.70-7.55 (1H, m), 7.15-7.00 (2H, m), 6.92 (1H, d, J=7.1 Hz), 4.32 (2H, s), 4.04 (3H, s), 3.90 (3H, s), 3.35-3.20 (1H, m), 1.27 (6H, d, J=7.0Hz) |
| 17-2 | 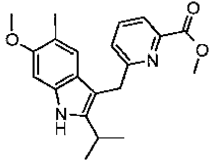 | 1H-NMR (CDCl3) δ ppm:8.00-7.90 (1H, m), 7.79 (1H, s), 7.65-7.55 (1H, m), 7.20-7.10 (1H, m), 7.09 (1H, s), 6.81 (1H, s), 4.35 (2H, s), 4.04 (3H, s), 3.84 (3H, s), 3.30-3.15 (1H, m), 2.23 (3H, s), 1.25(6H, d, J=7.0Hz) |
| 18-1 | 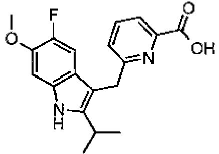 | 1H-NMR (DMSO-d6) δ ppm:10.72 (1H, s), 7.90-7.75 (2H, m), 7.35-7.25 (1H, m), 7.22 (1H, d, J=12.2Hz), 6.91 (1H, d, J=7.4Hz), 4.15 (2H, s), 3.80 (3H, s), 1.23 (6H, d, J=7.0Hz) |
| | | MS(ESI, m/z) = 343 (M+H)+ |
| 18-2 | 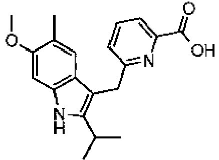 | 1H-NMR (DMSO-d6) δ ppm:10.50 (1H, s), 7.85-7.70 (2H, m), 7.25-7.15 (1H, m), 7.12 (1H, s), 6.76 (1H, s), 4.15 (2H, s), 3.75 (3H, s), 3.35-3.15 (1H, m), 2.13 (3H, s), 1.22 (6H, d, J=6.9Hz) |
| | | MS(ESI, m/z) = 339 (M+H)+ |

### Test Example 1

### Test for Confirmation of EP₁ Receptor Antagonism

### (1) Preparation of Rat EP₁ Expression vector

Using Rat Kidney BD Marathon-Ready cDNA (Nippon Becton Dickinson Company, Ltd.) as a template, a forward primer shown in SEQ ID NO. 1, and a reverse primer shown in SEQ ID NO. 2, a first run of PCR was carried out using KOD-Plus-Ver 2.0 (Toyobo Co., Ltd.). Further, using this amplification product as a template, a forward primer shown in SEQ ID NO. 3, and a reverse primer shown in SEQ ID NO. 4, a second run of PCR was carried out in the same manner. The amplification product obtained by the second run of PCR was incorporated into a vector (pcDNA3.1 D/V5-His-TOPO (registered trademark), Invitrogen Japan K. K.). By a conventional method, the vector containing this amplification product was introduced to E. coli (One Shot TOP10 Competent Cells, Invitrogen Corporation) to transform. This transformed E. coli was cultured in an LB agar medium for one day. After the culture, colonies were selected and cultured in an LB liquid medium containing 50 µg/mL of ampicillin. After the culture, the vector was purified using a QIAprep Spin Miniprep Kit (Qiagen K. K.). The base sequence of the insertion site of this vector (SEQ ID NO.5) was compared with the rat EP₁ base sequence (Ptger1) registered as an accession number NM_013100 in well-known database (NCBI), and as a result, they all matched except for a single base. Further, the amino acid sequence translated by the base sequence completely matched the amino acid sequence of the rat EP₁ receptor registered as an NCBI accession number NP_037232. Therefore, it was confirmed that the cloned gene sequence was a base sequence of the rat EP₁ receptor and the obtained amino acid sequence was that of the rat EP₁ receptor. The pcDNA3.1 D/V5-His-TOPO (registered trademark) to which the nucleic acid shown in SEQ ID NO.5 had been inserted was taken as a rat EP₁-expressing vector.

### (2) Preparation of Rat EP₁ Receptor-Expressing Cells

### (2-1) COS-1 Cell Culture

COS-1 cells (Dainippon Sumitomo Pharma Co., Ltd.) were cultured until it reached confluence in an incubator at 37°C under a 5% CO₂ gas condition, using a D-MEM liquid medium (high glucose and L-glutamine contained, Invitrogen Corporation) to which a penicillin-streptomycin solution (Invitrogen Corporation, final concentration: 100 U/mL as benzylpenicillin; 100 µg/mL as streptomycin) as an antibiotic, MEM nonessential amino acids (Invitrogen Corporation, final Concentration: 0.1 mM), and fetal calf serum (Sanko Junyaku Co., Ltd., final concentration: 10%) were added.

### (2-2) COS-1 Cell Subculture

The cells that had reached confluence were stripped with 0.05% trypsin/0.53 mM EDTA 4Na (Invitrogen Japan K. K.) and resuspended in the above liquid medium. The resuspended cells were diluted and cultured in the above liquid medium at a spread ratio from 1:4 to 1:8.

### (2-3) Preparation of Cells for Introduction of Rat EP₁-Expressing Vector

The cells that had reached confluence were stripped with 0.05% trypsin/0.53 mM EDTA-4Na, and resuspended in a D-MEM liquid medium (high glucose and L-glutamine contained, Invitrogen Corporation) to which an MEM nonessential amino acid (final concentration: 0.1 mM) and fetal calf serum (final concentration: 10%) were added. In each well of a Poly D-lysine-coated 96-well microplate (BD BioCoat (registered trademark), Nippon Becton Dickinson Company, Ltd.), this resuspended cell suspension culture was prepared to be 5x10⁴ cells/well in 100 µL of the liquid medium, and seeded thereon. After seeding, the cells were cultured in an incubator at 37°C under a 5% CO₂ gas condition. At a point when the cells for introduction of a rat EP₁-expressing vector were adhered (about 2 hours after seeding), introduction of the rat EP₁-expressing vector was carried out in the following order.

### (2-4) Introduction of Rat EP₁-Expressing Vector

For introduction of the rat EP₁-expressing vector, Lipofectamine 2000 (Invitrogen Japan K. K.) was used. The rat EP₁-expressing vector was diluted with OPTI-MEM (registered trademark) 1 Reduced-Serum Medium (Invitrogen Japan K. K.) to 200 ng/25 µL/well, and at the same time, Lipofectamine 2000 (Invitrogen Japan K. K.) was also diluted with OPTI-MEM (registered trademark) I Reduced-Serum Medium (Invitrogen Japan K. K.) to 0.5 µL/25 µL/well, followed by incubation at room temperature for 5 minutes. After the incubation for 5 minutes, in order to form a complex of the rat EP₁-expressing vector/Lipofectamine 2000, the diluted rat EP₁-expressing vector and the diluted Lipofectamine 2000 were mixed and incubated at room temperature for 30 minutes. After the incubation for 30 minutes, the complex of the rat EP₁-expressing vector/Lipofectamine 2000 was distributed to the above cells for introduction of the rat EP₁-expressing vector at 50 µL/well. The cells to which the complex of the rat EP₁-expressing vector/Lipofectamine 2000 had been distributed were cultured in an incubator at 37°C for 20 hours under a 5% CO₂ gas condition. After the culture for 20 hours, the cells were used for measurement of an intracellular calcium concentration as rat EP₁ receptor-expressing cells.

### (3) Study on Inhibitory Effect on Increase in Intracellular Calcium Concentration

Using the rat EP₁ receptor-expressing cells, the inhibitory effect of each test compound on the increased intracellular calcium concentration induced by prostaglandin E₂ was studied in Method A or Method B as shown below.

### Method A:

A 10 mM solution of each test compound in dimethyl sulfoxide was diluted with an assay buffer (20 mM HEPES/Hank's Balanced Salt Solution (HBSS), pH 7.2).

The rat EP1 receptor-expressing cells were washed with the assay buffer. 100 µL Of a fluorescent calcium indicator (Fluo-4 NW Calcium Assay Kit (Molecular Probes): prepared by the protocol of the same product, Invitrogen Corporation, 2.5 mM probenecid contained) was added to each well, followed by incubation in an incubator at 37°C for 60 minutes. Then, all the cell supernatants were aspirated and washed with the assay buffer. After the washing, 100 µL of an assay buffer containing 2.5 mM probenecid was added to each well, and the intracellular calcium concentration was measured immediately.

The intracellular calcium concentration was measured as a fluorescent signal using FlexStation (registered trademark) (manufactured by Molecular Devices). 50 µL Of each test compound that had been diluted with the assay buffer (final concentrations: 1 nM to 10 µM) was added to each well after 20 seconds from initiating the reading of the fluorescent signal, and the fluorescence signal was measured for 60 seconds. Then, 50 µL of a prostaglandin E₂ buffer solution were added to each well (final concentration 10 nM) and the fluorescence signal was measured for 60 seconds.

### Method B:

A 10 mM solution of each test compound in dimethyl sulfoxide was diluted in an assay buffer (20 mM HEPES/Hank's Balanced Salt Solution (HBSS), pH 7.2).

The rat EP₁ receptor-expressing cells were washed with the assay buffer. 100 µL Of a fluorescent calcium indicator (Calcium kit II, Fluo 4 (Dojindo Laboratories): prepared by the protocol of the same product, Invitrogen Japan K. K., 2.5 mM probenecid contained) was added to each well, followed by incubation in an incubator at 37°C for 60 minutes. Then, the intracellular calcium concentration was measured immediately.

The intracellular calcium concentration was measured as a fluorescent signal using FDSS (registered trademark) 7000 (manufactured by Hamamatsu Photonics K. K.). 50 µL Of each test compound (final concentrations: 1 nM to 10 µM) was added to each well after 20 seconds from initiating the reading of the fluorescent signal, and the fluorescence signal was measured for 60 seconds. Then, 50 µL of a prostaglandin E₂ buffer solution were added to each well (final concentration 10 nM) and the fluorescence signal was measured for 60 seconds.

In Method A or Method B, as a fluorescent signal obtained by the addition of the prostaglandin E₂ with the addition of the assay buffer instead of the test compound was taken as 100% and a signal obtained without the addition of any of the test compound and the prostaglandin E₂ was taken as 0%, the concentration of the test compound showing 50% inhibition from the concentration-response curve was taken as an IC₅₀ value. As the values of the EP₁ receptor antagonism, the obtained IC₅₀ values of each test compound were shown in Tables 137 to 138 below. Further, sodium 6-(6-chloro-3-isobutylindol-1-yl)pyridine-2-carboxylate (Compound 12g) described in Non-Patent literature 5 was tested in the similar way as Comparative Example 1. The results were shown in Table 138.

**[Table 137]**

| Ex. No | IC₅₀ (nM) | Method | Ex. No | IC₅₀ (nM) | Method |
|---|---|---|---|---|---|
| 4-1 | 10 | A | 11-34 | 36 | B |
| 4-2 | 32 | A | 11-37 | 50 | B |
| 4-12 | 40 | A | 16-2 | 23 | B |
| 4-17 | 15 | A | 16-6 | 44 | B |
| 4-27 | 28 | A | 16-8 | 32 | B |
| 4-34 | 44 | A | 16-9 | 41 | B |
| 4-35 | 18 | A | 16-11 | 27 | B |
| 11-1 | 11 | A | 16-12 | 23 | B |
| 11-3 | 42 | A | 16-13 | 36 | B |
| 11-5 | 14 | B | 16-16 | 25 | B |
| 11-10 | 15 | B | 16-18 | 30 | B |
| 11-11 | 32 | B | 16-21 | 24 | B |
| 11-13 | 31 | B | 16-23 | 34 | B |
| 11-15 | 48 | B | 16-24 | 22 | B |
| 11-17 | 50 | B | 16-28 | 30 | B |
| 11-20 | 26 | A | 16-29 | 27 | B |
| 11-21 | 23 | A | 16-38 | 36 | B |
| 11-24 | 41 | B | 16-44 | 11 | B |

**[Table 138]**

| Ex. No | IC₅₀ (nM) | Method | Ex. No | IC₅₀ (nM) | Method |
|---|---|---|---|---|---|
| 16-45 | 26 | B | 16-76 | 20 | B |
| 16-48 | 27 | B | 16-78 | 24 | B |
| 16-51 | 14 | B | 16-79 | 22 | B |
| 16-52 | 43 | B | 16-84 | 17 | B |
| 16-53 | 31 | B | 16-85 | 18 | B |
| 16-54 | 47 | B | 16-86 | 18 | B |
| 16-56 | 39 | B | 16-89 | 22 | B |
| 16-58 | 38 | B | 16-92 | 30 | B |
| 16-59 | 19 | B | 16-93 | 46 | B |
| 16-60 | 32 | B | 16-97 | 32 | B |
| 16-61 | 43 | B | 16-98 | 47 | B |
| 16-62 | 27 | B | 16-100 | 44 | B |
| 16-65 | 14 | B | 16-100 | 26 | B |
| 16-66 | 30 | B | 16-102 | 49 | B |
| 16-67 | 22 | B | 16-105 | 26 | B |
| 16-68 | 34 | B | 16-107 | 40 | B |
| 16-73 | 23 | B | 16-108 | 19 | B |
| 16-74 | 47 | B | 16-109 | 35 | B |
| 16-75 | 16 | B | 18-1 | 39 | B |
| | | | Comparative Example | 476 | B |

As shown in Tables 137 and 138, it is apparent that the compounds of the present invention exhibit potent EPₜ receptor antagonism, as compared with Comparative Example 1.

### Test Example 2

### Inhibitory Effect of Compound on Sulprostone-Induced Bladder Contraction

Female SD rats were used. Under urethane anesthesia (1.25 g/kg, administered subcutaneously), a tracheal cannula (Size 8, HIBIKI) and a femoral vein cannula for administration (23G needle-equipped PE50) were inserted thereinto. The bladder cannula (PE50) was inserted from the bladder apex. The bladder cannula was connected to a three-way stopcock, and then, one was connected to a pressure transducer and the other was connected to a syringe filled with saline. Saline was injected to the bladder at an injection rate of 3.6 mL/hour and the bladder contraction pressure was recorded at the time of injection with a recorder (RECTI-HORITZ-8K, NEC Corporation). After 10 minutes from stabilization of the bladder contraction pressure during urination, sulprostone was administered subcutaneously (0.3 mg/kg). Then, at the time point when the bladder contraction pressure became constant, a test agent was administered intravenously (1.0 mg/kg). An average bladder contraction pressure during the 10 minutes period before administration of sulprostone was taken as a baseline (0%). Further, an average bladder contraction pressure during the 10 minutes period before administration of the test agent was taken as a maximum bladder contraction pressure (100%). The average bladder contraction pressures were measured during 5 minutes before and after at 15 minutes and 60 minutes from administration of the test agent. The ratio of this measured value to the maximum bladder contraction pressure was calculated by the following equation and taken as an average bladder contraction rate after administration of the test agent: (Average Bladder Contraction Rate after Administration of Test Agent (%))=(Average Bladder Contraction Pressure after Administration of Test Agent)/(Maximum Bladder Contraction Pressure). In addition, the difference between the maximum bladder contraction rate (100%) and the average bladder contraction rate (%) after administration of the test agent was calculated by the following equation and taken as a bladder contraction inhibition rate of the test agent: (Bladder Contraction Inhibition Rate) = 100% - (Average Bladder Contraction Rate after Administration of Test Agent (%)). The results were shown in Table 139.

**[Table 139]**

| Ex No. | Bladder Contraction Inhibition Rate (%) | |
|---|---|---|
| | 15 Minutes | 30 Minutes |
| 4-1 | 90.1 | 94.2 |
| 4-2 | 78.3 | 80.4 |
| 11-1 | 86.1 | 76.8 |
| 11-5 | 79.7 | 81.9 |
| 11-20 | 81.4 | 82.9 |

From the results above, it was found that the compounds of the present invention had potent and sustained inhibition of the bladder contraction even when administered in vivo.

### Industrial Applicability

The compound of the present invention has a potent EP₁ receptor antagonism, and therefore, it is useful as an agent for treating or preventing diseases or symptoms caused by activation of an EP₁ receptor due to a stimulus of PGE₂. In particular, it is useful as an agent for treating or preventing lower urinary tract symptoms (LUTS), particularly overactive bladder syndrome (OABs).

### Sequence List Free Text

<Sequence List 1>
   SEQ ID No. 1 is a sequence of a forward primer (5' primer) used for amplification of DNA of SEQ ID NO. 5.
<Sequence List 2>
   SEQ ID NO. 2 is a sequence of a reverse primer (3' primer) used for amplification of DNA of SEQ ID NO. 5.
<Sequence List 3>
   SEQ ID No. 3 is a sequence of a forward primer (5' primer) used for amplification of DNA of SEQ ID NO. 5.
<SEQ ID NO. 4>
   SEQ ID NO. 4 is a sequence of a reverse primer (3' primer) used for amplification of DNA of SEQ ID NO. 5.
<SEQ ID NO. 5>
   SEQ ID NO. 5 is a DNA sequence for expressing a rat EP1 receptor which is amplified using the primers of SEQ ID NO.1, SEQ ID NO.2, SEQ ID NO.3, and SEQ ID NO. 4.

## Claims

1. A compound represented by the general formula (I) or a pharmaceutically acceptable salt thereof: 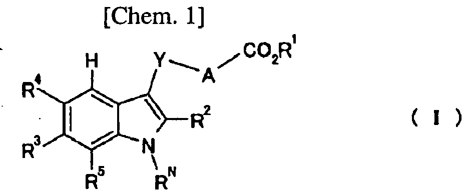 [wherein
A represents a group selected from the group consisting of the following a) to h): 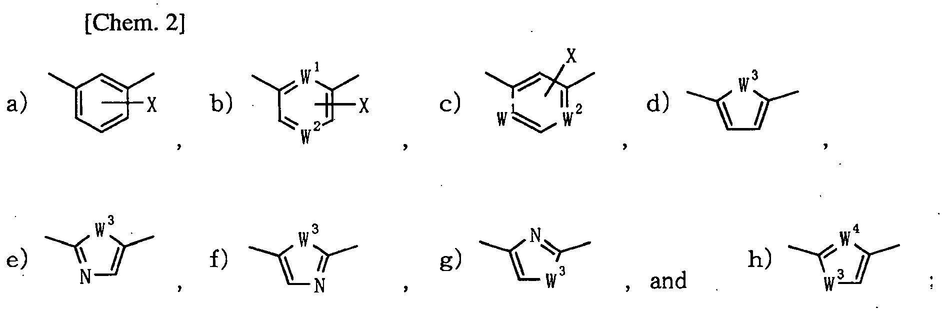
one of W¹ and W² represents a nitrogen atom and the other represents =CH- or a nitrogen atom;
W³ represents an oxygen atom or a sulfur atom;
W⁴ represents =CH- or a nitrogen atom;
X represents a hydrogen atom or a halogen atom;
Y represents a C₁₋₆ alkylene group or a halo-C₁₋₆ alkylene group;
R^{N} represents a hydrogen atom or a C₁₋₆ alkyl group;
R¹ represents a hydrogen atom, a C₁₋₆ alkyl group or a C₇₋₁₀ aralkyl group;
R² represents a group selected from the group consisting of the following i) to m):
i) a branched C₃₋₆ alkyl group,
j) a C₃₋₆ cycloalkyl group,
k) a phenyl group, in which the ring is unsubstituted or substituted with 1 to 5 groups independently selected from the group consisting of: a halogen atom, a C₁₋₆ alkyl group, a halo-C₁₋₆ alkyl group, a hydroxy-C₁₋₆ alkyl group, a C₁₋₆ alkoxy group and a cyano group,
l) a 6-membered aromatic heterocyclic group, in which the ring is unsubstituted or substituted with 1 to 4 groups independently selected from the group consisting of: a halogen atom, a C₁₋₆ alkyl group, a halo-C₁₋₆ alkyl group, a hydroxy-C₁₋₆ alkyl group, a C₁₆ alkoxy group and a cyano group, and
m) a 5-membered aromatic heterocyclic group, in which the ring is unsubstituted or substituted with 1 to 3 groups independently selected from the group consisting of: a halogen atom, a C₁₋₆ alkyl group, a halo-C₁-₆ alkyl group, a hydroxy-C₁₋₆ alkyl group, a C₁₆ alkoxy group and a cyano group;
R³ represents a halogen atom, a C₁₋₆ alkyl group, a halo-C₁₋₆ alkyl group, a hydroxy-C₁₋₆ alkyl group, a C₁₋₆ alkoxy group, a halo-C₁₋₆ alkoxy group, a C₁₋₆ alkylsulfanyl group, a C₁₋₆ alkylsulfinyl group, a C₁₋₆ alkylsulfonyl group, a C₃₋₆ cycloalkyl group, a cyano group, an amino group or a nitro group;
R⁴ represents a hydrogen atom, a halogen atom, a C₁₋₆ alkyl group or a C₁₋₆ alkoxy group; and
R⁵ represents a hydrogen atom, a halogen atom, a C₁₋₆ alkyl group or a C₁₋₆ alkoxy group].

2. The compound as claimed in claim 1 or a pharmaceutically acceptable salt thereof, wherein A is a group selected from the group consisting of the following a) to d):

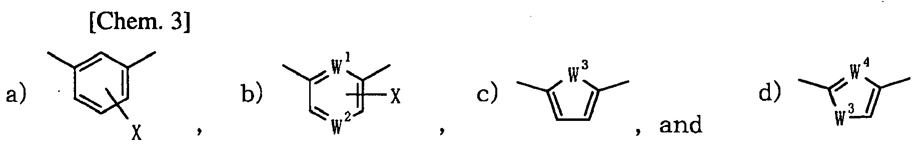

3. The compound as claimed in claim 2 or a pharmaceutically acceptable salt thereof, wherein A is a group selected from the group consisting of the following a) to c): 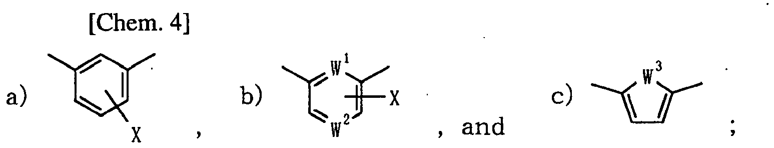
W¹ is a nitrogen atom;
W² is =CH-; and
X is a hydrogen atom.

4. The compound as claimed in claim 3 or a pharmaceutically acceptable salt thereof, wherein R² is a group selected from the group consisting of the following a) to c):
a) a branched C₃₋₆ alkyl group,
b) a phenyl group, and
c) a 5-membered aromatic heterocyclic group or a 6-membered aromatic heterocyclic group;
R³ is a halogen atom, a C₁₋₆ alkyl group, a halo-C₁₋₆ alkyl group, a C₁₋₆ alkoxy group, a halo-C₁₋₆ alkoxy group, or a cyano group;
R⁴ is a hydrogen atom; and
R⁵ is a hydrogen atom.

5. The compound as claimed in claim 4 or a pharmaceutically acceptable salt thereof, wherein R¹ is a hydrogen atom.

6. The compound as claimed in claim 5 or a pharmaceutically acceptable salt thereof, wherein Y is a methylene group and R^{N} is a hydrogen atom.

7. The compound as claimed in claim 6 or a pharmaceutically acceptable salt thereof, wherein R² is a phenyl group, a 5-membered aromatic heterocyclic group, or a 6-membered aromatic heterocycle.

8. The compound as claimed in claim 7 or a pharmaceutically acceptable salt thereof, wherein R³ is a halogen atom or a C₁₋₆ alkoxy group.

9. The compound as claimed in claim 6 or a pharmaceutically acceptable salt thereof, wherein R² is an isopropyl group, an isobutyl group, a sec-butyl group, or a tert-butyl group.

10. The compound as claimed in claim 9 or a pharmaceutically acceptable salt thereof, wherein R³ is a halogen atom or a C₁₋₆ alkoxy group.

11. The compound as claimed in claim 2 or a pharmaceutically acceptable salt thereof, wherein A is a group represented by the following formula:

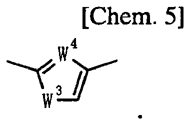

12. The compound as claimed in claim 11 or a pharmaceutically acceptable salt thereof, wherein R¹ is a hydrogen atom.

13. The compound as claimed in claim 3 or a pharmaceutically acceptable salt thereof, wherein R² is a group selected from the group consisting of the following a) to d): 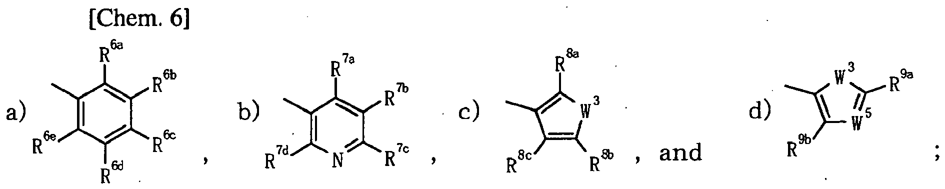
W⁵ is a nitrogen atom or -CR^{9c}=; and
R^{6a}, R^{6b}, R^{6c}, R^{6d}, R^{6e}, R^{7a}, R^{7b}, R^{7c}, R^{7d}, R^{8a}, R^{8b}, R^{8c}, R^{9a}, R^{9b} and R^{9c} are each independently a hydrogen atom, a halogen atom, a C₁-₆ alkyl group, a halo-C₁-₆ alkyl group, a hydroxy-C₁-₆ alkyl group, a C₁₋₆ alkoxy group or a cyano group.

14. The compound as claimed in claim 13 or a pharmaceutically acceptable salt thereof, wherein R¹ is a hydrogen atom.

15. A pharmaceutical composition comprising the compound as claimed in any one of claims 1 to 14 or a pharmaceutically acceptable salt thereof as an active ingredient.

16. An EP₁ receptor antagonist comprising the compound as claimed in any one of claims 1 to 14 or a pharmaceutically acceptable salt thereof as an active ingredient.

17. An agent for treating or preventing lower urinary tract symptoms, comprising the compound as claimed in any one of claims 1 to 14 or a pharmaceutically acceptable salt thereof as an active ingredient.

## Patentansprüche

1. Verbindung, dargestellt durch die allgemeine Formel (I), oder ein pharmazeutisch verträgliches Salz davon: 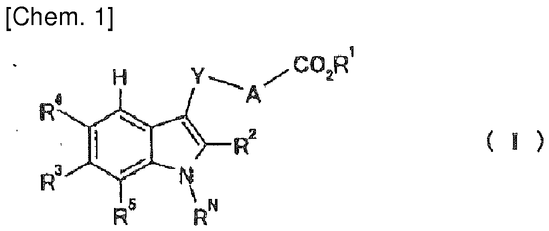 [worin
A eine Gruppe darstellt, ausgewählt aus der Gruppe, bestehend aus den folgenden a) bis h): 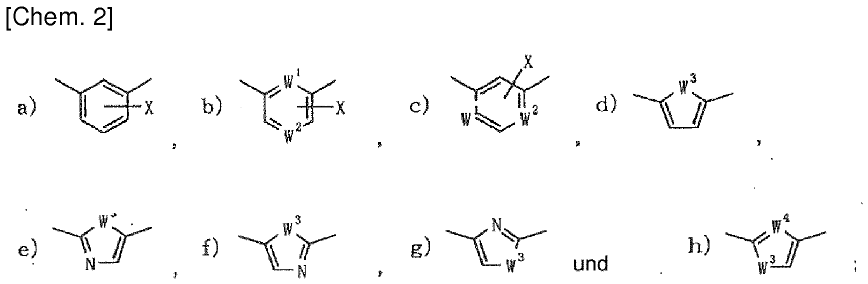
worin eines von W¹ und W² ein Stickstoffatom darstellt und das andere =CH- oder
ein Stickstoffatom darstellt;
W³ ein Sauerstoffatom oder ein Schwefelatom darstellt;
W⁴ =CH- oder ein Stickstoffatom darstellt;
X ein Wasserstoffatom oder ein Halogenatom darstellt;
Y eine C₁₋₆-Alkylengruppe oder eine Halo- C₁₋₆-Alkylengruppe darstellt;
R^{N} ein Wasserstoffatom oder eine C₁₋₆-Alkylgruppe darstellt;
R¹ ein Wasserstoffatom, eine C₁₋₆-Alkylgruppe oder eine C₇₋₁₀-Aralkylgruppe darstellt;
R² eine Gruppe darstellt, ausgewählt aus der Gruppe, bestehend aus den folgenden i) bis m):
i) einer verzweigten C₃₋₆-Alkylgruppe;
j) einer C₃₋₆-Cycloalkylgruppe;
k) einer Phenylgruppe, in der der Ring unsubstituiert oder substituiert ist mit 1 bis 5 Gruppen, unabhängig ausgewählt aus der Gruppe, bestehend aus: einem Halogenatom, einer C₁₋₆-Alkylgruppe, einer Halo-C₁₋₆-alkylgruppe, einer Hydroxy-C₁₋₆-alkylgruppe, einer C₁₋₆-Alkoxygruppe und einer Cyanogruppe.,
I) einer 6-gliedrigen aromatischen heterozyklischen Gruppe, in welcher der Ring unsubstituiert oder substituiert ist mit 1 bis 4 Gruppen, unabhängig ausgewählt aus der Gruppe, bestehend aus: einem Halogenatom, einer C₁₋₆-Alkylgruppe, einer Halo-C₁₋₆-alkylgruppe, einer Hydroxy-C₁₋₆-alkylgruppe, einer C₁₋₆-Alkoxygruppe und einer Cyanogruppe, und
m) einer 5-gliedrigen aromatischen heterozyklischen Gruppe, in welcher der Ring unsubstituiert oder substituiert ist mit 1 bis 3 Gruppen, unabhängig ausgewählt aus der Gruppe, bestehend aus: einem Halogenatom, einer C₁₋₆-Alkylgruppe, einer Halo-C₁₋₆-alkylgruppe, einer Hydroxy-C₁₋₆-alkylgruppe, einer -C₁₋₆-Alkoxygruppe und einer Cyanogruppe;
R³ ein Halogenatom, eine C₁₋₆-Alkylgruppe, eine Halo-C₁₋₆-alkylgruppe, eine Hydroxy-C₁₋₆-alkylgruppe, eine C₁₋₆-Alkoxygruppe, eine Halo-C₁₋₆-alkoxygruppe, eine C₁₋₆-Alkylsulfanylgruppe, eine C₁₋₆-Alkylsulfinylgruppe, eine C₁₋₆-Alkylsulfonylgruppe, eine C₃₋₆-Cycloalkylgruppe, eine Cyanogruppe, eine Aminogruppe oder eine Nitrogruppe ist;
R⁴ ein Wasserstoffatom, ein Halogenatom, eine C₁₋₆-Alkylgruppe oder eine C₁₋₆-Alkoxygruppe darstellt; und
R⁵ ein Wasserstoffatom, ein Halogenatom, eine C₁₋₆-Alkylgruppe oder eine C₁₋₆-Alkoxygruppe darstellt].

2. Verbindung nach Anspruch 1 oder ein pharmazeutisch verträgliches Salz davon, worin A eine Gruppe ist, ausgewählt aus der Gruppe, bestehend aus den folgenden a) bis d):

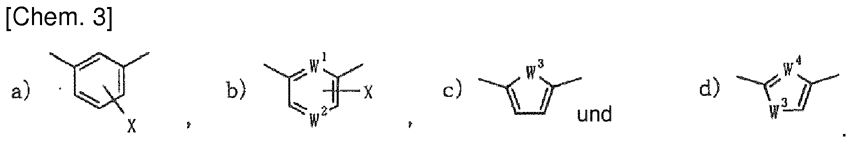

3. Verbindung nach Anspruch 2 oder ein pharmazeutisch verträgliches Salz davon, worin A eine Gruppe ist, ausgewählt aus der Gruppe, bestehend aus den folgenden a) bis c): 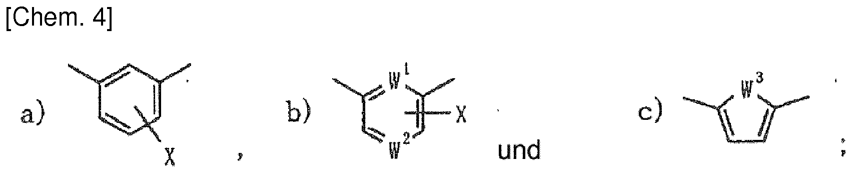
worin W¹ ein Stickstoffatom ist;
W² =CH- ist; und
X ein Wasserstoffatom ist.

4. Verbindung nach Anspruch 3 oder ein pharmazeutisch verträgliches Salz davon, worin R² eine Gruppe ist, ausgewählt aus der Gruppe, bestehend aus den folgenden a) bis c):
a) einer verzweigten C₃₋₆-Alkylgruppe,
b) einer Phenylgruppe und
c) einer 5-gliedrigen aromatischen heterozyklischen Gruppe oder einer 6-gliedrigen aromatischen heterozyklischen Gruppe;
R³ ein Halogenatom, eine C₁₋₆-Alkylgruppe, eine Halo-C₁-₆-alkylgruppe, eine C₁₋₆-Alkoxygruppe, eine Halo-C₁₋₆-Alkoxygruppe oder eine Cyanogruppe ist;
R⁴ ein Wasserstoffatom ist; und
R⁵ ein Wasserstoffatom ist.

5. Verbindung nach Anspruch 4 oder ein pharmazeutisch verträgliches Salz davon, worin R¹ ein Wasserstoffatom ist.

6. Verbindung nach Anspruch 5 oder ein pharmazeutisch verträgliches Salz davon, worin Y eine Methylengruppe ist und R^{N} ein Wasserstoffatom ist.

7. Verbindung nach Anspruch 6 oder ein pharmazeutisch verträgliches Salz davon, worin R² eine Phenylgruppe, eine 5-gliedrige aromatische heterozyklische Gruppe oder ein 6-gliedriger aromatischer Heterozyklus ist.

8. Verbindung nach Anspruch 7 oder ein pharmazeutisch verträgliches Salz davon, worin R³ ein Halogenatom oder eine C₁₋₆-Alkoxygruppe ist.

9. Verbindung nach Anspruch 6 oder ein pharmazeutisch verträgliches Salz davon, worin R² eine Isopropylgruppe, eine Isobutylgruppe, eine sec-Butylgruppe oder eine *tert*-Butylgruppe ist.

10. Verbindung nach Anspruch 9 oder ein pharmazeutisch verträgliches Salz davon, worin R³ ein Halogenatom oder eine C₁₋₆-Alkoxygruppe ist.

11. Verbindung nach Anspruch 2 oder ein pharmazeutisch verträgliches Salz davon, worin A eine Gruppe ist, dargestellt durch die folgende Formel:

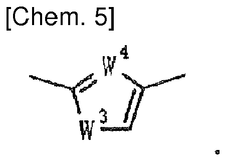

12. Verbindung nach Anspruch 11 oder ein pharmazeutisch verträgliches Salz davon, worin R¹ ein Wasserstoffatom ist.

13. Verbindung nach Anspruch 3 oder ein pharmazeutisch verträgliches Salz davon, worin R² eine Gruppe ist, ausgewählt aus der Gruppe, bestehend aus den folgenden a) bis d): 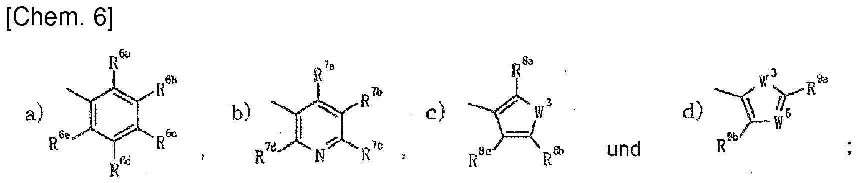
worin W⁵ ein Stickstoffatom oder -CR^{9c}= ist; und
R^{6a}, R^{6b}, R^{6c}, R^{6d}, R^{6e}, R^{7a}, R^{7b}, R^{7c}, R^{7d}, R^{8a}, R^{8b}, R^{8c}, R^{9a}, R^{9b} und R^{9c} jeweils unabhängig ein Wasserstoffatom, ein Halogenatom, eine C₁₋₆-Alkylgruppe , eine Halo-C₁₋₆-alkylgruppe, eine Hydroxy-C₁₋₆-Alkylgruppe, eine C₁₋₆-Alkoxygruppe oder eine Cyanogruppe darstellen.

14. Verbindung nach Anspruch 13 oder ein pharmazeutisch verträgliches Salz davon, worin R¹ ein Wasserstoffatom ist.

15. Pharmazeutische Zusammensetzung, umfassend die Verbindung nach einem der Ansprüche 1 bis 14 oder ein pharmazeutisch verträgliches Salz davon als einen Wirkstoff.

16. EP₁-Rezeptor-Antagonist, umfassend die Verbindung nach einem der Ansprüche 1 bis 14 oder ein pharmazeutisch verträgliches Salz davon als einen Wirkstoff.

17. Mittel zum Behandeln oder Verhindern von Symptomen im unteren Harntrakt, umfassend die Verbindung nach einem der Ansprüche 1 bis 14 oder ein pharmazeutisch verträgliches Salz davon als einen Wirkstoff.

## Revendications

1. Composé représenté par la formule générale (I) ou un sel pharmaceutiquement acceptable de celui-ci : 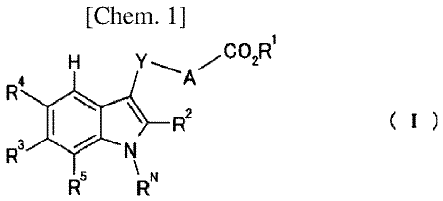 dans lequel
A représente un groupe choisi dans le groupe constitué des a) à h) suivants : 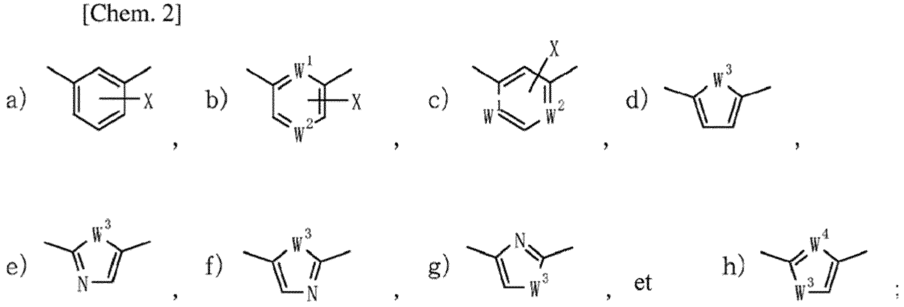
l'un de W¹ et W² représente un atome d'azote et l'autre représente =CH- ou un atome d'azote ;
W³ représente un atome d'oxygène ou un atome de soufre ;
W⁴ représente =CH- ou un atome d'azote ;
X représente un atome d'hydrogène ou un atome d'halogène ;
Y représente un groupe alkylène en C₁₋₆ ou un groupe halogéno-(alkylène en C₁₋₆) ;
R^{N} représente un atome d'hydrogène ou un groupe alkyle en C₁₋₆ ;
R¹ représente un atome d'hydrogène, un groupe alkyle en C₁₋₆ ou un groupe aralkyle en C₇₋₁₀ ;
R² représente un groupe choisi dans le groupe constitué des i) à m) suivants :
i) un groupe alkyle en C₃₋₆ ramifié,
j) un groupe cycloalkyle en C₃₋₆,
k) un groupe phényle, dans lequel le cycle est non substitué ou substitué par 1 à 5 groupes indépendamment choisis dans le groupe constitué de : un atome d'halogène, un groupe alkyle en C₁₋₆, un groupe halogéno-(alkyle en C₁₋₆), un groupe hydroxy-(alkyle en C₁₋₆), un groupe alcoxy en C₁₋₆ et un groupe cyano,
1) un groupe hétérocyclique aromatique à 6 chaînons, dans lequel le cycle est non substitué ou substitué par 1 à 4 groupes indépendamment choisis dans le groupe constitué de : un atome d'halogène, un groupe alkyle en C₁₋₆, un groupe halogéno-(alkyle en C₁₋₆), un groupe hydroxy-(alkyle en C₁₋₆), un groupe alcoxy en C₁₋₆ et un groupe cyano, et
m) un groupe hétérocyclique aromatique de 5 chaînons, dans lequel le cycle est non substitué ou substitué par 1 à 3 groupes indépendamment choisis dans le groupe constitué de : un atome d'halogène, un groupe alkyle en C₁₋₆, un groupe halogéno-(alkyle en C₁₋₆), un groupe hydroxy-(alkyle en C₁₋₆), un groupe alcoxy en C₁₋₆ et un groupe cyano ;
R³ représente un atome d'halogène, un groupe alkyle en C₁₋₆, un groupe halogéno-(alkyle en C₁₋₆), un groupe hydroxy-(alkyle en C₁₋₆), un groupe alcoxy en C₁₋₆, un groupe halogéno-(alcoxy en C₁₋₆), un groupe alkylsulfanyle en C₁₋₆, un groupe alkylsulfinyle en C₁₋₆, un groupe alkylsulfonyle en C₁₋₆, un groupe cycloalkyle en C₃₋₆, un groupe cyano, un groupe amino ou un groupe nitro ;
R⁴ représente un atome d'hydrogène, un atome d'halogène, un groupe alkyle en C₁₋₆ ou un groupe alcoxy en C₁₋₆ ; et
R⁵ représente un atome d'hydrogène, un atome d'halogène, un groupe alkyle en C₁₋₆ ou un groupe alcoxy en C₁₋₆.

2. Composé selon la revendication 1 ou un sel pharmaceutiquement acceptable de celui-ci, dans lequel A est un groupe choisi dans le groupe constitué des a) à d) suivants :

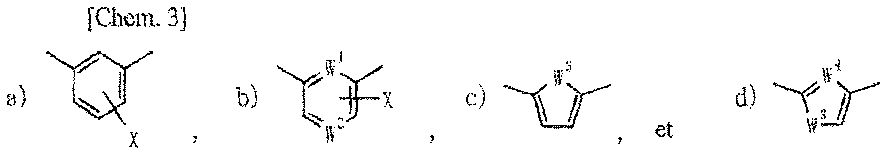

3. Composé selon la revendication 2 ou un sel pharmaceutiquement acceptable de celui-ci, dans lequel A est un groupe choisi dans le groupe constitué des a) à c) suivants : 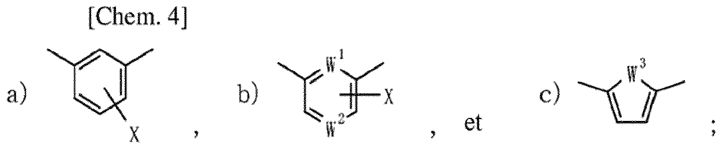
W¹ est un atome d'azote ;
W² est =CH- ; et
X est un atome d'hydrogène.

4. Composé selon la revendication 3 ou un sel pharmaceutiquement acceptable de celui-ci, dans lequel R² est un groupe choisi dans le groupe constitué des a) à c) suivants :
a) un groupe alkyle en C₃₋₆,
b) un groupe phényle, et
c) un groupe hétérocyclique aromatique à 5 chaînons ou un groupe hétérocyclique aromatique à 6 chaînons ;
R³ est un atome d'halogène, un groupe alkyle en C₁₋₆, un groupe halogéno- (alkyle en C₁₋₆), un groupe alcoxy en C₁₋₆, un groupe halogéno-(alcoxy en C₁₋₆), ou un groupe cyano ;
R⁴ est un atome d'hydrogène ; et
R⁵ est un atome d'hydrogène.

5. Composé selon la revendication 4 ou un sel pharmaceutiquement acceptable de celui-ci, dans lequel R¹ est un atome d'hydrogène.

6. Composé selon la revendication 5 ou un sel pharmaceutiquement acceptable de celui-ci, dans lequel Y est un groupe méthylène et R^{N} est un atome d'hydrogène.

7. Composé selon la revendication 6 ou un sel pharmaceutiquement acceptable de celui-ci, dans lequel R² est un groupe phényle, un groupe hétérocyclique aromatique à 5 chaînons, ou un hétérocycle aromatique à 6 chaînons.

8. Composé selon la revendication 7 ou un sel pharmaceutiquement acceptable de celui-ci, dans lequel R³ est un atome d'halogène ou un groupe alcoxy en C₁₋₆.

9. Composé selon la revendication 6 ou un sel pharmaceutiquement acceptable de celui-ci, dans lequel R² est un groupe isopropyle, un groupe isobutyle, un groupe sec-butyle, ou un groupe tert-butyle.

10. Composé selon la revendication 9 ou un sel pharmaceutiquement acceptable de celui-ci, dans lequel R³ est un atome d'halogène ou un groupe alcoxy en C₁₋₆.

11. Composé selon la revendication 2 ou un sel pharmaceutiquement acceptable de celui-ci, dans lequel A est un groupe représenté par la formule suivante :

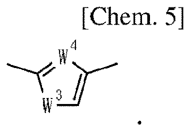

12. Composé selon la revendication 11 ou un sel pharmaceutiquement acceptable de celui-ci, dans lequel R¹ est un atome d'hydrogène.

13. Composé selon la revendication 3 ou un sel pharmaceutiquement acceptable de celui-ci, dans lequel R² est un groupe choisi dans le groupe constitué des a) à d) suivants : 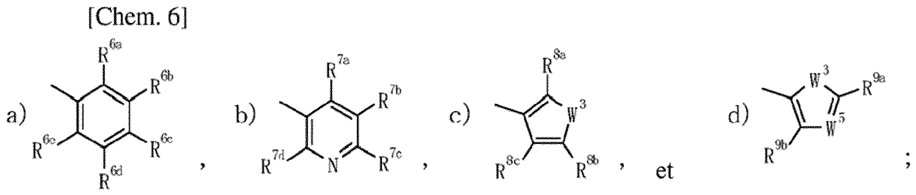
W⁵ est un atome d'azote ou -CR^{9c}= ; et
R^{6a}, R^{6b}, R^{6c}, R^{6d}, R^{6e}, R^{7a}, R^{7b}, R^{7c}, R^{7d}, R^{8a}, R^{8b}, R^{8c}, R^{9a}, R^{9b} et R^{9c} sont chacun indépendamment un atome d'hydrogène, un atome d'halogène, un groupe alkyle en C₁₋₆, un groupe halogéno-(alkyle en C₁₋₆), un groupe hydroxy-(alkyle en C₁₋₆), un groupe alcoxy en C₁₋₆ ou un groupe cyano.

14. Composé selon la revendication 13 ou un sel pharmaceutiquement acceptable de celui-ci, dans lequel R¹ est un atome d'hydrogène.

15. Composition pharmaceutique comprenant le composé selon l'une quelconque des revendications 1 à 14 ou un sel pharmaceutiquement acceptable de celui-ci en tant que substance active.

16. Antagoniste de récepteur EP₁ comprenant le composé selon l'une quelconque des revendications 1 à 14 ou un sel pharmaceutiquement acceptable de celui-ci en tant que substance active.

17. Agent pour traiter ou prévenir des symptômes du tractus urinaire inférieur, comprenant le composé selon l'une quelconque des revendications 1 à 14 ou un sel pharmaceutiquement acceptable de celui-ci en tant que substance active.


### SEQUENCE LISTING

<110> Kissei Pharmaceutical Co., Ltd.
<120> INDOLE DERIVATIVES, OR THE PHARMACEUTICALLY ACCEPTABLE SALTS
<130> PCT-A1013-00
<160> 5
<170> PatentIn version 3.1
<210> 1
   <211> 18
   <212> DNA
   <213> Artificial
<220>
   <223> 5' primer
<400> 1
   ttggccactg atatgagc 18
<210> 2
   <211> 18
   <212> DNA
   <213> Artificial
<220>
   <223> 3' primer
<400> 2
   gctttgggca cattcaca 18
<210> 3
   <211> 20
   <212> DNA
   <213> Artificial
<220>
   <223> 5' primer
<400> 3
   caccactgat atgagcccct 20
<210> 4
   <211> 19
   <212> DNA
   <213> Artificial
<220>
   <223> 3' primer
<400> 4
   gcctagcttt gggcacatt 19
<210> 5
   <211> 2414
   <212> DNA
   <213> Rattus norvegicus
<400> 5
   aacgccaata gggactttcc attgacgtca atgggtggag tatttacggt aaactgccca 60
   cttggcagta catcaagtgt atcatatgcc aagtacgccc cctattgacg tcaatgacgg 120
   taaatggccc gcctggcatt atgcccagta catgacctta tgggactttc ctacttggca 180
   gtacatctac gtattagtca tcgctattac catggtgatg cggttttggc agtacatcaa 240
   tgggcgtgga tagcggtttg actcacgggg atttccaagt ctccacccca ttgacgtcaa 300
   tgggagtttg ttttggcacc aaaatcaacg ggactttcca aaatgtcgta acaactccgc 360
   cccattgacg caaatgggcg gtaggcgtgt acggtgggag gtctatataa gcagagctct 420
   ctggctaact agagaaccca ctgcttactg gcttatcgaa attaatacga ctcactatag 480
   ggagacccaa gctggctagt taagcttggt accgagctcg gatccagtac ccttcaccac 540
   tgatatgagc ccctacgggc ttaacctgag cctagtggat gaggcaacaa cgtgtgtaac 600
   acccagggtc cccaatacat ctgtggtgct gccaacaggc ggtaacggca catcaccagc 660
   gctgcctatc ttctccatga cgctgggtgc tgtgtccaac gtgctggcgc tggcgctgct 720
   ggcccaggtt gcaggcagac tgcggcgccg ccgctcgact gccaccttcc tgttgttcgt 780
   cgccagcctg cttgccatcg acctagcagg ccatgtgatc ccgggcgcct tggtgcttcg 840
   cctgtatact gcaggacgtg cgcccgctgg cggggcctgt catttcctgg gcggctgtat 900
   ggtcttcttt ggcctgtgcc cacttttgct tggctgtggc atggccgtgg agcgctgcgt 960
   gggtgtcacg cagccgctga tccacgcggc gcgcgtgtcc gtagcccgcg cacgcctggc 1020
   actagccctg ctggccgcca tggctttggc agtggcgctg ctgccactag tgcacgtggg 1080
   tcactacgag ctacagtacc ctggcacttg gtgtttcatt agccttgggc ctcctggagg 1140
   ttggcgccag gcgttgcttg cgggcctctt cgccggcctt ggcctggctg cgctccttgc 1200
   cgcactagtg tgtaatacgc tcagcggcct ggcgctcctt cgtgcccgct ggaggcggcg 1260
   tcgctctcga cgtttccgag agaacgcagg tcccgatgat cgccggcgct ggggggtccg 1320
   tggactccgc ttggcctccg cctcgtctgc gtcatccatc acttcaacca cagctgccct 1380
   ccgcagctct cggggaggcg gctccgcgcg cagggttcac gcacacgacg tggaaatggt 1440
   gggccagctc gtgggcatca tggtggtttc gtgcatctgc tggagccccc tgctggtatt 1500
   ggtggtgttg gccatcgggg gctggaactc taactccctg cagcggccgc tctttctggc 1560
   tgtacgcctc gcgtcgtgga accagatcct ggacccatgg gtgtacatcc tgctgcgcca 1620
   ggctatgctg cgccaacttc ttcgcctcct acccctgagg gttagtgcca agggtggtcc 1680
   aacggagctg agcctaacca agagtgcctg ggaggccagt tcactgcgta gctcccggca 1740
   cagtggcttc agccacttgt gaatgtgccc aaagctaggc aagggtcaag acaattctgc 1800
   agatatccag cacagtggcg gccgctcgag tctagagggc ccgcggttcg aaggtaagcc 1860
   tatccctaac cctctcctcg gtctcgattc tacgcgtacc ggtcatcatc accatcacca 1920
   ttgagtttaa acccgctgat cagcctcgac tgtgccttct agttgccagc catctgttgt   1980
   ttgcccctcc cccgtgcctt ccttgaccct ggaaggtgcc actcccactg tcctttccta   2040
   ataaaatgag gaaattgcat cgcattgtct gagtaggtgt cattctattc tggggggtgg   2100
   ggtggggcag gacagcaagg gggaggattg ggaagacaat agcaggcatg ctggggatgc   2160
   ggtgggctct atggcttctg aggcggaaag aaccagctgg ggctctaggg ggtatcccca   2220
   cgcgccctgt agcggcgcat taagcgcggc gggtgtggtg gttacgcgca gcgtgaccgc   2280
   tacacttgcc agcgccctag cgcccgctcc tttcgctttc ttcccttcct ttctcgccac   2340
   gttcgccggc tttccccgtc aagctctaaa tcgggggctc cctttagggt tccgatttag   2400
   tgctttacgg cacc                                                     2414